# EUROPÄISCHE PATENTSCHRIFT

(11) **EP 0 738 259 B1**
(45) Veröffentlichungstag und Bekanntmachung des Hinweises auf die Patenterteilung: **16.08.2000**
(21) Anmeldenummer: 95906288.6
(22) Anmeldetag: 03.01.1995
(51) Int. Cl.: C07C 251/34, C07C 251/50, A01N 37/00, A01N 43/00

(54) **PHENYLESSIGSÄUREDERIVATE, VERFAHREN UND ZWISCHENPRODUKTE ZU IHRER HERSTELLUNG UND SIE ENTHALTENDE MITTEL**
PHENYL ACETIC ACID DERIVATIVES, PROCESS AND INTERMEDIATE PRODUCTS FOR THEIR PRODUCTION AND AGENTS CONTAINING THEM
DERIVES D'ACIDE PHENYLACETIQUE, LEUR PROCEDE DE PREPARATION ET PRODUITS INTERMEDIAIRES UTILISES A CET EFFET, ET AGENTS LES CONTENANT

(30) Priorität: 04.02.1994 DE 4403447; 17.06.1994 DE 4421180
(43) Veröffentlichungstag der Anmeldung: 23.10.1996
(60) Teilanmeldung: 99107871.8
(73) Patentinhaber: BASF AKTIENGESELLSCHAFT, 67056 Ludwigshafen (DE)
(72) Erfinder: BAYER, Herbert, D-68159 Mannheim (DE); SAUTER, Hubert, D-68167 Mannheim (DE); MÜLLER, Ruth, D-56626 Andernach (DE); GRAMMENOS, Wassilios, D-67063 Ludwigshafen (DE); HARREUS, Albrecht, D-67063 Ludwigshafen (DE); KIRSTGEN, Reinhard, D-67434 Neustadt (DE); RÖHL, Franz, D-67105 Schifferstadt (DE); AMMERMANN, Eberhard, D-64646 Heppenheim (DE); LORENZ, Gisela, D-67434 Hambach (DE)
(86) Internationale Anmeldenummer: EP9500013
(87) Internationale Veröffentlichungsnummer: WO9521153

(56) Entgegenhaltungen:
- EP-A- 0 370 629
- EP-A- 0 463 488
- WO-A-90/07493
- WO-A-92/18487
- WO-A-95/18789

## Beschreibung

Die vorliegende Erfindung betrifft Phenylessigsäurederivate der Formel I 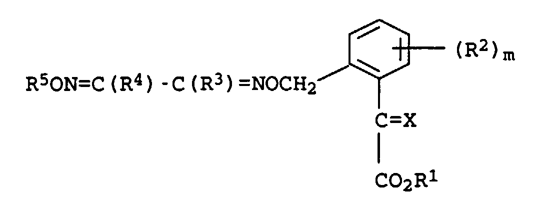 in der die Substituenten und der Index die folgende Bedeutung haben:
- X: NOCH₃, CHOCH₃, CHCH₃ und CHCH₂CH₃;
- R¹: Wasserstoff und C₁-C₄-Alkyl;
- R²: Cyano, Nitro, Trifluormethyl, Halogen, C₁-C₄-Alkyl und C₁-C₄-Alkoxy;
- m: 0, 1 oder 2, wobei die Reste R² verschieden sein können, wenn m für 2 steht;
- R³: Wasserstoff, Cyano, Nitro, Hydroxy, Amino, Halogen,
C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino oder Di-C₁-C₄-alkylamino;
- R⁴: Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR⁶)-Aₙ-R⁷;
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
- R⁵: Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶)-Aₙ-R⁷;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶)-Aₙ-R⁷;
wobei
- A: für Sauerstoff, Schwefel oder Stickstoff steht und wobei der Stickstoff Wasserstoff oder C₁-C₆-Alkyl trägt;
- n: 0 oder 1 bedeutet;
- R⁶: Wasserstoff oder C₁-C₆-Alkyl bedeutet und
- R⁷: Wasserstoff oder C₁-C₆-Alkyl bedeutet,
wobei X nicht CHOCH₃ oder NOCH₃ bedeutet, wenn
der Index m Null bedeutet, und
- R³: Wasserstoff, Cyano, C₁-C₄-Alkyl, C₁-C₄-Halogenalkyl oder Methylthio,
- R⁴: Wasserstoff, unsubstituiertes C₁-C₆-Alkyl, unsubstituiertes C₃-C₆-Cycloalkyl, Thienyl oder
Phenyl, das ggf. substituiert ist durch:
Halogen, Cyano, Nitro, C₁-C₄-Alkyl, C₁-C₄-Alkoxy, C₁-C₂-Halogenalkyl, C₁-C₂-Halogenalkoxy, C₃-C₆-Alkenyloxy, C₃-C₆-Alkinyloxy, C₃-C₄-Alkylendioxy; oder
folgende über Sauerstoff, C₁-C₄-Alkoxy, C₁-C₄-Oxyalkyl, S(O)ₙ, C₁-C₄-Alkyl-S(O)ₙ, S(O)ₙ-C₁-C₄-Alkyl, wobei n für 0, 1 oder 2 steht, gebundene Reste:
   C₁-C₆-Alkyl, C₁-C₆-Halogenalkyl, C₃-C₆-Cycloalkyl oder C₂-C₆-Alkenyl oder C₂-C₄-Alkinyl-C₁-C₂-alkyl, wobei diese Reste 1 bis 3-fach halogeniert sein können; oder
   ggf. durch Halogen, Trimethylsilyl, C₁-C₆-Alkyl, C₁-C₆-Halogenalkyl, C₁-C₆-Alkoxy, C₁-C₆-Halogenalkoxy, ggf. substituiertes Cyclopropyl oder Cyclopropyl-C₁-C₃-alkyl substituierte Aryl- oder Heterocyclylreste;
und
- R⁵: Wasserstoff, C₁-C₆-Alkyl, C₁-C₆-Halogenalkyl; C₁-C₄-Alkoxy-C₁-C₂-alkyl, ggf. 1- bis 3-fach halogeniertes C₂-C₄-Alkenyl-C₁-C₂-alkyl, C₂-C₄-Alkinyl-C₁-C₂-alkyl, ggf. 1- bis 4-fach halogeniertes C₃-C₆-Cycloalkyl, ggf. 1- bis 4-fach halogeniertes C₃-C₆-Cycloalkyl-C₁-C₂-alkyl, Cyano-C₁-C₄-alkyl, C₁-C₄-Alkoxycarbonyl-C₁-C₂-alkyl;
Phenyl-C₁-C₃-alkyl, das substituiert sein kann durch: Halogen, Cyano, Nitro, C₁-C₃-Alkyl, C₁-C₄-Alkoxy, C₁-C₄-Halogenalkyl, wobei die Phenylgruppe ggf. 1-bis 3-fach durch gleiche oder verschiedene Reste substituiert sein kann;
Phenyl, das ggf. 1- oder 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt;
oder
Pyridyl, das 1- bis 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt;
oder
- R³: Wasserstoff, C₁-C₄-Alkyl oder Cyano,
- R⁴: Cyano oder Heterocyclyl und
- R⁵: C₁-C₆-Alkyl, C₁-C₆-Halogenalkyl, C₁-C₄-Alkoxy-C₁-C₂-alkyl, unsubstituiertes oder mit 1 bis 3 Halogenatomen substituiertes C₂-C₆-Alkenyl, C₃-C₆-Alkinyl, unsubstituiertes oder mit 1 bis 4 Halogenatomen substituiertes C₃-C₆-Cycloalkyl-C₁-C₄-alkyl bedeuten;
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Aus der Literatur sind Phenylessigsäurederivate zur Schädlingsbekämpfung bekannt (EP-A 422 597, EP-A 463 488, EP-A 370 629, EP-A 460 575, EP-A 472 300, WO-A 90/07,493, WO-A 92/13,830, WO-A 92/18,487). In den nachveröffentlichten Schriften WO-A 95/18,789 und WO-A 96/11,183 sind weitere Phenylessigsäurederivate zur Schädlingsbekämpfung beschrieben.

Der vorliegenden Erfindung lagen neue Verbindungen mit verbesserter Wirkung als Aufgabe zugrunde.

Demgemäß wurden die eingangs definierten Phenylessigsäurederivate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -C(X)-CO₂R¹ oder die Gruppierung -CH₂ON=C(R³)-C(R⁴)=NOR⁵ aufgebaut wird.

Der Aufbau der Gruppierung -C(X)-CO₂R¹ ist beispielsweise aus der eingangs zitierten Literatur bekannt.

Die Art der Synthese der -CH₂ON=C(R³)-C(R⁴)=NOR⁵ Seitenkette richtet sich im wesentlichen nach der Art der Substituenten R³ und R⁴.
1. Für den Fall, daß R³ und R⁴ nicht Halogen bedeuten, geht man beim Aufbau der Gruppierung -CH₂ON=C(R³)-C(R⁴)=NOR⁵ im allgemeinen so vor, daß man ein Benzylderivat der Formel II mit einem Hydroxyimim der Formel III umsetzt. 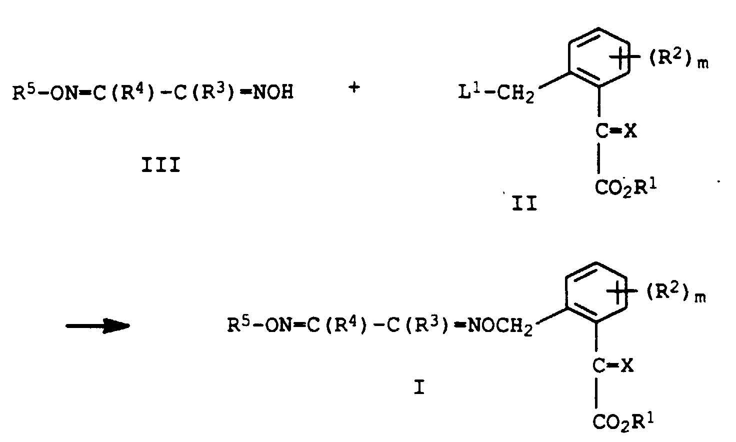
   L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einen inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370f und Houben-Weyl, Bd. 10/1, S. 1189f beschriebenen Methoden.
   Das benötigte Hydroxyimim III erhält man beispielsweise durch Umsetzung eines entsprechenden Dihydroxyimins IV mit einem nukleophil substituierten Reagens VI 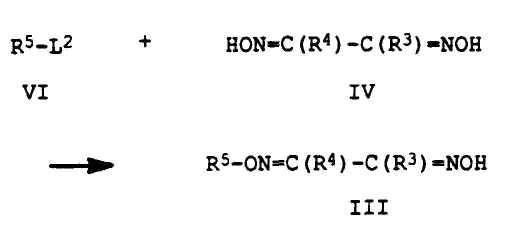
   L² in der Formel VI steht für eine nukleophil austauschbare Abgangsgruppe, z.B. Halogen oder Sulfonatgruppen, vorzugsweise Chlor, Brom, Iod, Mesylat, Tosylat oder Triflat.
   Die Umsetzung erfolgt in an sich bekannter Weise in einem inerten organischen Lösungsmittel in Gegenwart einer Base, z.B. Kaliumcarbonat, Kaliumhydroxid, Natriumhydrid, Pyridin und Triethylamin gemäß den in Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
1.1 Alternativ können die Verbindungen I auch dadurch erhalten werden, daß das Benzylderivat II zunächst mit dem Dihydroxyiminoderivat IV in ein entsprechendes Benzyloxim der Formel V umgesetzt wird, wobei V anschließend mit dem nukleophil substituierten Reagens VI zu I umgesetzt wird. 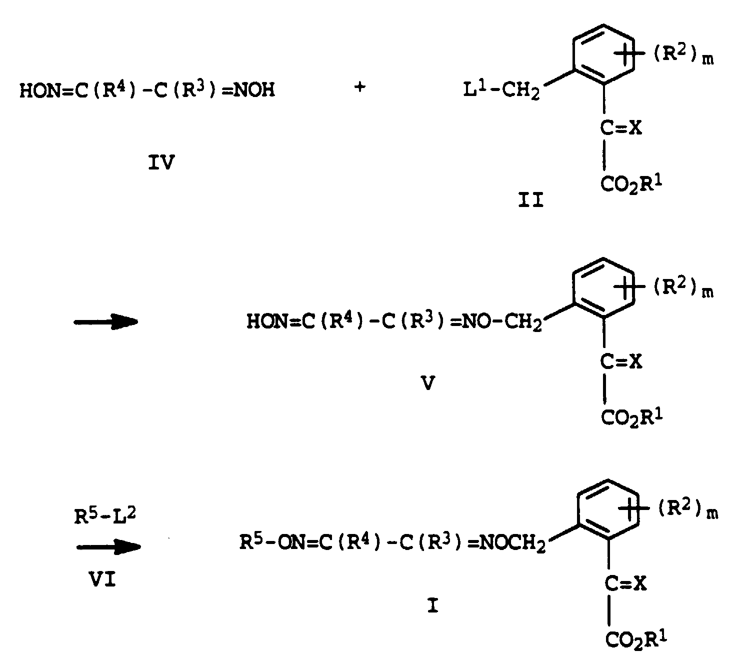
   Die Umsetzung erfolgt in an sich bekannter Weise in einem inerten organischen Lösungsmittel in Gegenwart einer Base z.B. Kaliumcarbonat, Kaliumhydroxid, Natriumhydrid, Pyridin und Triethylamin gemäß den in Houben-Weyl, Bd. 10/1, S. 1189f; Houben-Weyl, Bd. E 14b, S. 307f, S. 370f und S. 385f; Houben-Weyl, Bd. 10/4, S. 55f, S. 180f und S. 217f; Houben-Weyl, Bd. E 5, S. 780f, beschriebenen Methoden.
1.2 Analog ist es ebenfalls möglich, das benötigte Hdroxyimim der Formel III aus einem Carbonylhydroxyimin VII durch Umsetzung mit einem Hydroxylamin IXa oder seinem Salz IXb herzustellen. 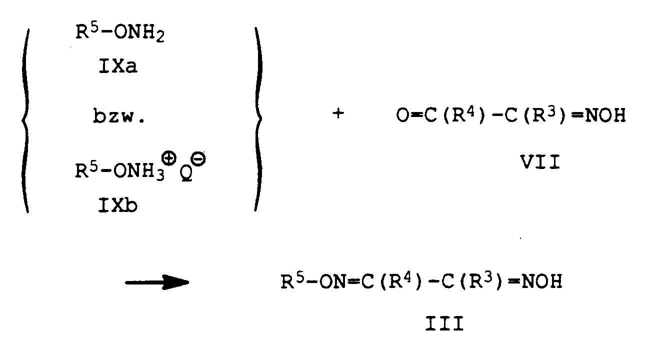
   Q^{⊖} in der Formel IXb steht für das Anion einer Säure, insbesondere einer anorganischen Säure, z.B. Halogenid wie Chlorid.
   Die Umsetzung erfolgt in an sich bekannter Weise in einem inerten organischen Lösungsmittel gemäß den in EP-A 513 580; Houben-Weyl, Bd. 10/4, S. 73f; Houben-Weyl, Bd. E 14b, S. 369f und S. 385f beschriebenen Methoden.
1.3 Alternativ können die Verbindungen I auch dadurch erhalten werden, daß das Benzylderivat II zunächst mit dem Carbonylhydroxyiminoderivat VII in ein entsprechendes Benzyloxyimin der Formel VIII umgesetzt wird, wobei VIII anschließend mit dem Hydroxylamin IXa bzw. dessen Salz IXb zu I umgesetzt wird. 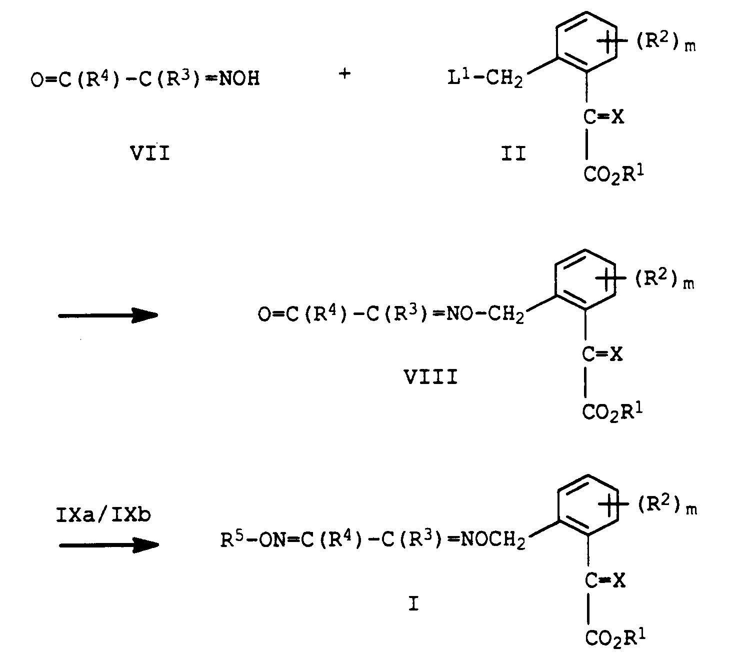
   Die Umsetzung erfolgt in an sich bekannter Weise in einem inerten organischen Lösungsmittel gemäß den in Houben-Weyl, Bd. E 14b, S. 369f; Houben-Weyl, Bd. 10/1, S. 1189f und Houben-Weyl, Bd. 10/4, S. 73f oder EP-A 513 580 beschriebenen Methoden.
1.4 Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung X. 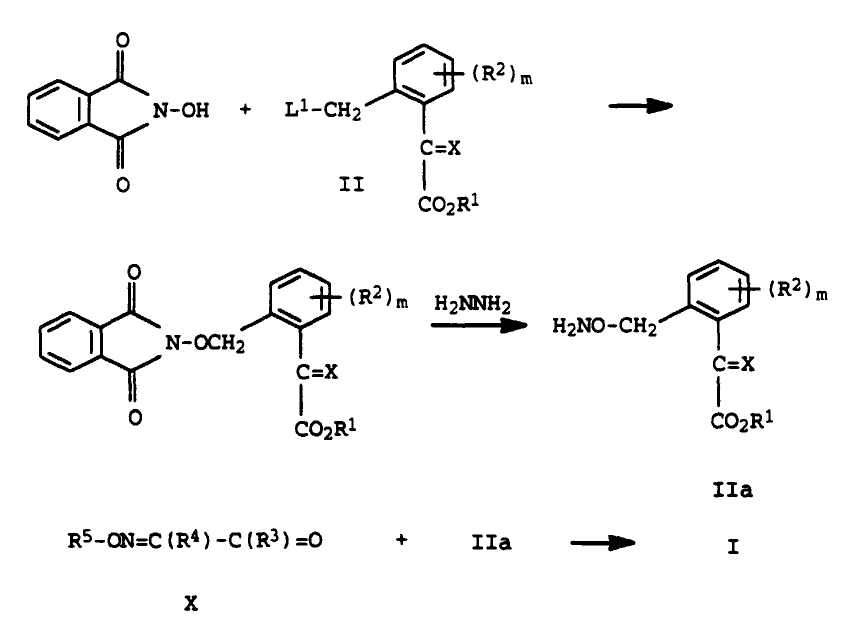
   Die Umsetzung erfolgt in an sich bekannter Weise in einem inerten organischen Lösungsmittel gemäß den in EP-A 463 488, DE-Anm. Nr. 42 28 867.3 beschriebenen Methoden.
   Die benötigte Carbonylverbindung X erhält man beispielsweise durch Umsetzung einer entsprechenden Hydroxyiminocarbonylverbindung VIIa mit einem nukleophil substituierten Reagens VI 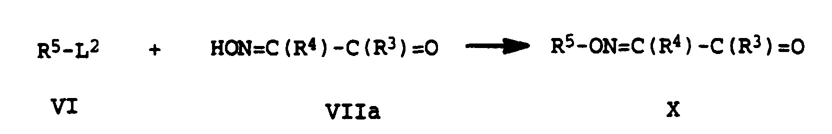 oder durch Umsetzung einer entsprechenden Dicarbonylverbindung XI mit einem Hydroxylamin IXa oder dessen Salz IXb 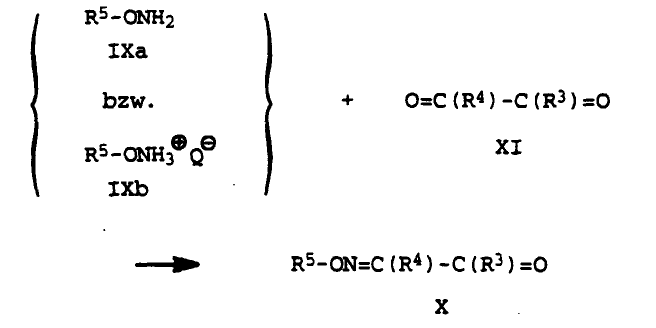
   Die Umsetzungen erfolgen in an sich bekannter Weise in einem inerten organischen Lösungsmittel gemäß den in EP-A 513 580, Houben-Weyl, Bd. 10/4, S. 55f, S. 73f, S. 180f und S. 217f; Houben-Weyl, Bd. E 14b, S. 307f und 369f, Houben-Weyl, Bd. E 5, S. 780f beschriebenen Methoden.
1.5 Entsprechend können die Verbindungen I auch dadurch erhalten werden, daß das Benzylhydroxylamin IIa zunächst mit dem Hydroxyiminoderivat VIIa in das entsprechende Benzyloxyiminodetivat der Formel V umgesetzt wird, wobei V anschließend mit dem nukleophil substituierten Reagens VI wie vorstehend beschrieben zu I umgesetzt wird. 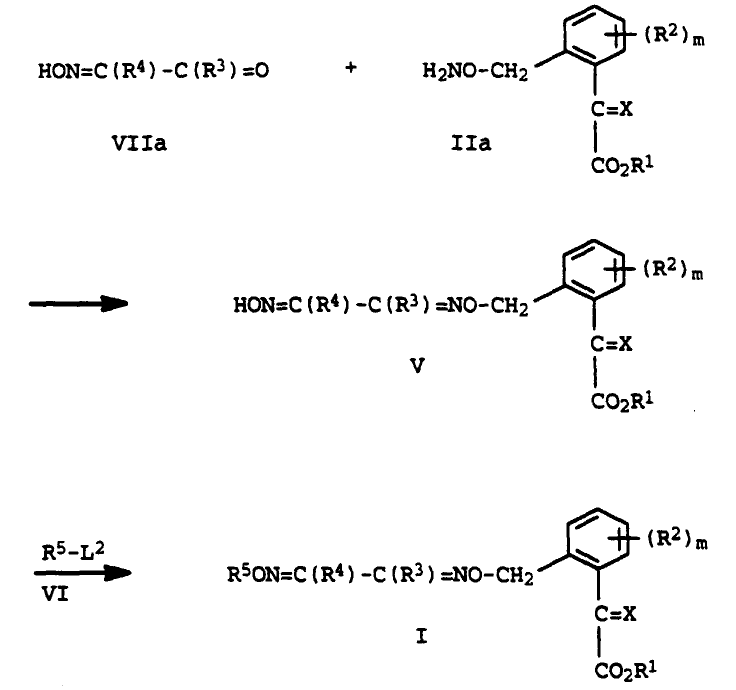
1.6 Analog können die Verbindungen I ebenfalls dadurch hergestellt werden, daß das Benzylhydroxylamin IIa zunächst mit dem Dicarbonylderivat der Formel XI in das Benzyloxyiminoderivat der Formel VIII überführt wird und VIII anschließend mit dem Hydroxylamin IXa oder dessen Salz IXb wie vorstehend beschrieben zu I umgesetzt wird. 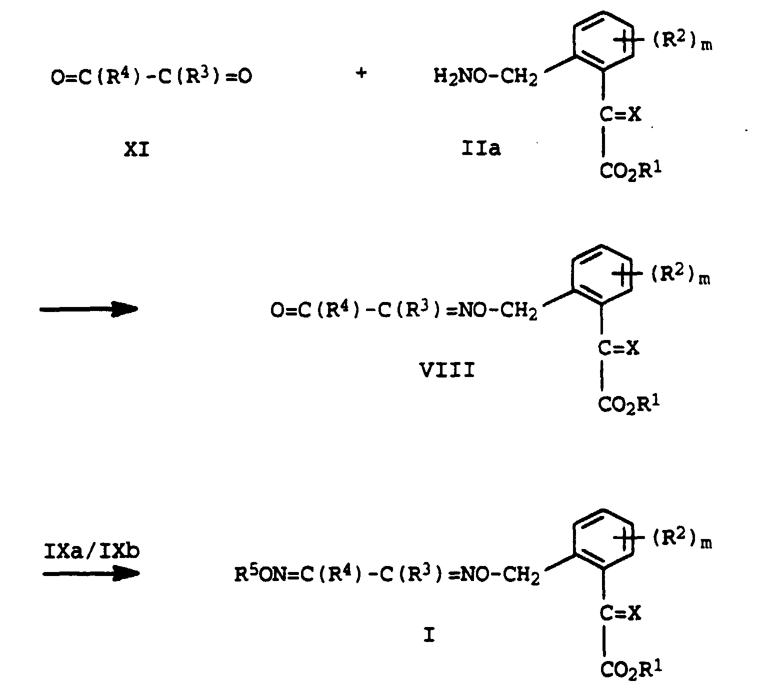
2. Verbindungen, in denen R³ und/oder R⁴ für ein Halogenatom stehen, erhalt man aus den entsprechenden Vorstufen, in denen der betreffende Rest für eine Hydroxygruppe steht nach an sich bekannten Methoden (vgl. Houben-Weyl, Vol. E5, S. 631; J. Org. Chem. 36, 233 (1971); J. Org. Chem. 57, 3245 (1992)). Vorzugsweise werden die entsprechenden Umsetzungen zum Halogenderivat auf den Stufen I und VIII vorgenommen.
3. Verbindungen, in denen R³ und/oder R⁴ über ein O-, S- oder N-Atom an das Molekülgerüst gebunden sind, erhält man aus den entsprechenden Vorstufen, in denen der betreffende Rest für ein Halogenatom steht nach an sich bekannten Methoden (vgl. Houben-Weyl, Bd. E5, S. 826 f und 1280 f, J. Org. Chem. 36, 233 (1971), J. Org. Chem. 46, 3623 (1981)). Vorzugsweise werden die entsprechenden Umsetzungen der Halogenderivat auf den Stufen I und VIII vorgenommen.
4. Verbindungen, in denen R³ und/oder R⁴ über ein Sauerstoffatom an das Molekül gebunden sind, erhält man zum Teil auch aus den entsprechenden Vorstufen, in denen der betreffende Rest für eine Hydroxygruppe steht nach an sich bekannten Methoden (vgl. Houben-Weyl, Bd. E5, S. 826-829, Aust. J. Chem. 27, 1341-9 (1974)). Vorzugsweise werden die entsprechenden Umsetzungen zu den Alkoxyderivaten auf den Stufen I und VIII vorgenommen.
5. Verbindungen, in denen R³ nicht Halogen bedeutet, erhält man bevorzugt dadurch, daß man eine Verbindung X gemäß den in EP-A 493 711 beschriebenen Methoden mit einem Lacton XII zunächst in die entsprechende Benzoesäure XIII überführt und XIII über die entsprechenden Halogenide in die Cyanocarbonsäuren XIV überführt, welche im Wege der Pinner-Reaktion (Angew. Chem. 94, 1 (1982)) in die α-Ketoester XV und anschließend in die Derivate I überführt werden (vgl. EP 348 766, DE 37 05 389, EP 178 826, DE 36 23 921). 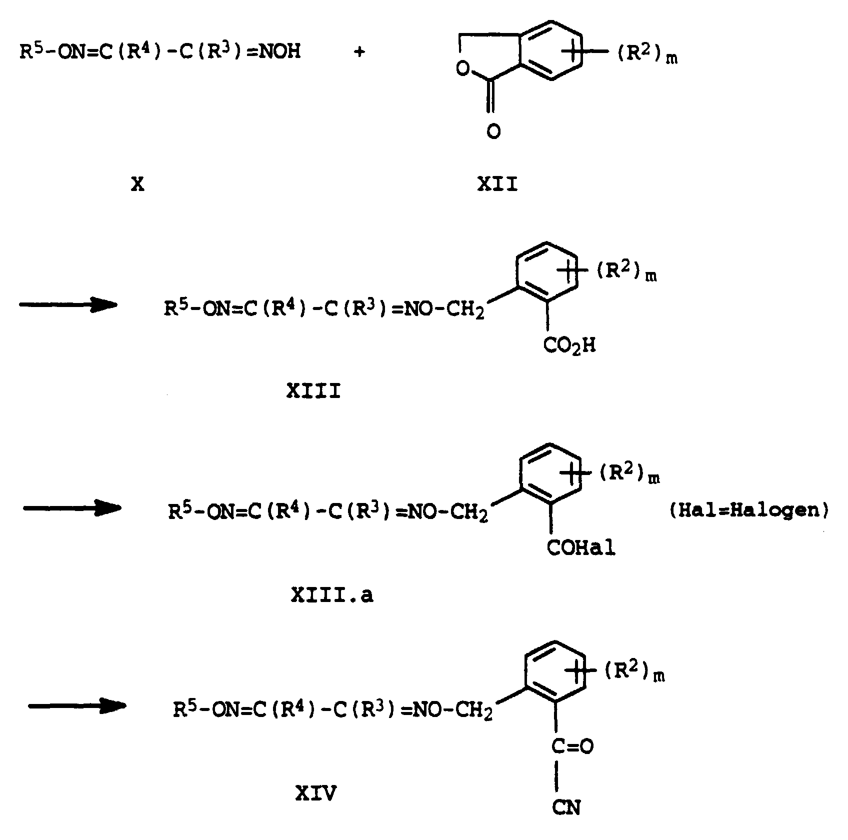 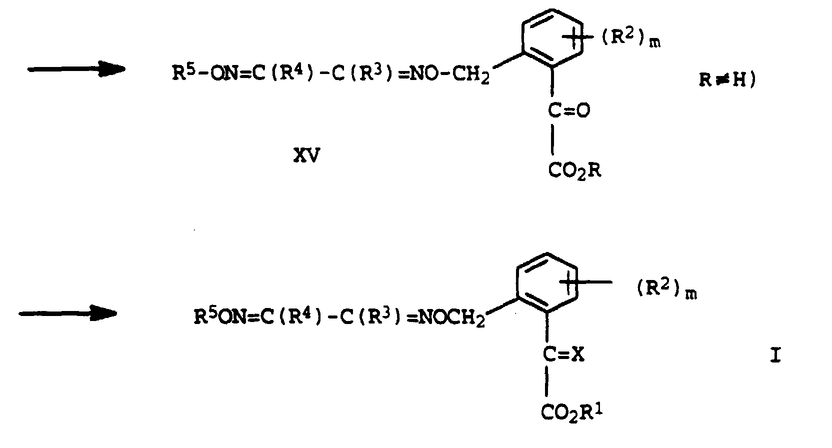 Verbindungen I, in denen R¹ Wasserstoff bedeutet, erhält man nach diesem Verfahren durch Verseifung der Ester XV und anschließende Umsetzung zu I.

Die Verbindungen II sind bekannt (EP-A 513 580, EP-A 477 631, EP-A 463 488, EP-A 370 629, EP-A 460 575) oder können nach den dort beschriebenen Methoden hergestellt werden.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise wahrend der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=X Doppelbindung werden hinsichtlich ihrer Wirksamkeit die E-Isomere der Verbindungen I bevorzugt (Konfiguration bezogen auf die -OCH₃, die -CH₃ bzw. die -CH₂CH₃ Gruppe im Verhältnis zur -CO₂R¹ Gruppe).

In Bezug auf die -C(R³)=NOCH₂- Doppelbindung werden hinsichtlich ihrer Wirksamkeit die cis-Isomere der Verbindungen I bevorzugt (Konfiguration bezogen auf den Rest R³ im Verhältnis zur -OCH₂-Gruppe).

Bei der eingangs angegebenen Definitionen der Verbindungen I wurden Sammelbegriffe verwendet, die allgemein repräsentativ für die folgenden Gruppen stehen:
**Halogen:** Fluor, Chlor, Brom und Jod;
**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;
**Halogenalkyl:** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;
**Alkoxy:** geradkettige oder verzweigte Alkylgruppen mit 1 bis 4 oder 6 Kohlenstoffatomen wie vorstehend genannt, welche über ein Sauerstoffatom (-O-) an das Gerüst gebunden sind, z.B. C₁-C₆-Alkoxy wie Methyloxy, Ethyloxy, Propyloxy, 1-Methylethyloxy, Butyloxy, 1-Methyl-propyloxy, 2-Methylpropyloxy, 1,1-Dimethylethyloxy, Pentyloxy, 1-Methylbutyloxy, 2-Methylbutyloxy, 3-Methylbutyloxy, 2,2-Di-methylpropyloxy, 1-Ethylpropyloxy, Hexyloxy, 1,1-Dimethylpropyloxy, 1,2-Dimethylpropyloxy, 1-Methylpentyloxy, 2-Methylpentyloxy, 3-Methylpentyloxy, 4-Methylpentyloxy, 1,1-Dimethylbutyloxy, 1,2-Dimethylbutyloxy, 1,3-Dimethylbutyloxy, 2,2-Dimethylbutyloxy, 2,3-Dimethylbutyloxy, 3,3-Dimethylbutyloxy, 1-Ethyl-butyloxy, 2-Ethylbutyloxy, 1,1,2-Trimethylpropyloxy, 1,2,2-Trimethylpropyloxy, 1-Ethyl-1-methylpropyloxy und 1-Ethyl-2-methylpropyloxy;
**Cycloalkyl:** monocyclische Alkylgruppen mit 3 bis 6 Kohlenstoffringgliedern, z.B. Cyclopropyl, Cyclobutyl, Cyclopentyl und Cyclohexyl;
**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-1-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1- Ethyl-1-methyl-2-propenyl, 1-Ethyl-2-methyl-1-propenyl und 1-Ethyl-2-methyl-2-propenyl;
**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;
**Cycloalkenyl bzw. Cycloalkenyloxy, Cycloalkenylthio und Cycloalkenylamino:** mnonocyclische Alkenylgruppen mit 3 bis 6 Kohlenstoffringgliedern, welche direkt bzw. (Cycloalkenyloxy) über ein Sauerstoffatom oder (Cycloalkenylthio) ein Schwefelatom oder Cycloalkenylamino) über ein Stickstoffatom an das Gerüst gebunden sind, z.B. Cyclopropenyl, Cyclobutenyl, Cyclopentenyl oder Cyclohexenyl.
**Heterocyclyl bzw. Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,
**Aryl bzw. Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;
**Hetaryl bzw. Hetaryloxy, Hetarylthio, Hetarylcarbonyl und** **Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.

- 5-gliedriges Heteroaryl, enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartem Kohlenstoffringglied durch eine Buta-1,3-dien-1,4-diylgruppe verbrückt sein können;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome: 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
- 6-gliedriges Heteroaryl, enthaltend ein bis drei bzw. ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
bzw. die entsprechenden Oxy-, Thio-, Carbonyl- oder Sulfonylgruppen.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung sind Verbindungen der Formel I bevorzugt, in denen m für 0 steht.

Gleichermaßen bevorzugt sind Verbindungen der Formel I, in denen R¹ für Methyl steht.

Daneben werden Verbindungen I bevorzugt, in denen R³ für Wasserstoff, Hydroxy, Cyclopropyl, Chlor, Methyl, Ethyl, 1-Methylethyl, Methoxy, Methylthio oder Phenyl steht.

Außerdem werden Verbindungen I bevorzugt, in denen R³ für Methyl steht.

Des weiteren werden Verbindungen I bevorzugt, in denen R³ für Methoxy steht.

Außerdem werden Verbindungen I bevorzugt, in denen R³ für Hydroxy steht.

Des weiteren werden Verbindungen I bevorzugt, in denen R³ für Chlor steht.

Des weiteren werden Verbindungen I bevorzugt, in denen R⁴ für Wasserstoff, Hydroxy, Cyclopropyl, Chlor, Methyl, Ethyl, iso-Propyl, Methoxy oder Methylthio steht.

Außerdem werden Verbindungen I bevorzugt, in denen R⁴ für Methyl steht.

Des weiteren werden Verbindungen I bevorzugt, in denen R⁴ für Methoxy steht.

Außerdem werden Verbindungen I bevorzugt, in denen R⁴ für Hydroxy steht.

Des weiteren werden Verbindungen I bevorzugt, in denen R⁴ für Ethyl steht.

Außerdem werden Verbindungen I bevorzugt, in denen R⁴ für Iso-Propyl steht.

Außerdem werden Verbindungen I bevorzugt, in denen R⁴ für Cyclopropyl steht.

Des weiteren werden Verbindungen I bevorzugt, in denen R⁴ für ggf. subst. Aryl oder Hetaryl steht.

Des weiteren werden Verbindungen I bevorzugt, in denen R⁴ für ggf. subst. Pyridyl, Pyrimidyl, Pyrazinyl, Pyridazinyl oder Triazinyl steht.

Des weiteren werden Verbindungen I bevorzugt, in denen R⁴ für ggf. subst. Furyl, Thienyl oder Pyrrolyl steht. Des weiteren werden Verbindungen I bevorzugt, in denen R⁴ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁴ für ggf. subst. Oxdiazolyl, Thiadiazolyl oder Triazolyl steht.

Außerdem werden Verbindungen I bevorzugt, in denen R⁴ für Phenyl steht, welches unsubstituiert ist oder ein oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino, C₁-C₄-Alkylsulfonyl, C₁-C₄-Alkoxycarbonyl, C₁-C₄-Alkylaminocarbonyl or Di-C₁-C₄-Alkylaminocarbonyl.

Außerdem werden Verbindungen I bevorzugt, in denen R⁵ für Wasserstoff, C₁-C₆-Alkyl, Arylalkyl, Hetarylalkyl, Aryloxyalkyl, Hetaryloxyalkyl, Aryl oder Hetaryl steht.

Des weiteren werden Verbindungen I bevorzugt, in denen R⁵ für C₁-C₆-Alkyl steht.

Außerdem werden Verbindungen I bevorzugt, in denen R⁵ für Methyl oder Ethyl steht.

Des weiteren werden Verbindungen I bevorzugt, in denen R⁵ für Arylalkyl oder Hetarylalkyl steht.

Außerdem werden Verbindungen I bevorzugt, in denen R⁵ für Aryloxyalkyl oder Hetaryloxyalkyl steht.

Außerdem werden Verbindungen I bevorzugt, in denen R⁵ für Aryl oder Hetaryl steht.

Daneben werden Verbindungen der Formel I bevorzugt, in denen X für NOCH₃ steht.

Daneben werden Verbindungen der Formel I bevorzugt, in denen X für CHOCH₃ steht.

Daneben werden Verbindungen der Formel I bevorzugt, in denen X für CHCH₃ oder CHCH₂CH₃ steht.

Insbesondere sind im Hinblick auf ihre Verwendung die in den folgenden Tabellen zusammengestellten Verbindungen I bevorzugt.

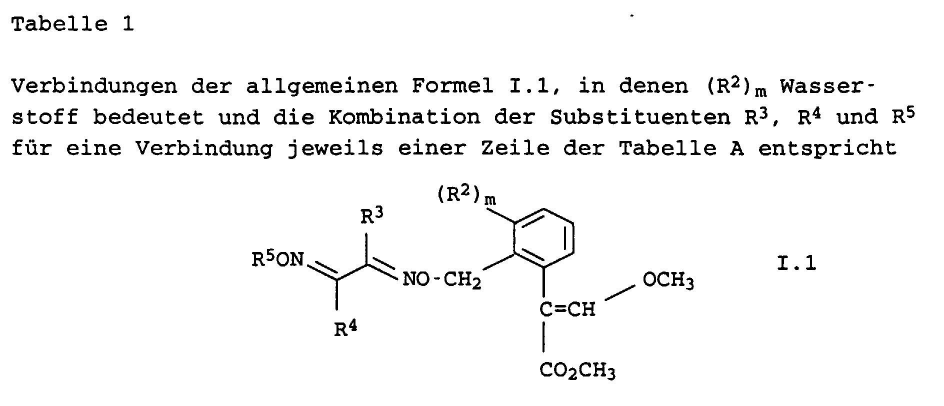

**Tabelle 2**

| |
|---|
| Verbindungen der allgemeinen Formel I.1, in denen (R²)ₘ Chlor |
| bedeutet und die Kombination der Substituenten R³, R⁴ und R⁵ für |
| eine Verbindung jeweils einer Zeile der Tabellen A und B entspricht. |

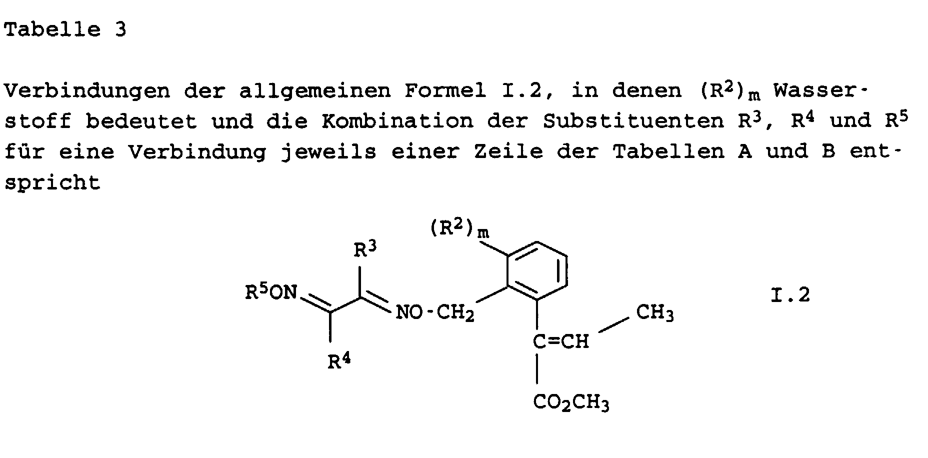

**Tabelle 4**

| |
|---|
| Verbindungen der allgemeinen Formel I.2, in denen (R²)ₘ Chlor |
| bedeutet und die Kombination der Substituenten R³, R⁴ und R⁵ für |
| eine Verbindung jeweils einer Zeile der Tabellen A und B entspricht. |

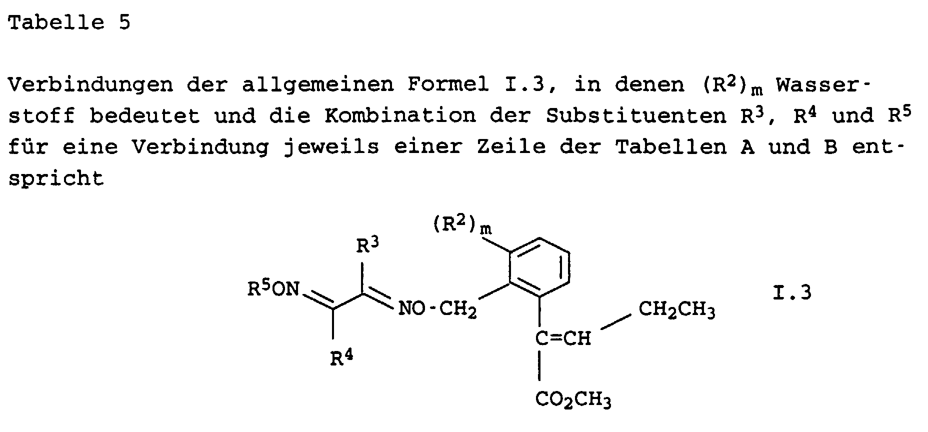

**Tabelle 6**

| |
|---|
| Verbindungen der allgemeinen Formel I.3, in denen (R²)ₘ Chlor |
| bedeutet und die Kombination der Substituenten R³, R⁴ und R⁵ für |
| eine Verbindung jeweils einer Zeile der Tabellen A und B entspricht. |

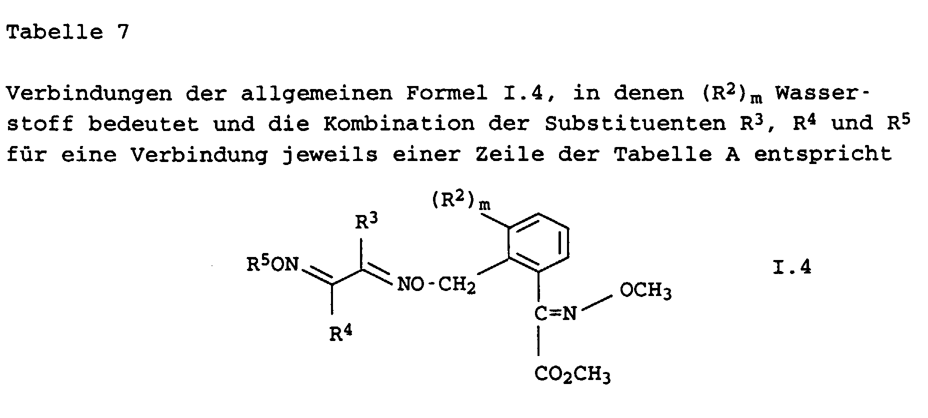

**Tabelle 8**

| |
|---|
| Verbindungen der allgemeinen Formel I.4, in denen (R²)ₘ Chlor |
| bedeutet und die Kombination der Substituenten R³, R⁴ und R⁵ für |
| eine Verbindung jeweils einer Zeile der Tabellen A und B entspricht. |

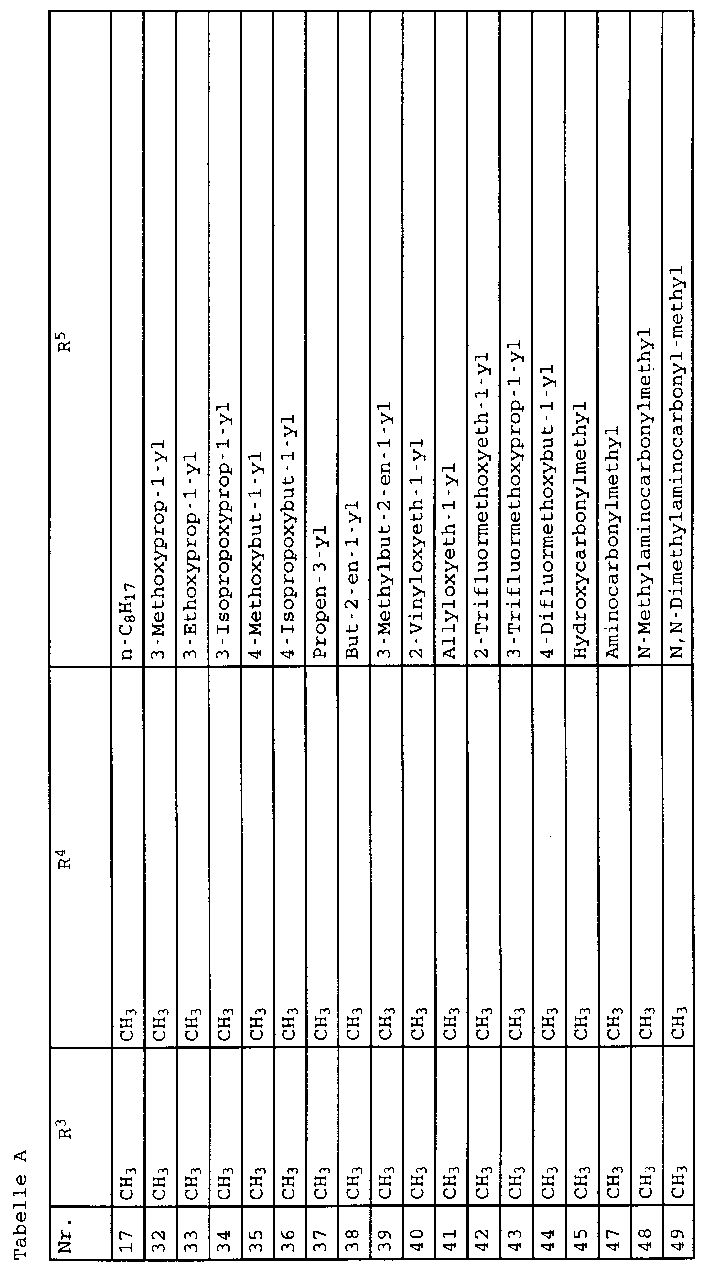

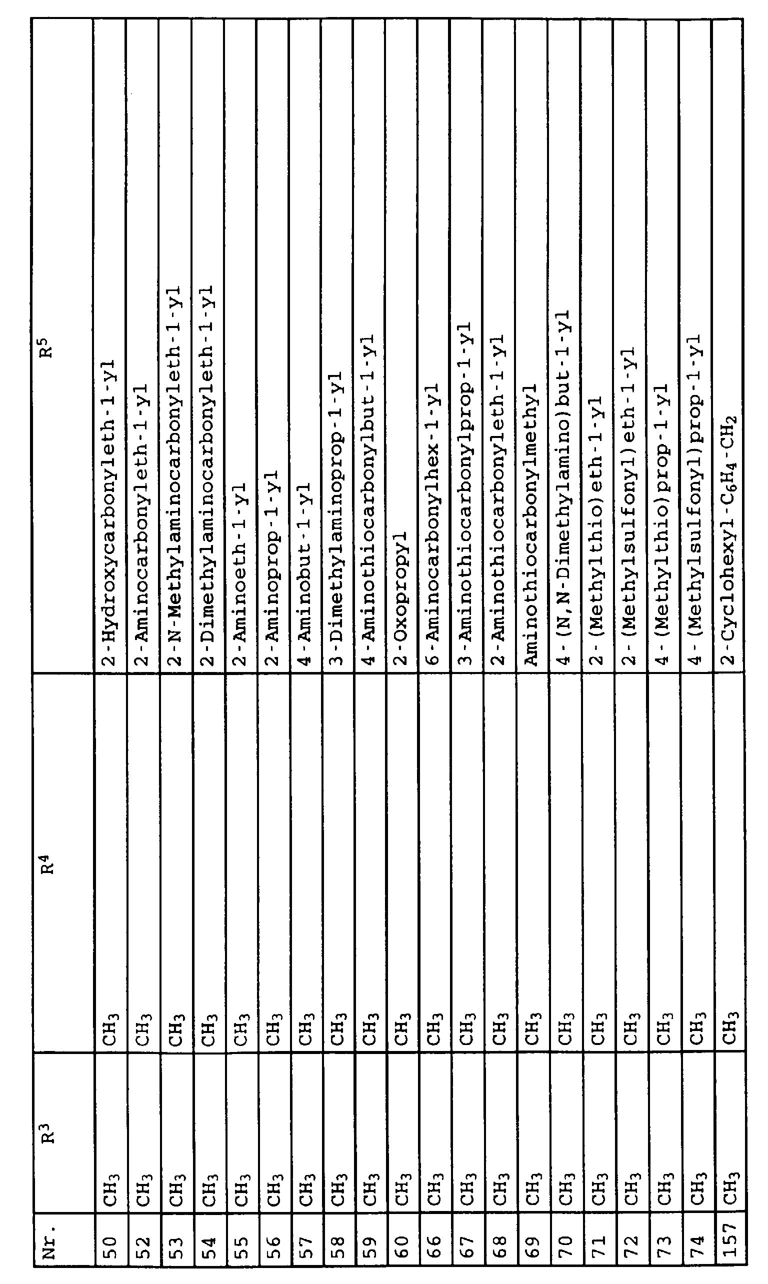

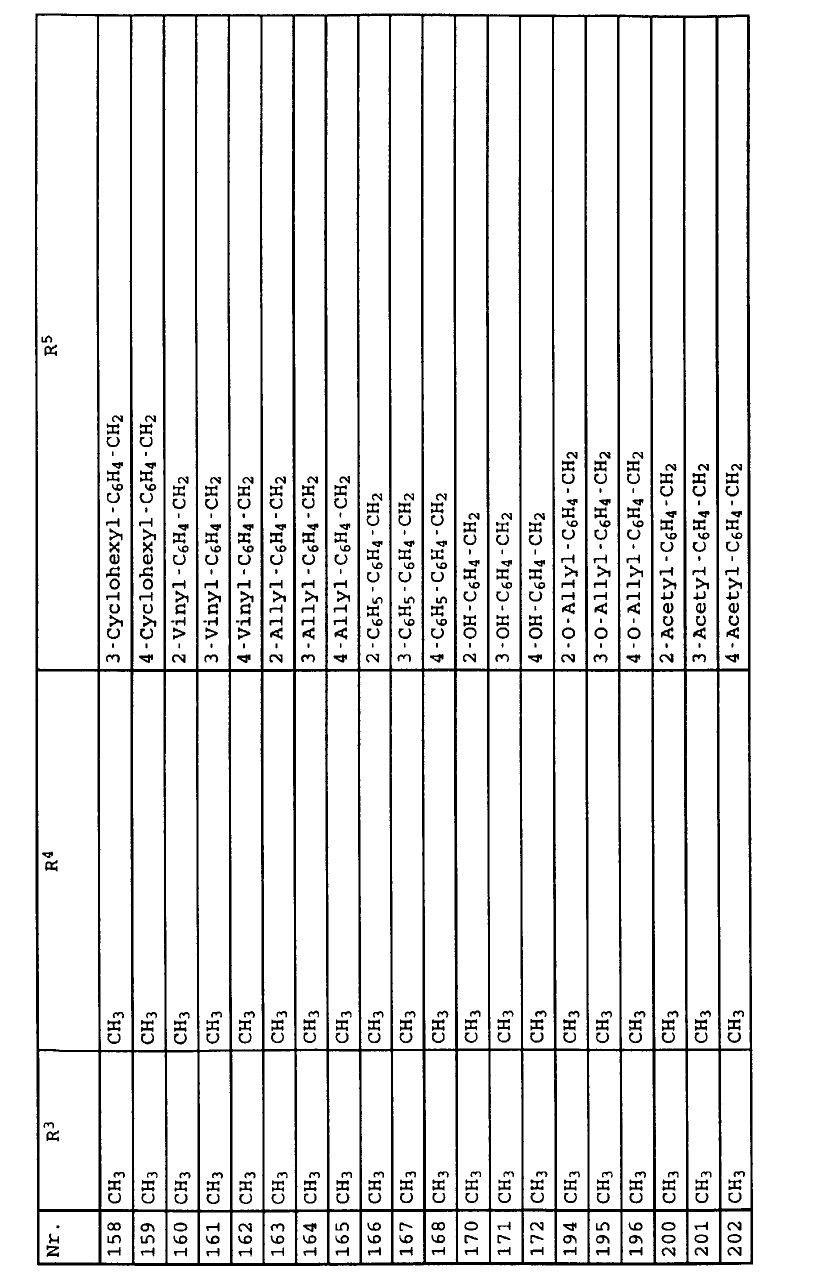

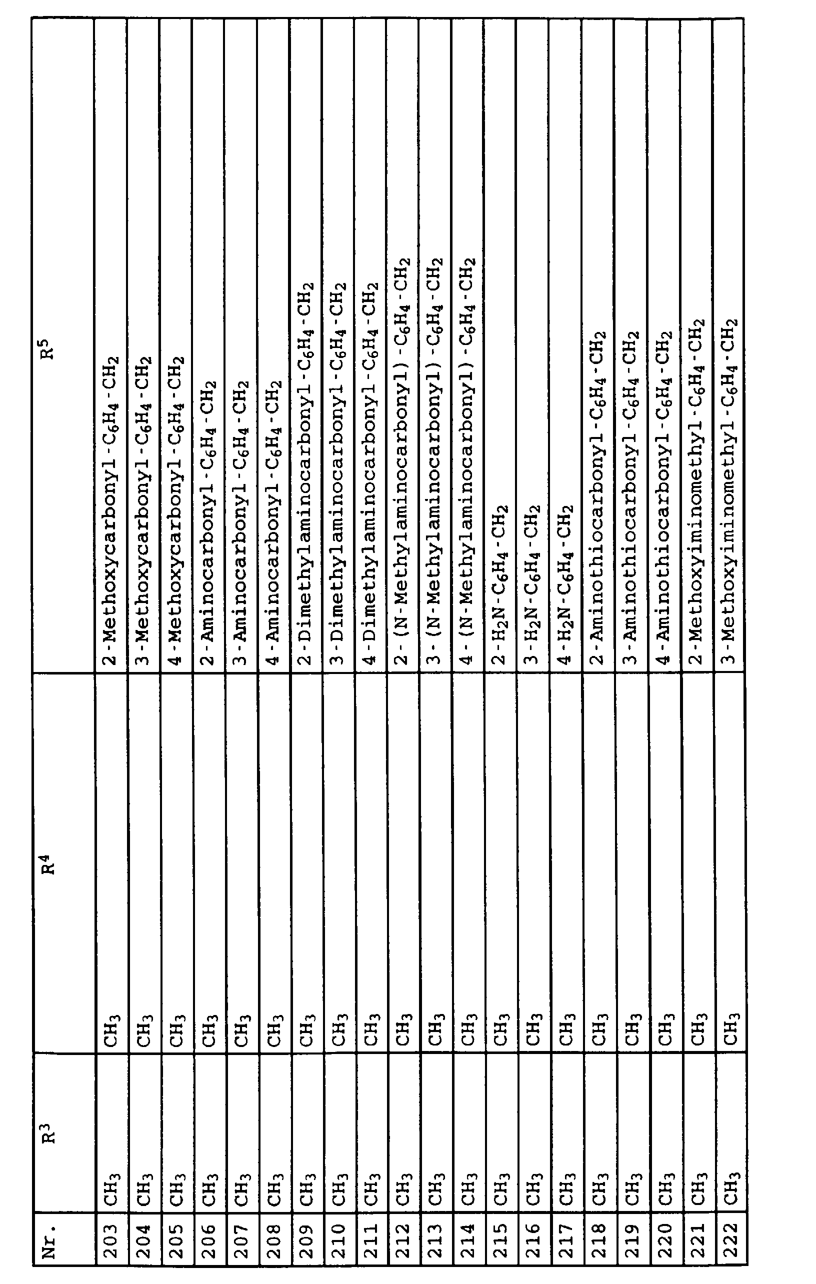

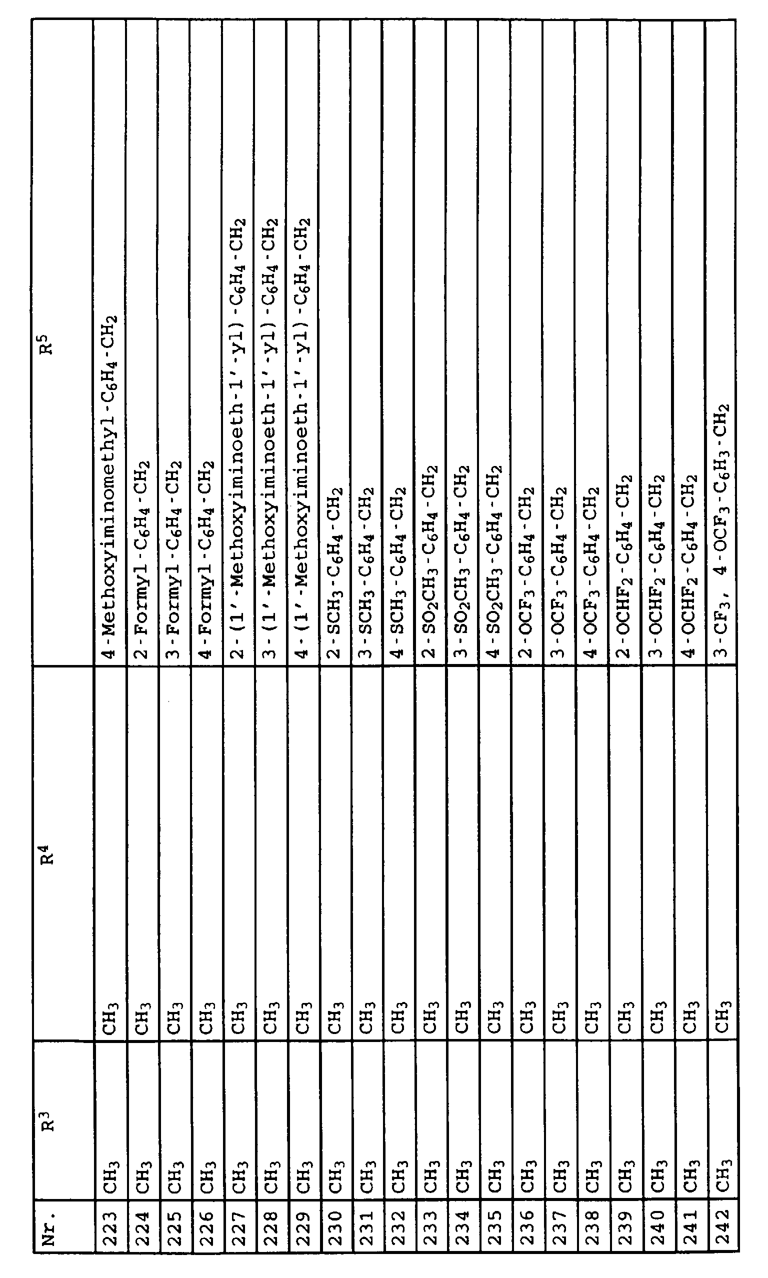

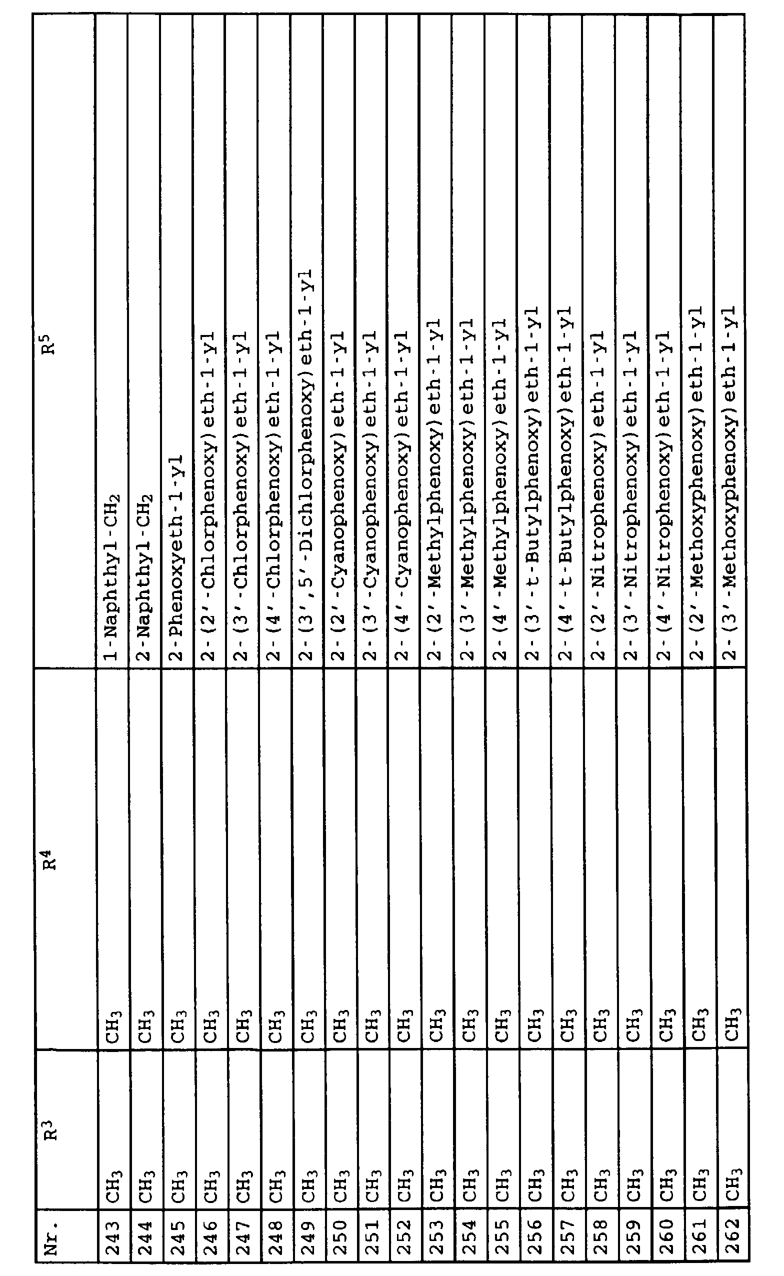

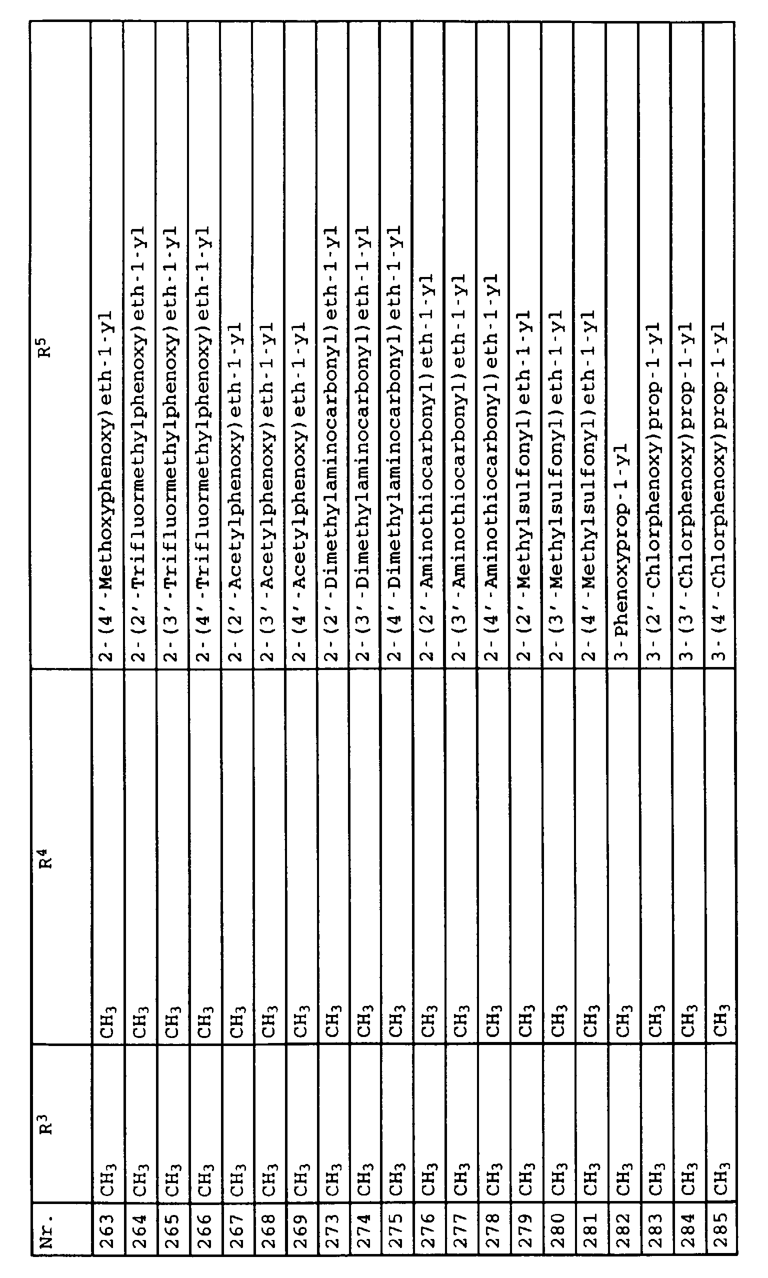

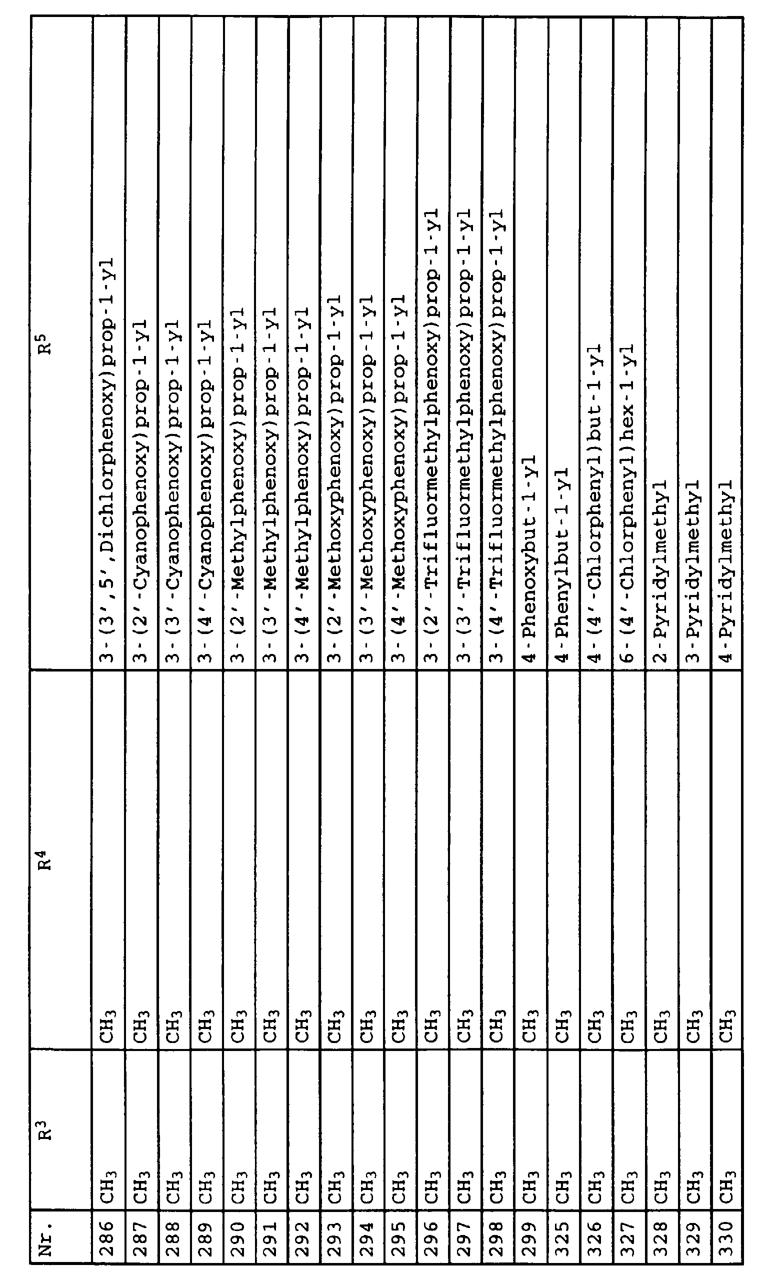

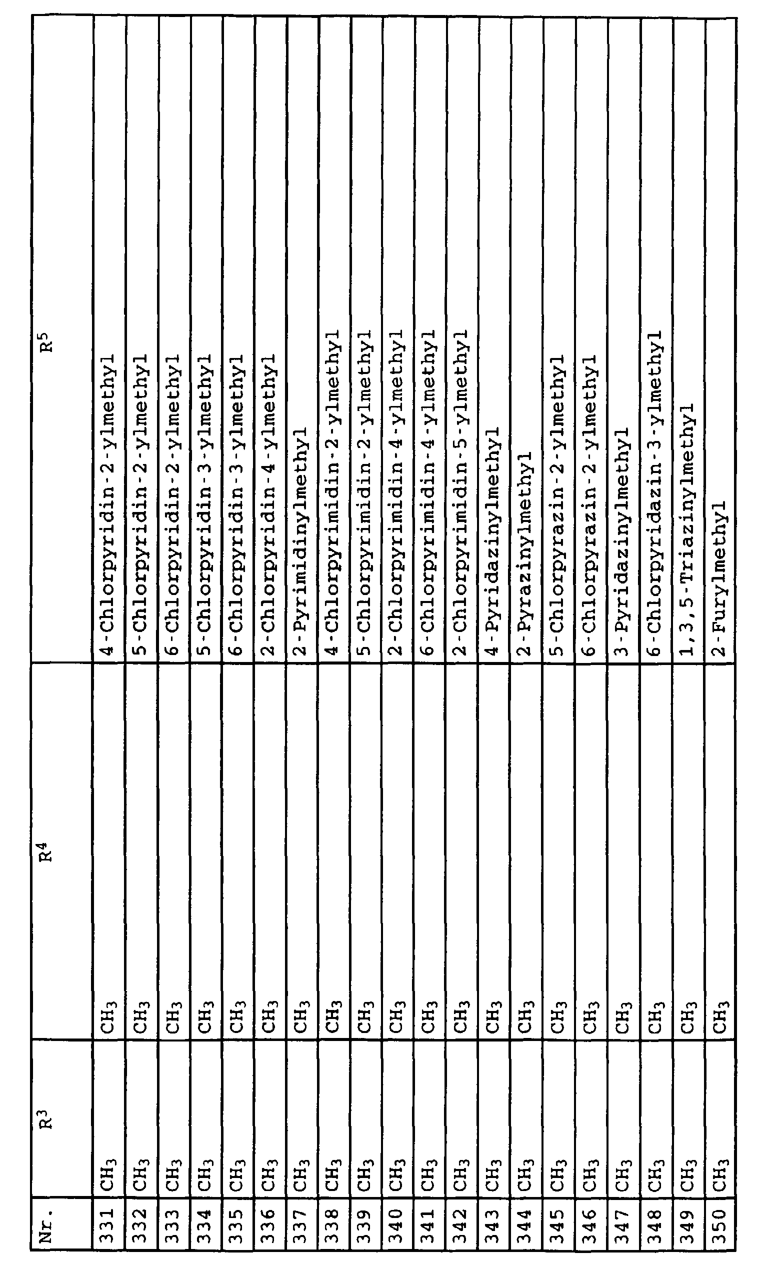

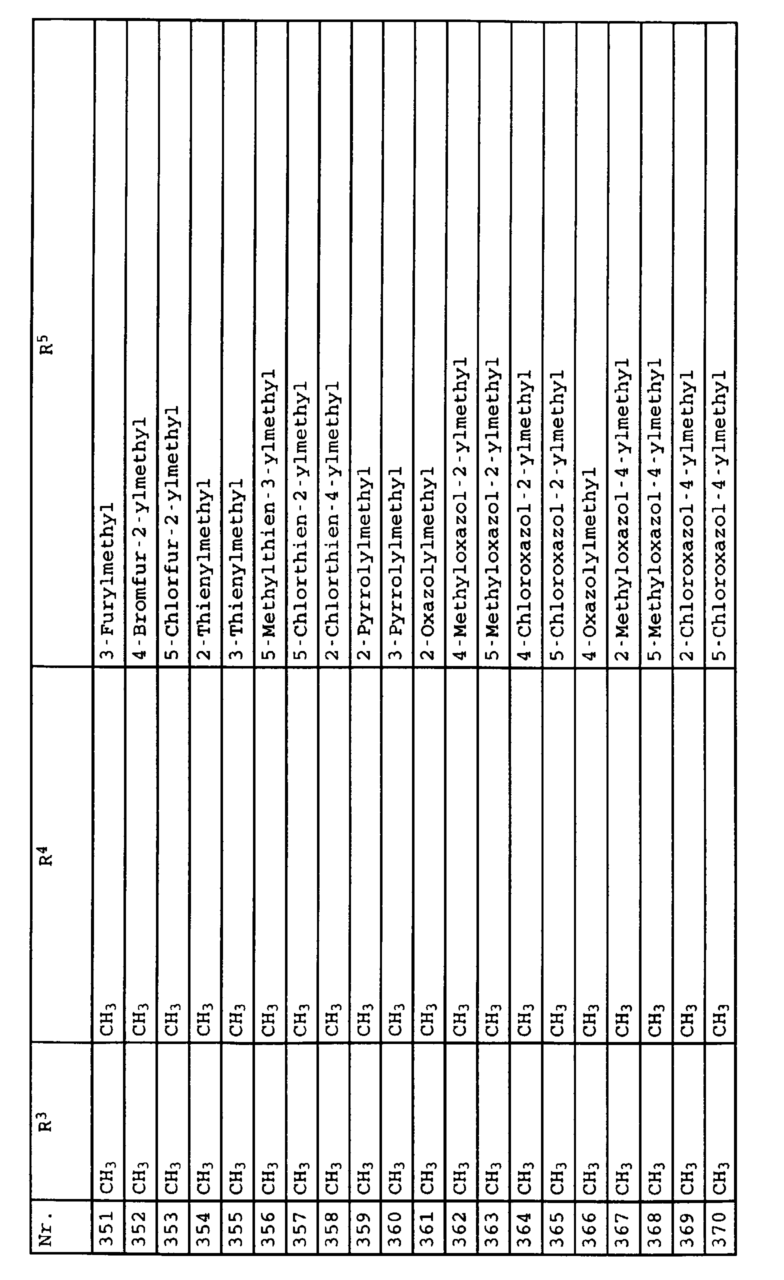

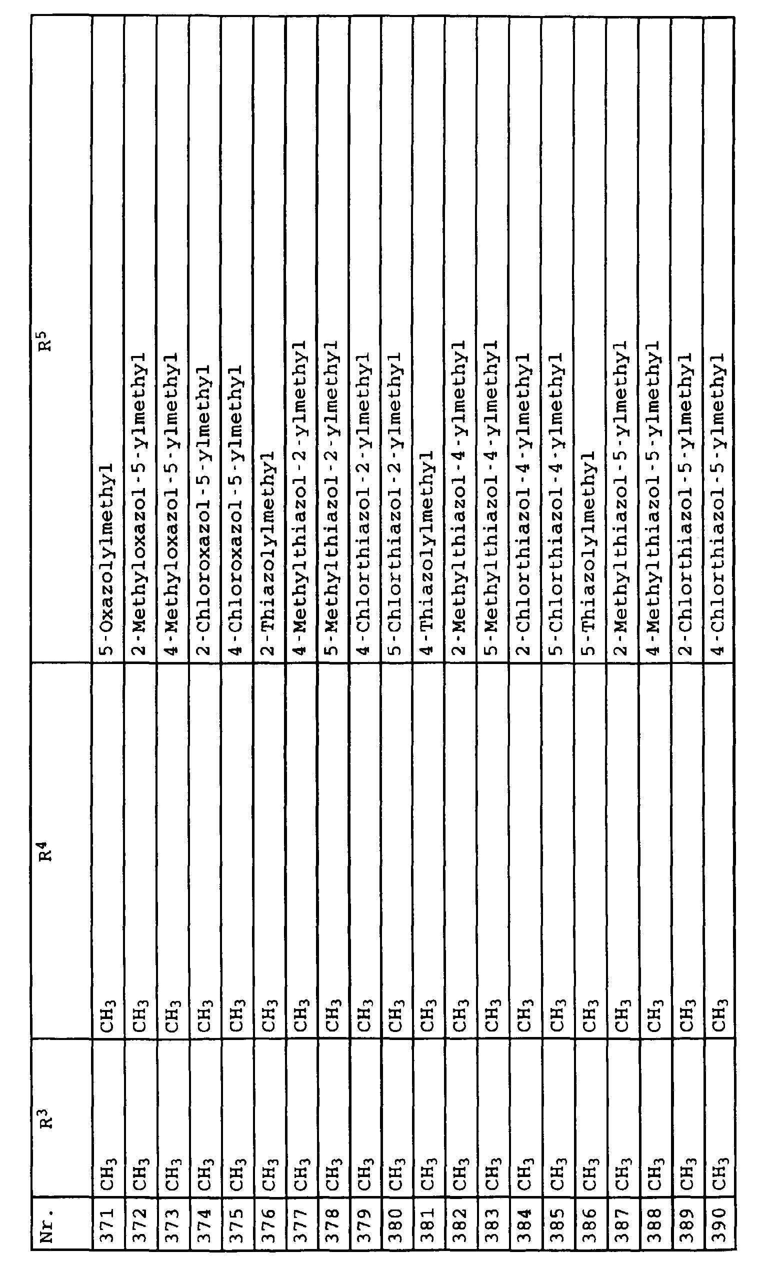

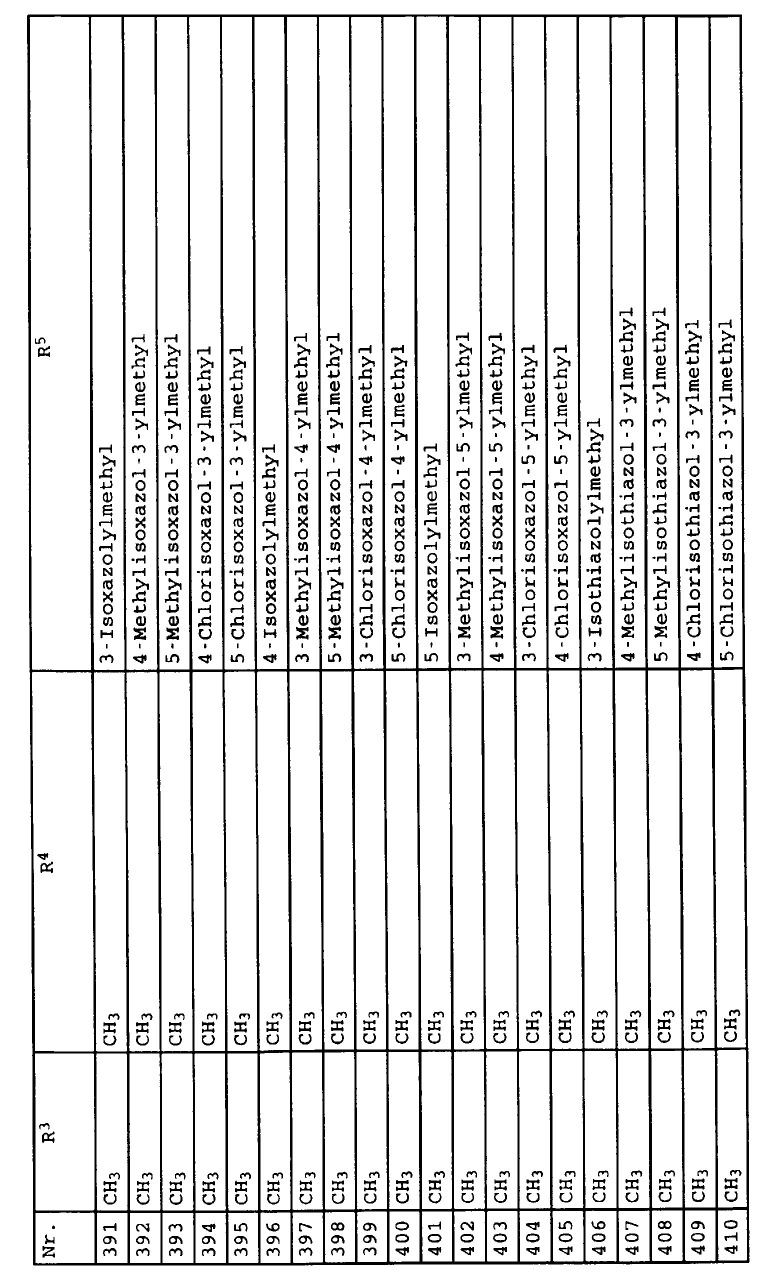

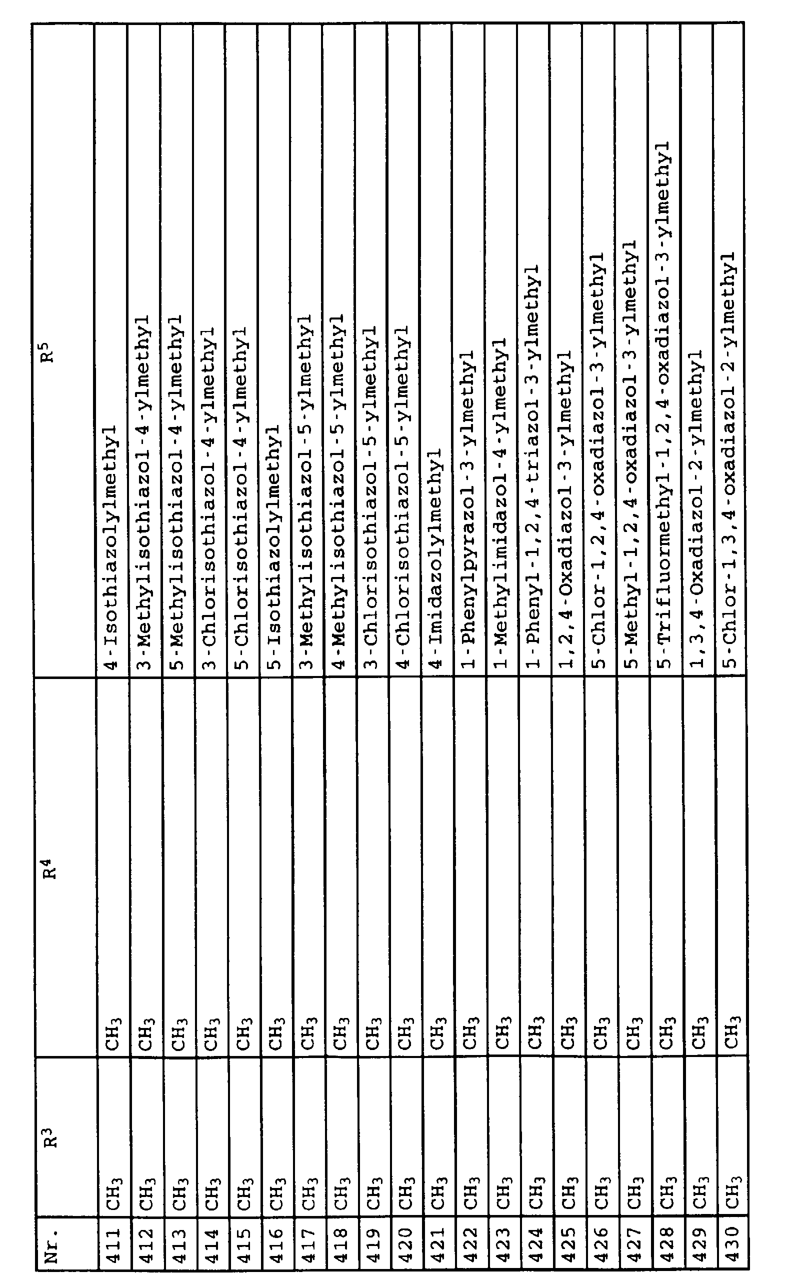

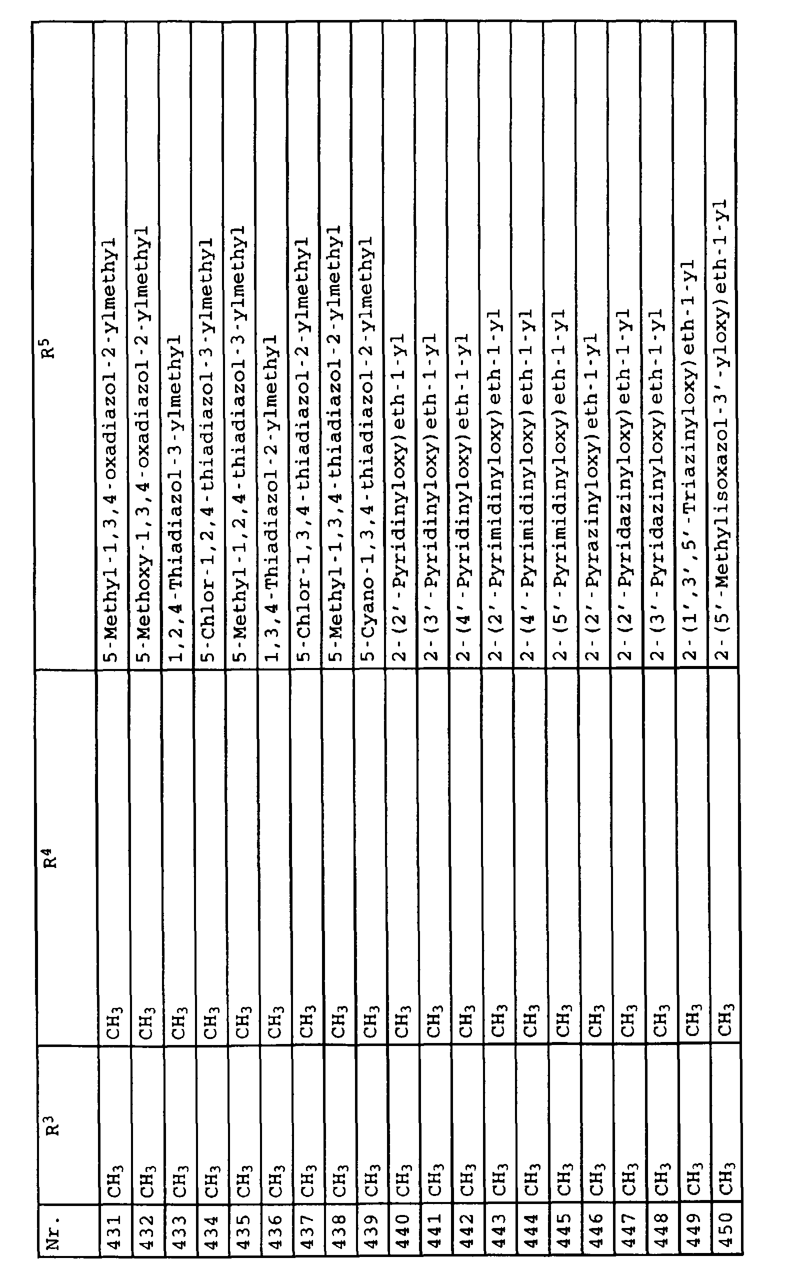

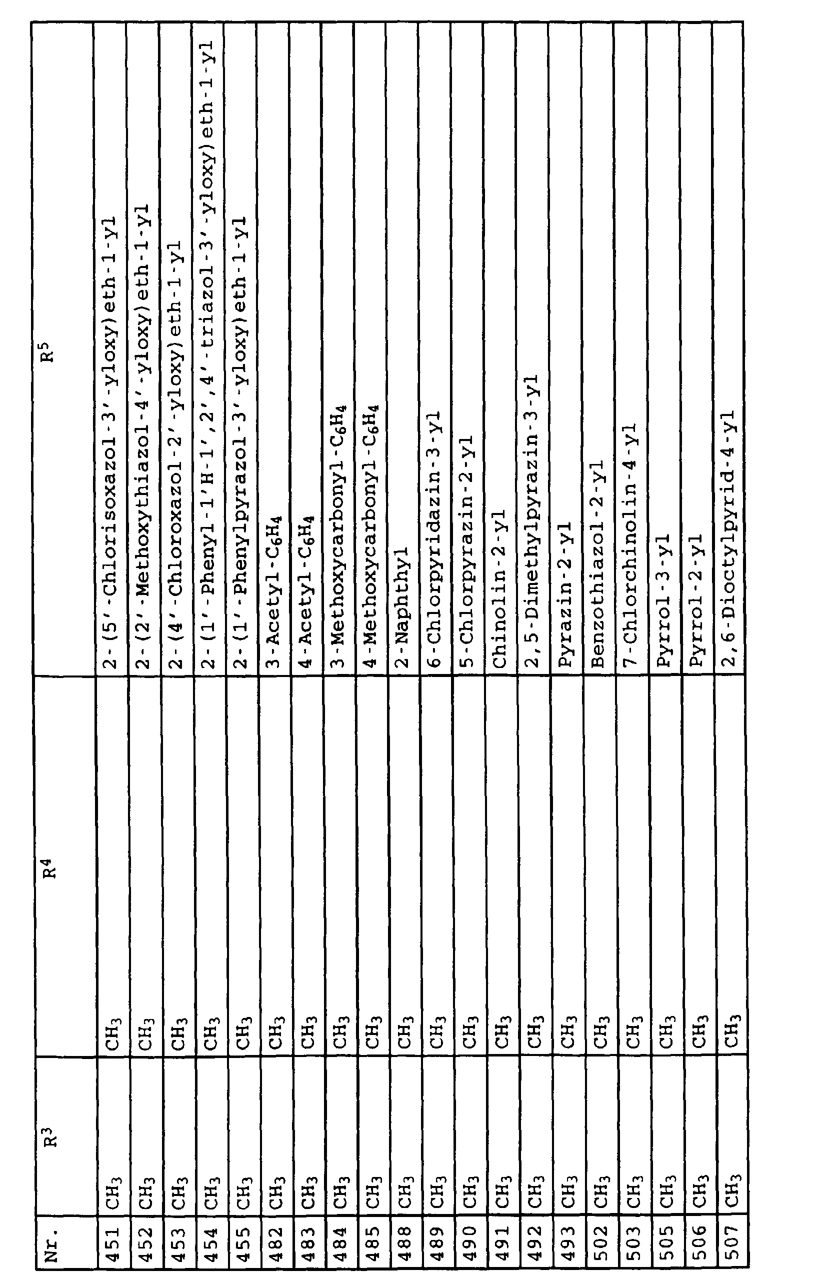

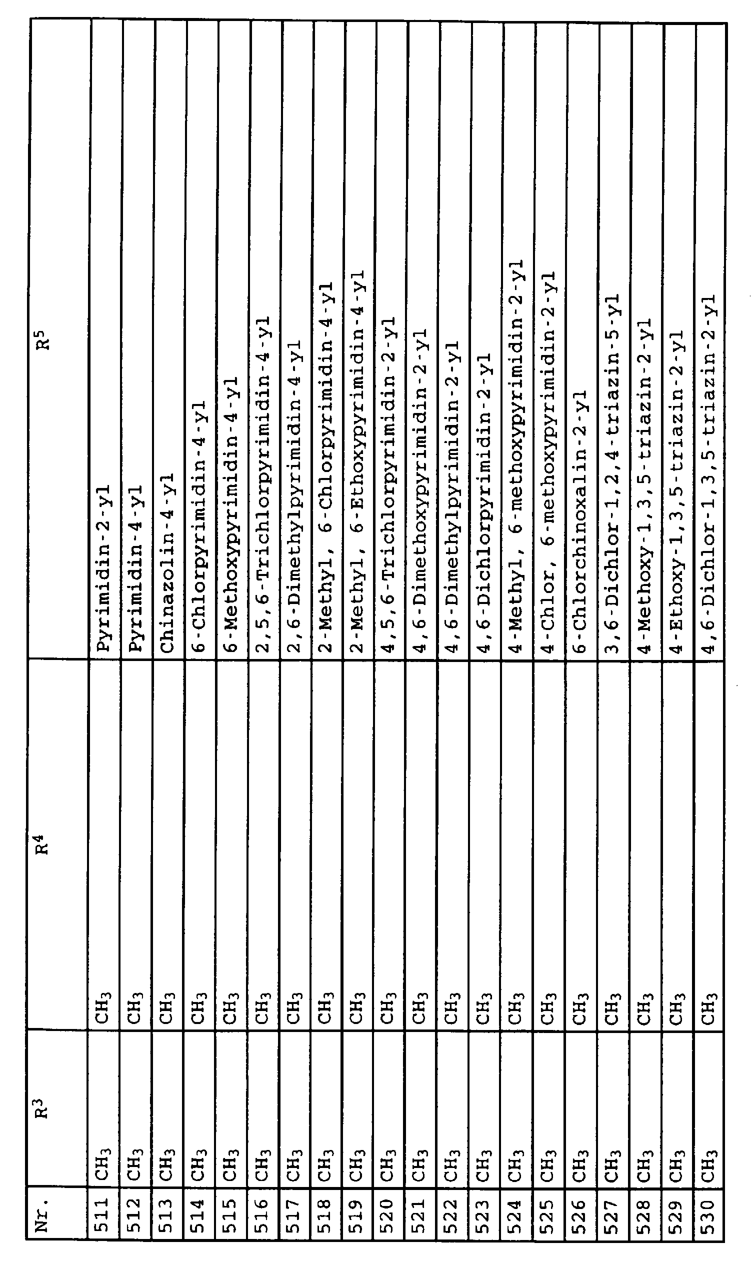

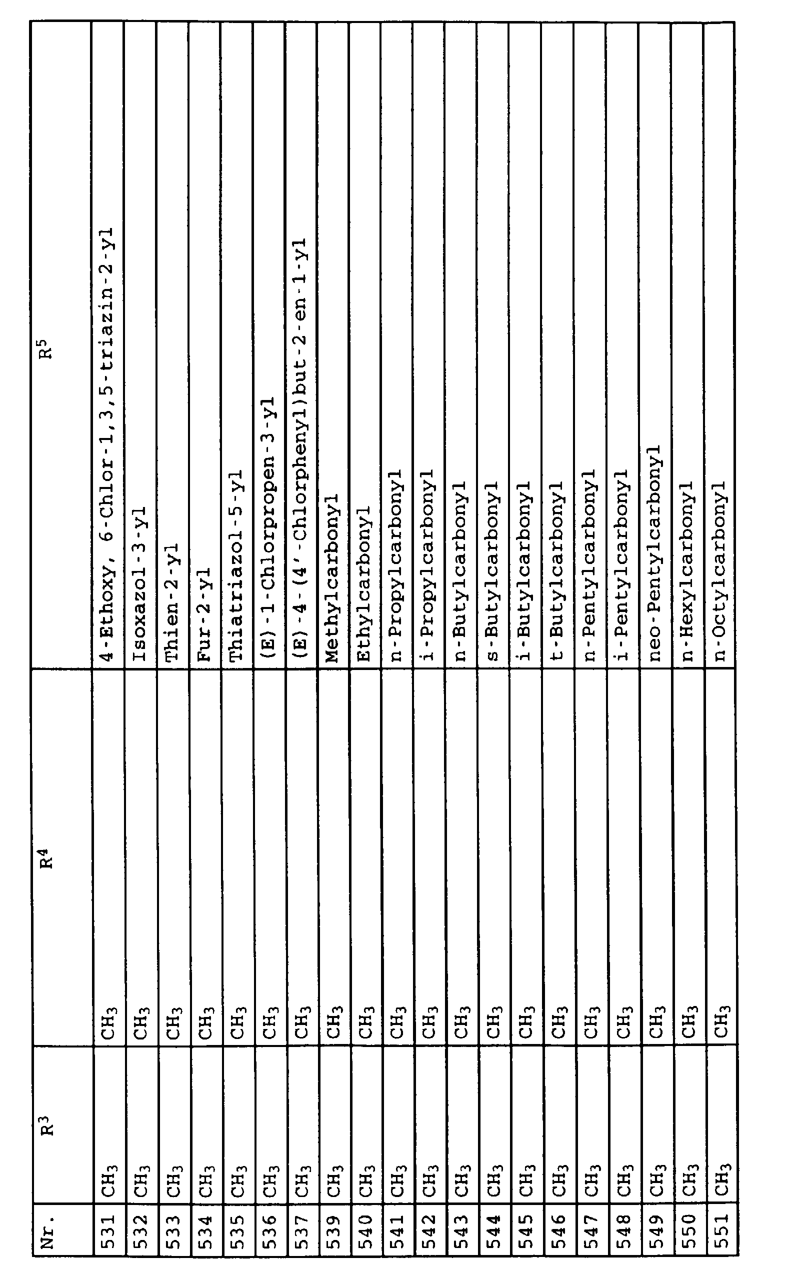

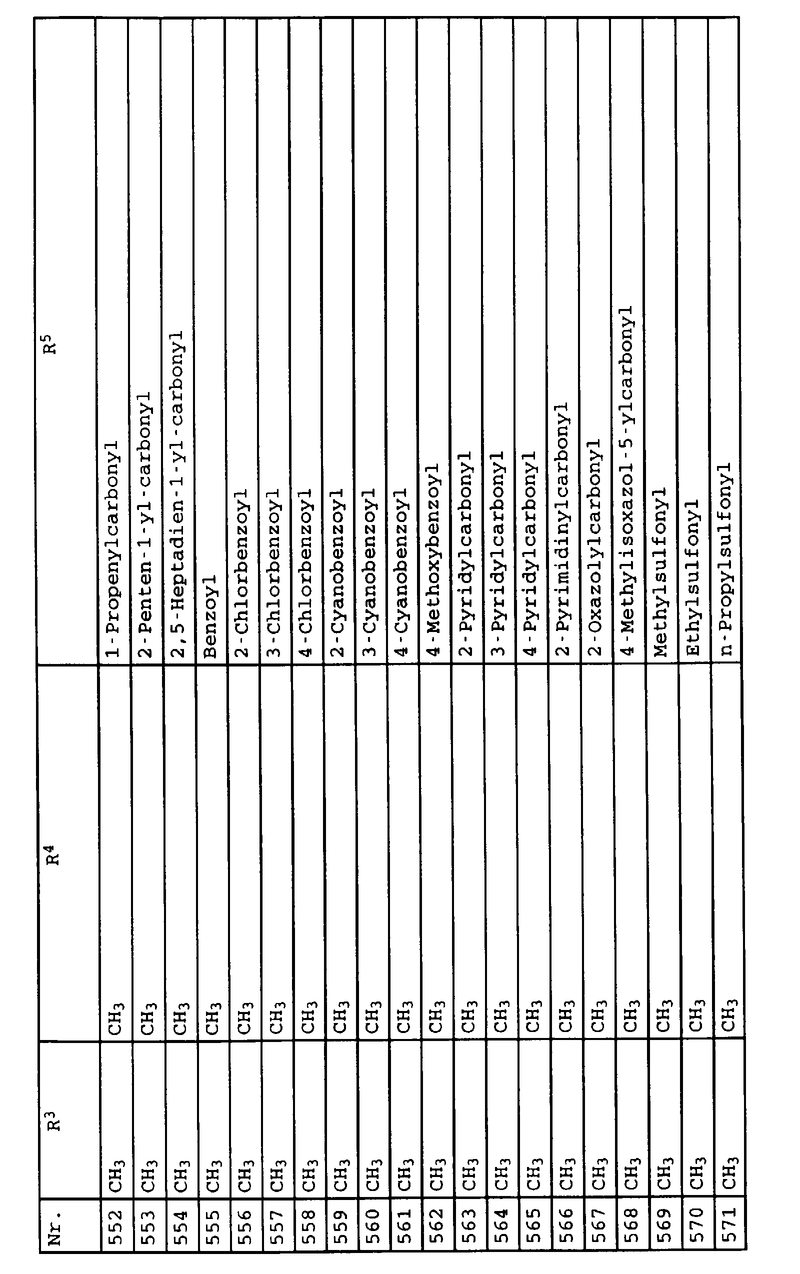

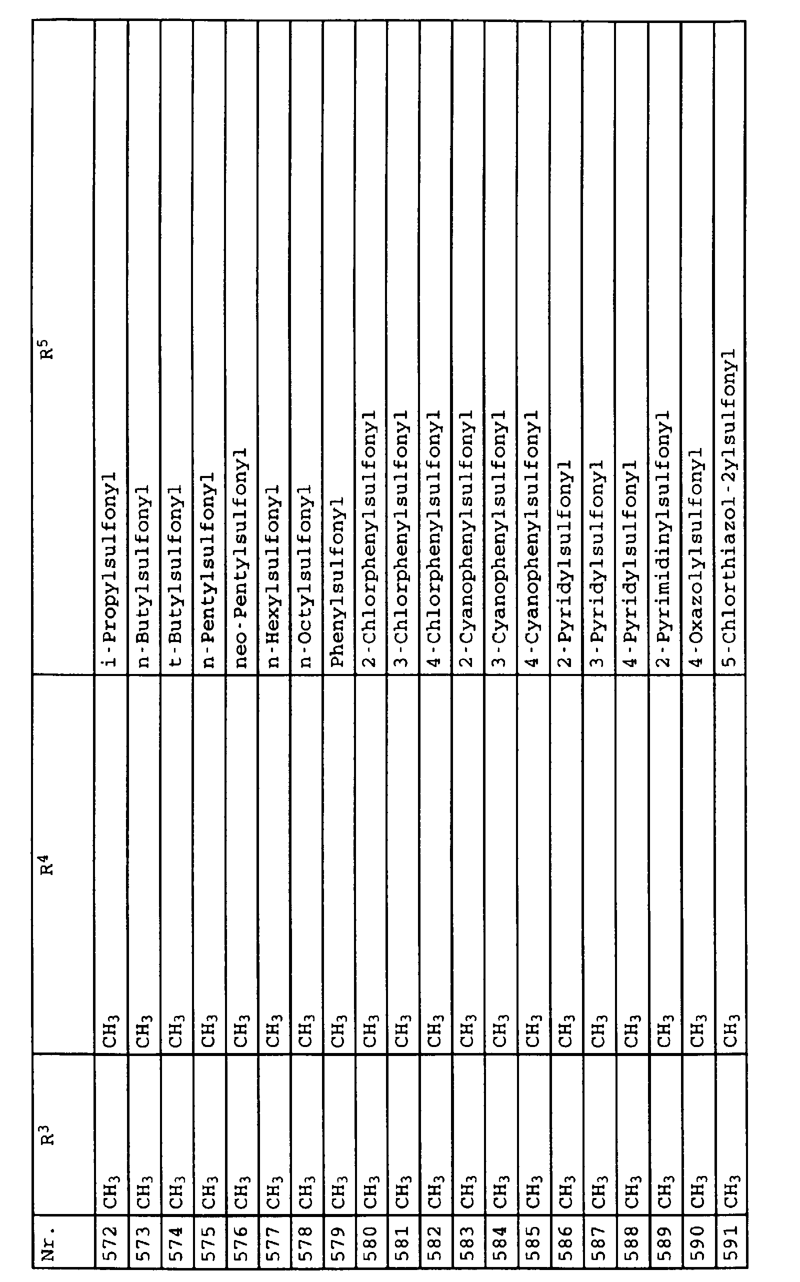

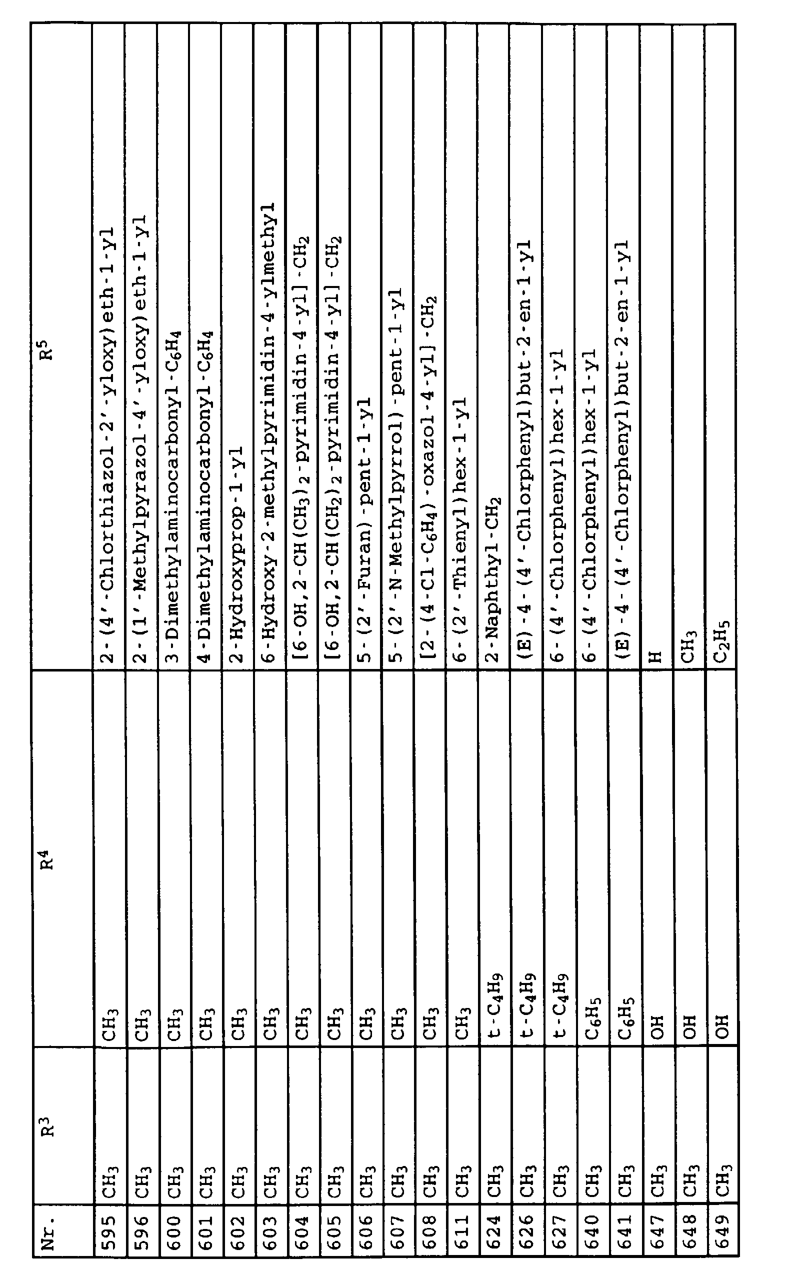

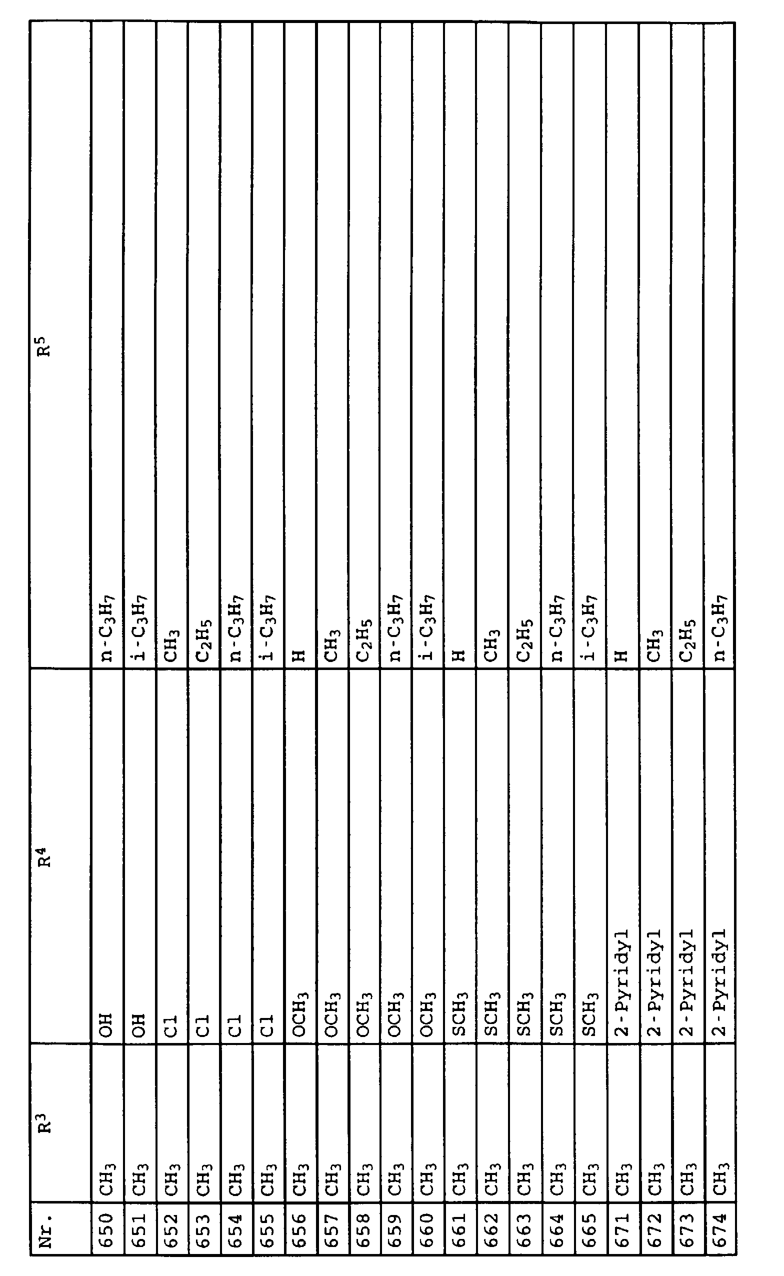

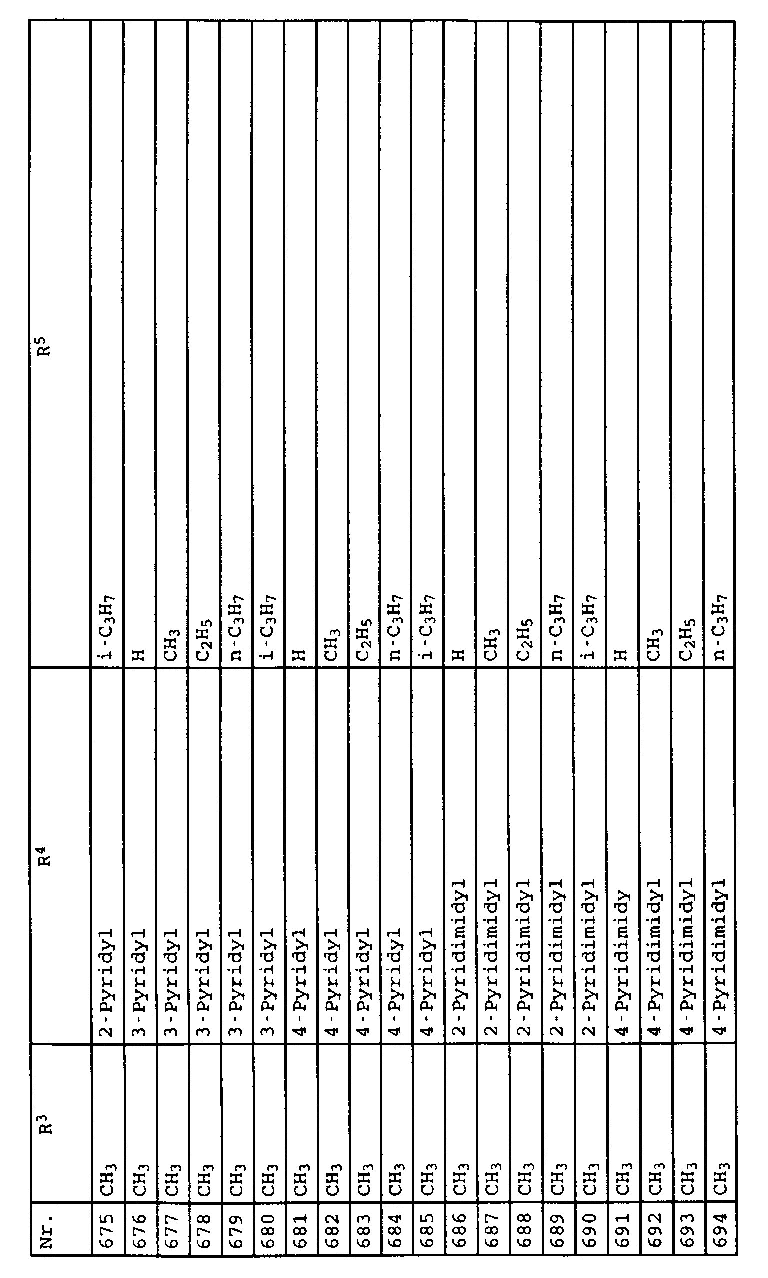

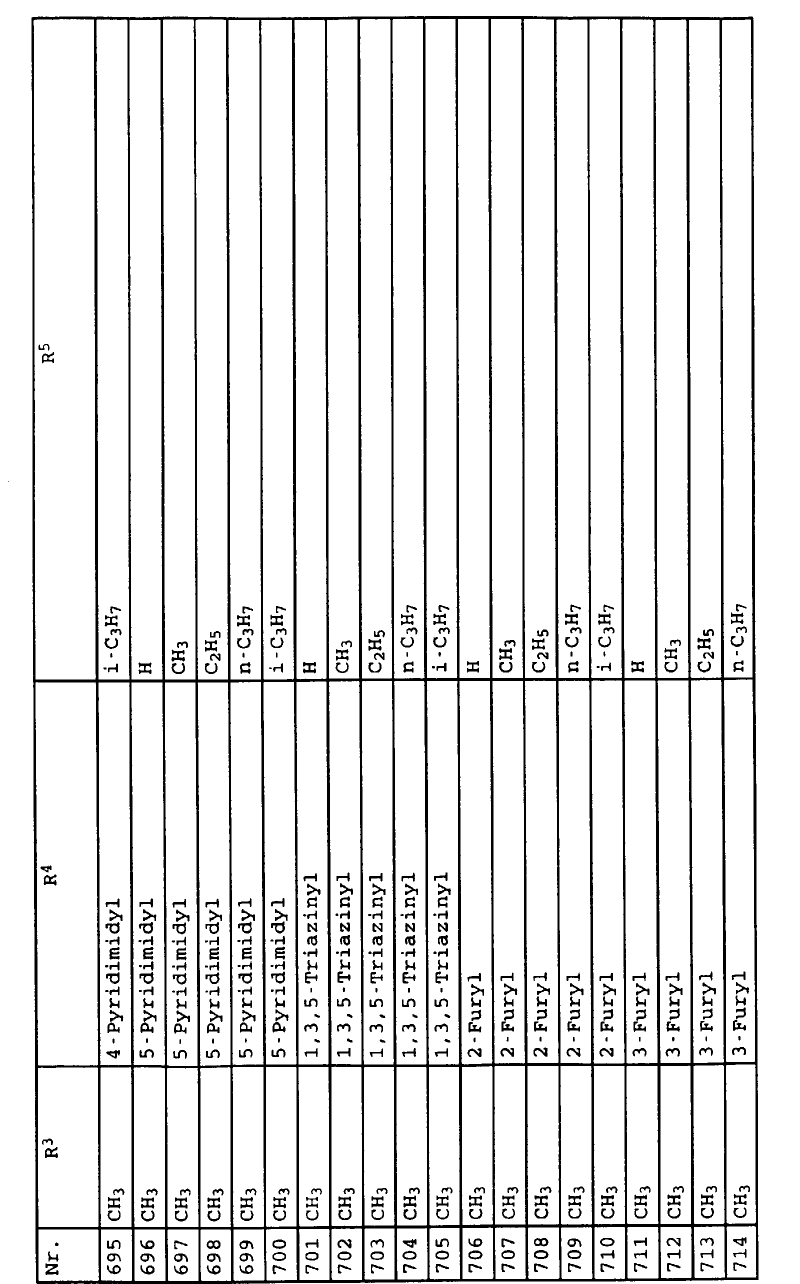

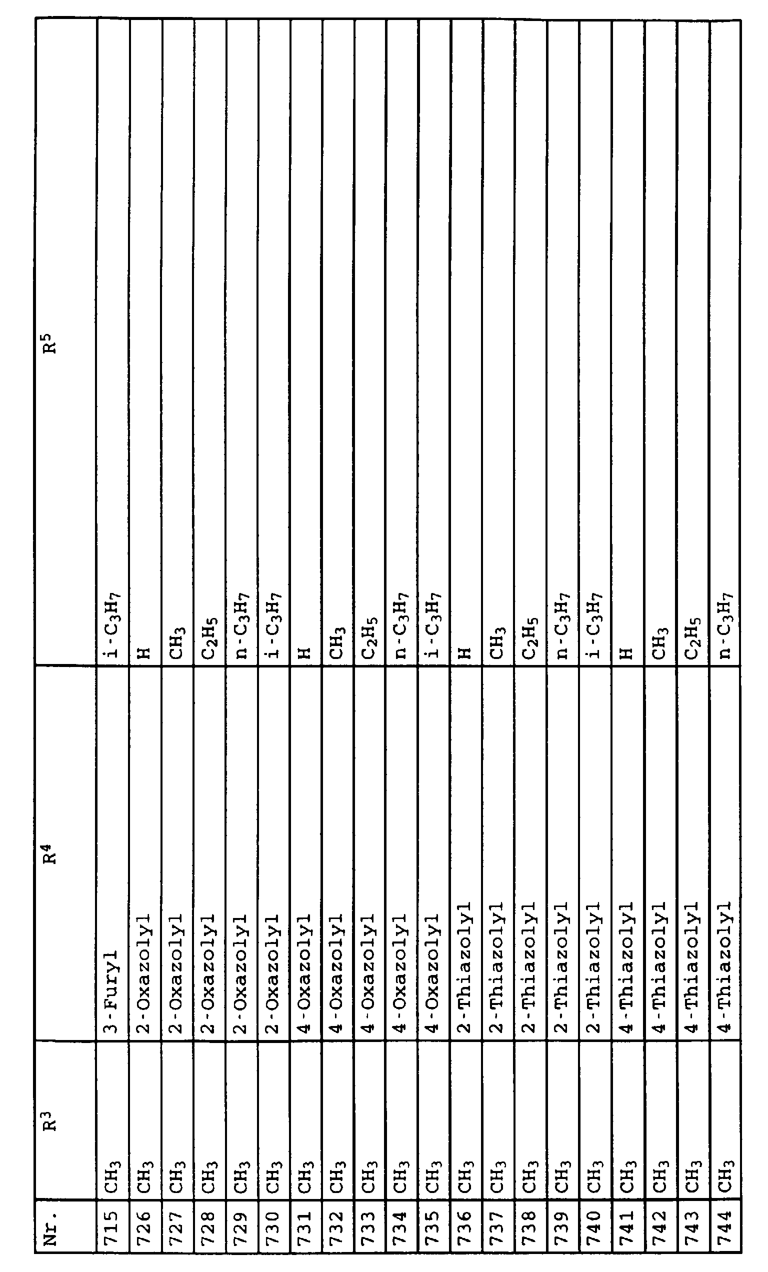

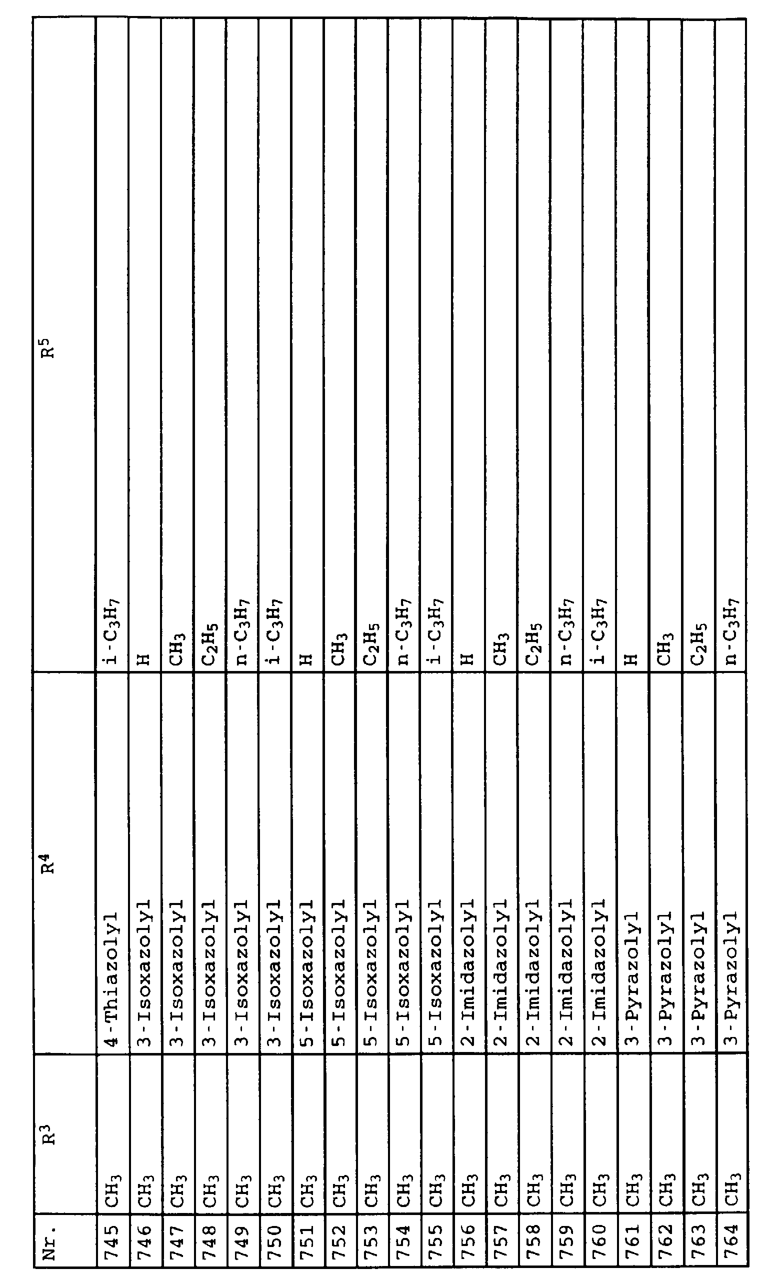

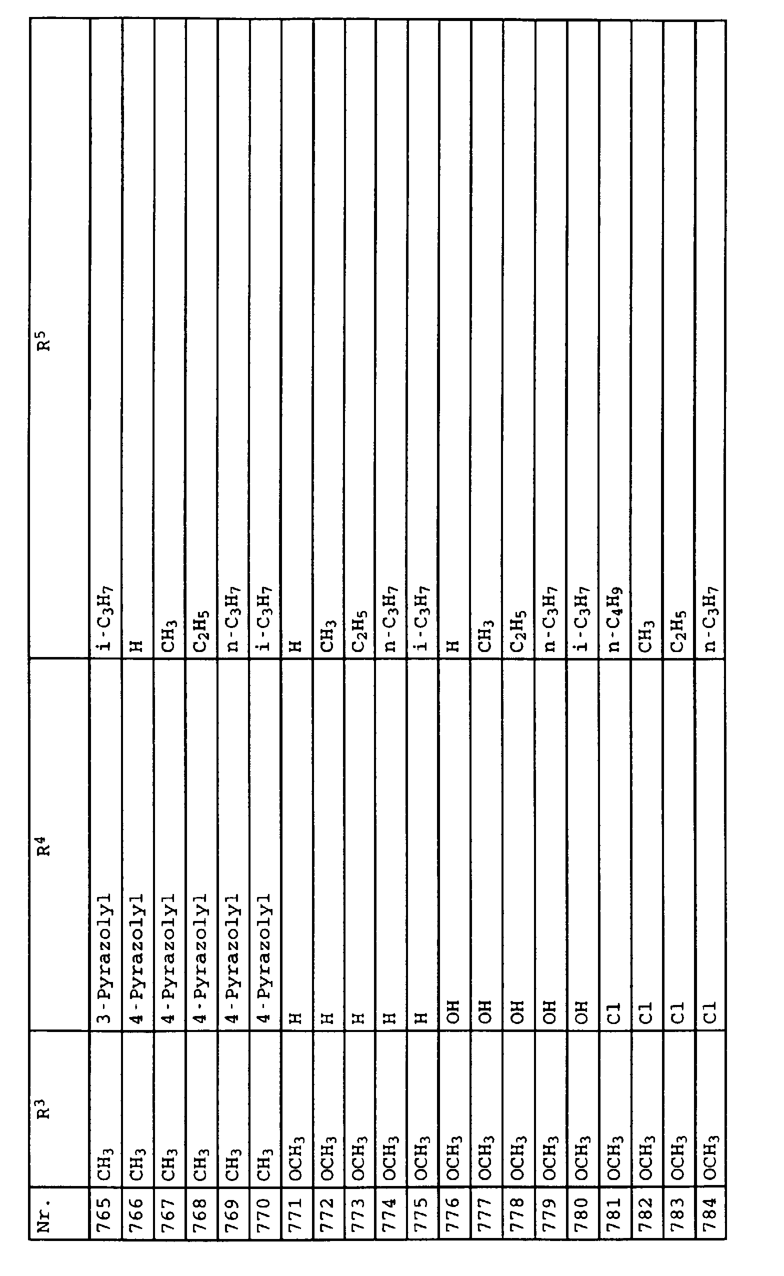

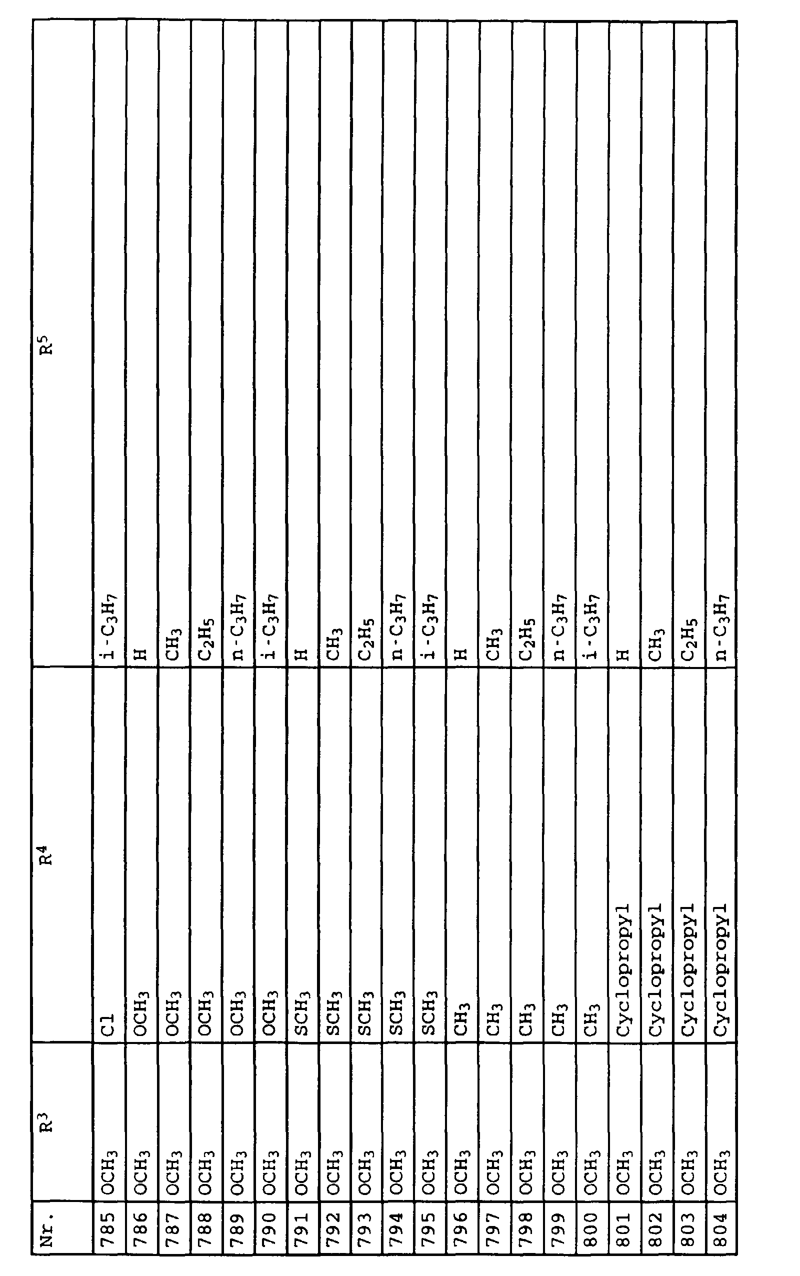

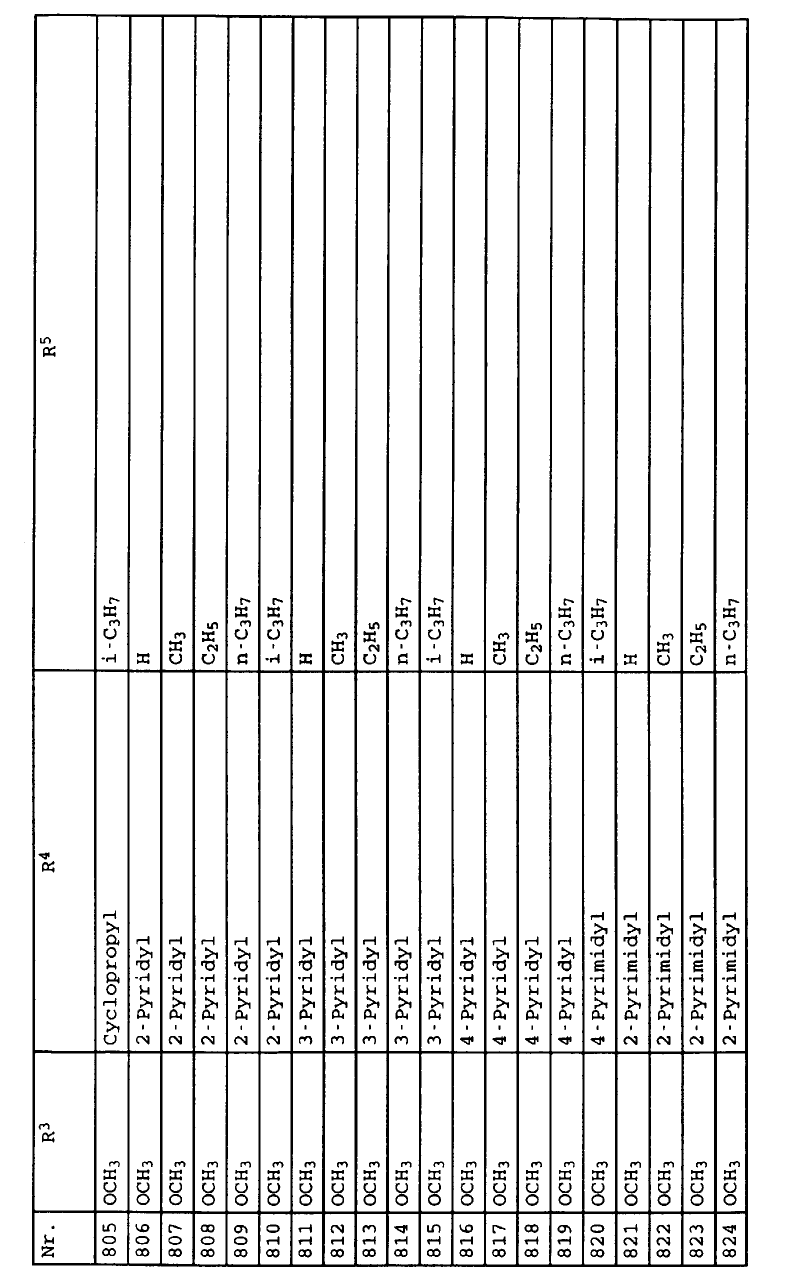

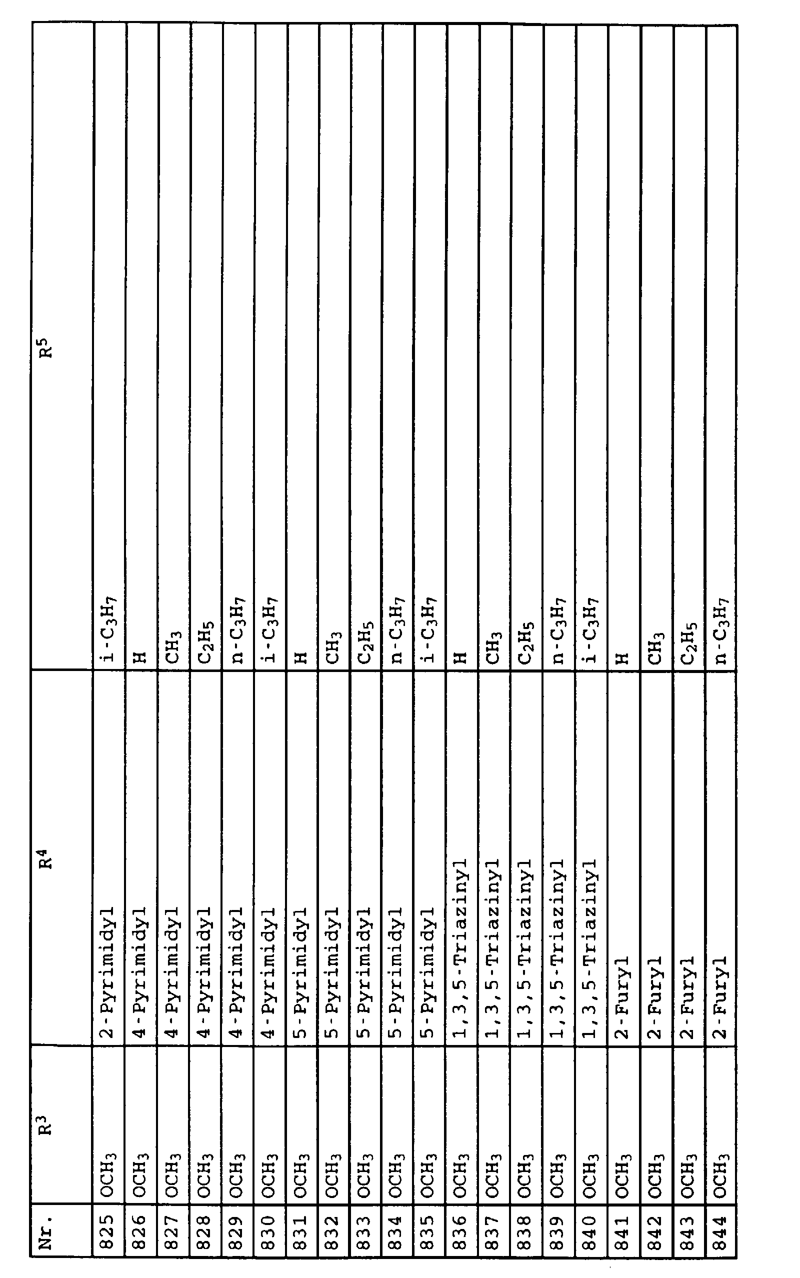

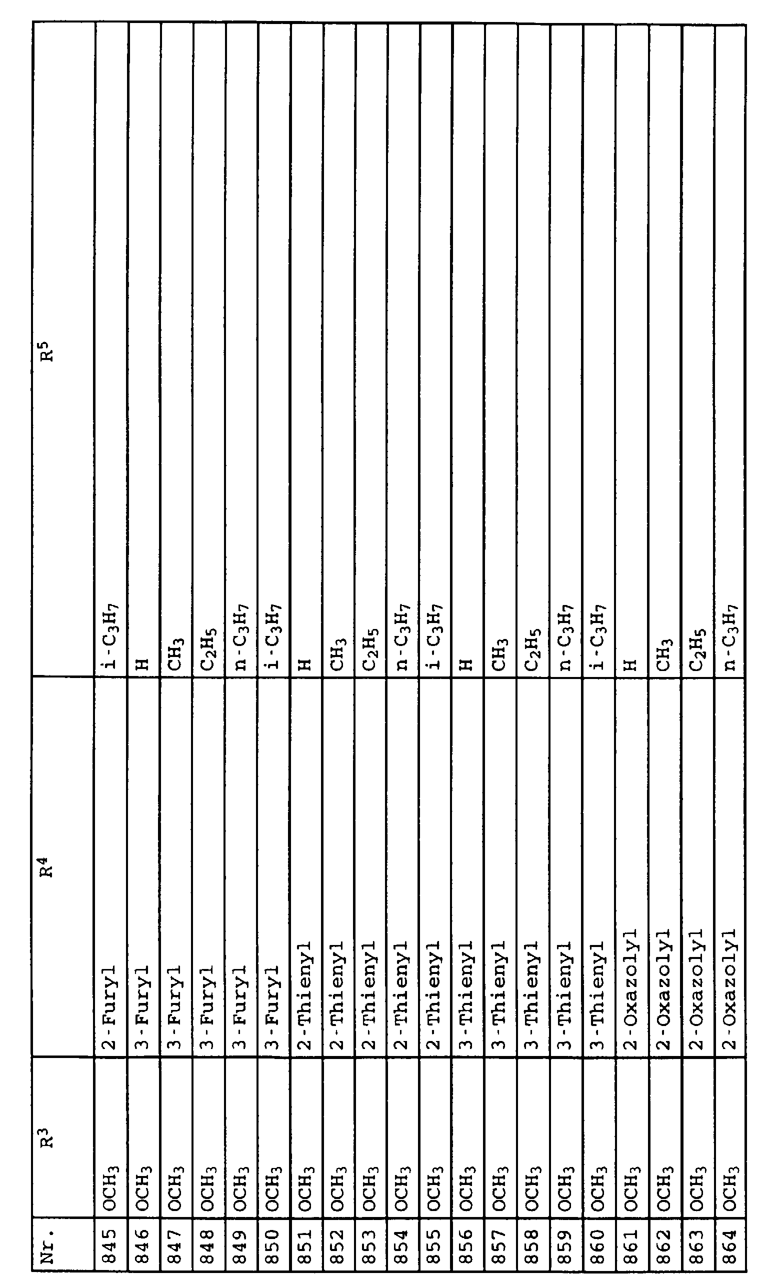

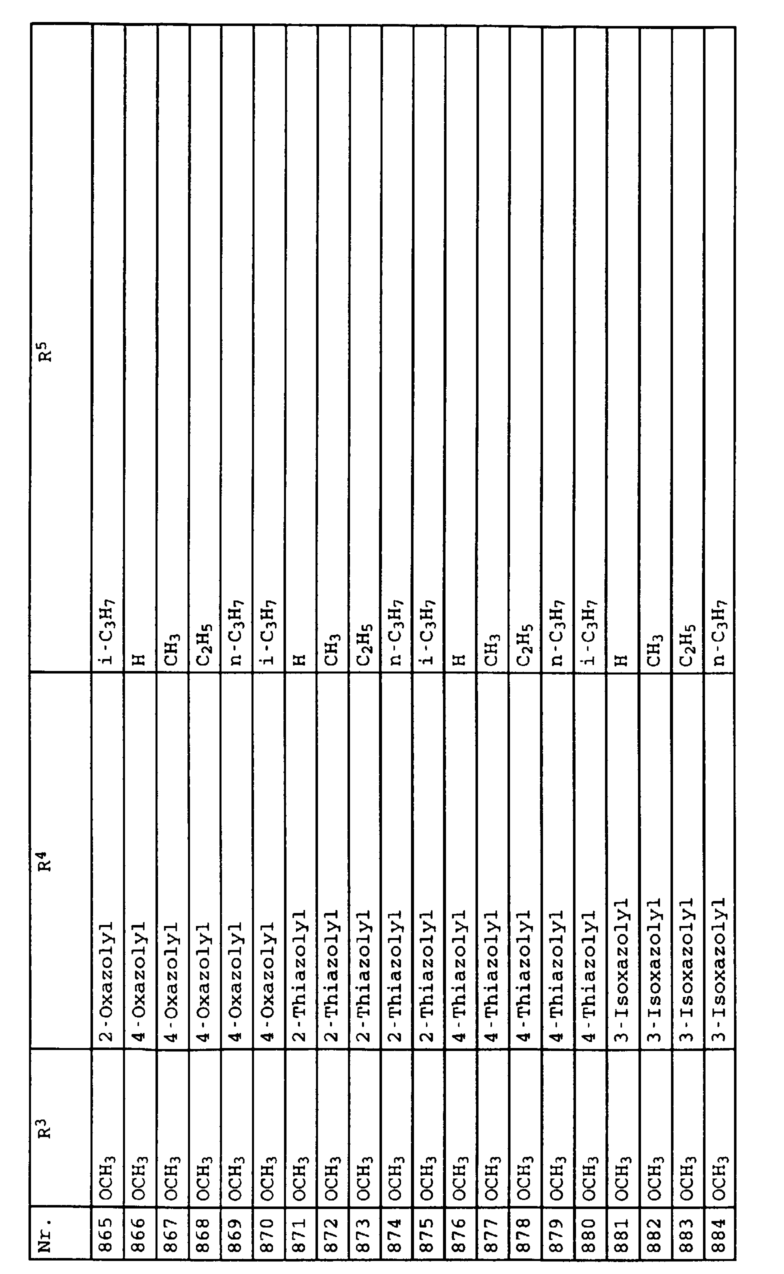

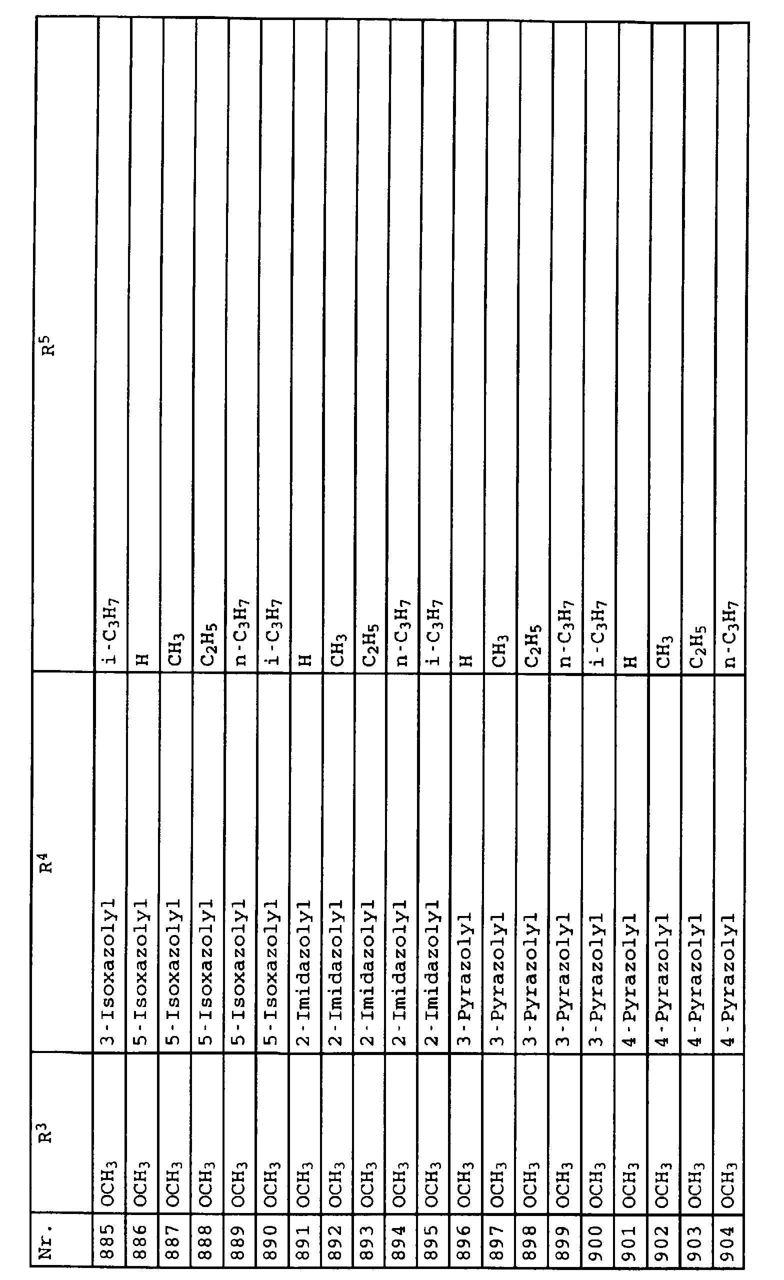

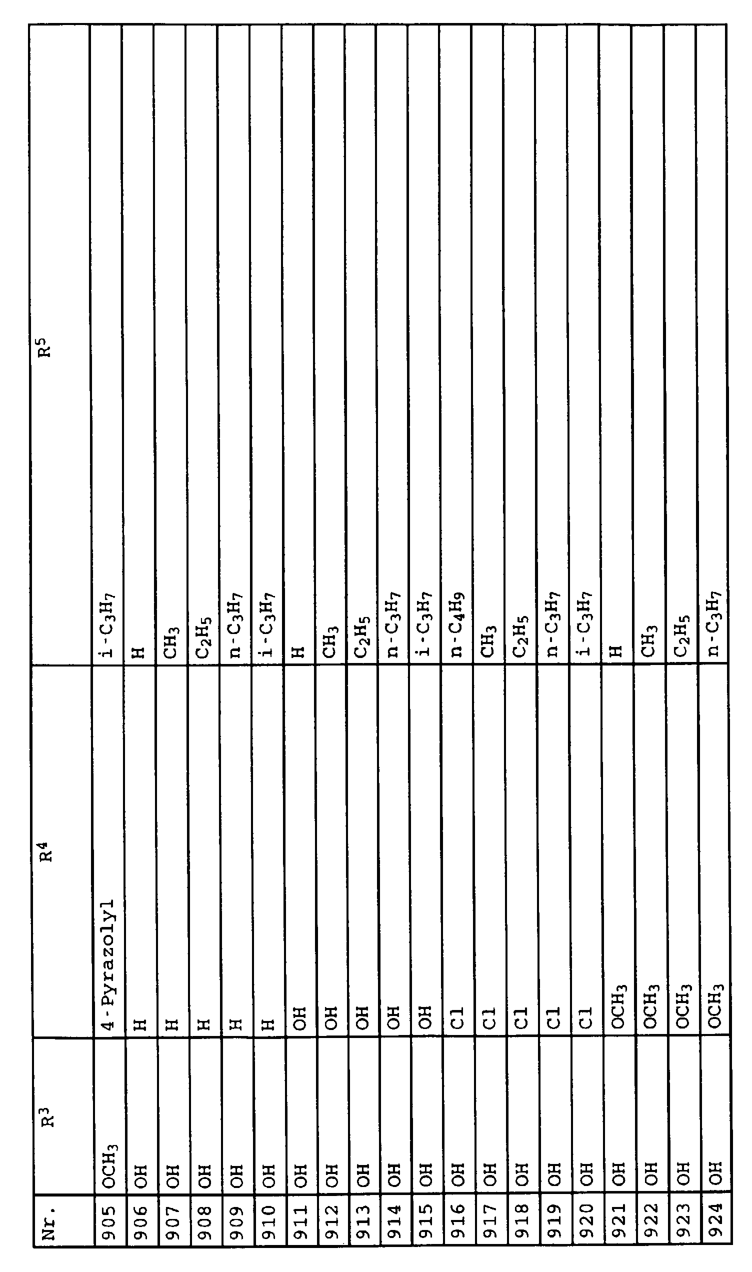

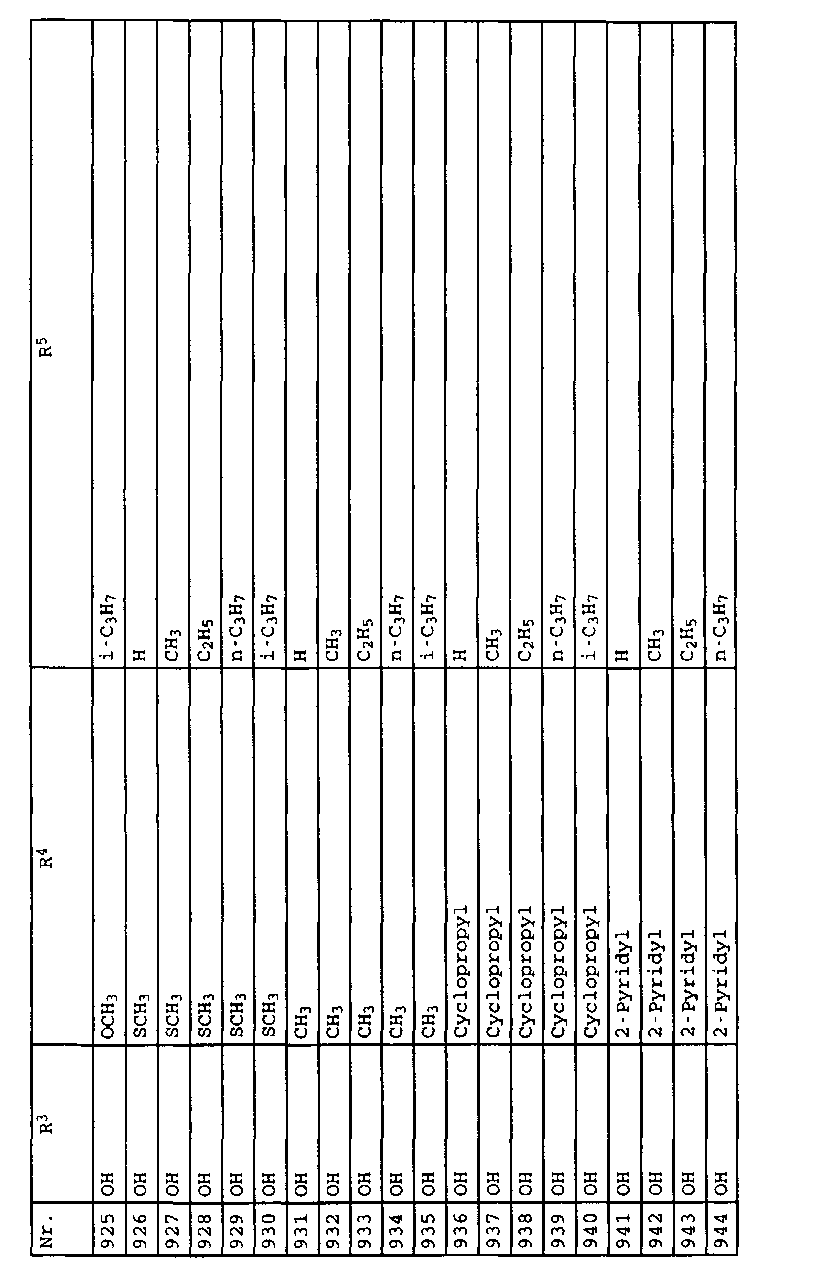

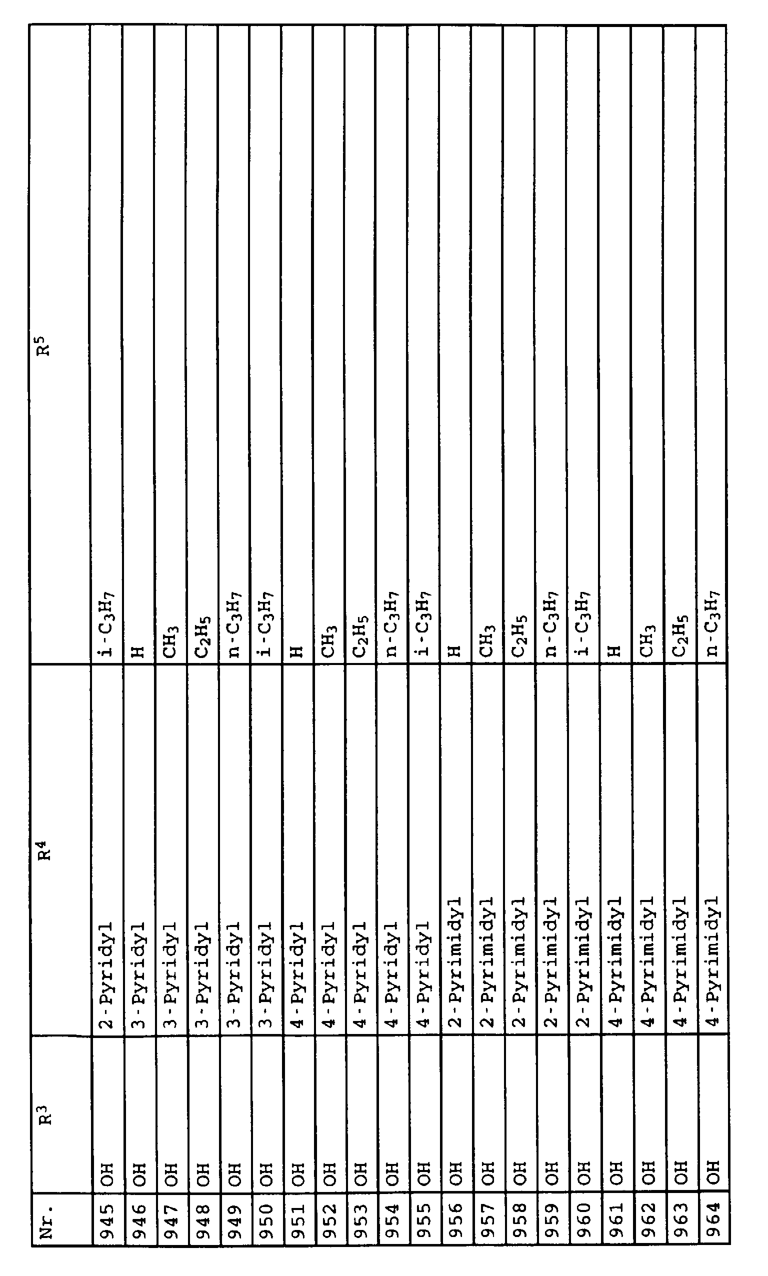

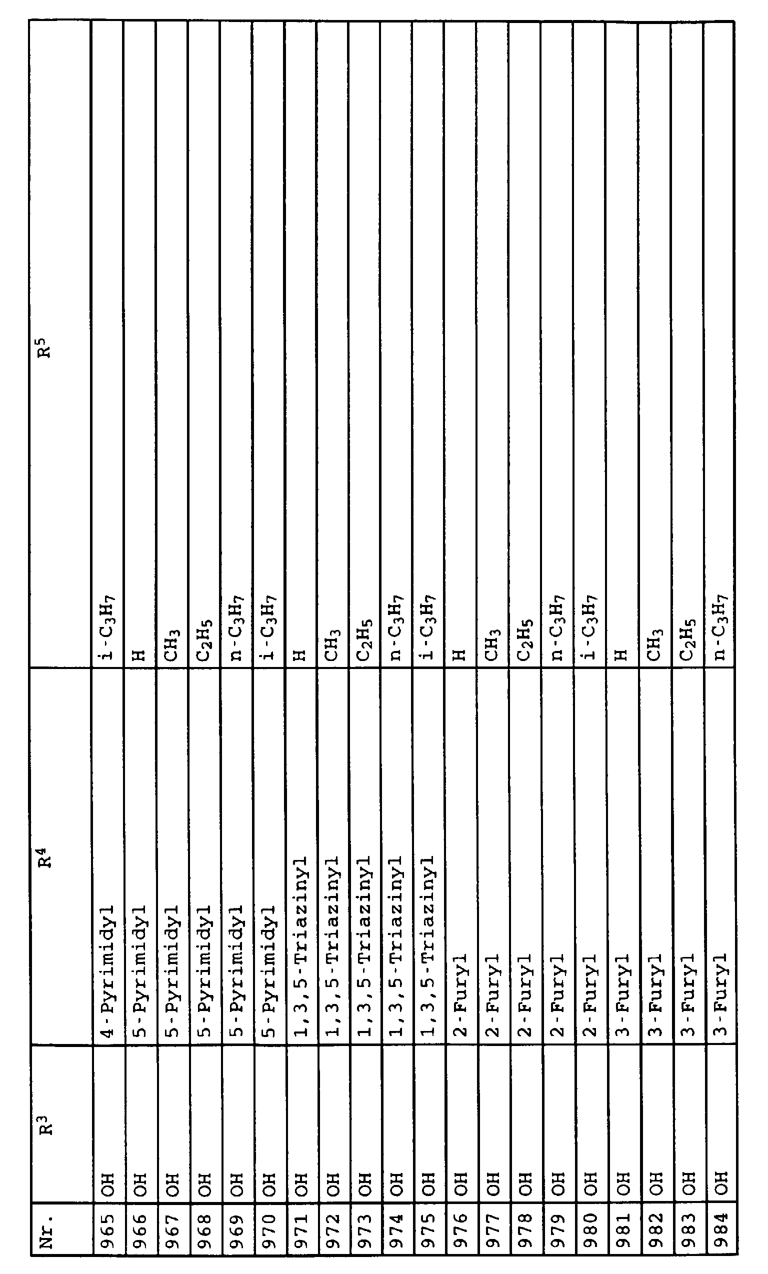

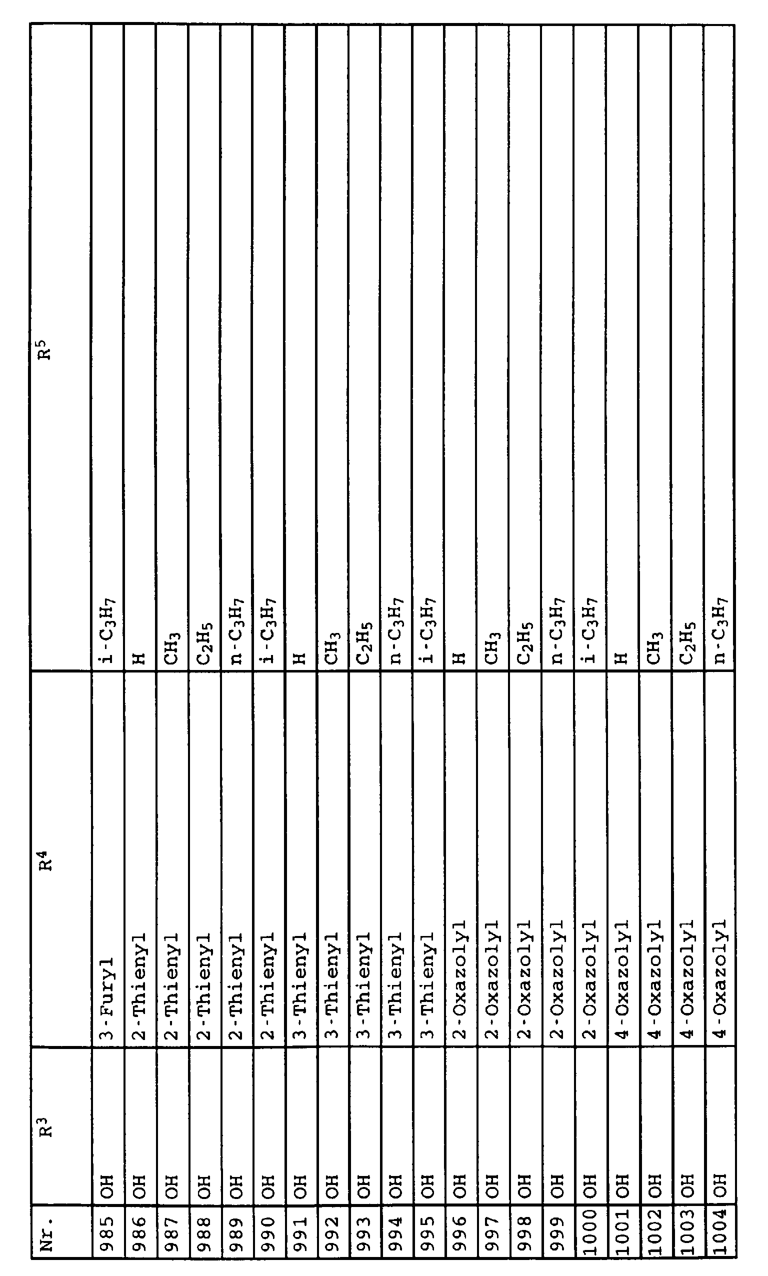

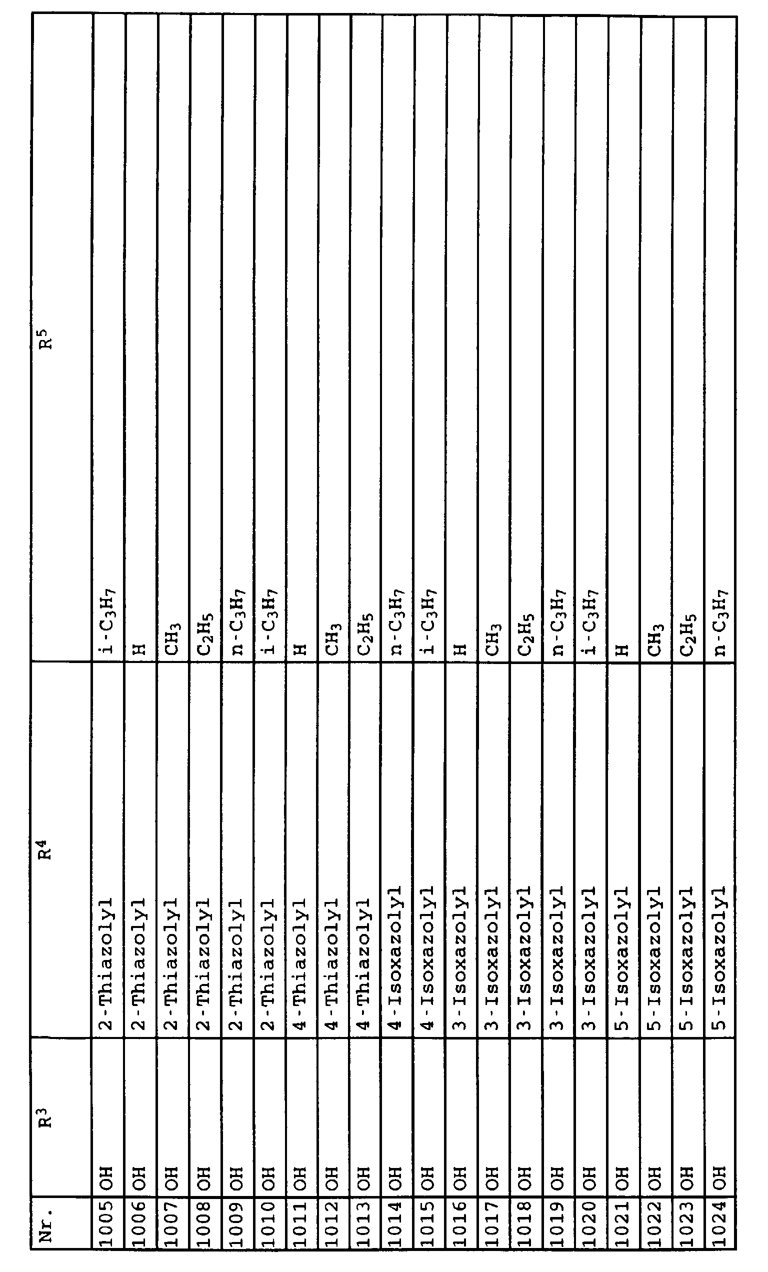

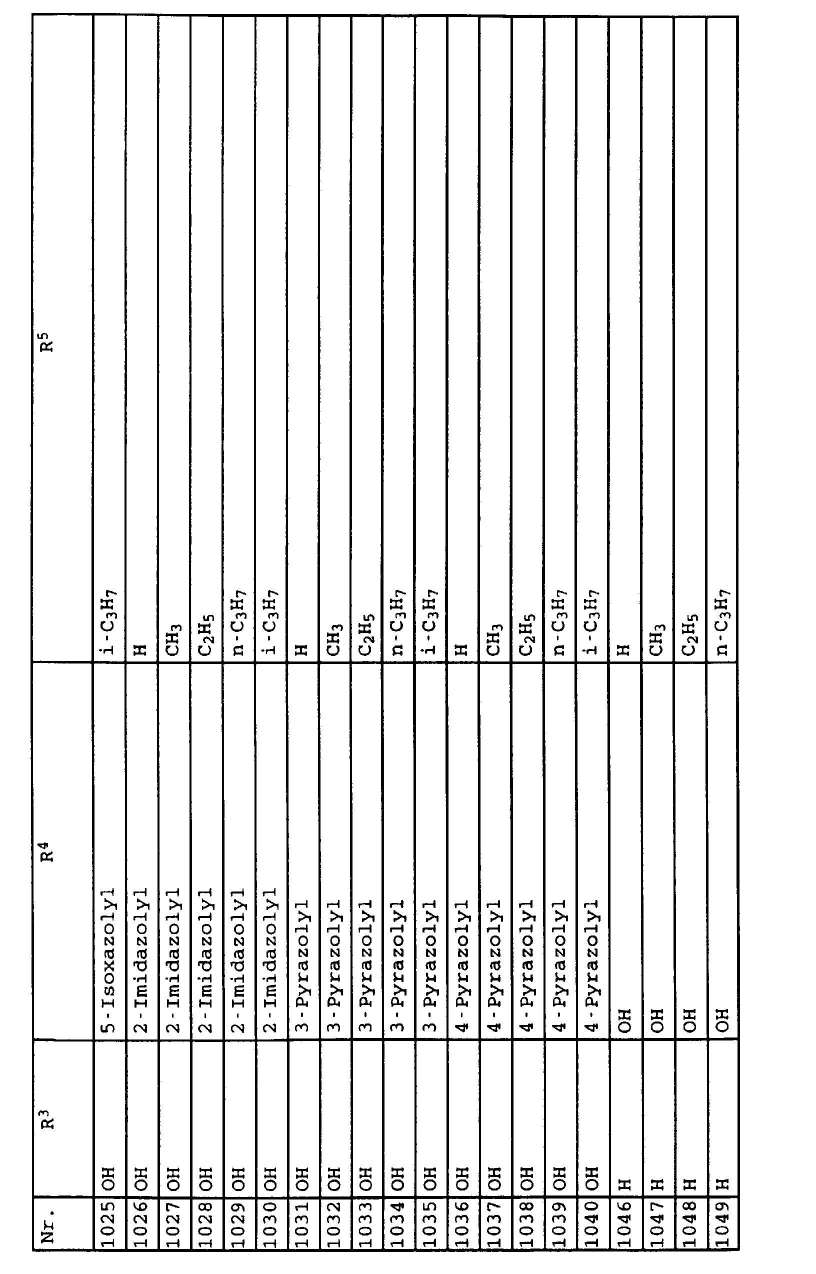

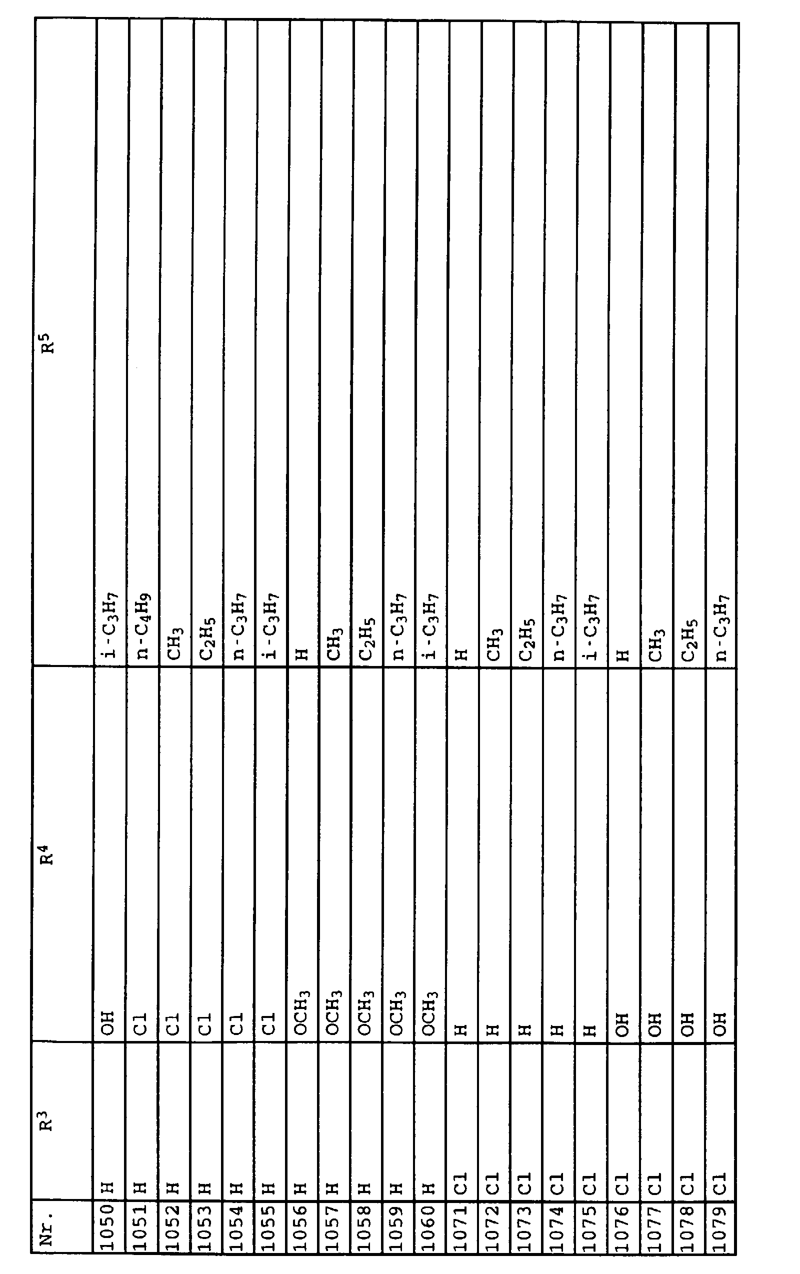

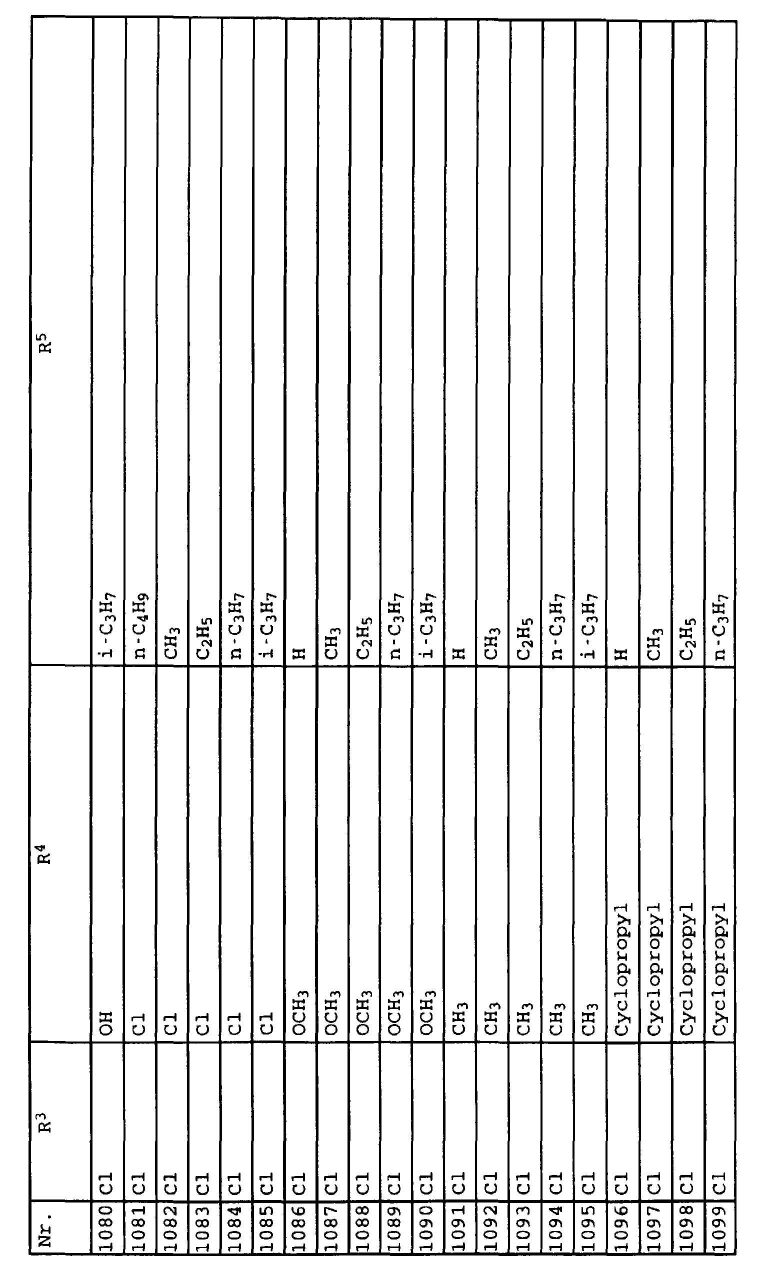

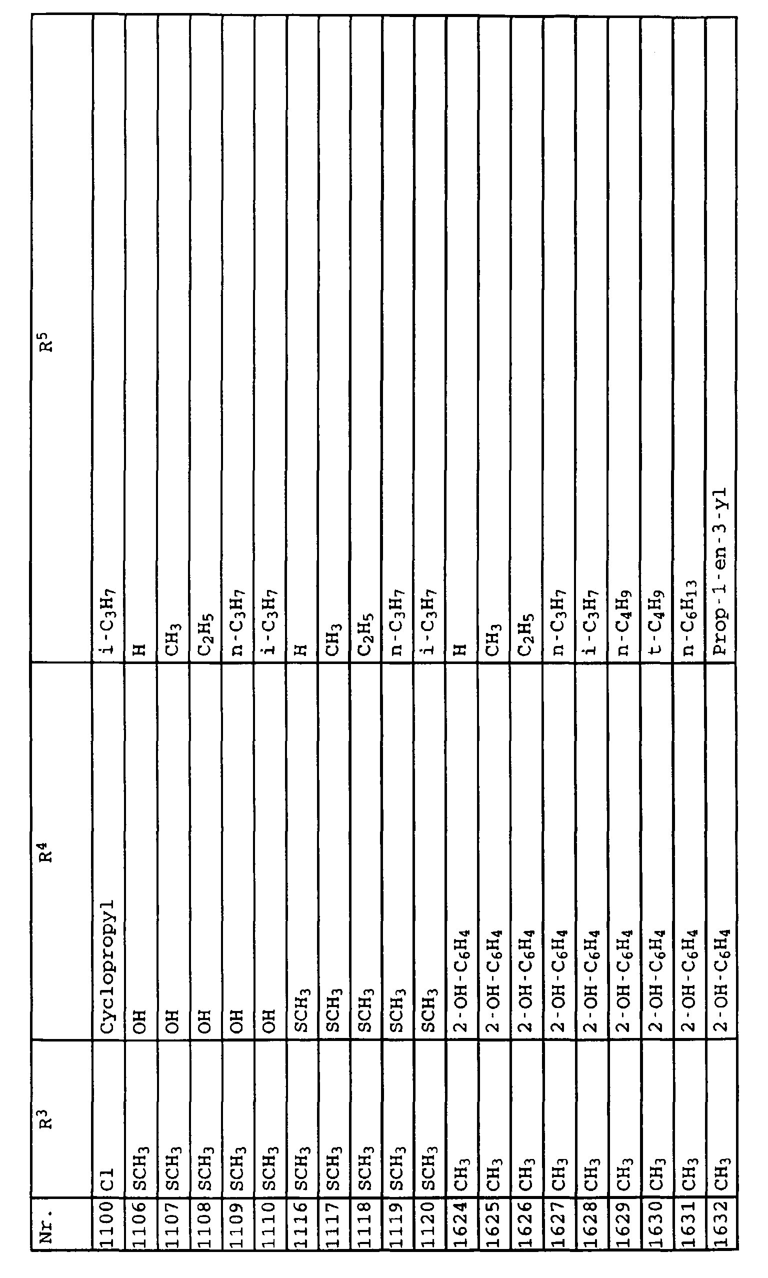

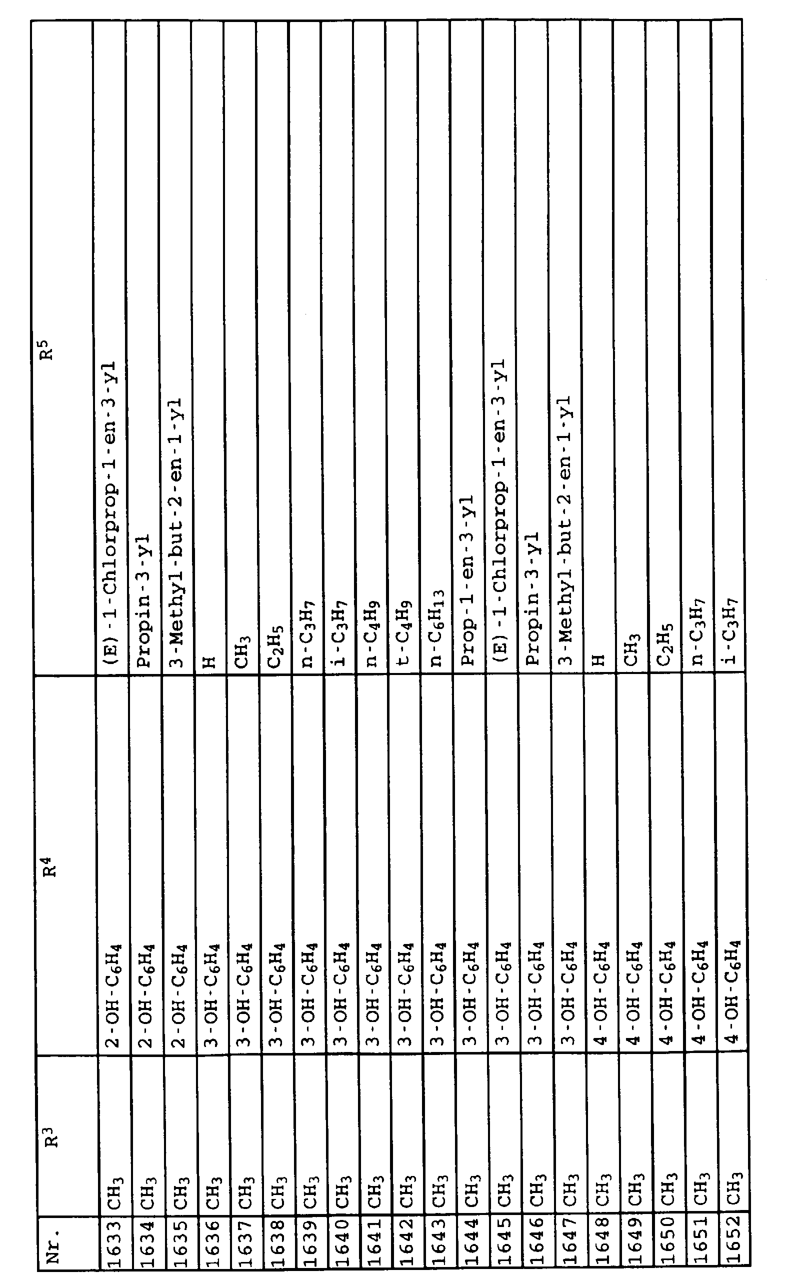

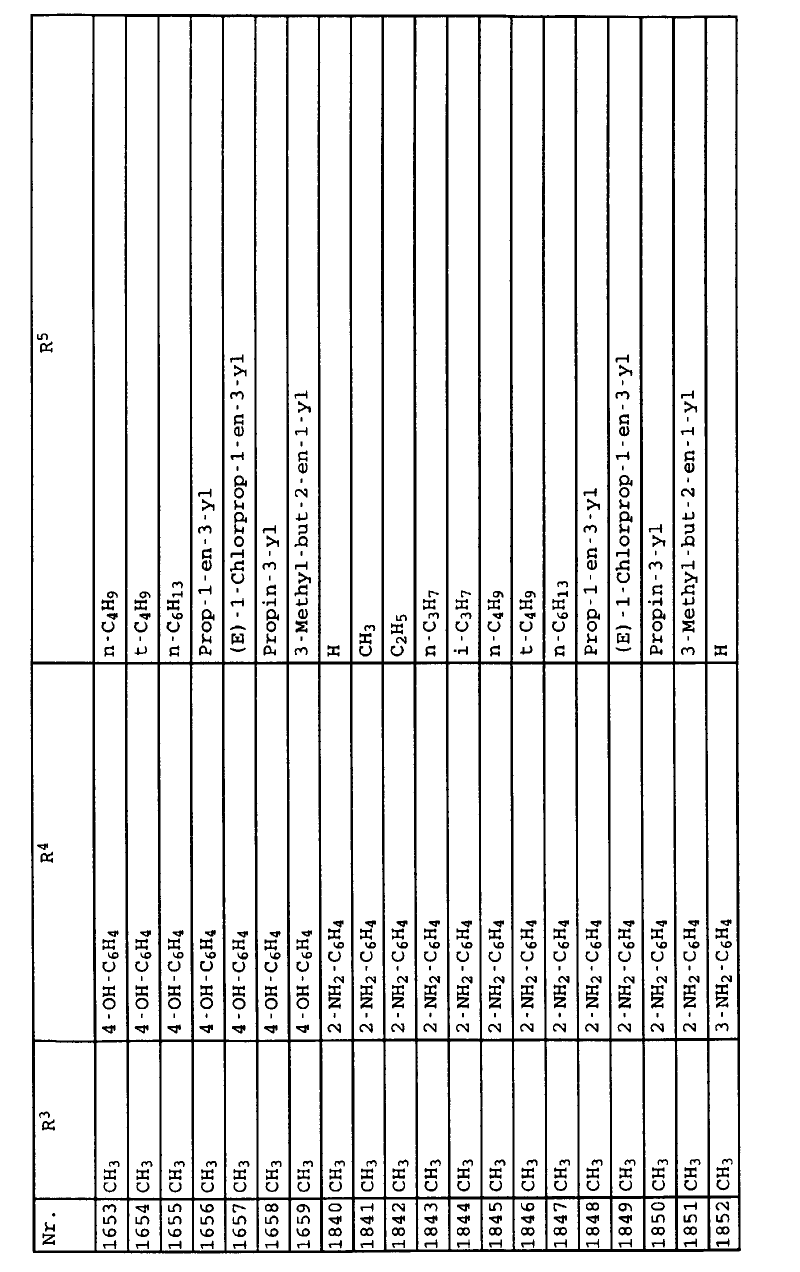

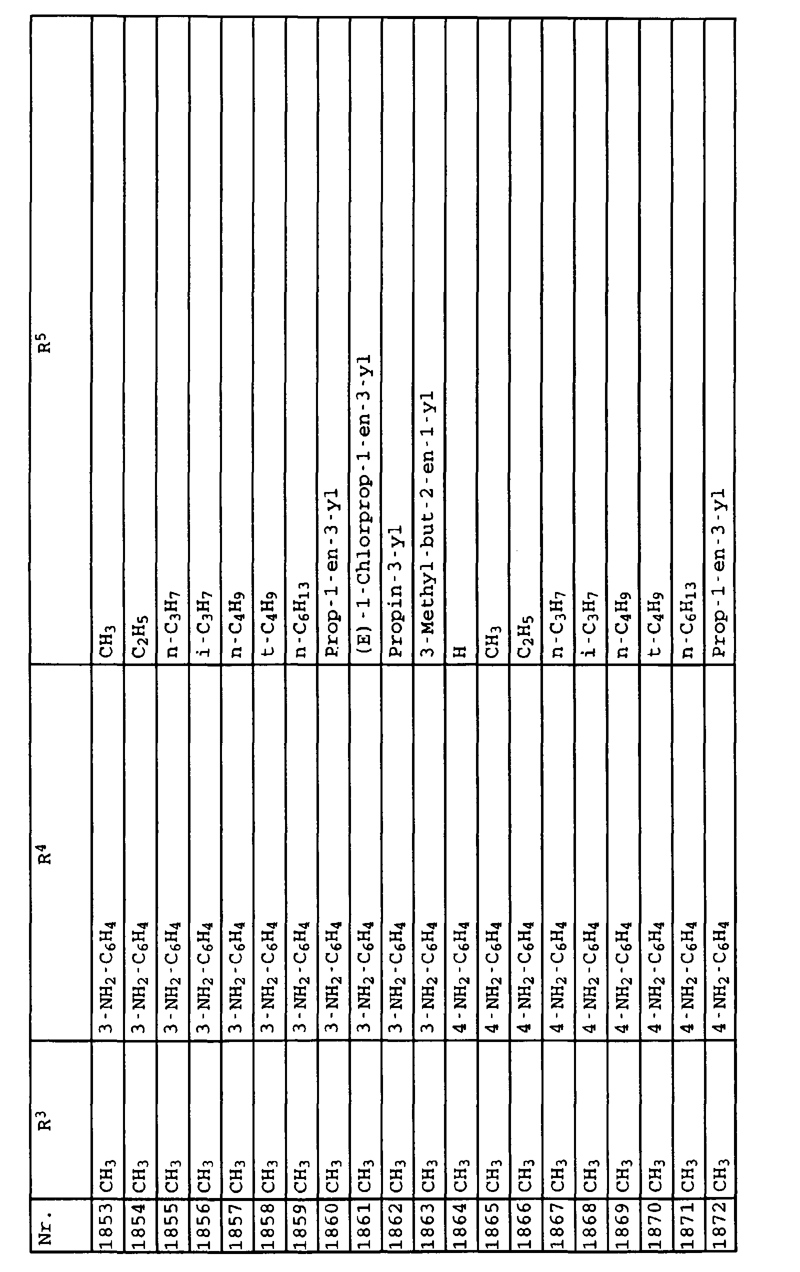

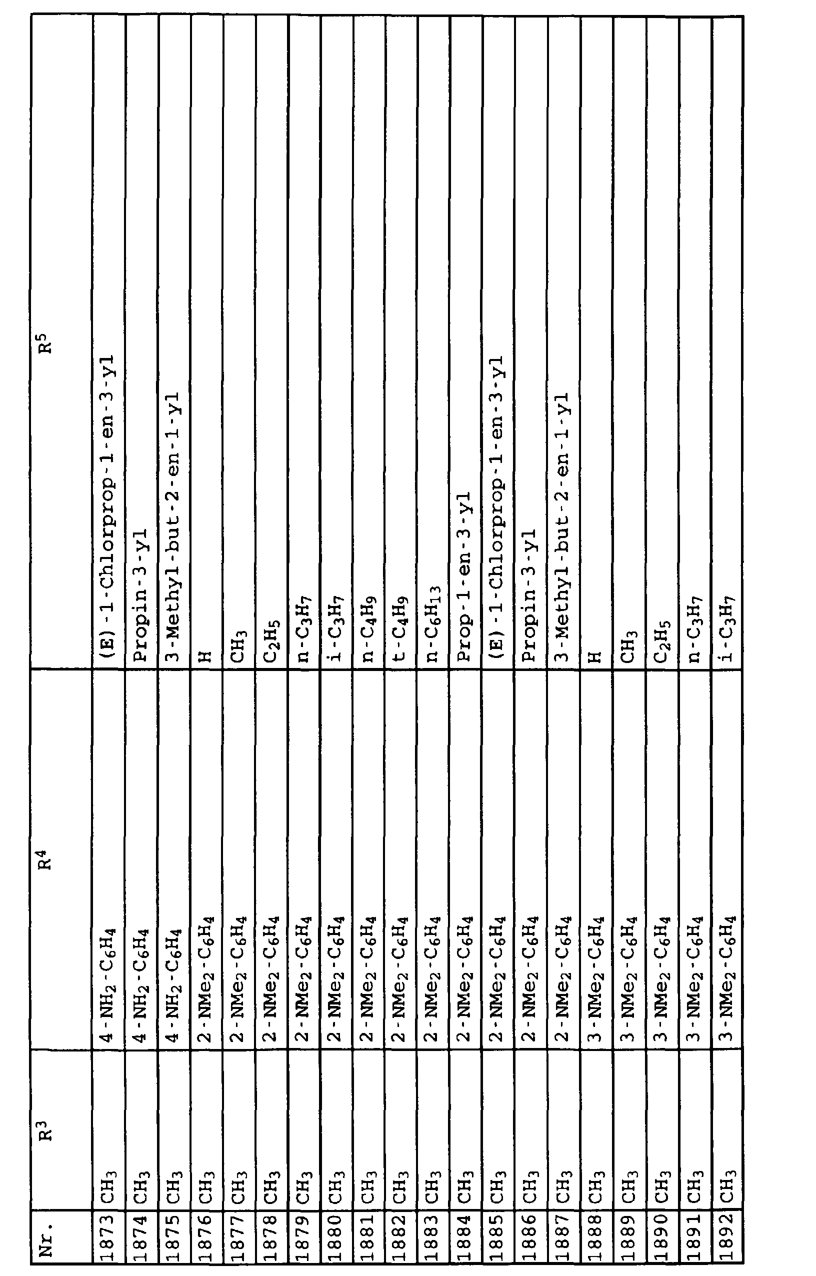

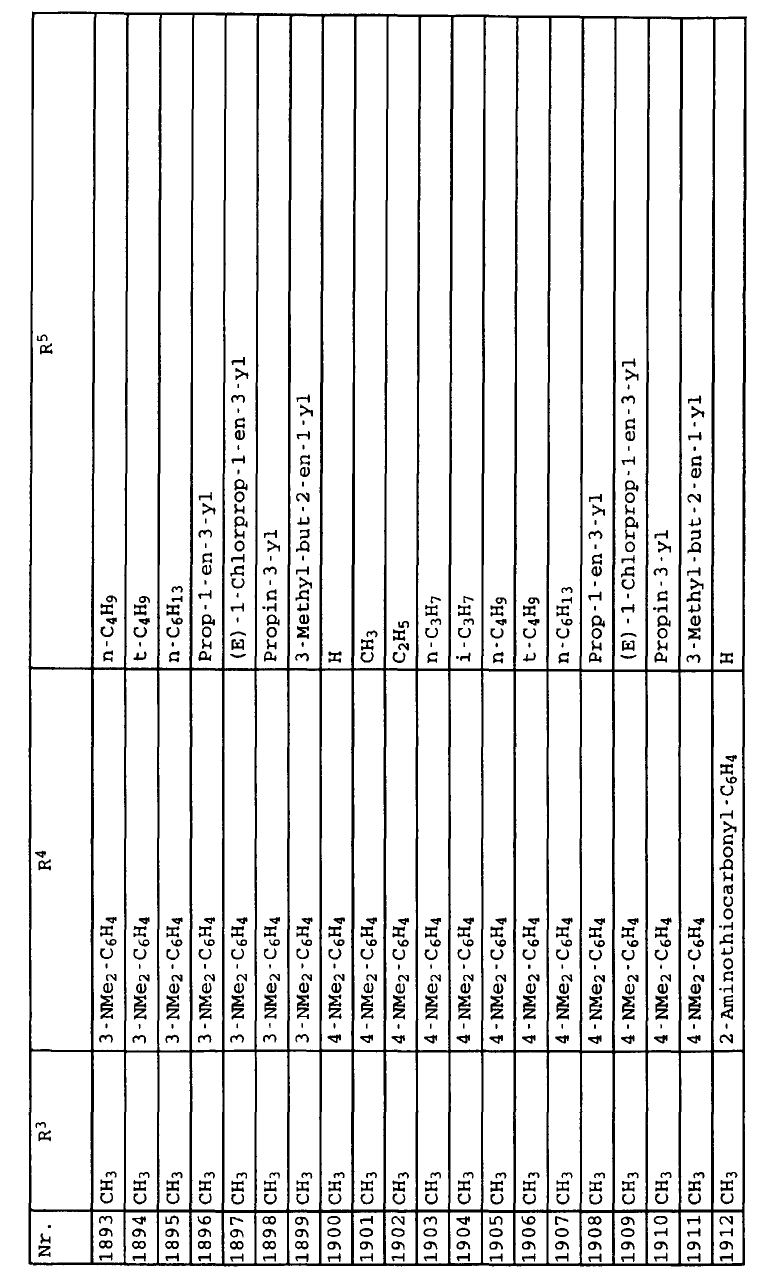

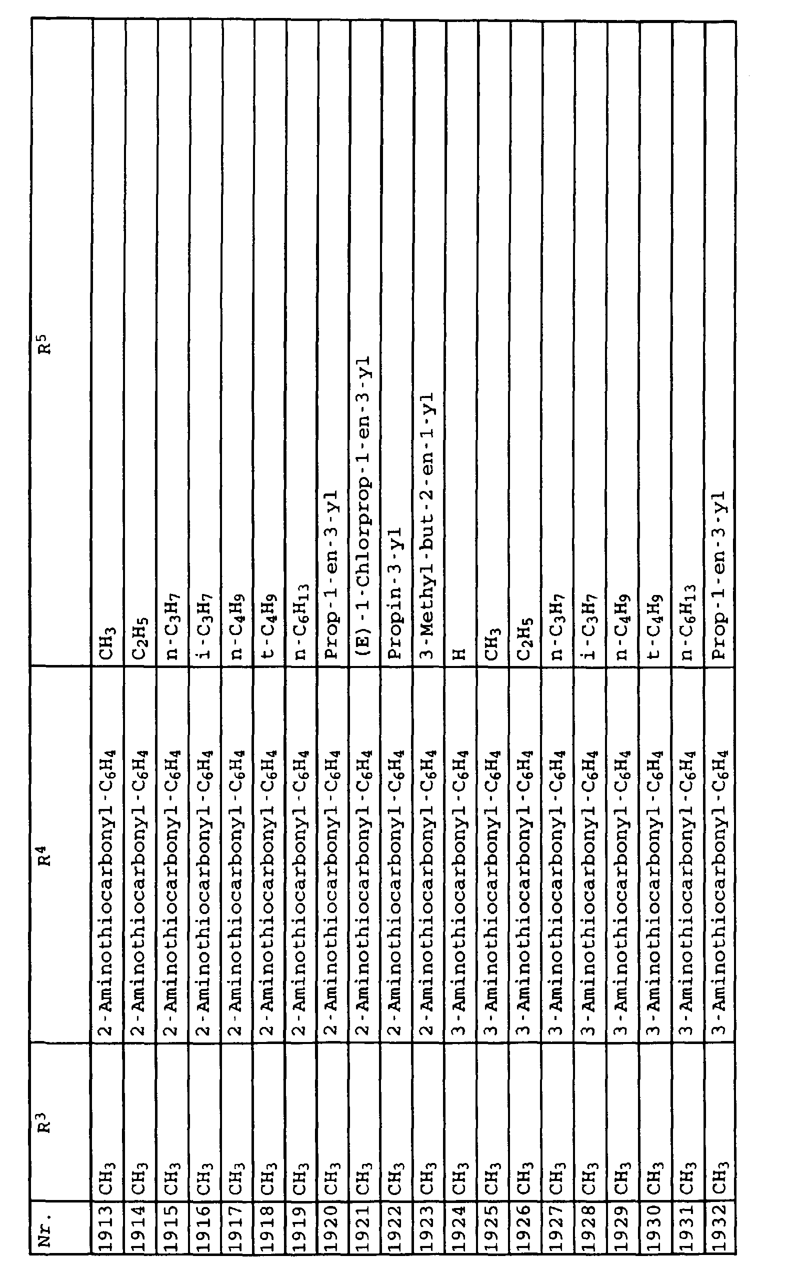

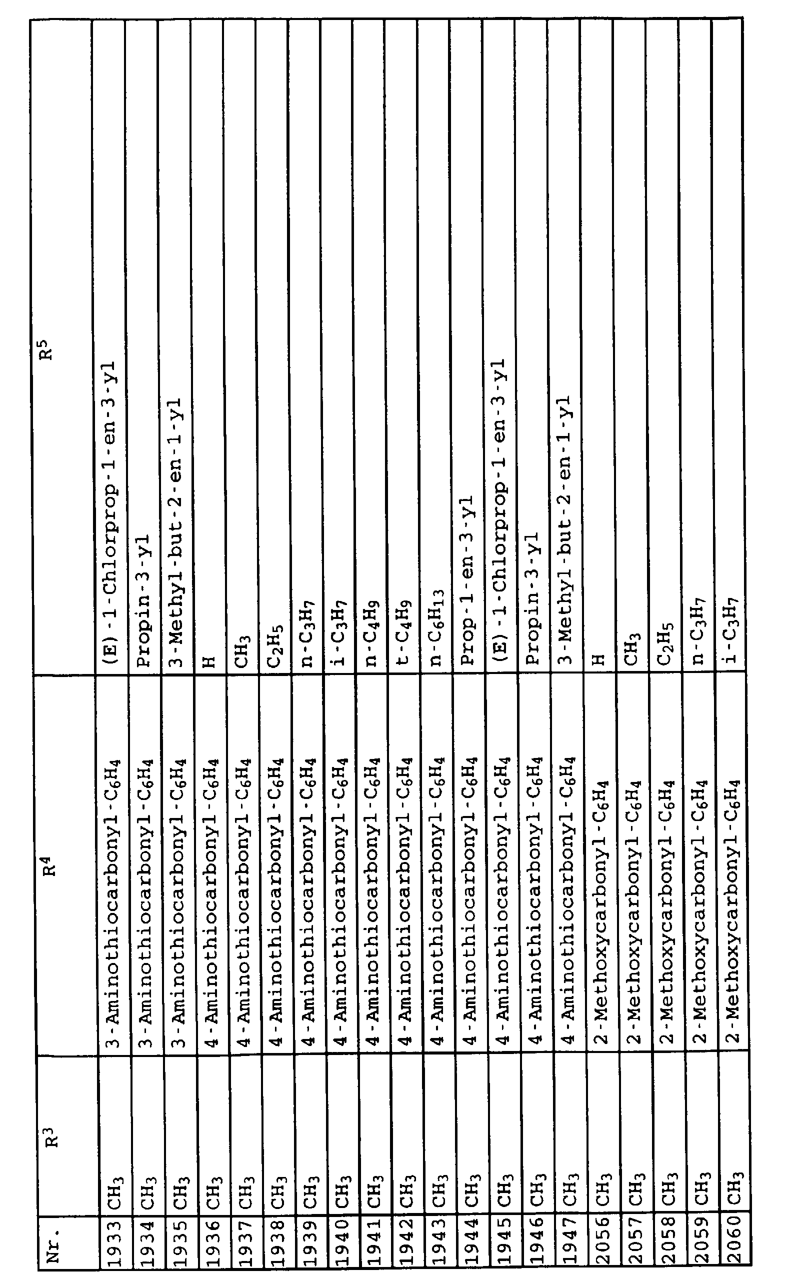

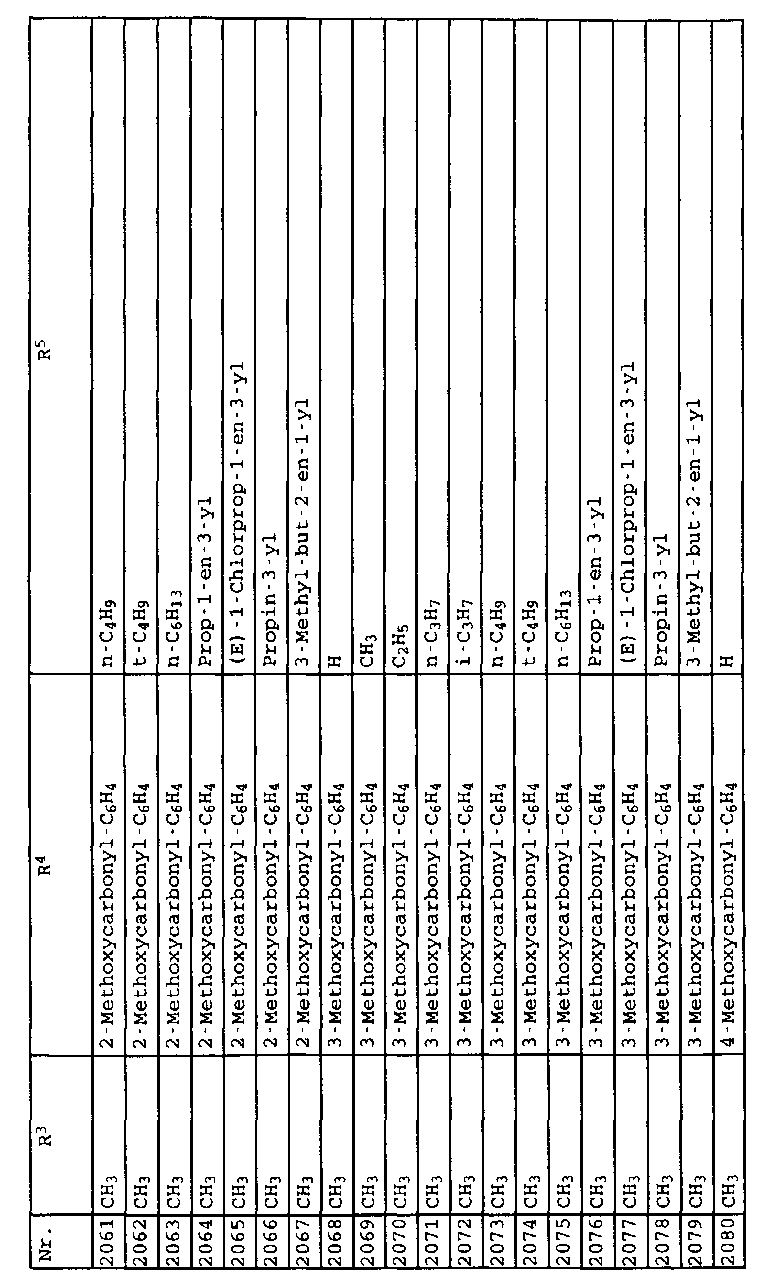

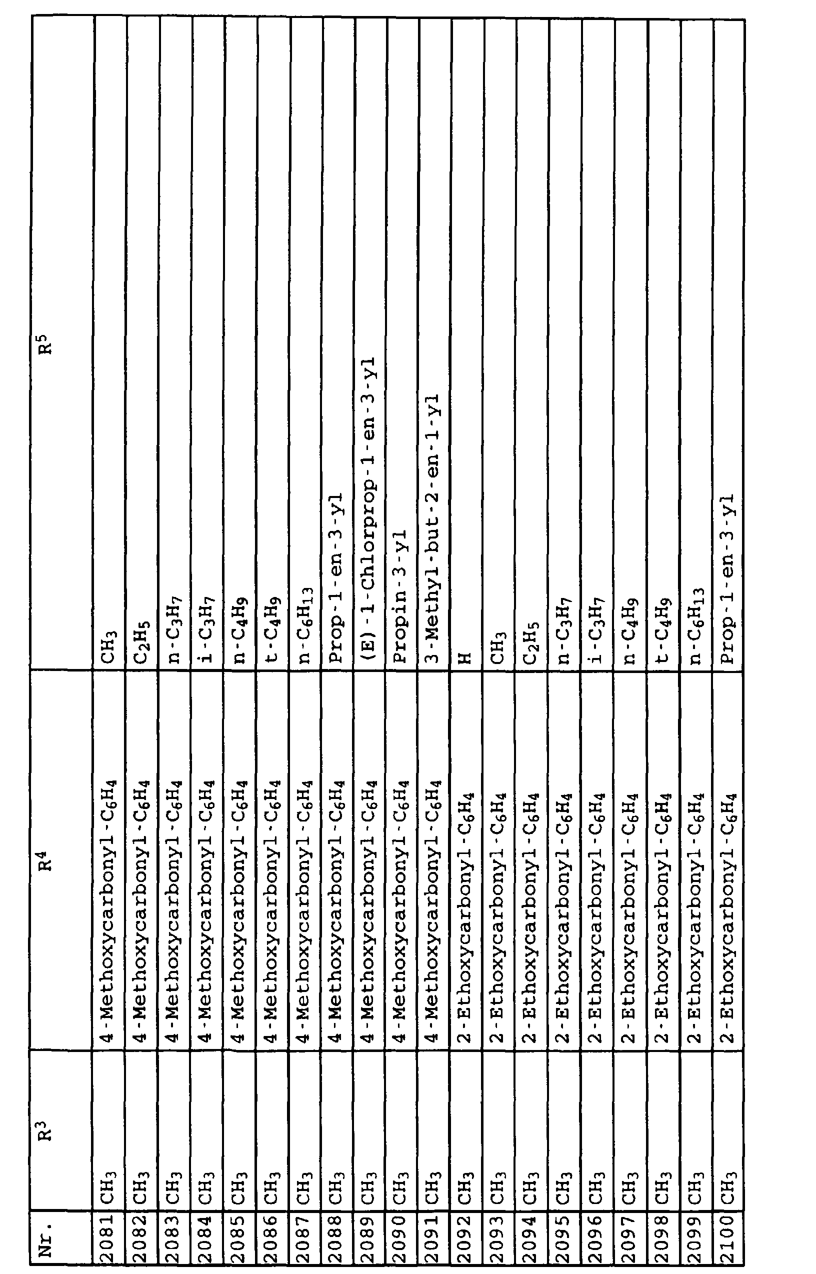

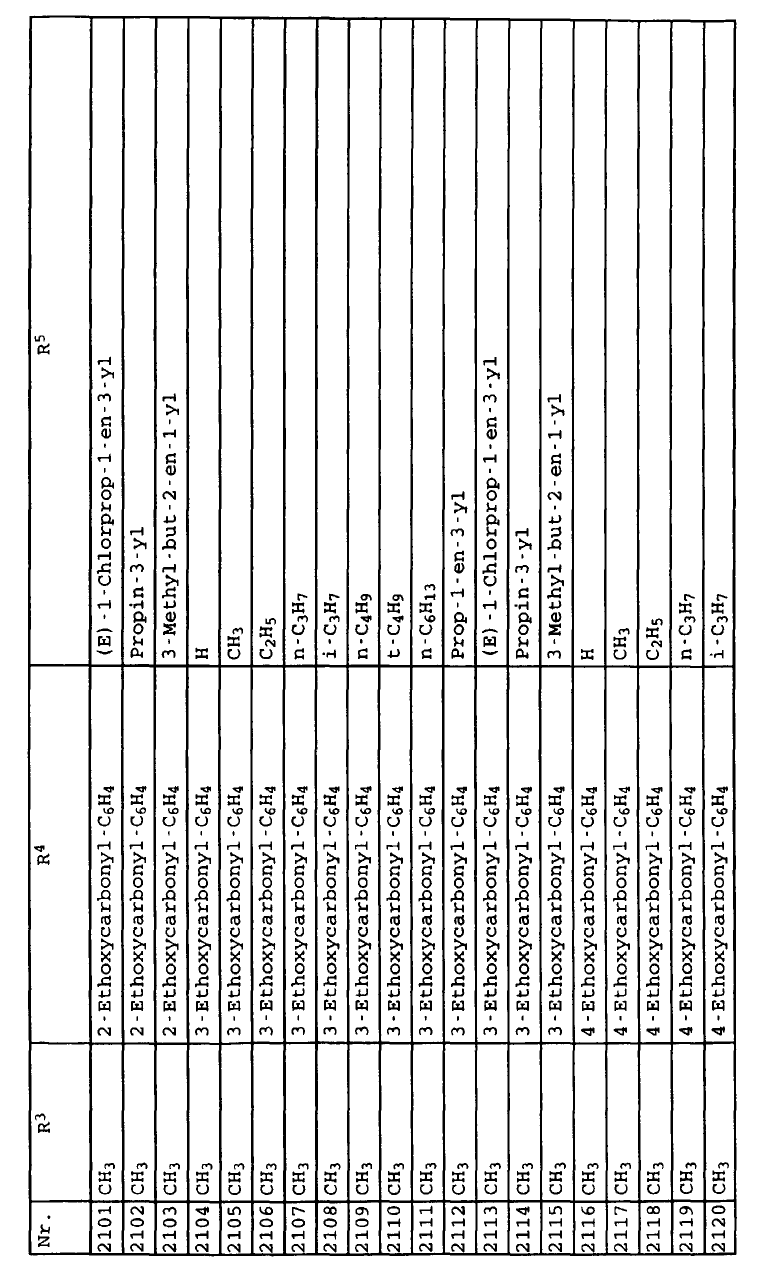

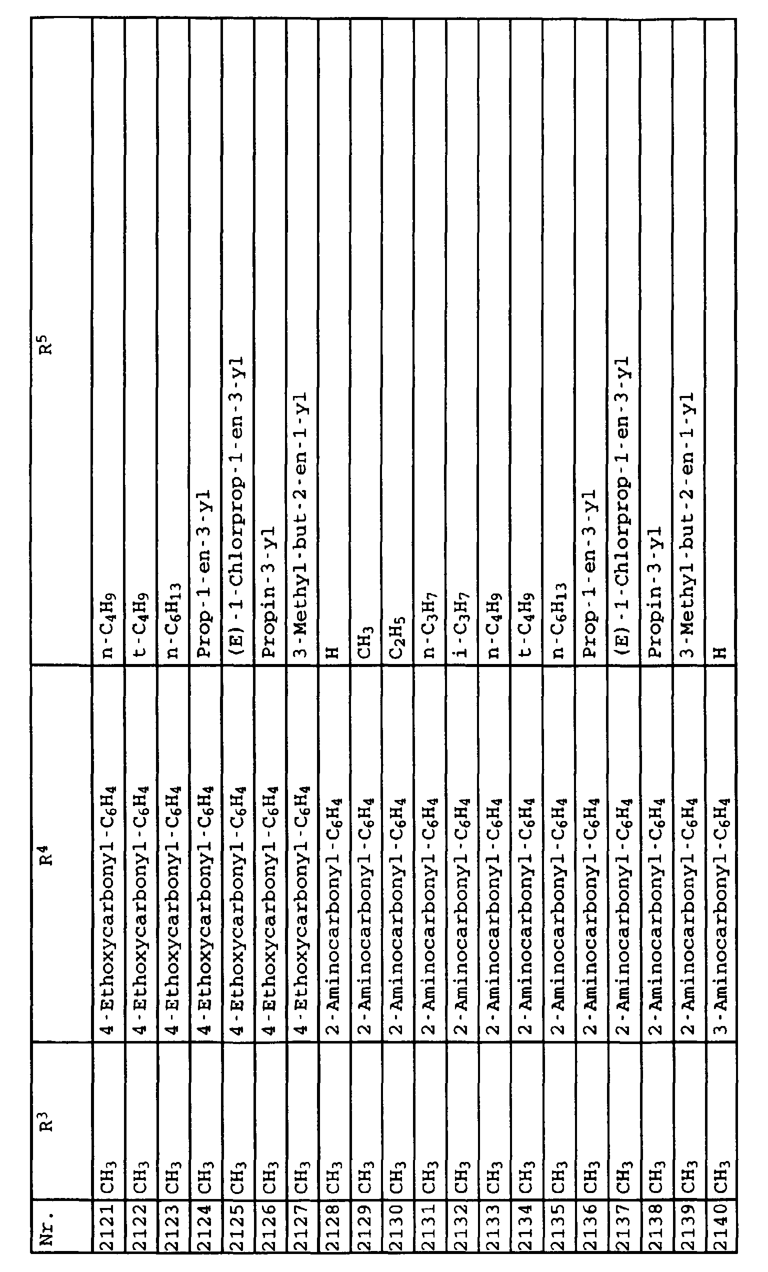

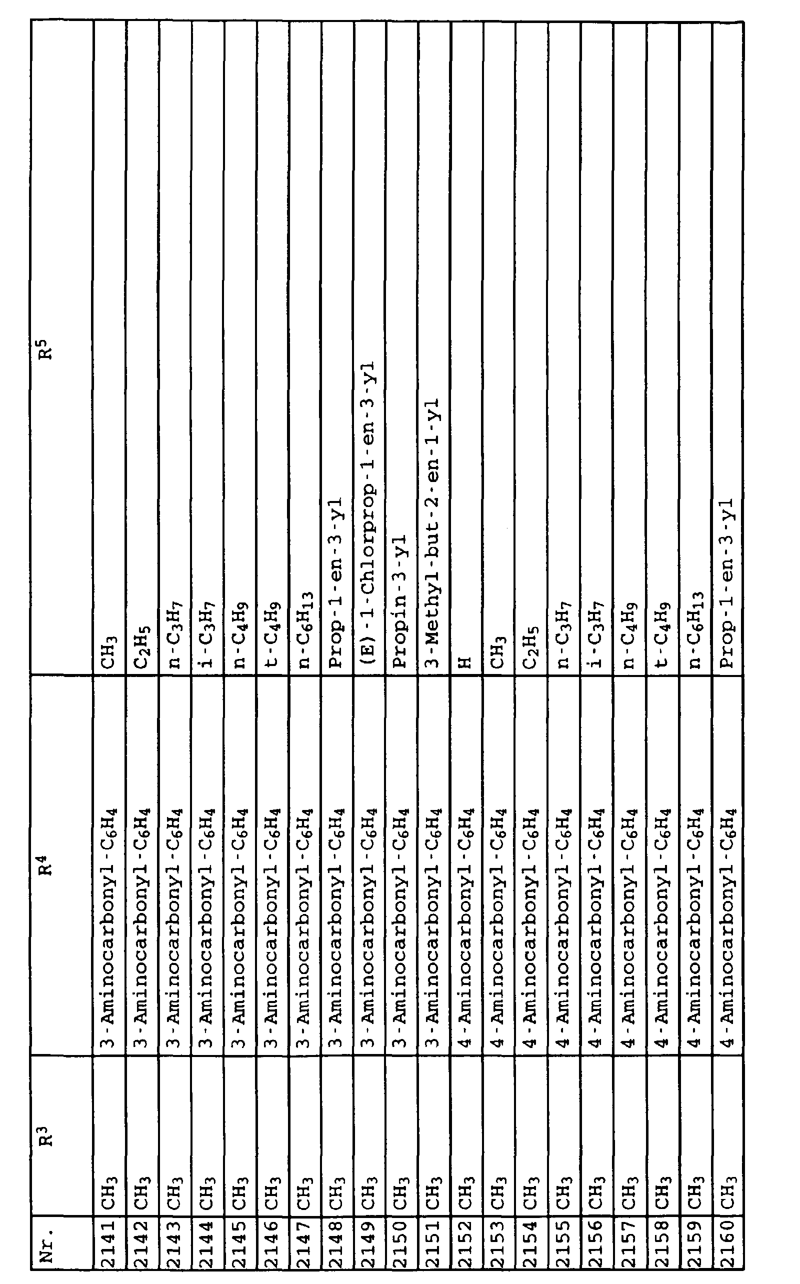

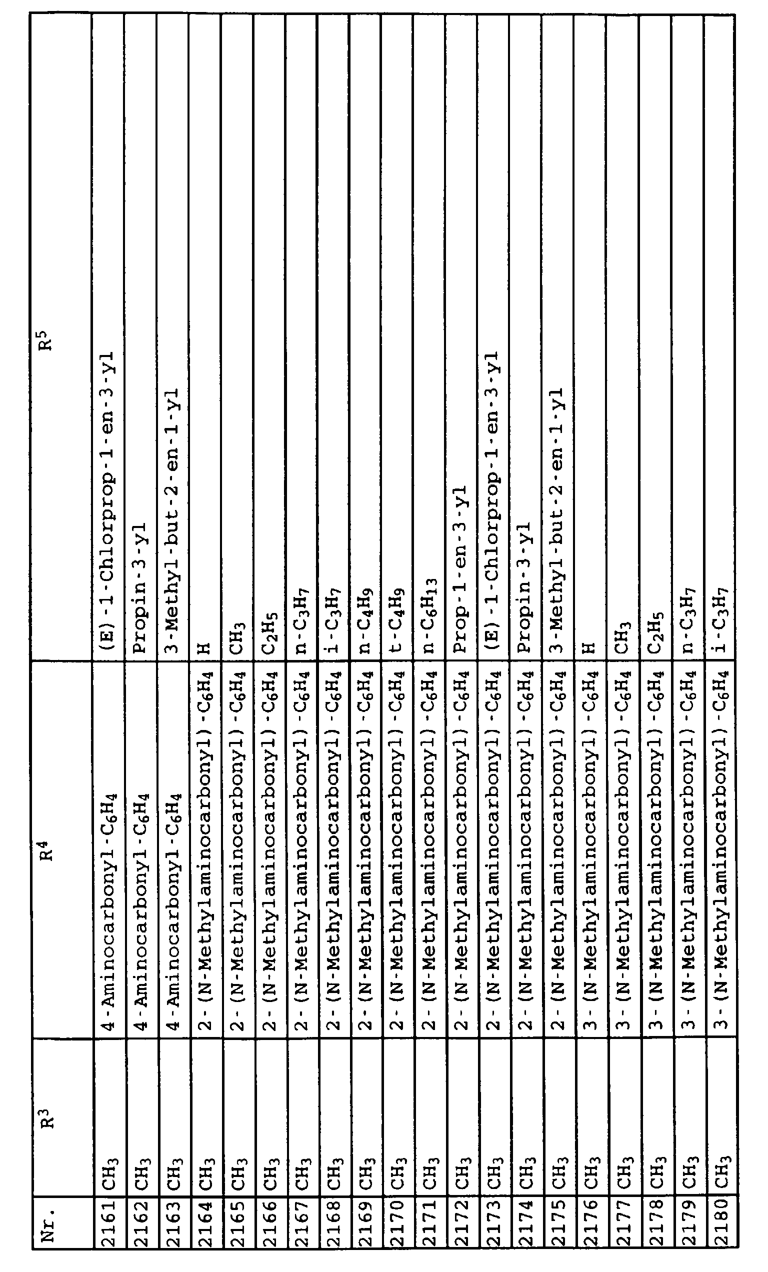

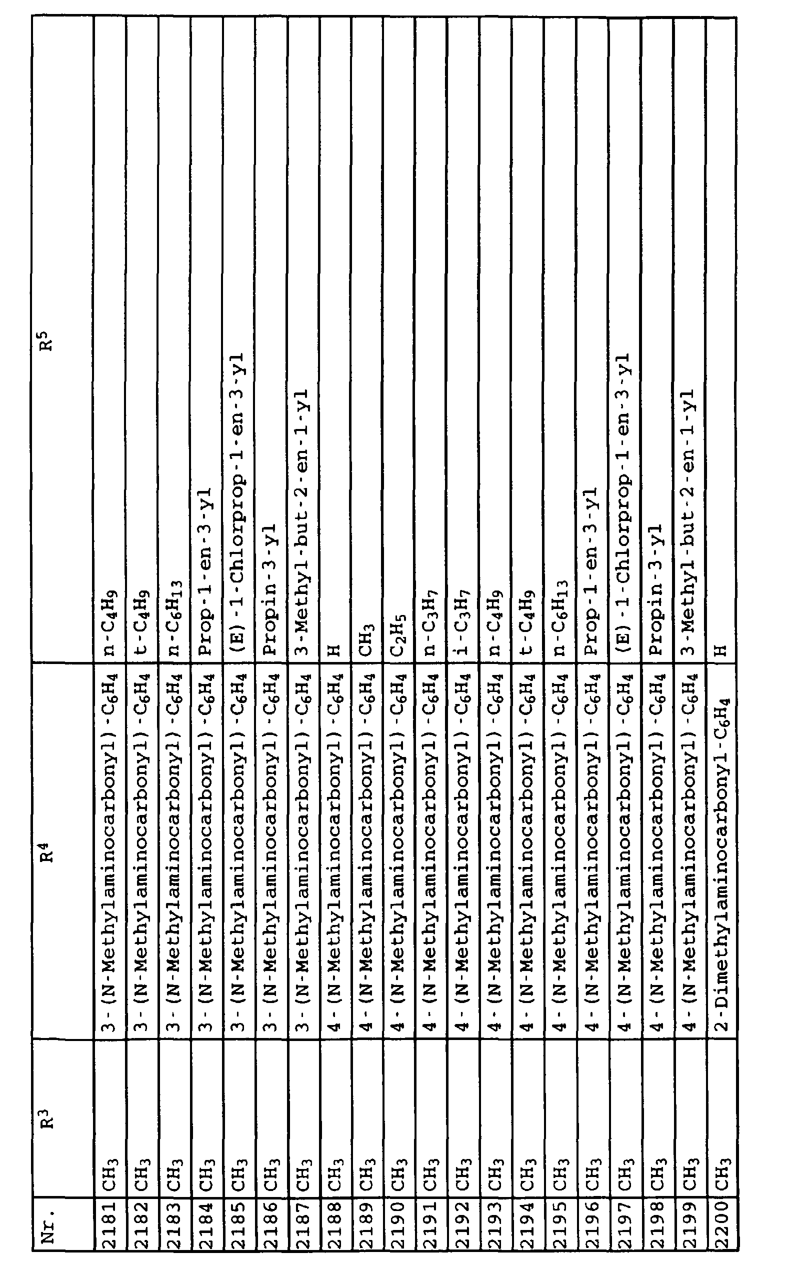

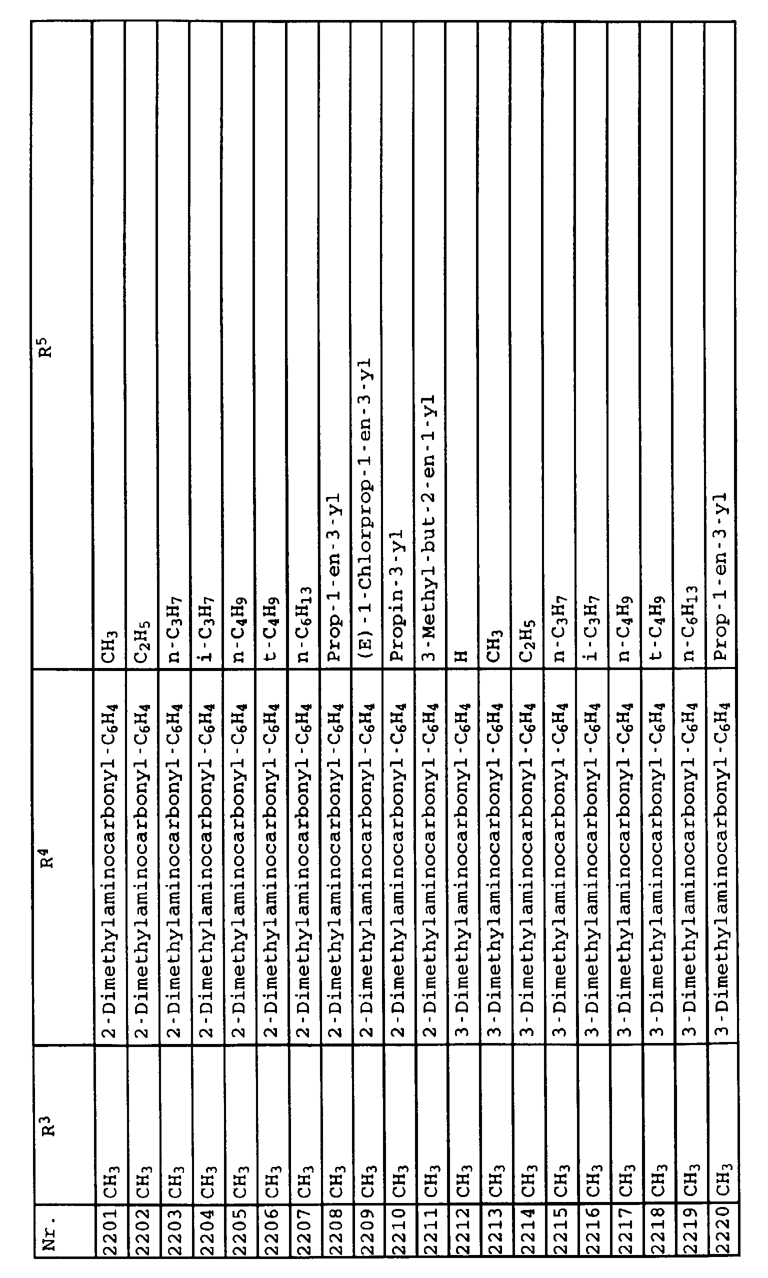

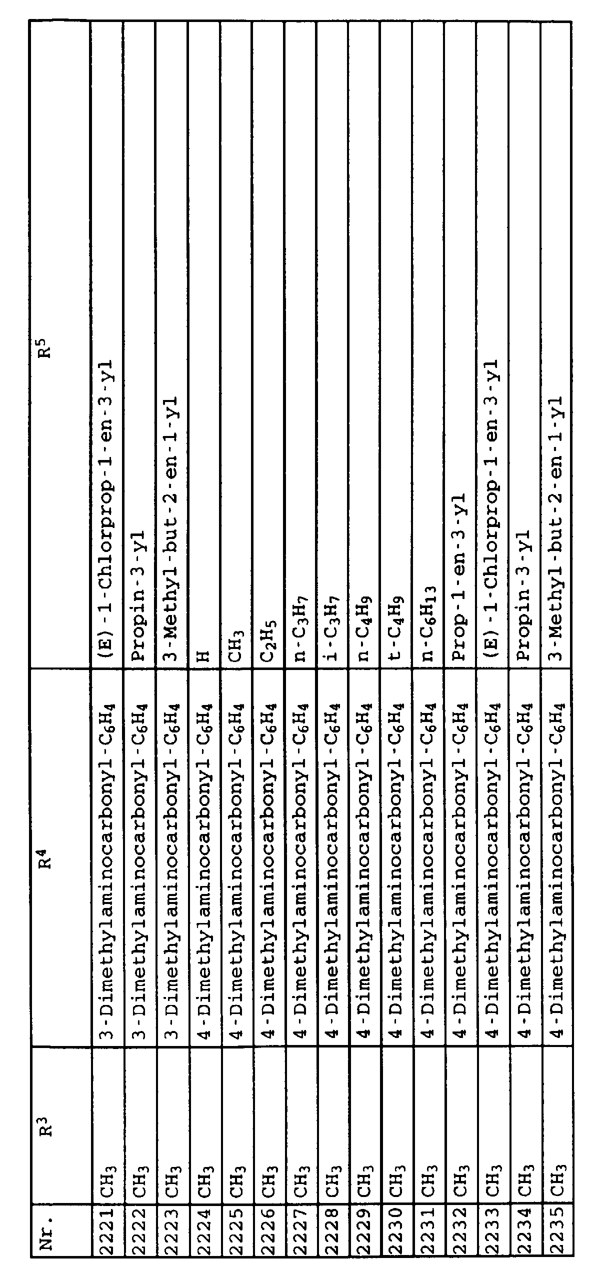

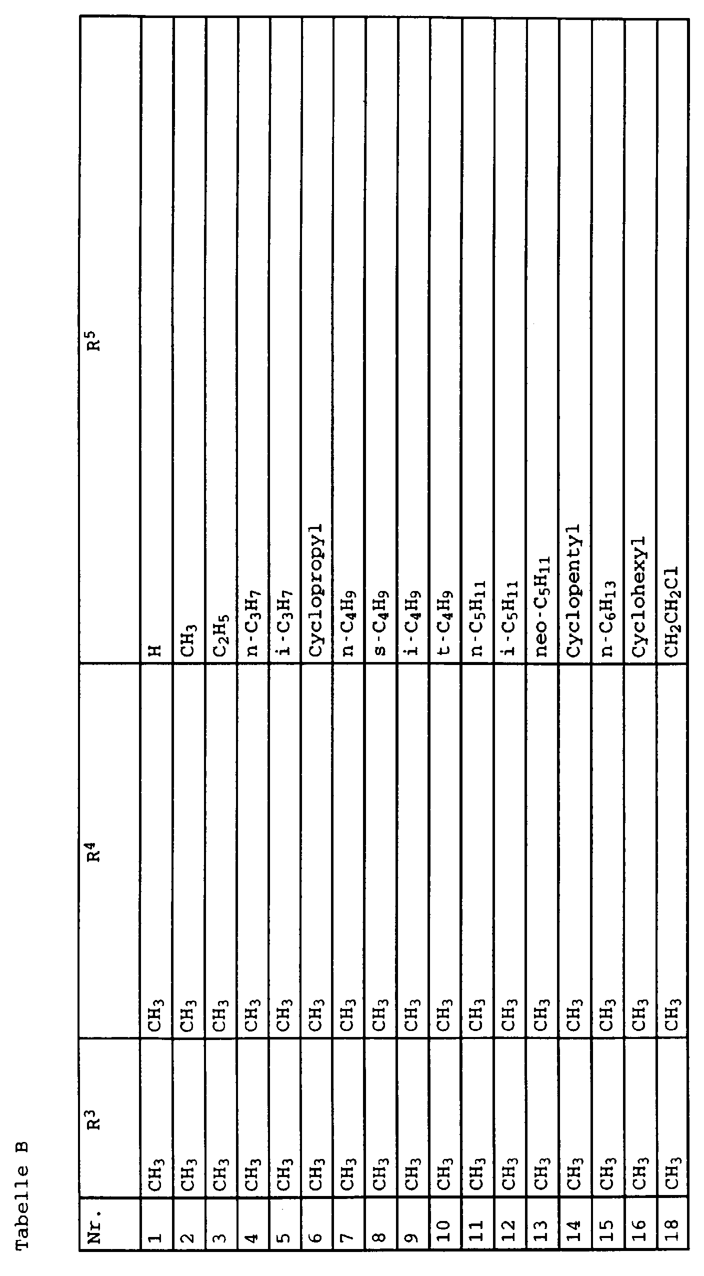

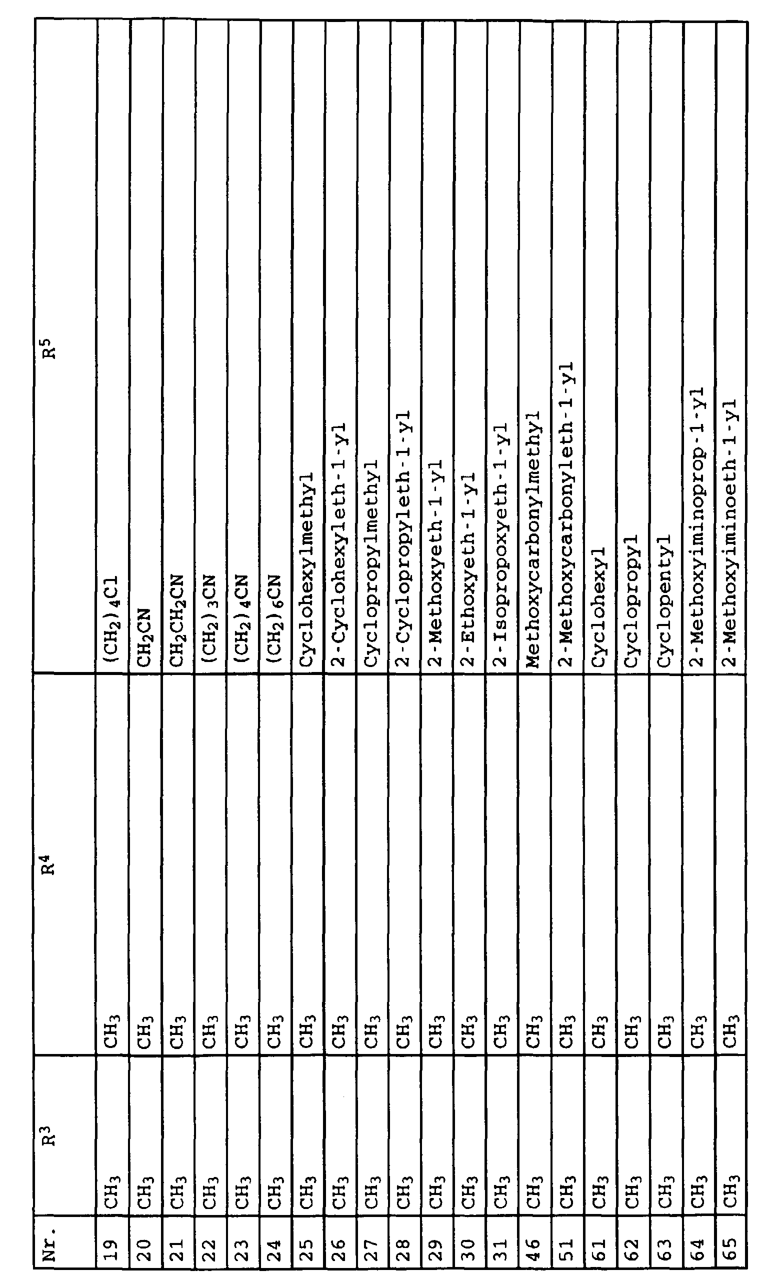

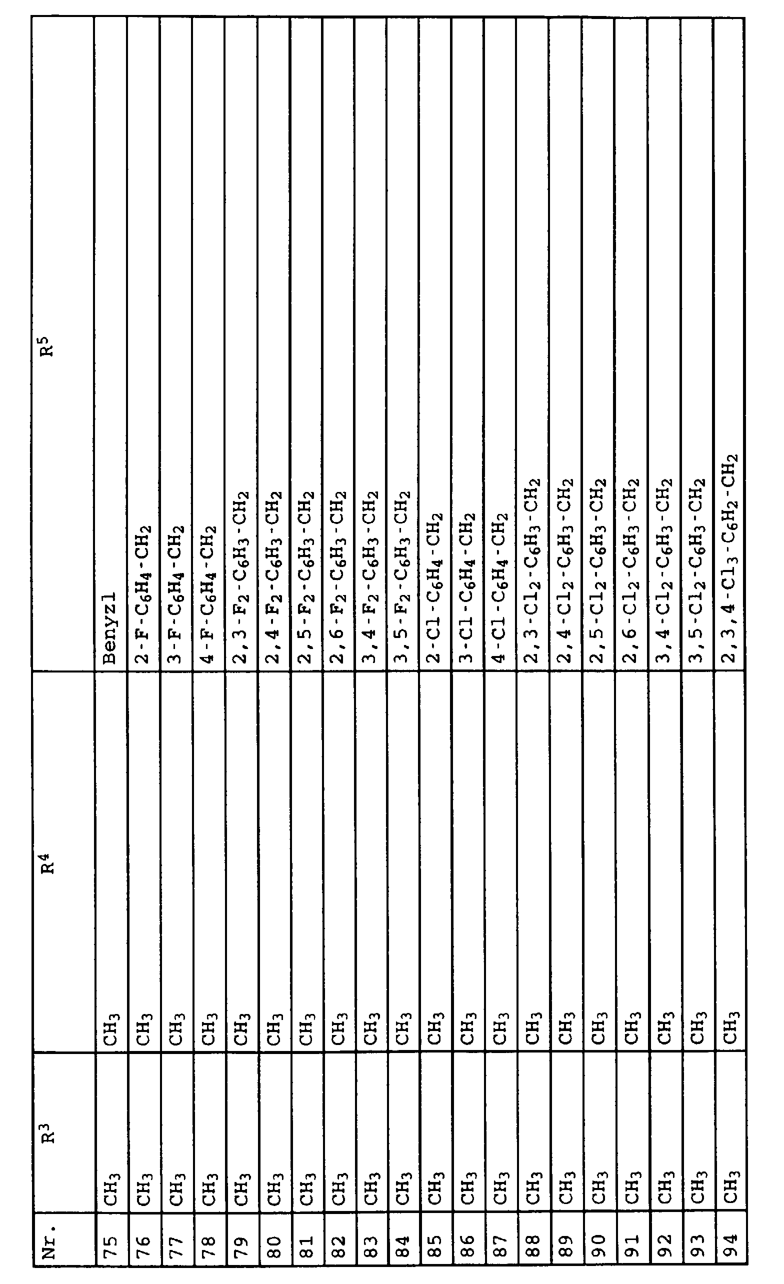

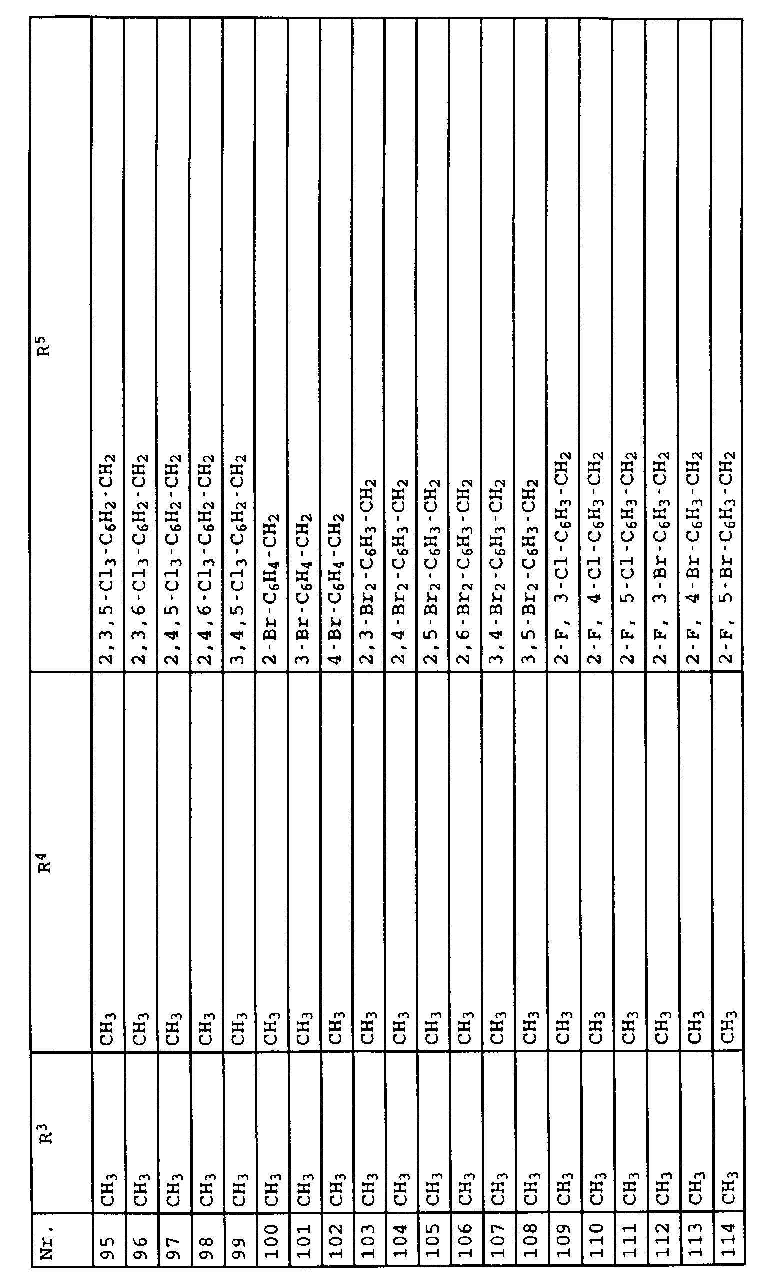

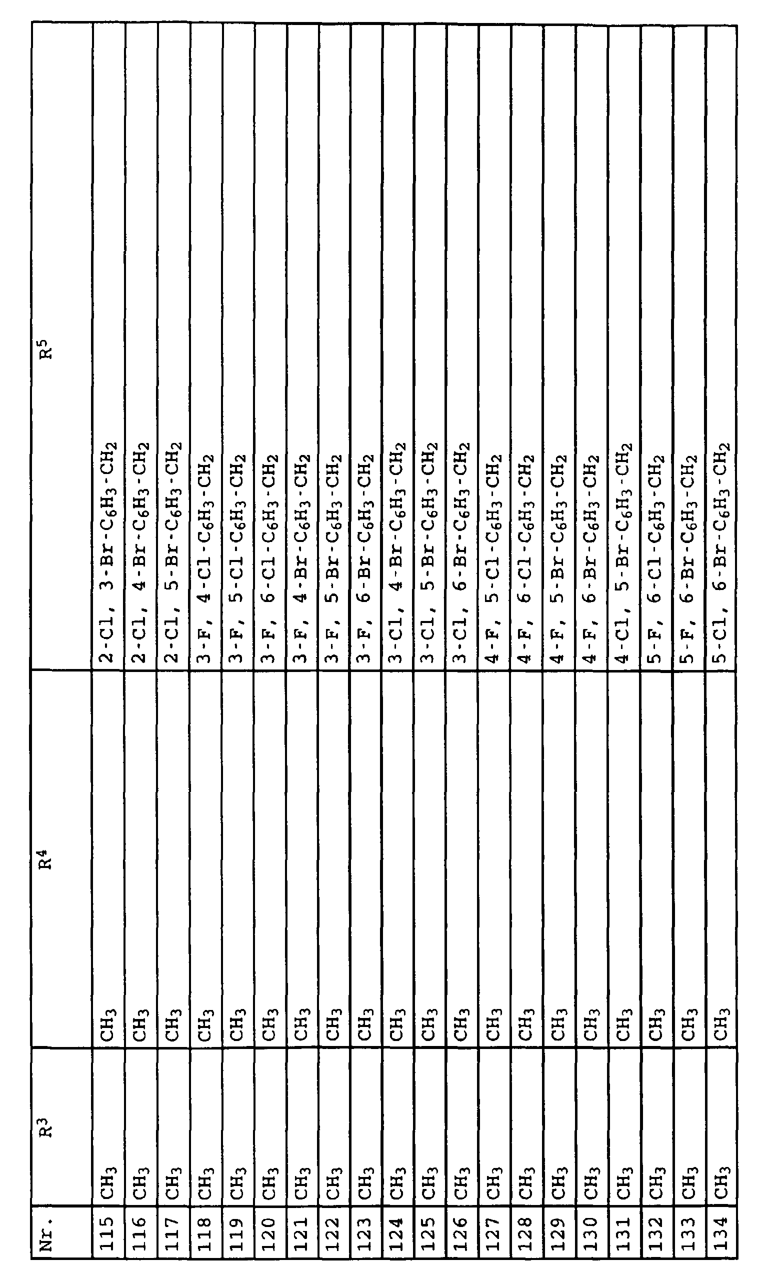

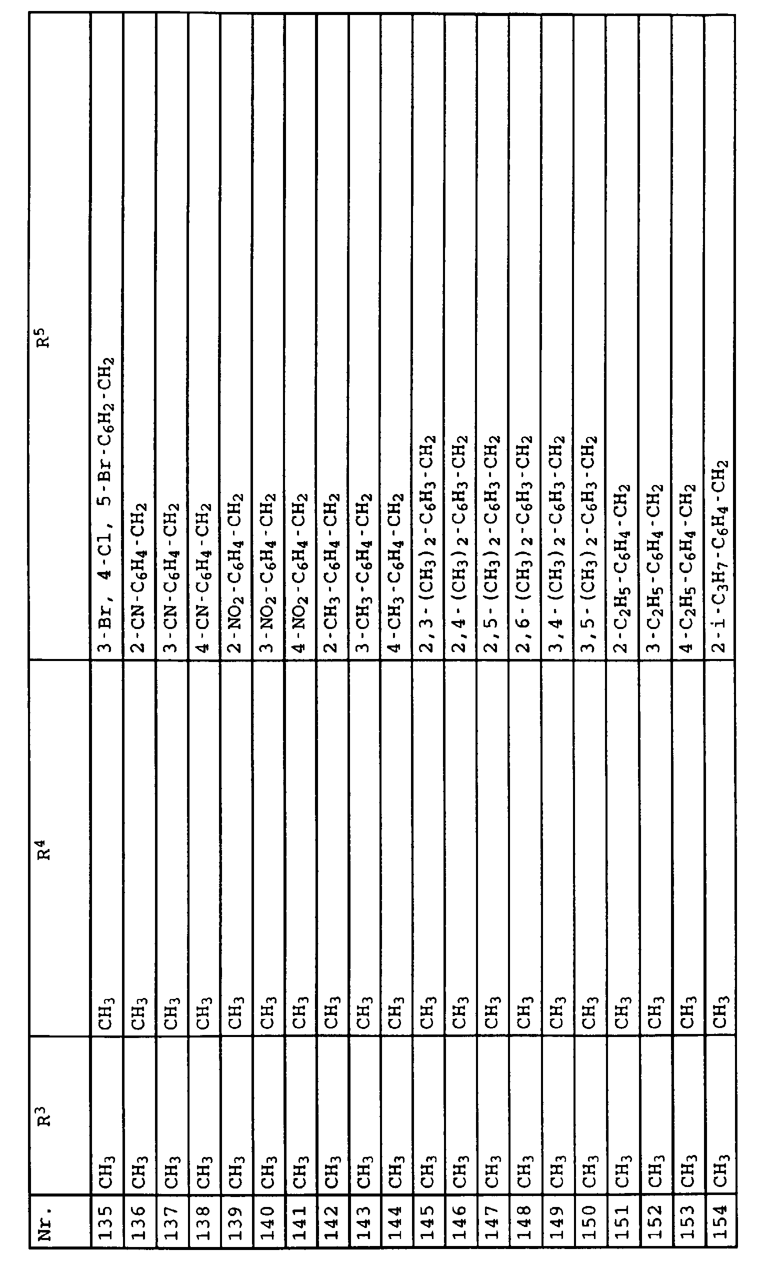

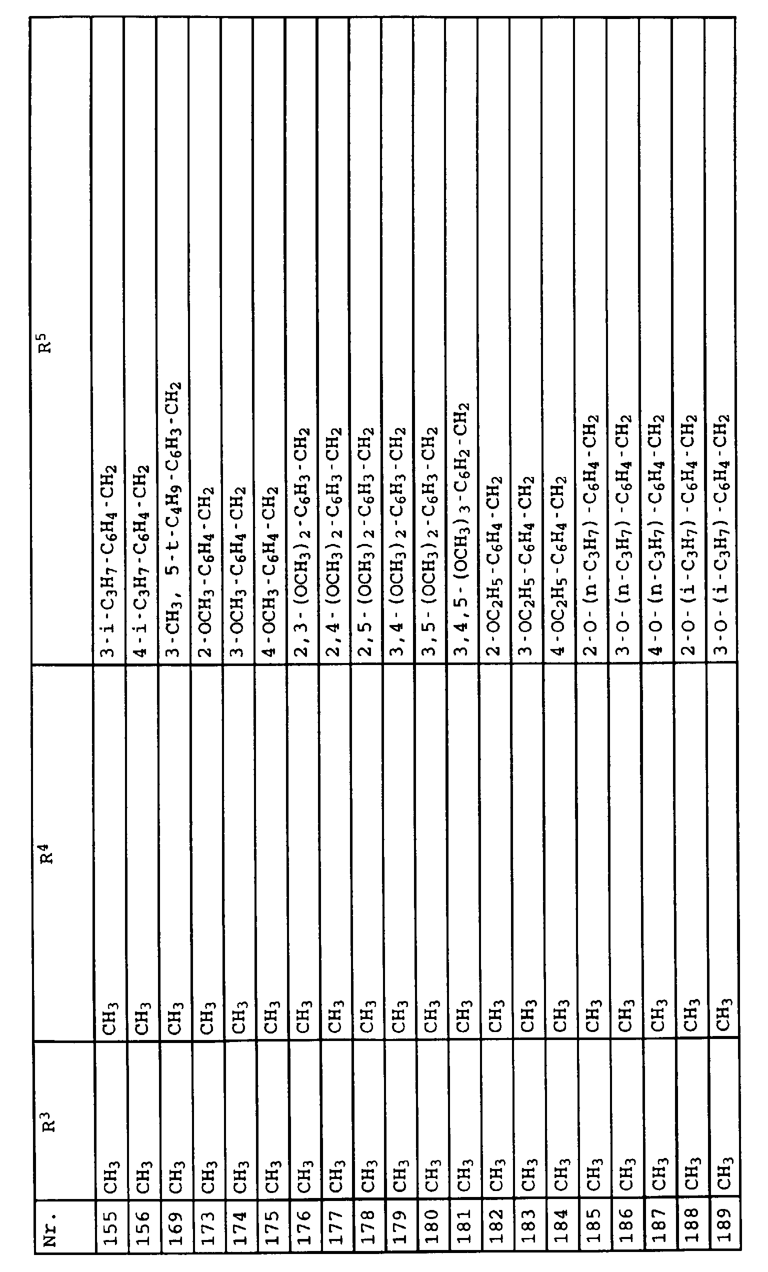

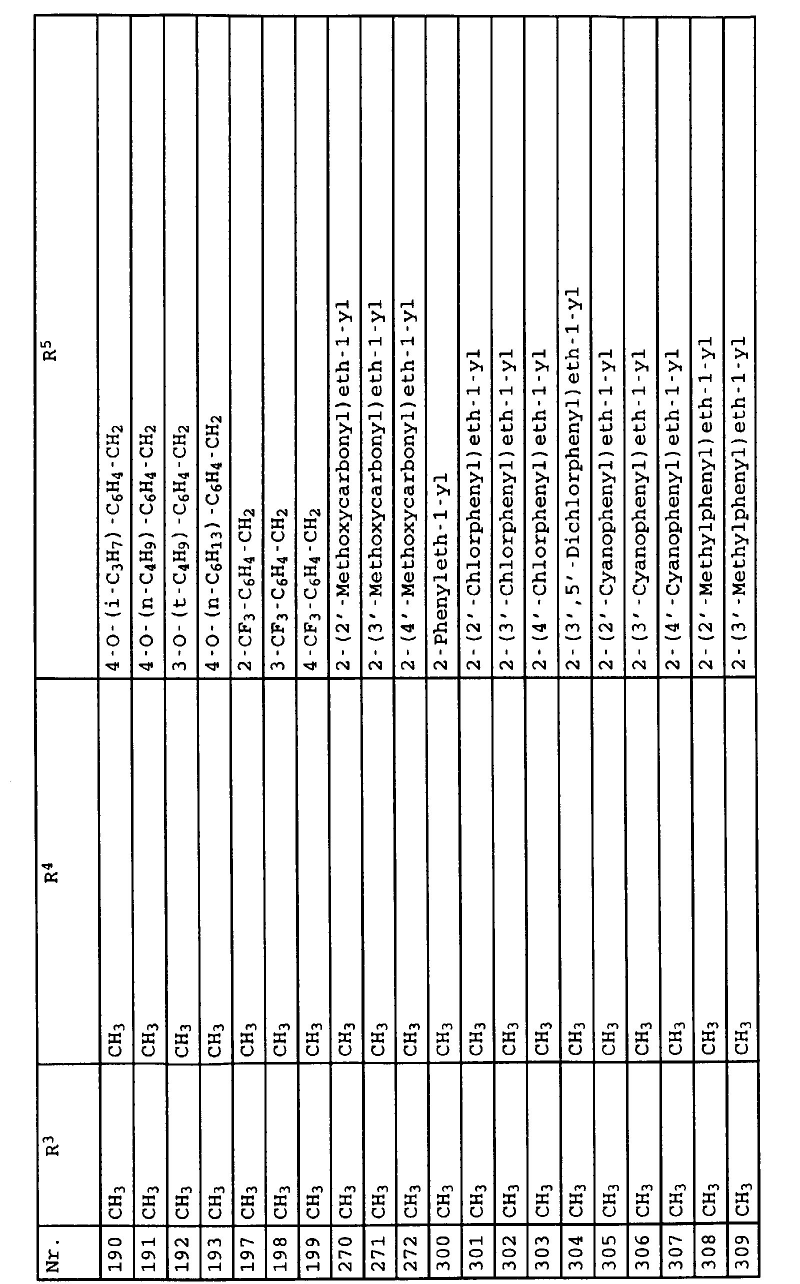

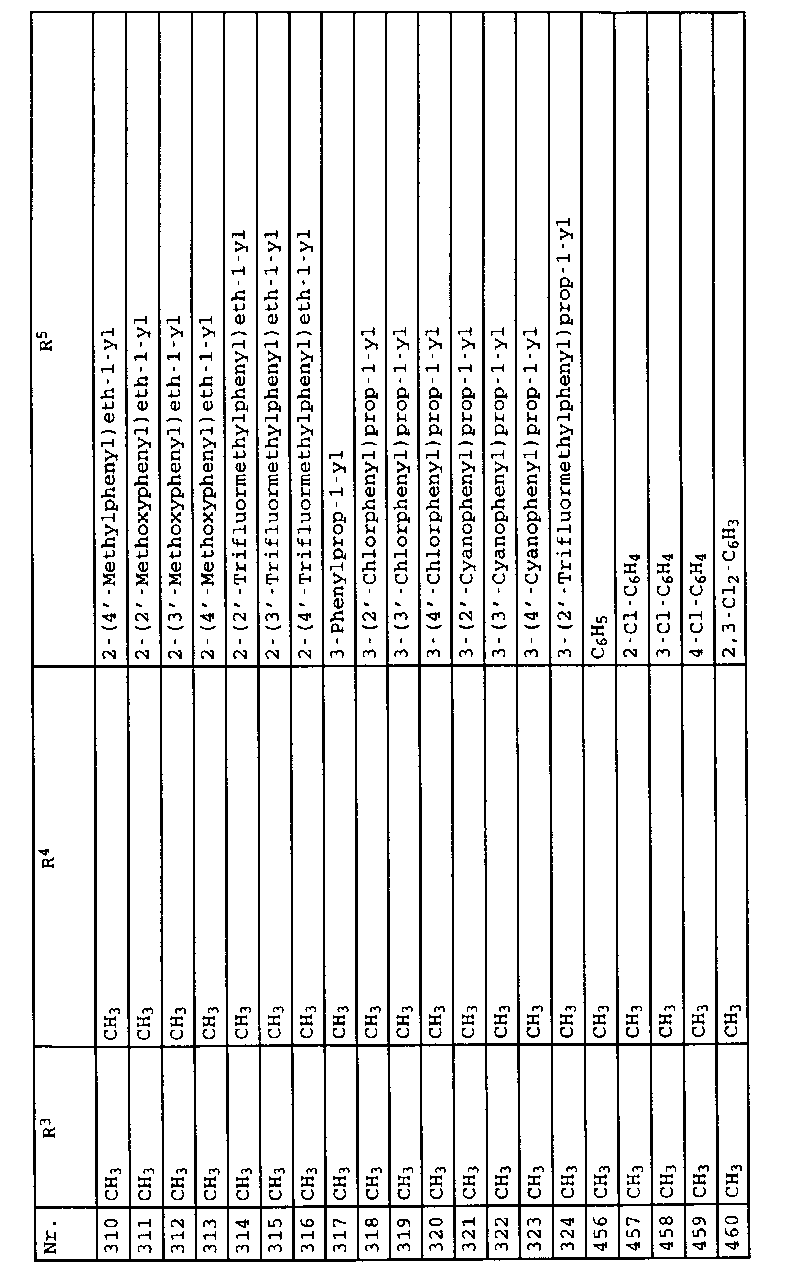

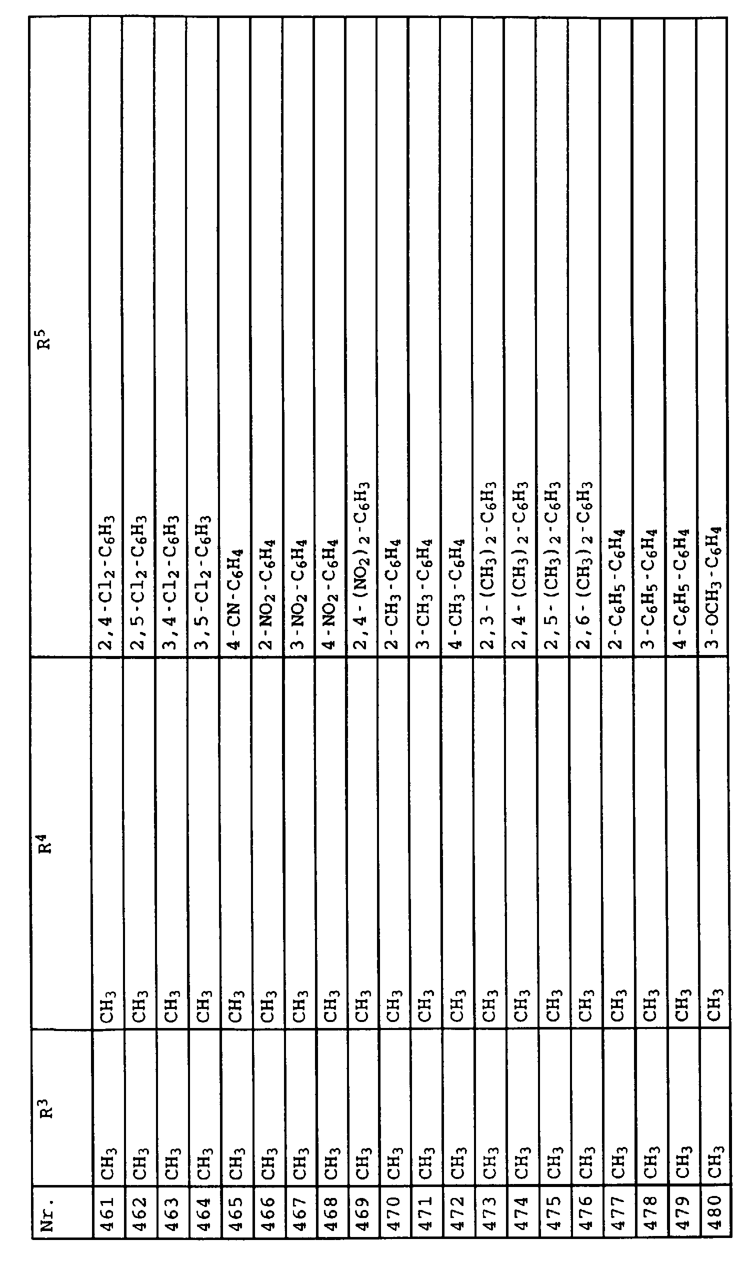

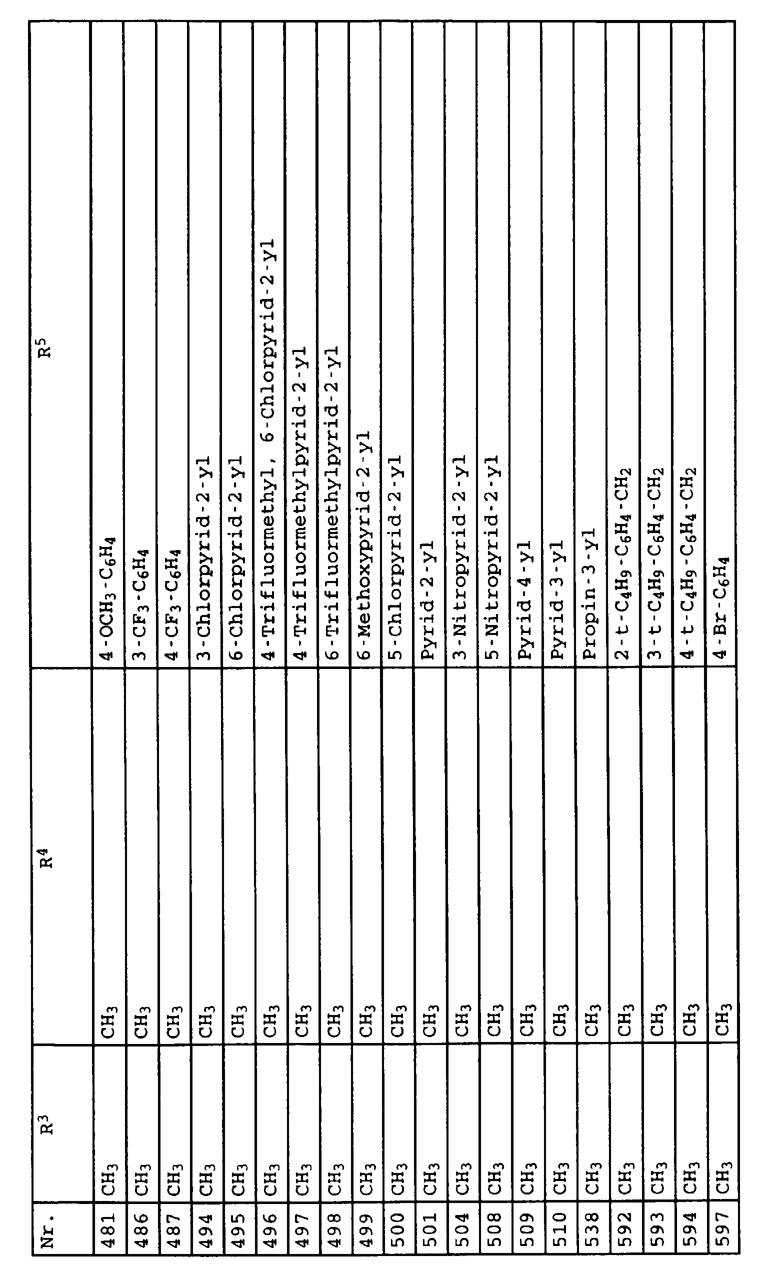

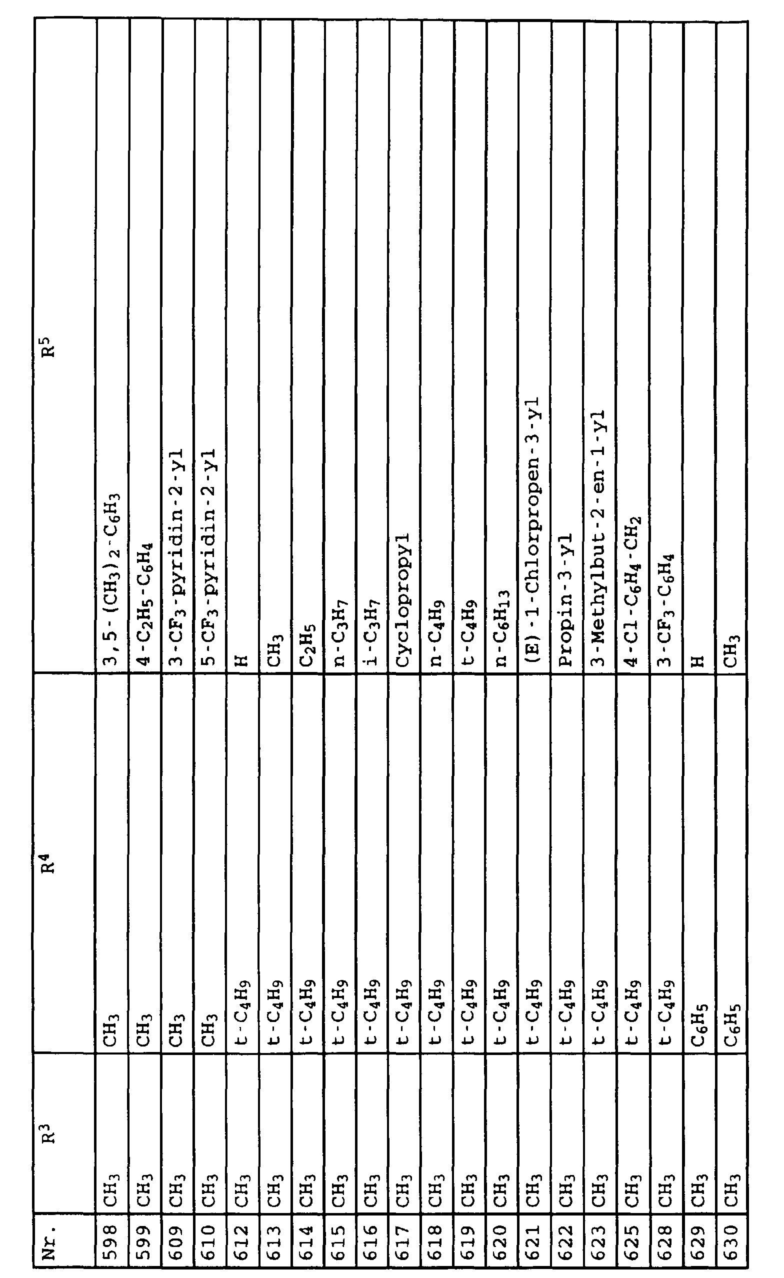

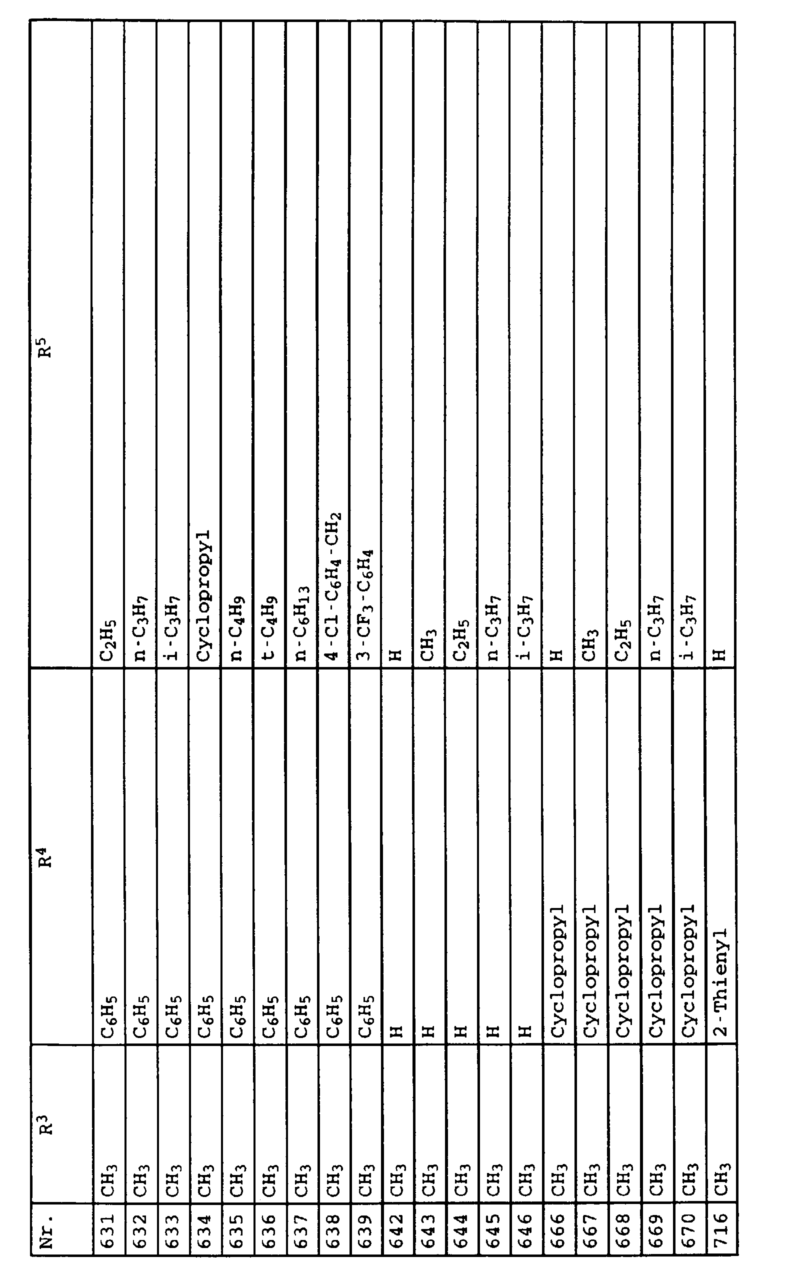

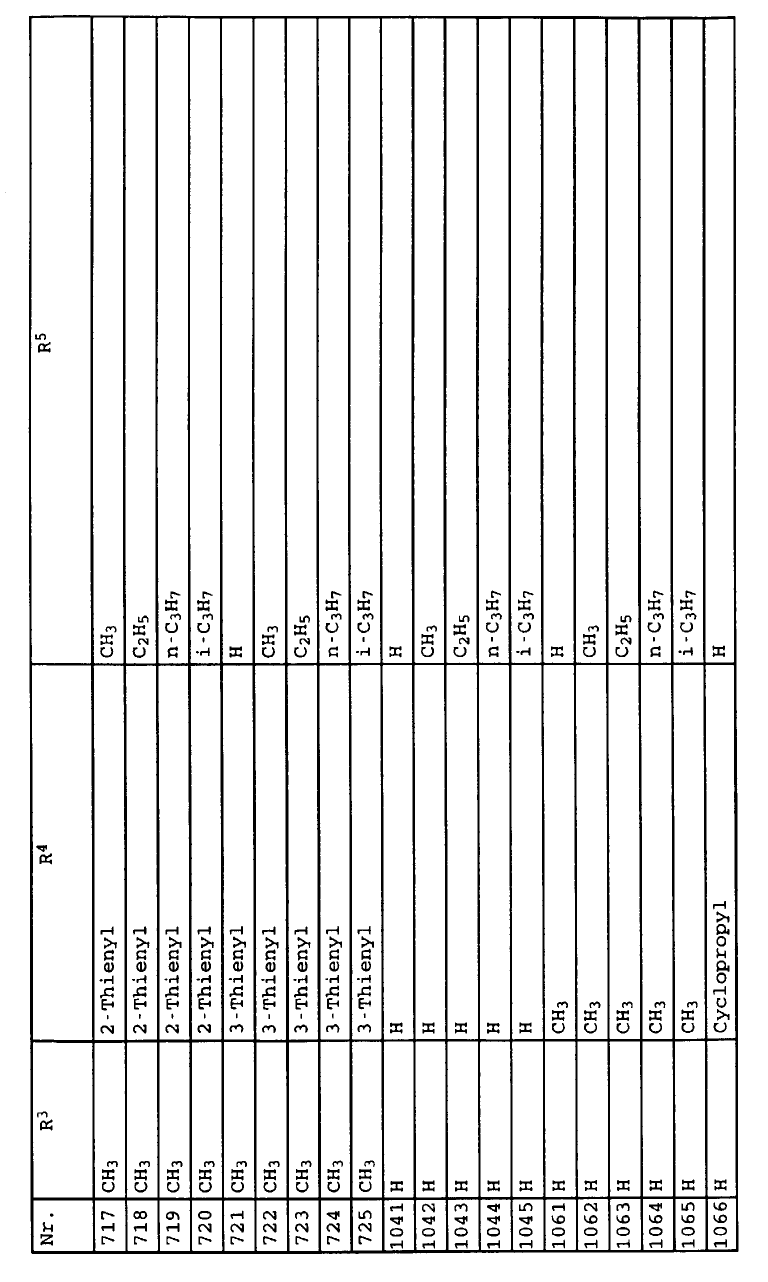

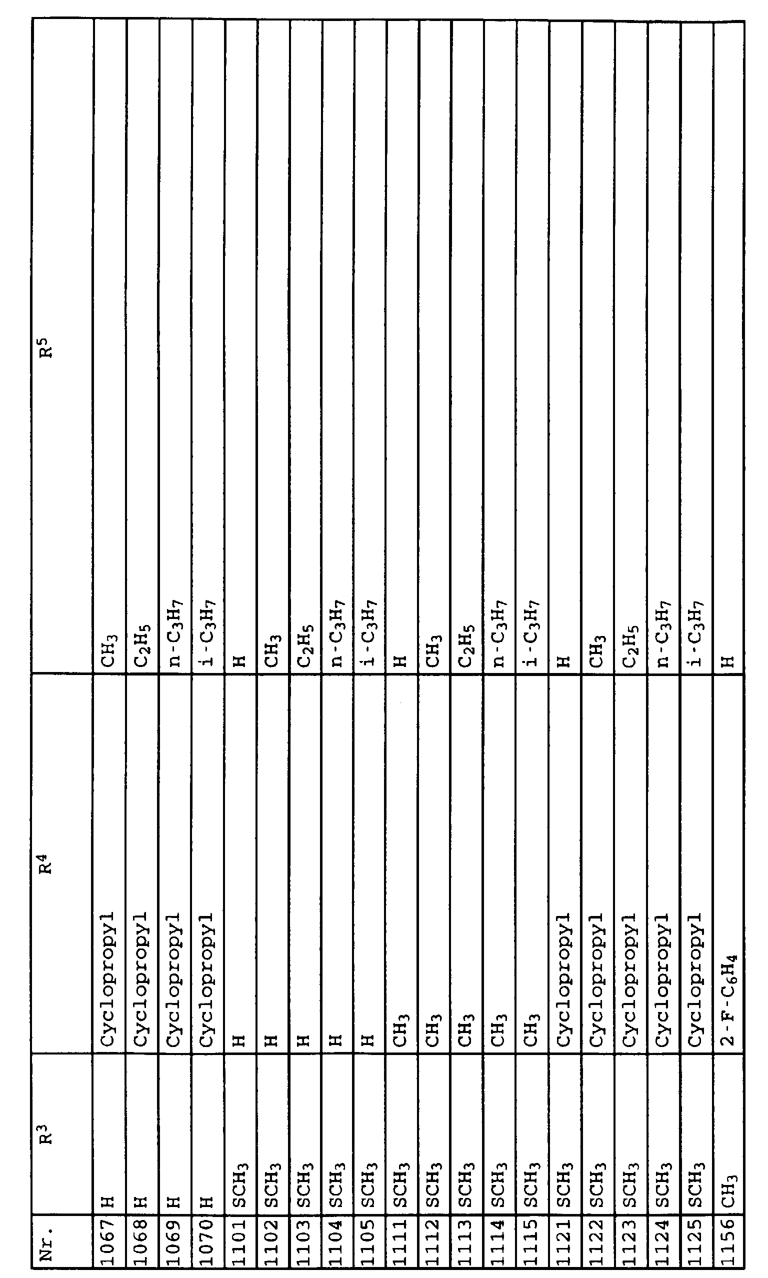

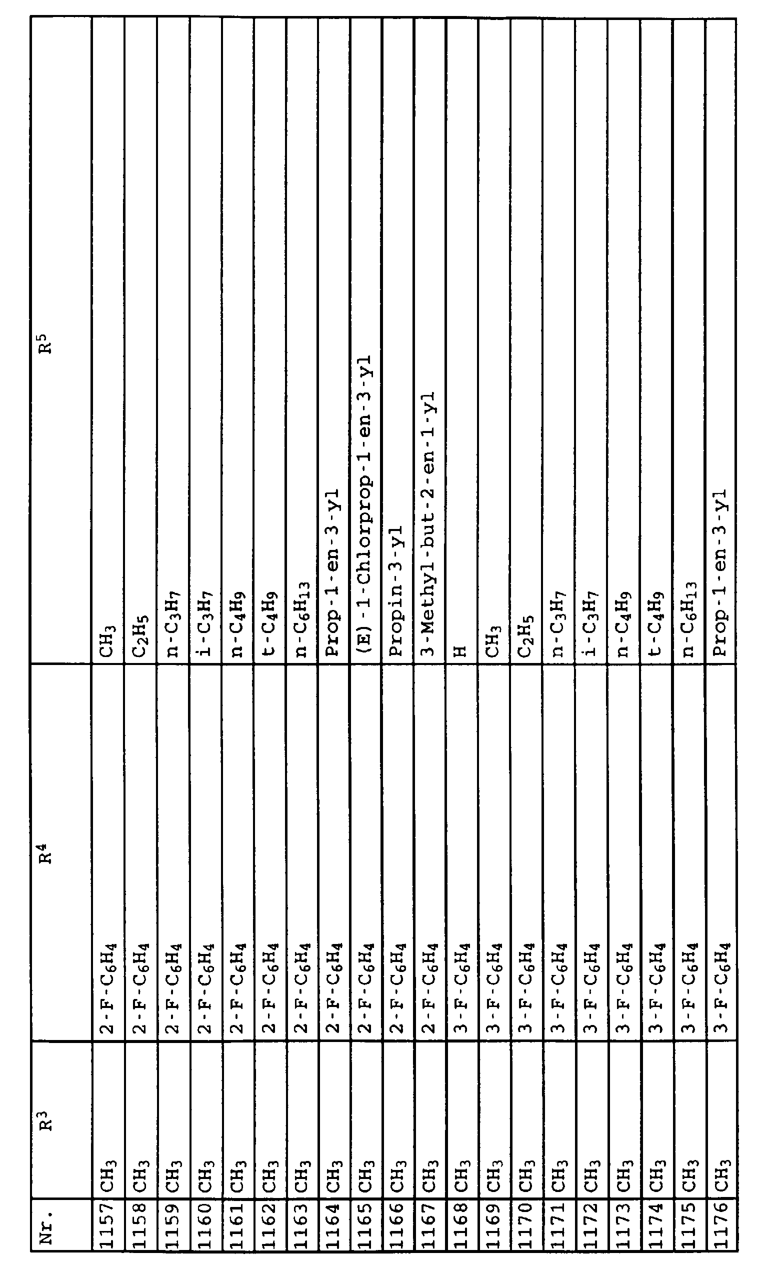

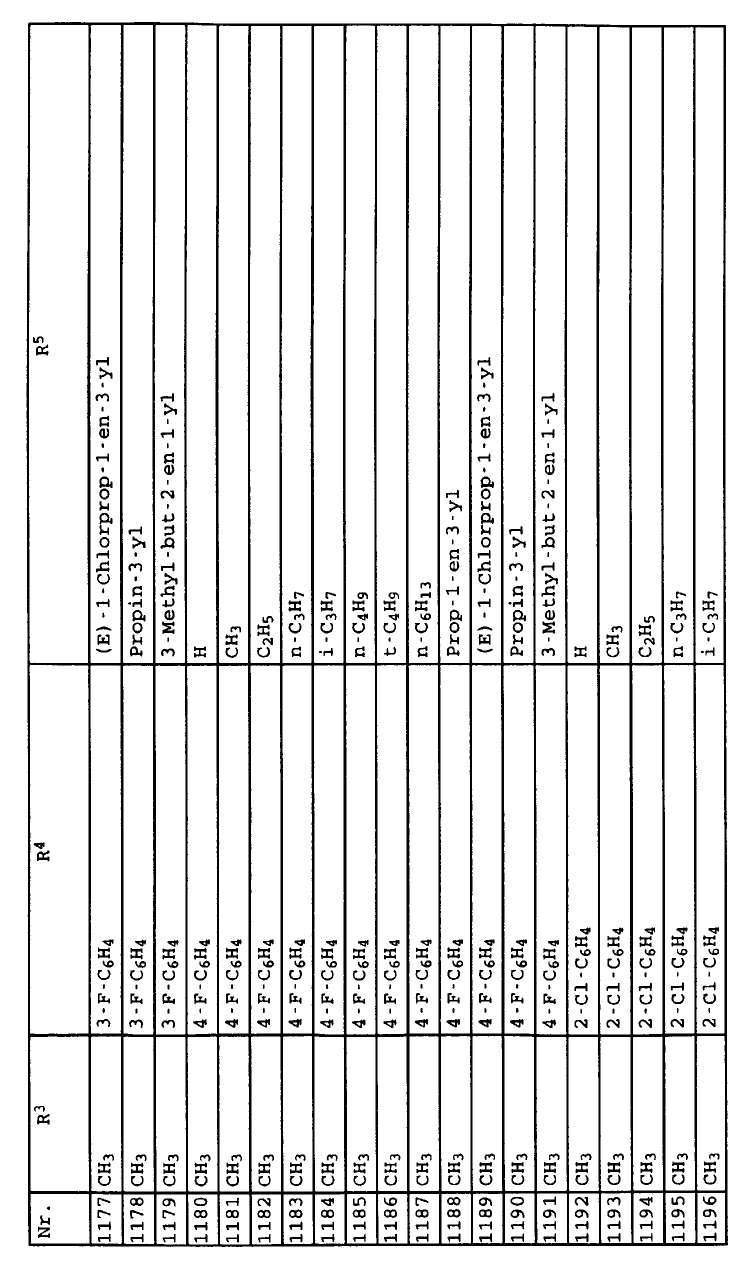

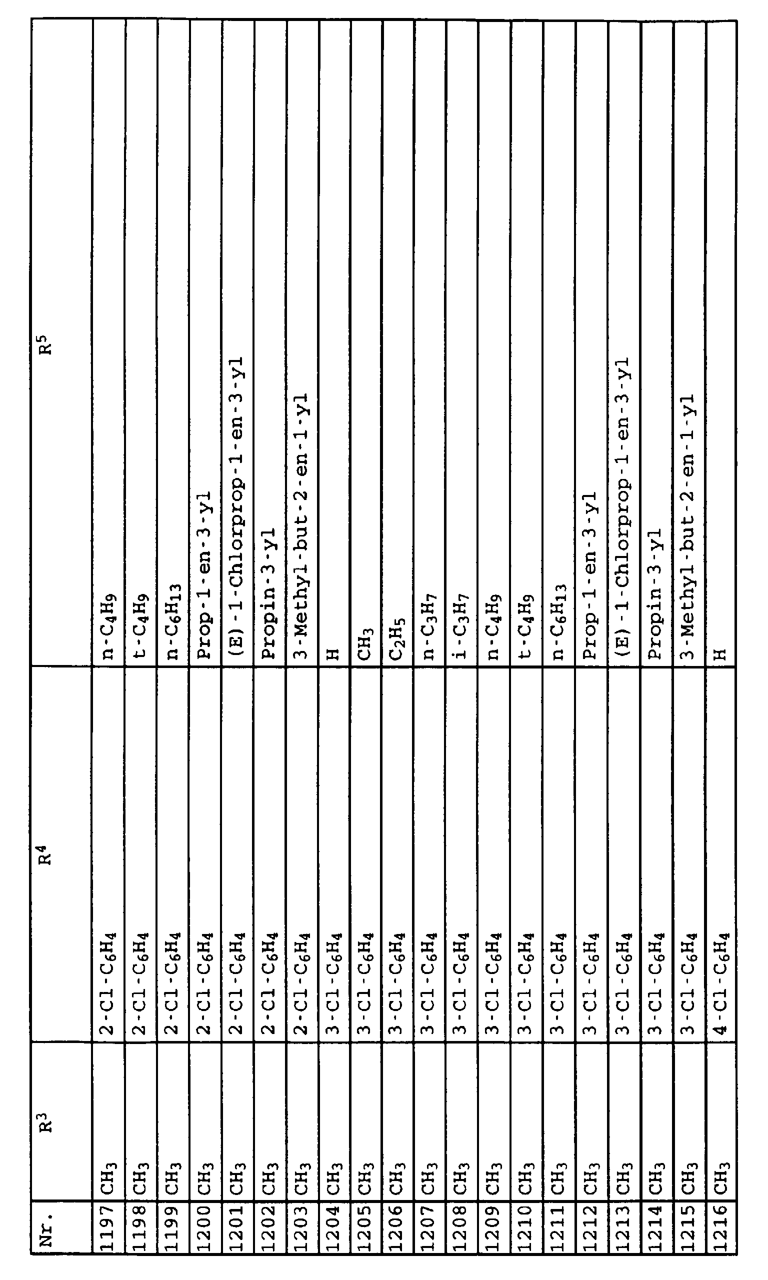

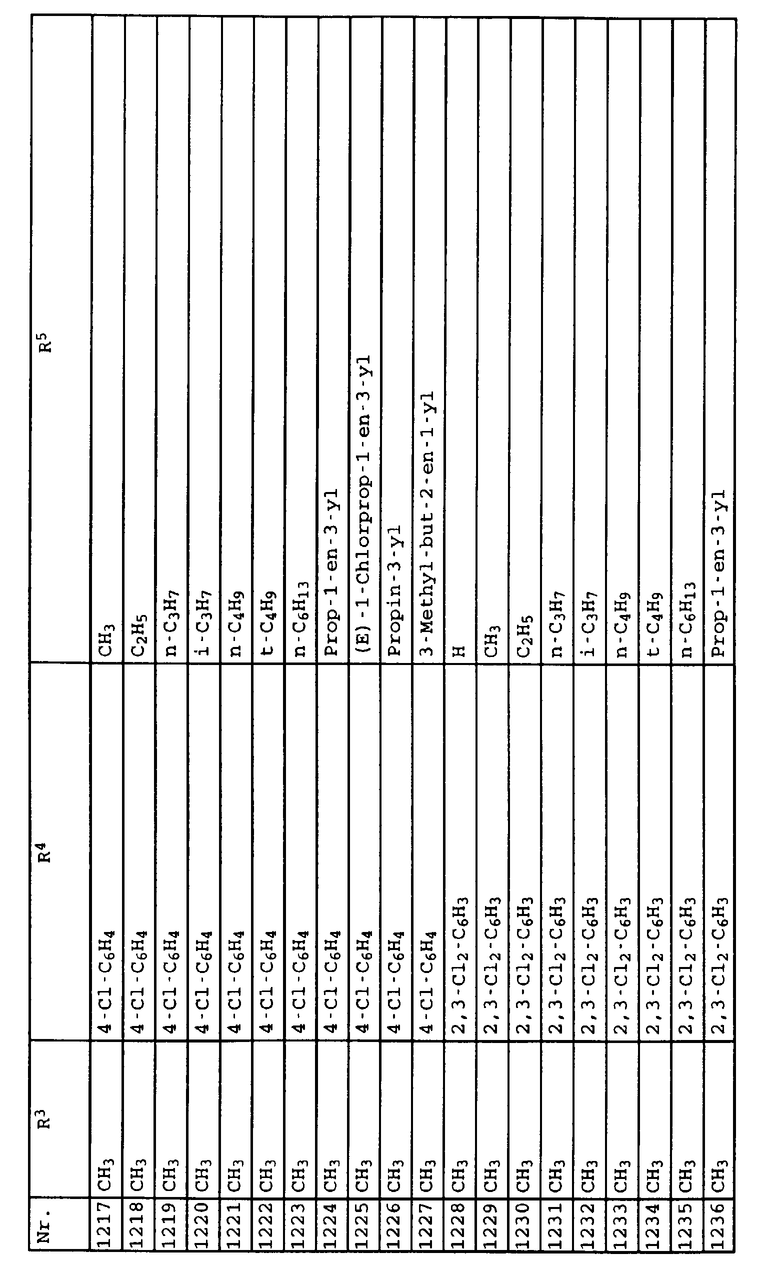

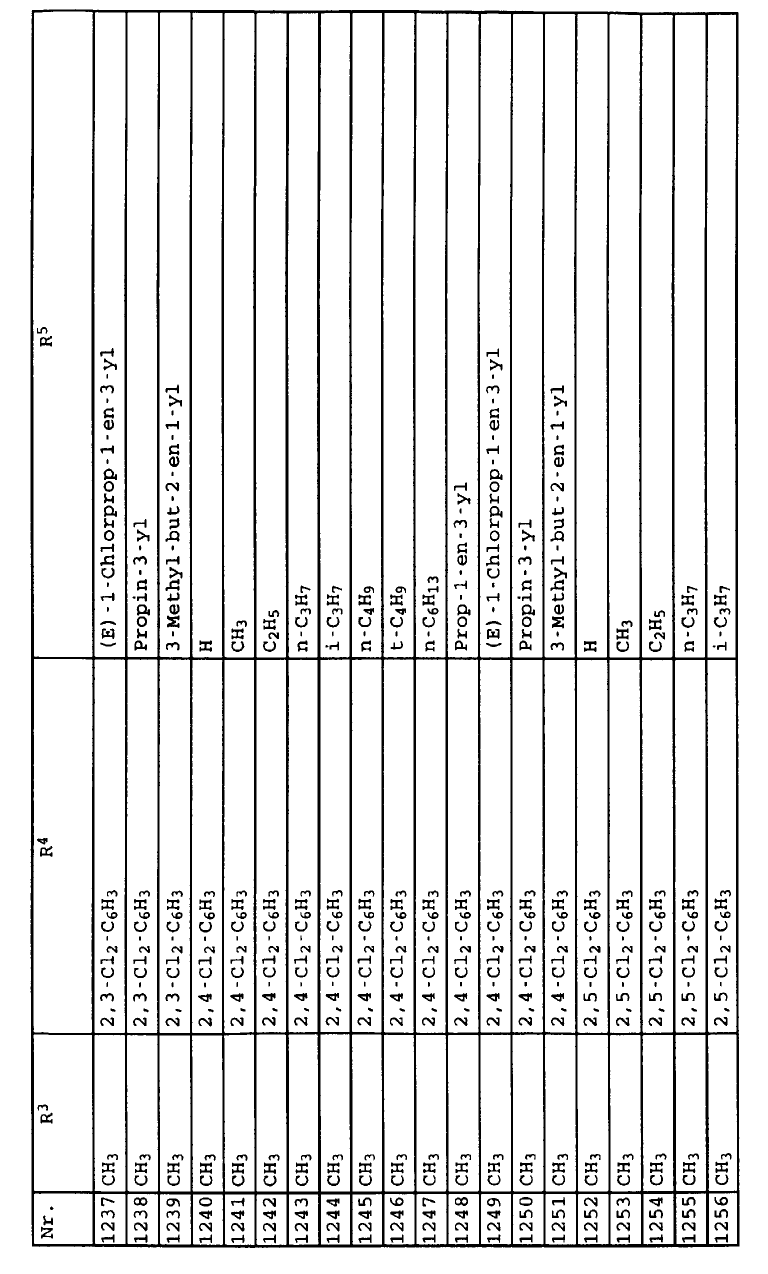

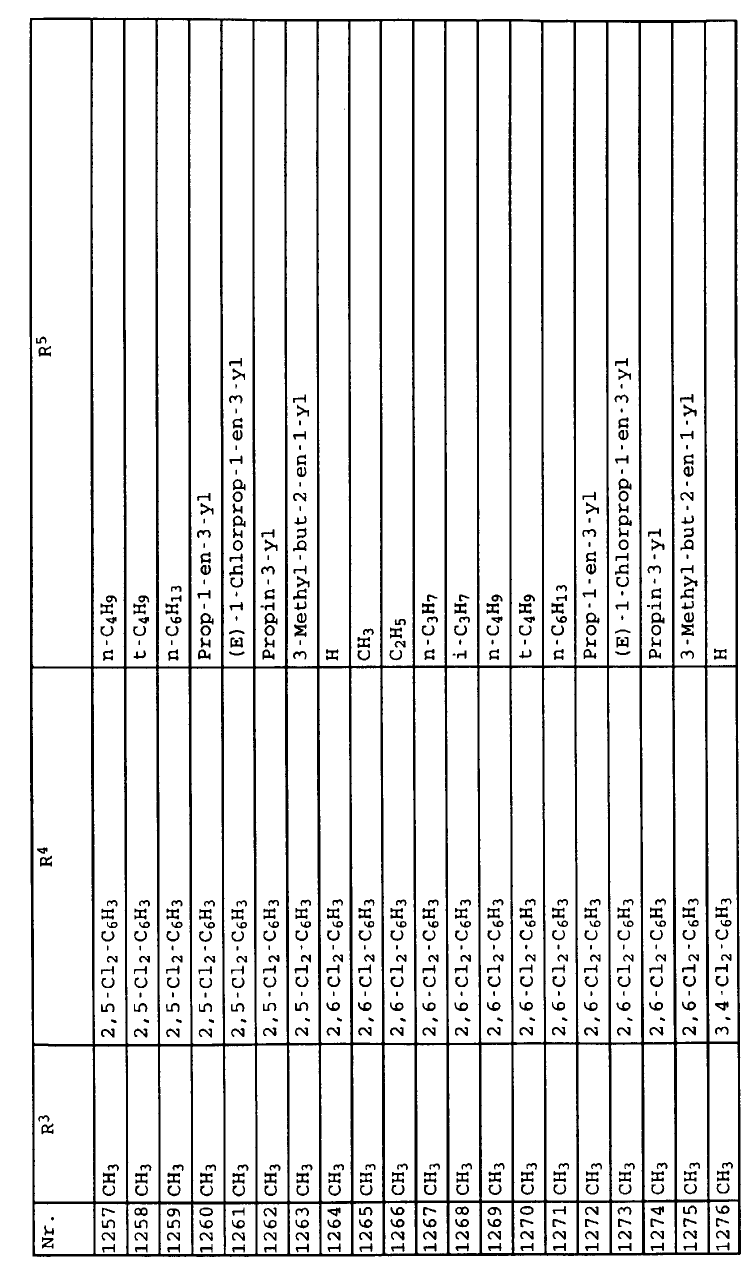

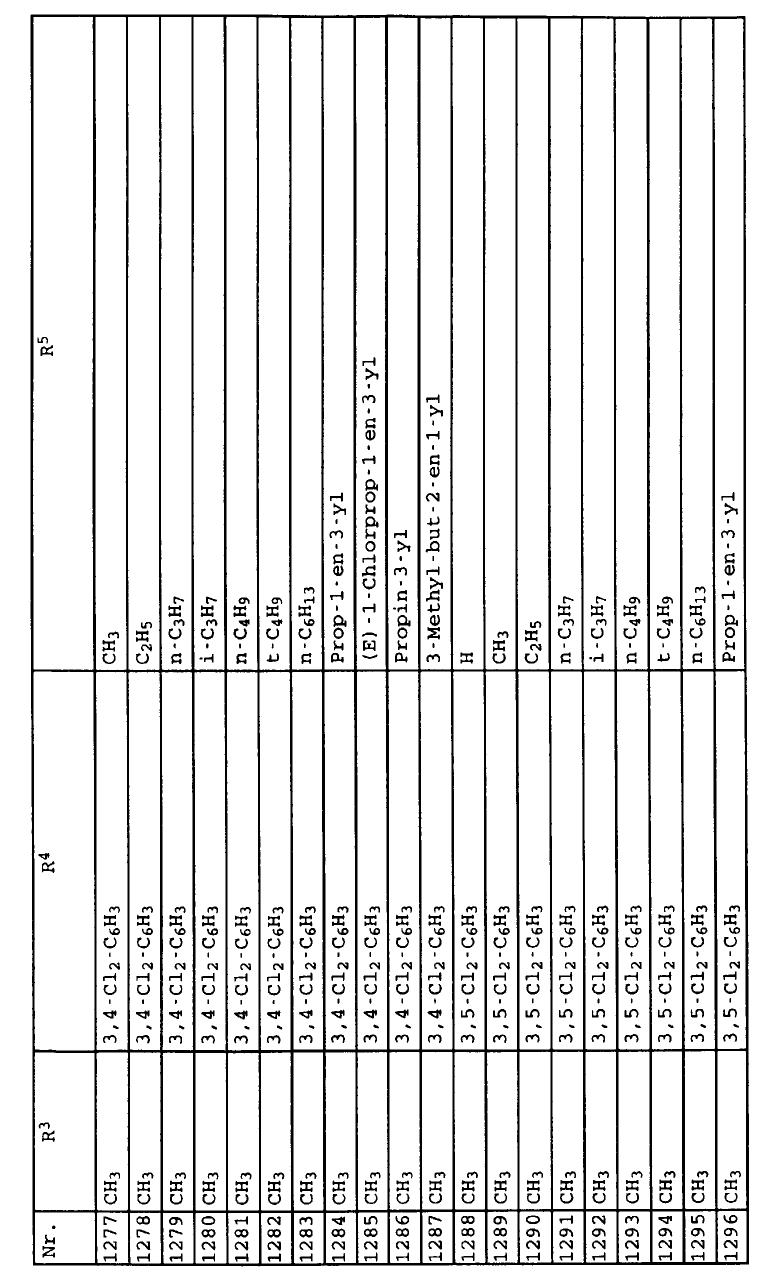

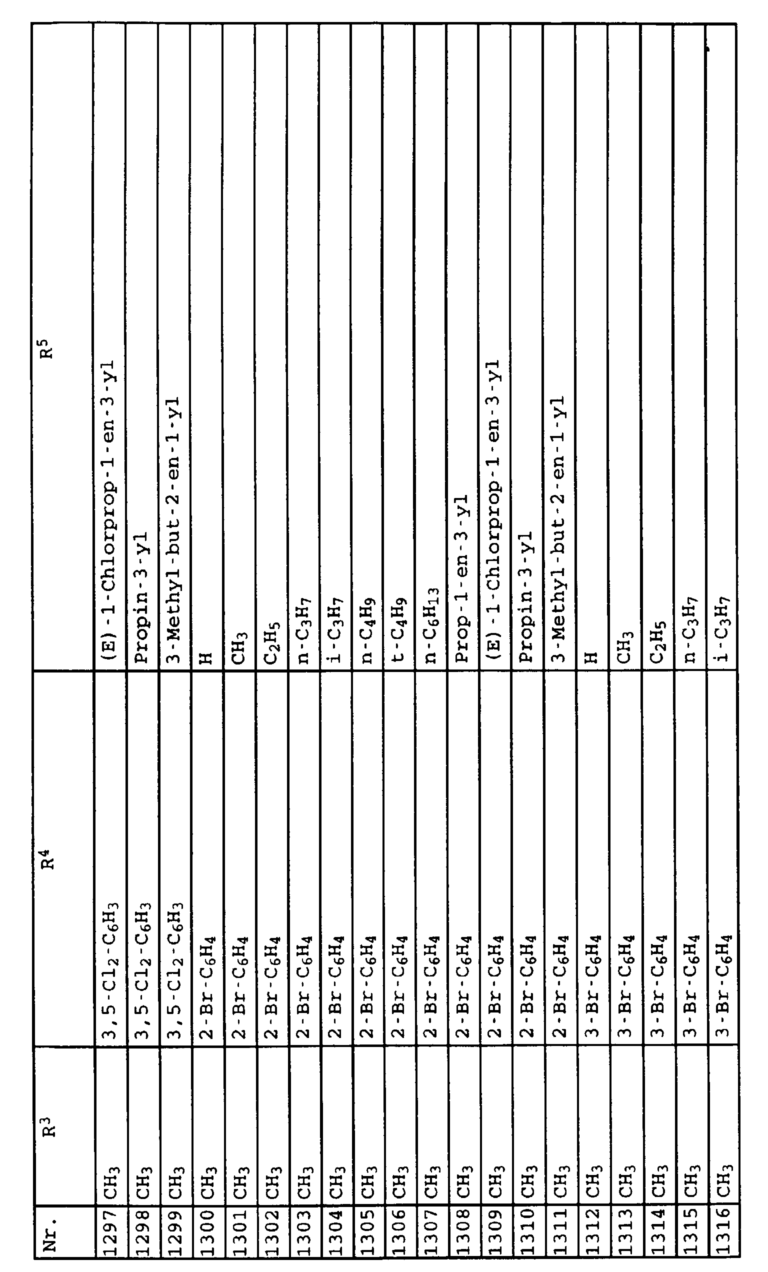

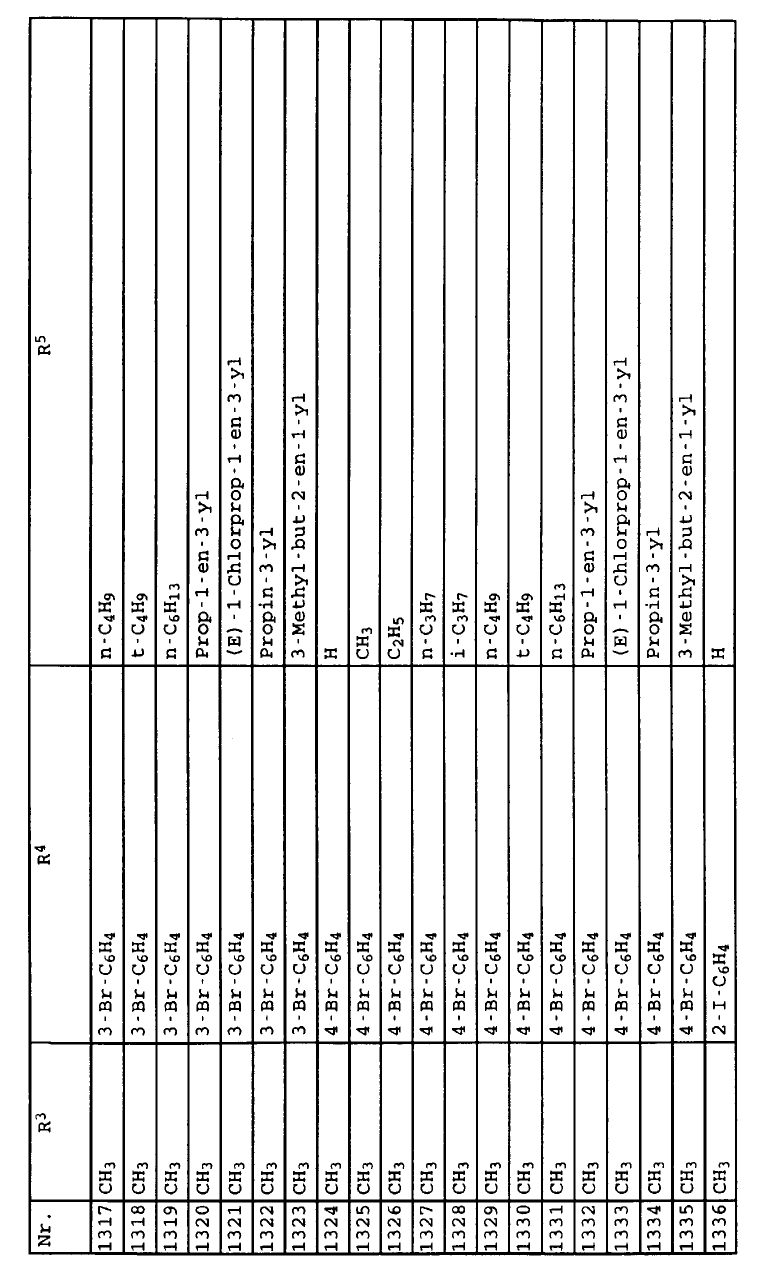

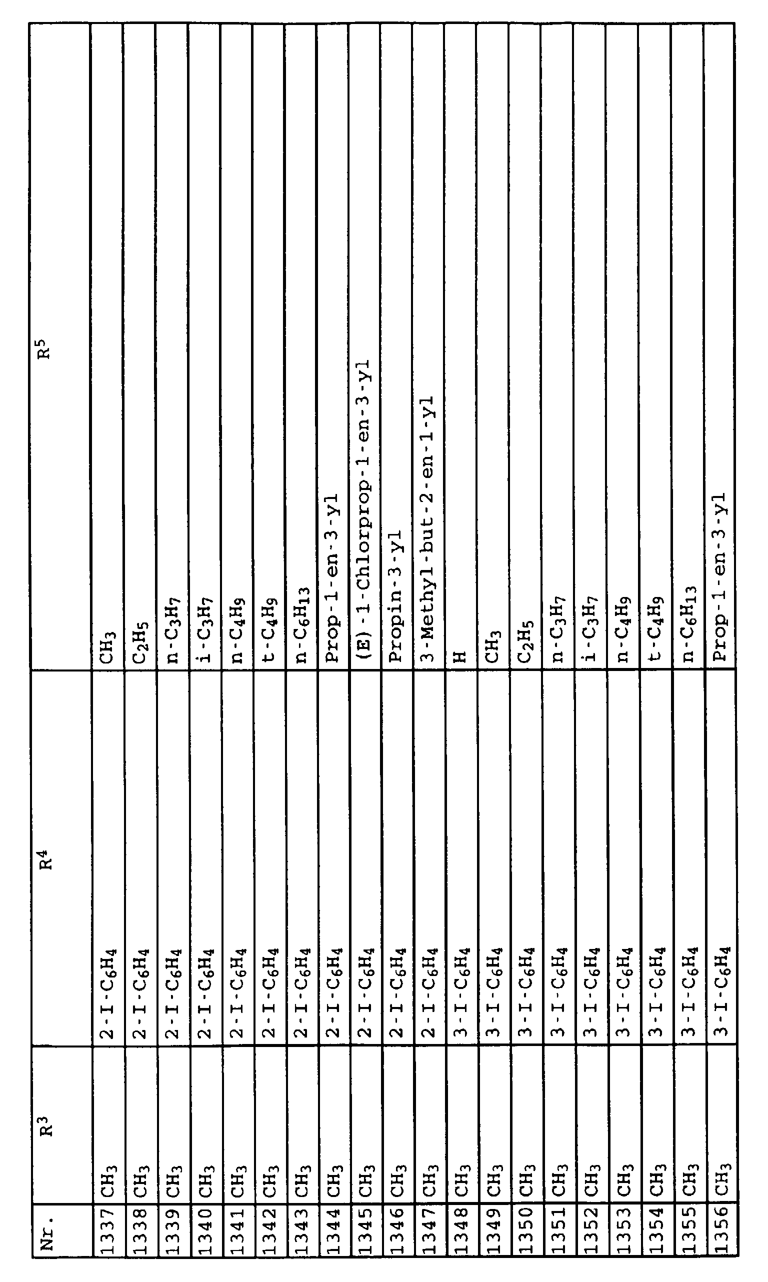

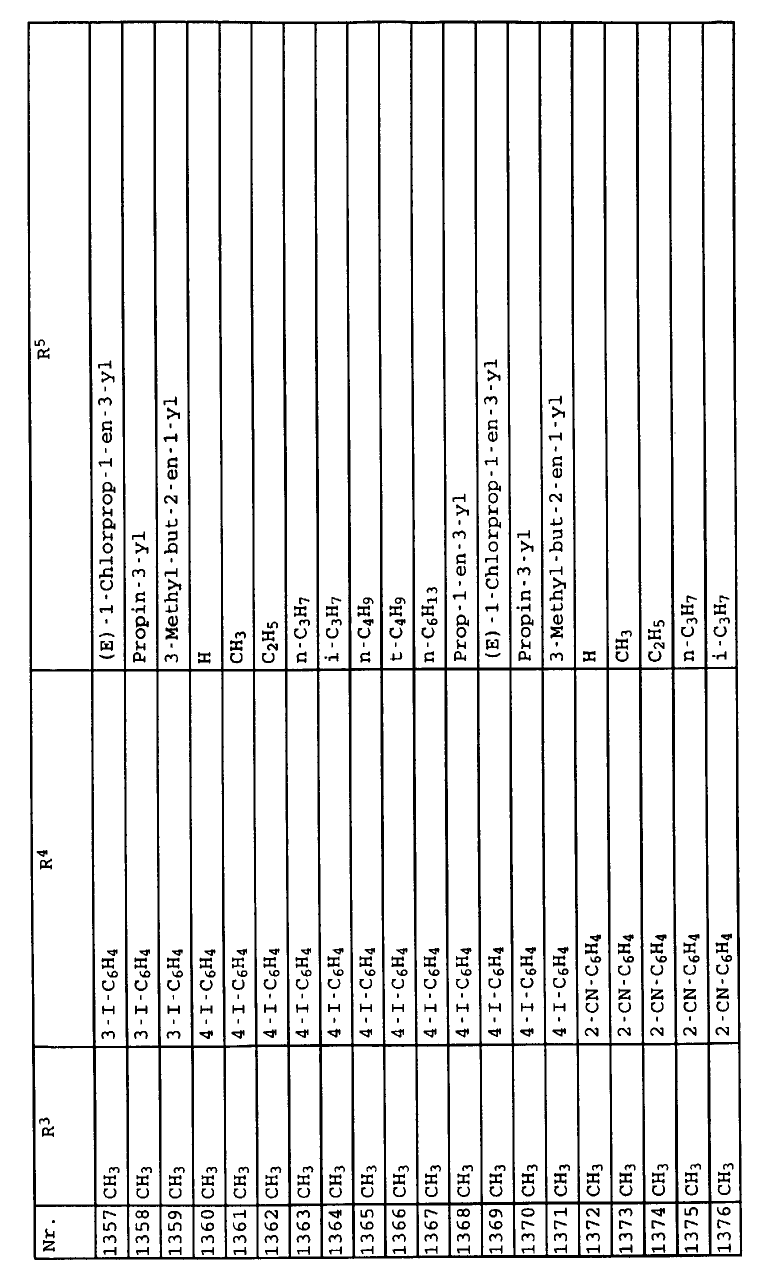

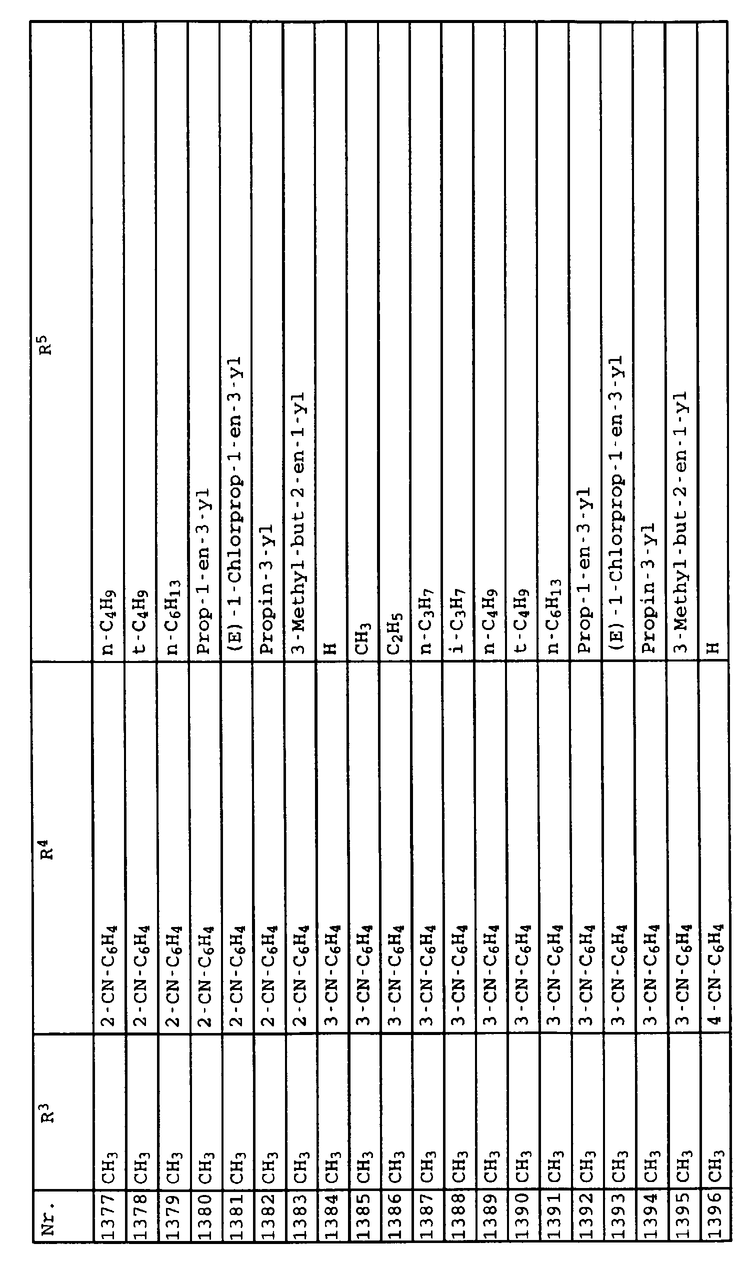

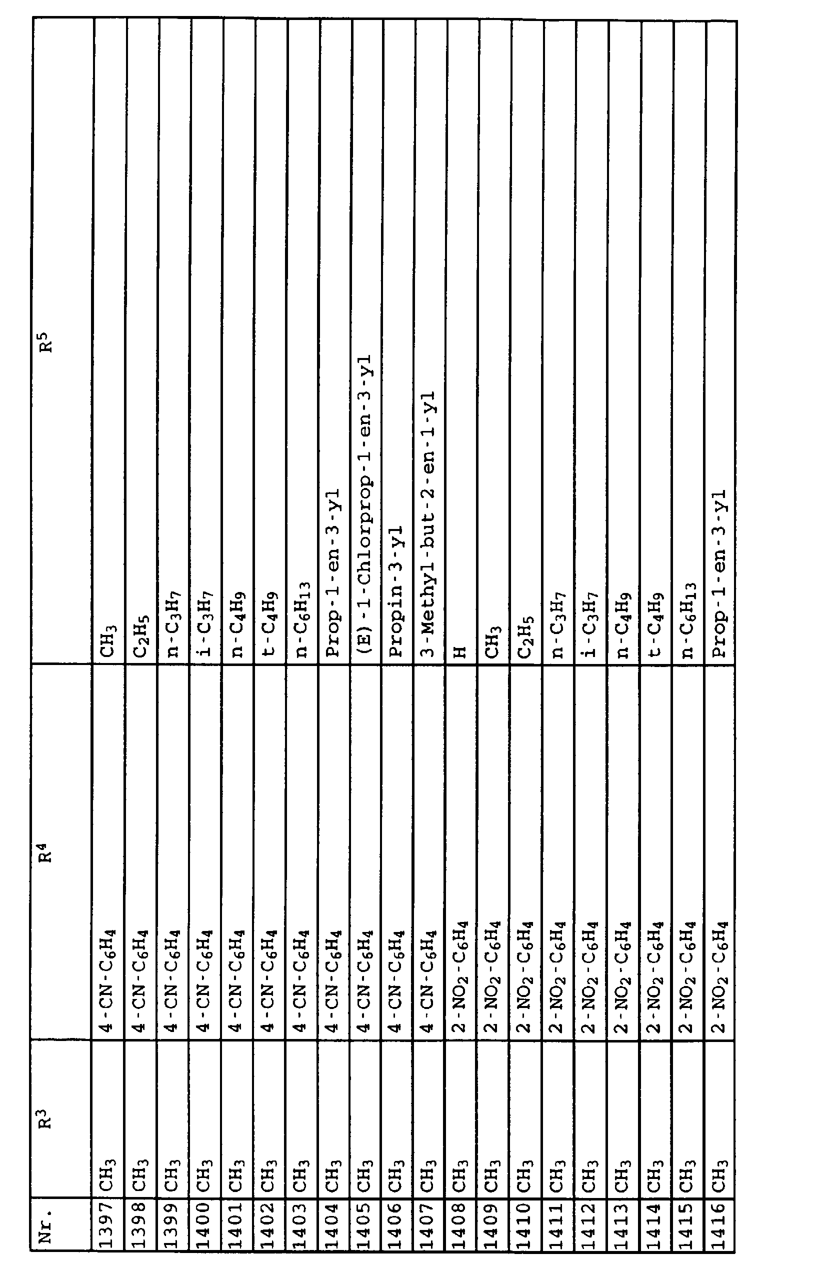

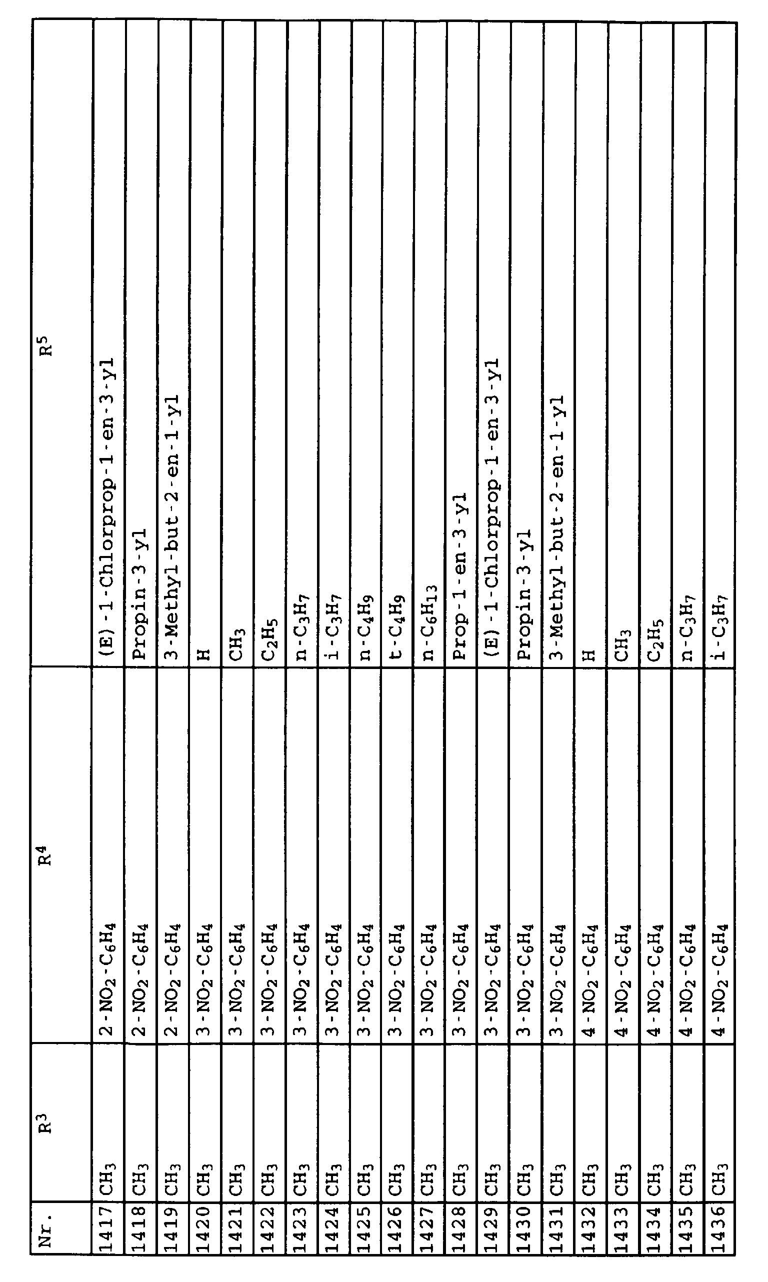

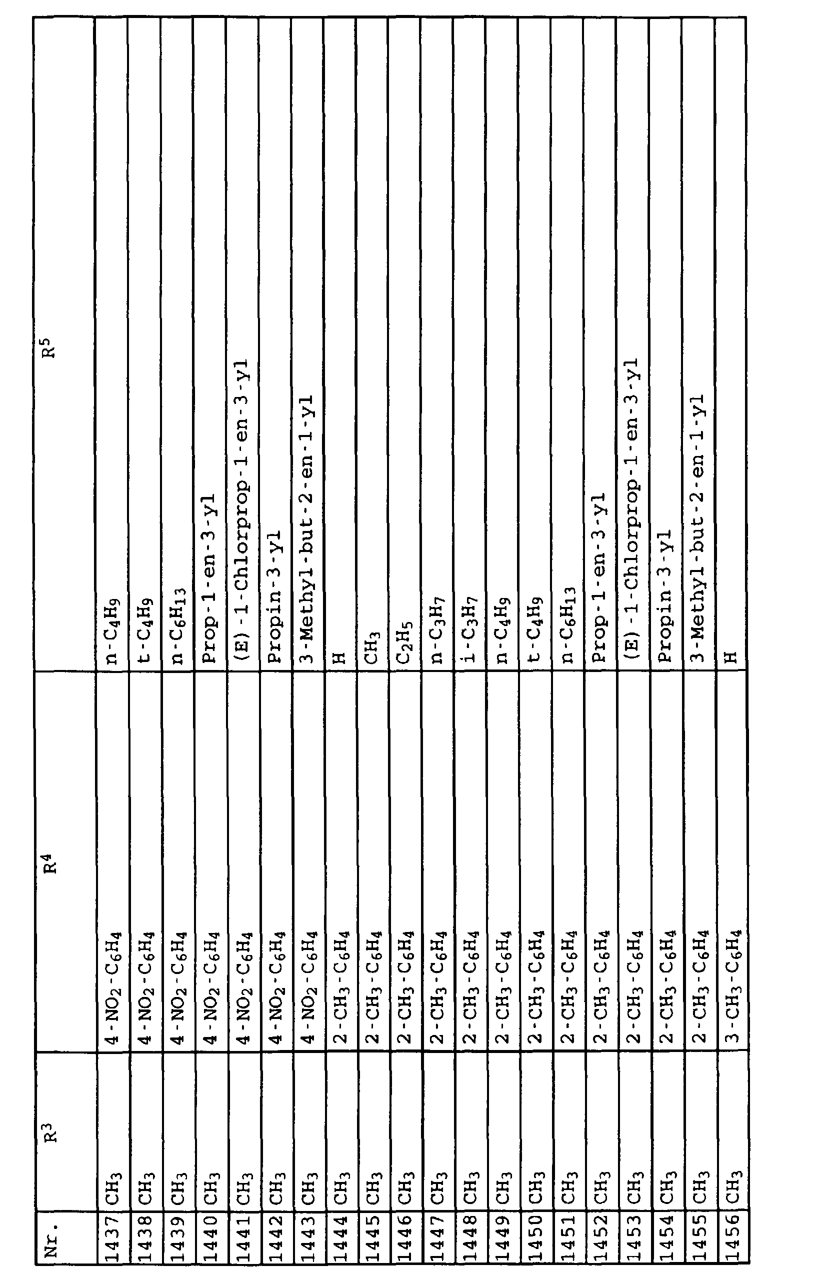

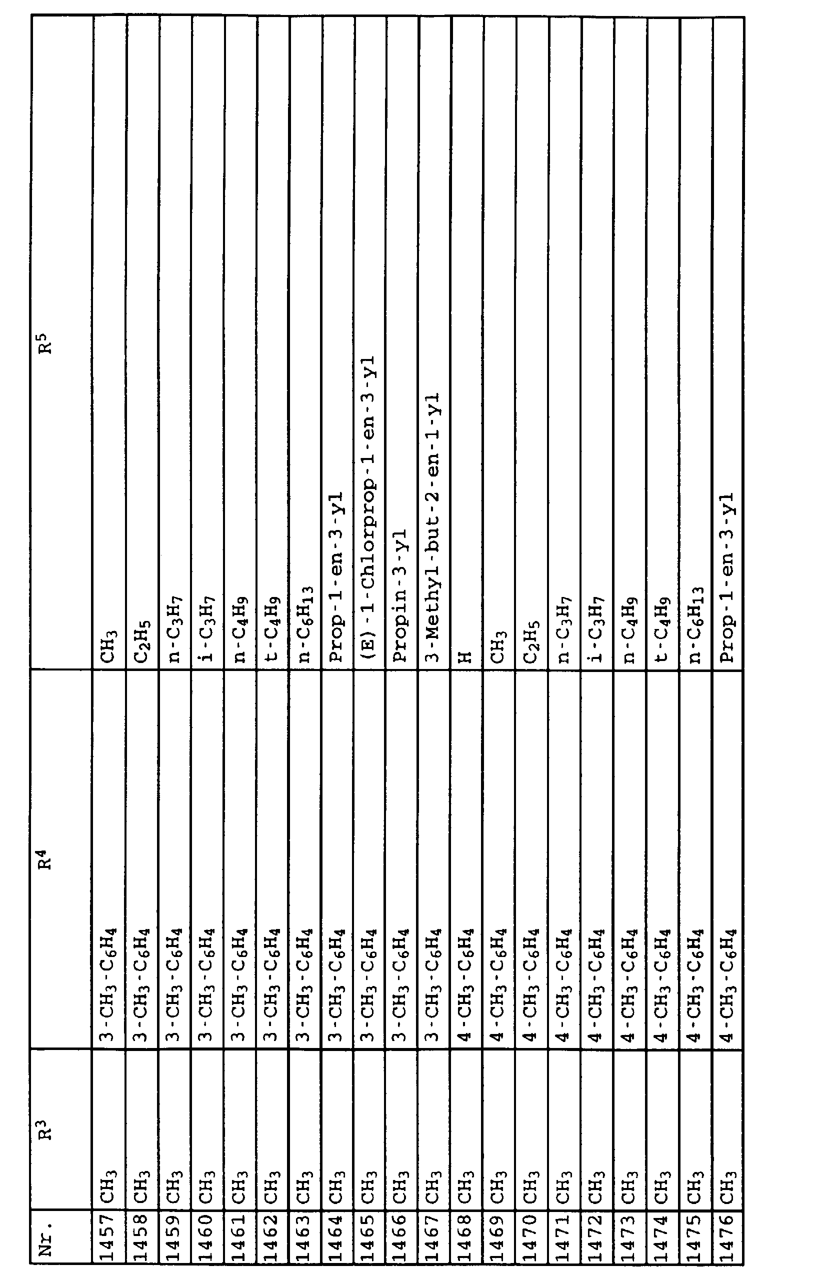

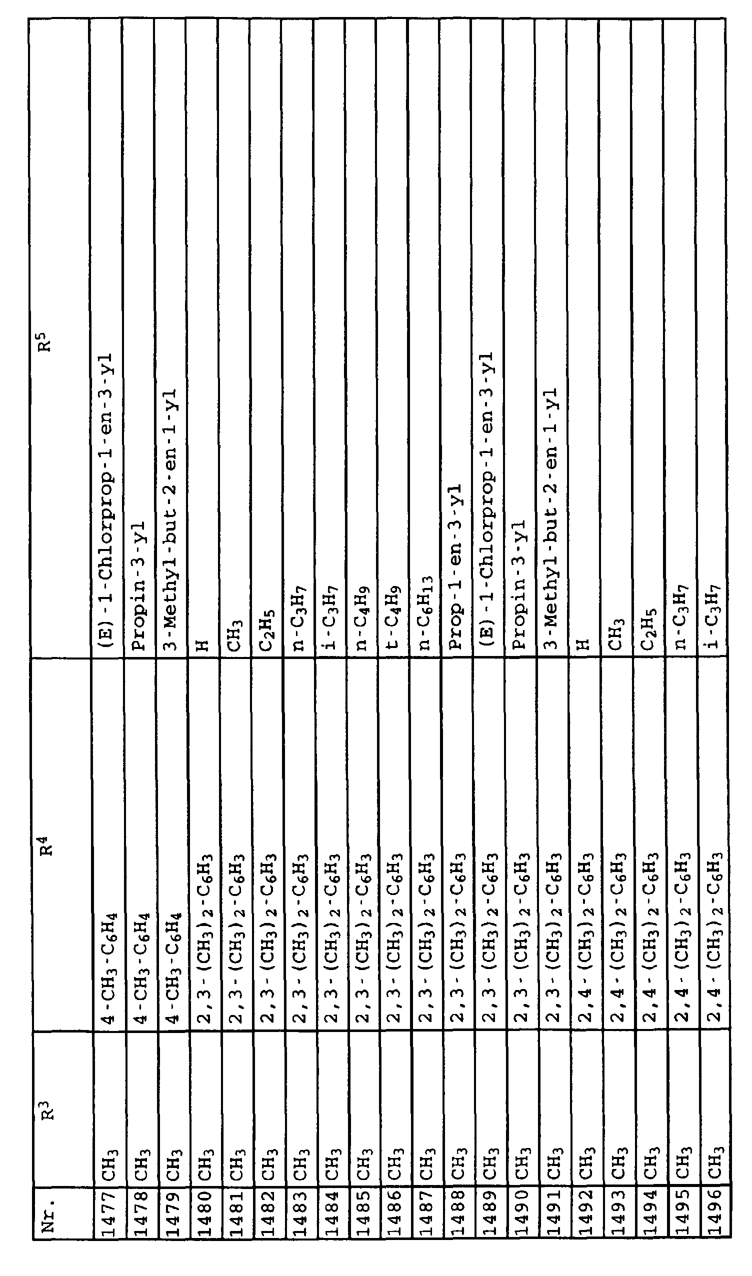

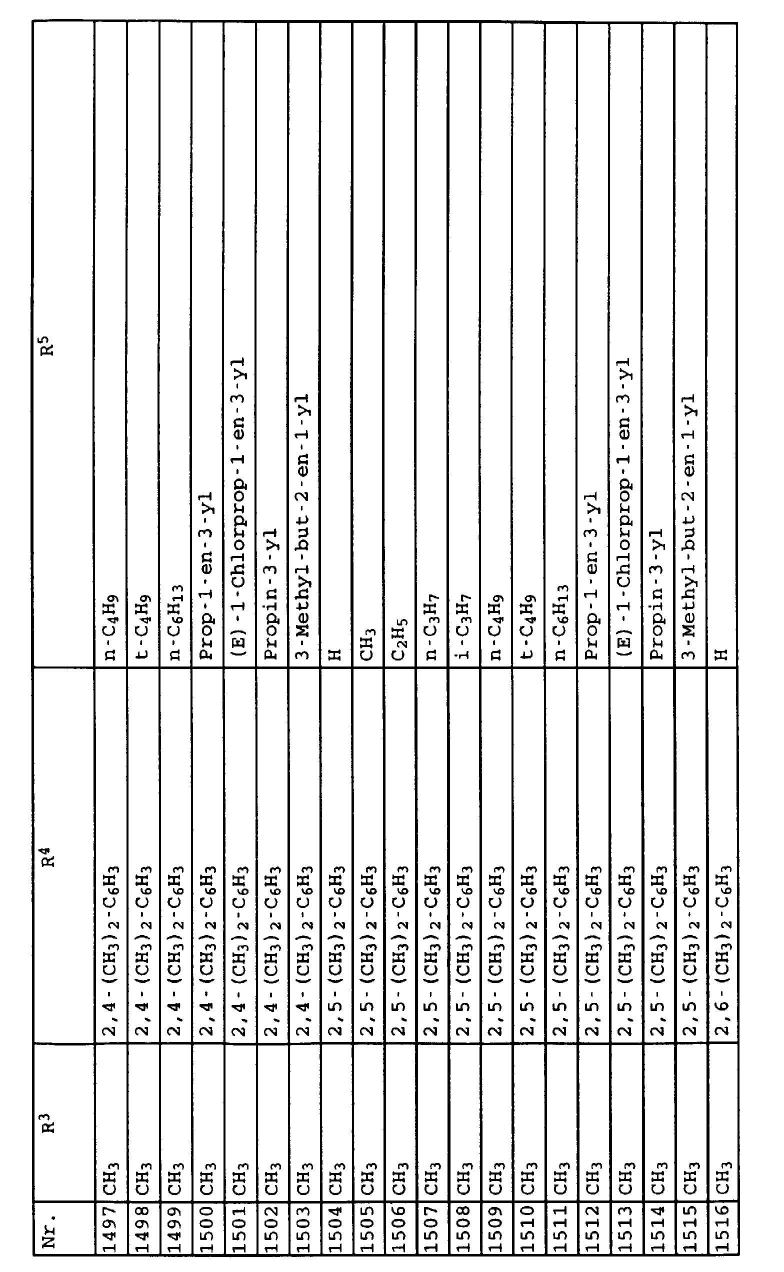

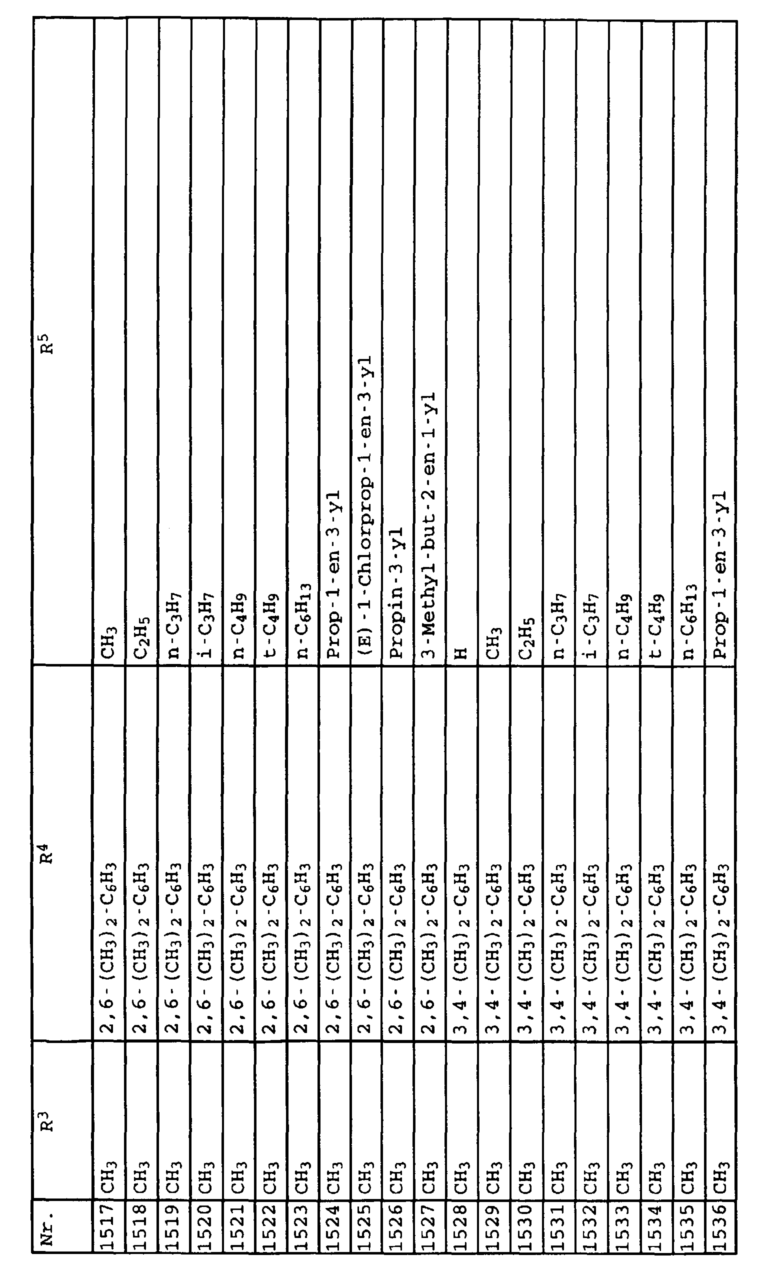

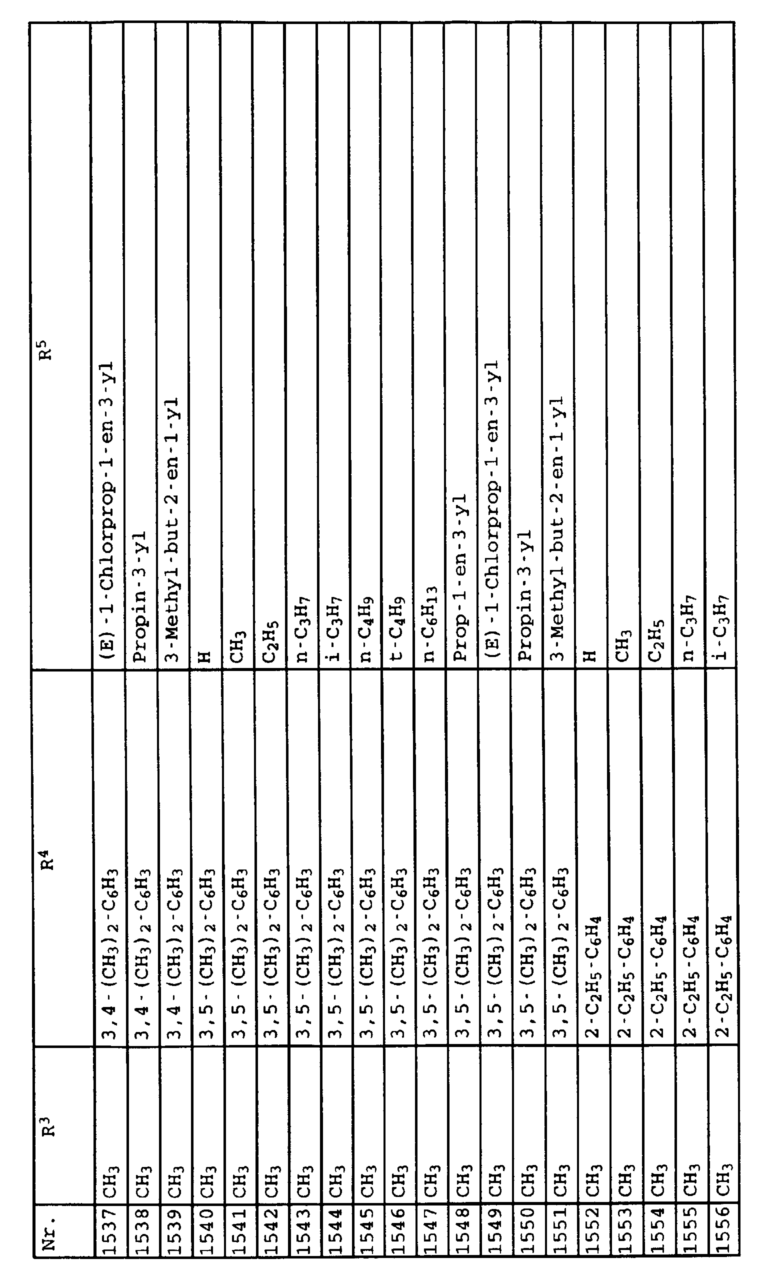

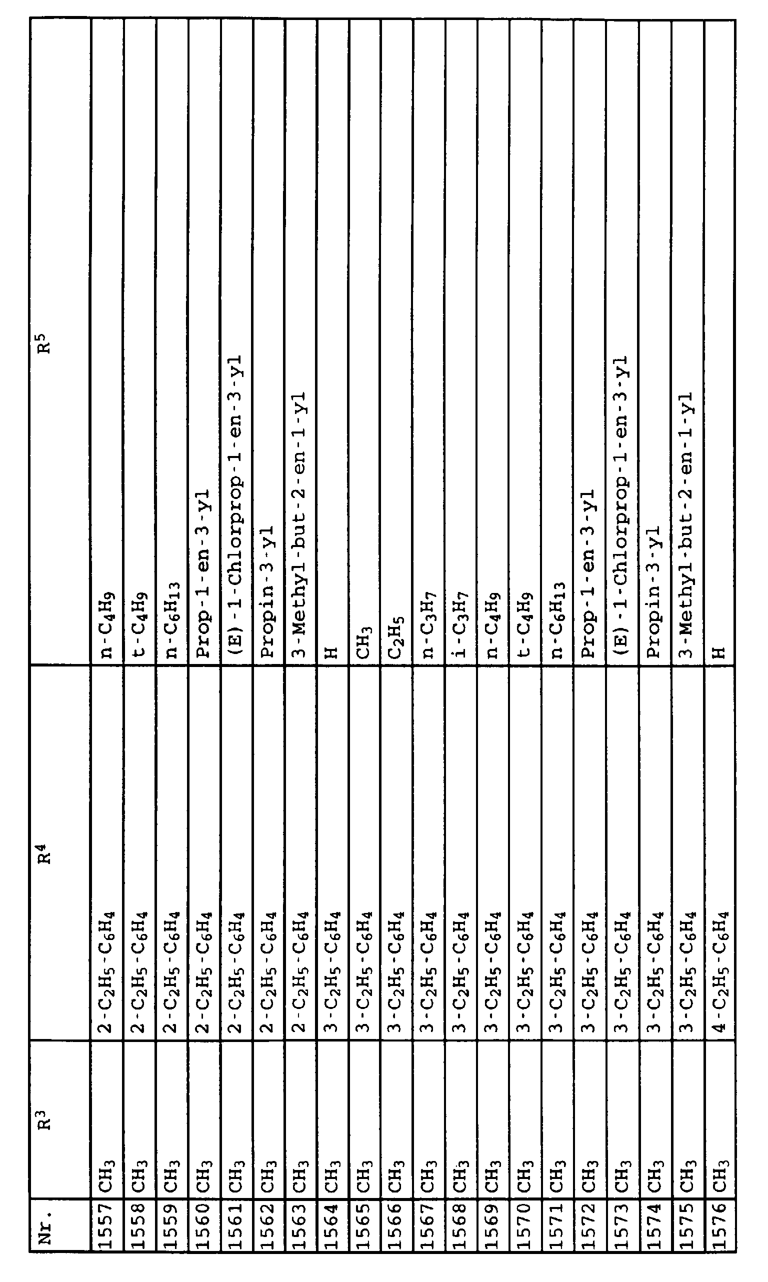

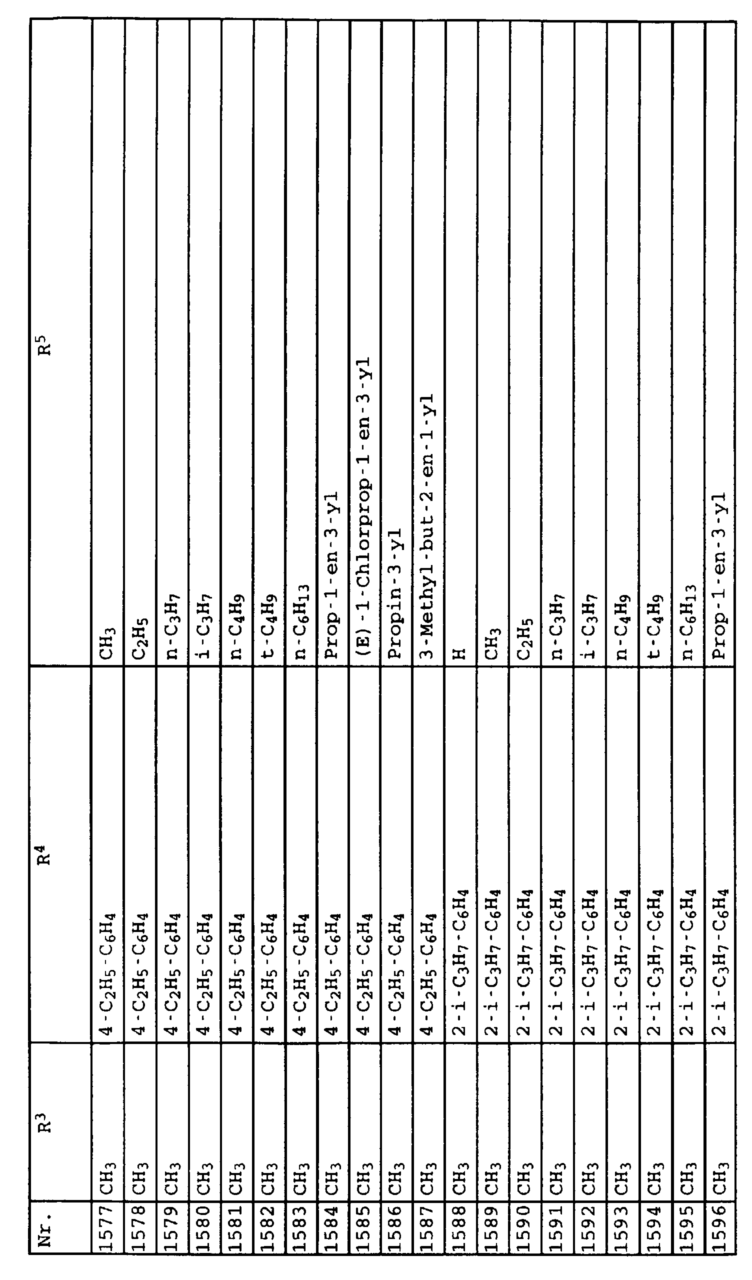

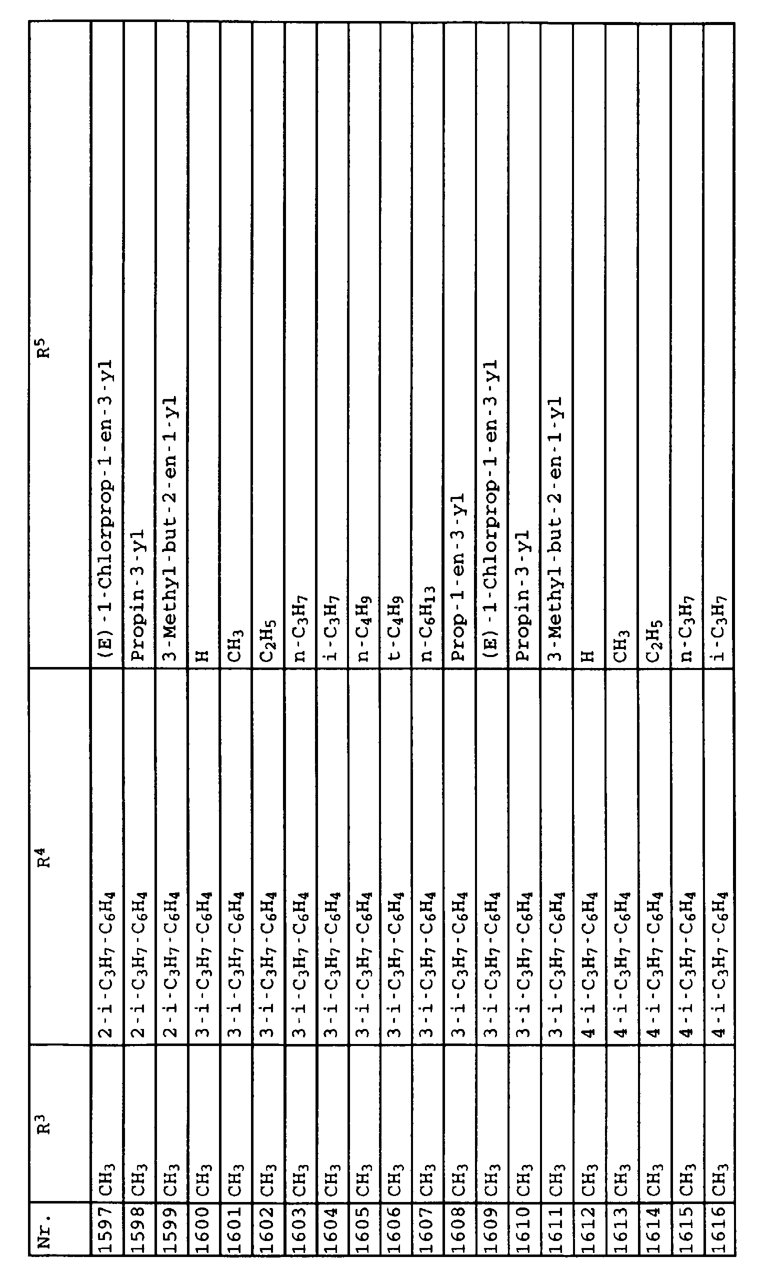

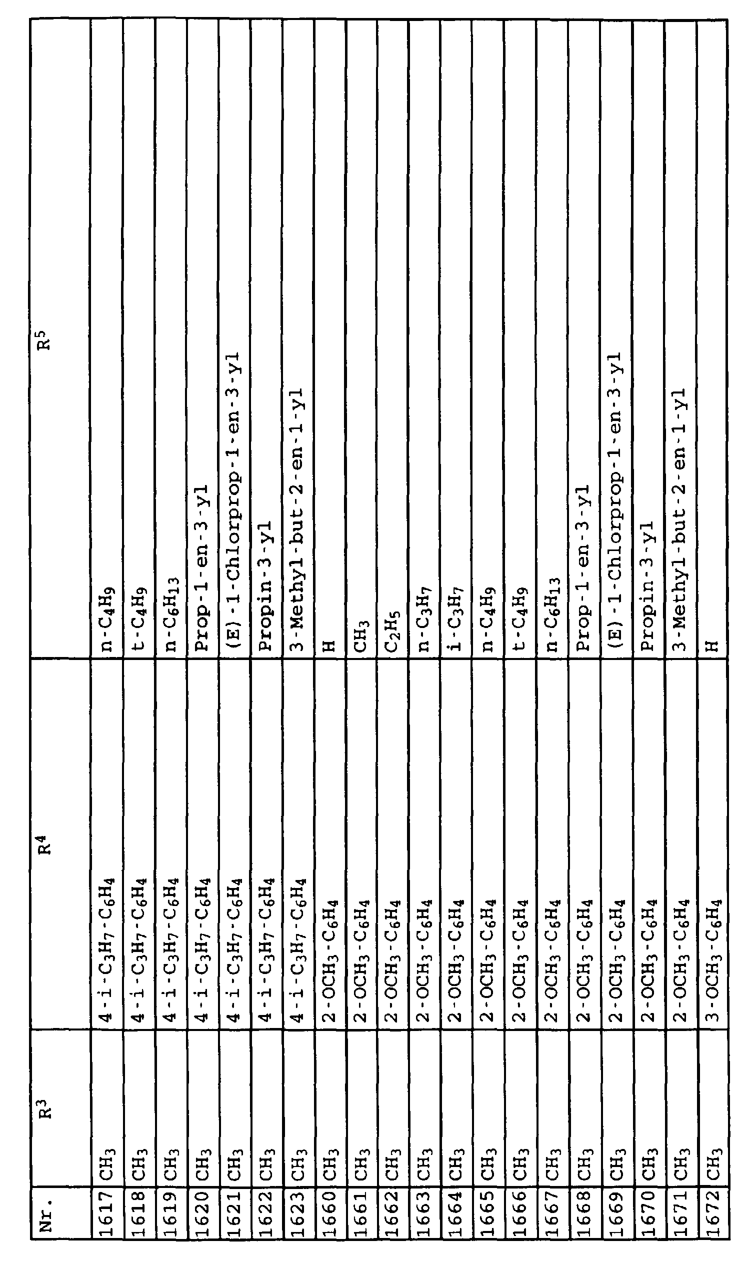

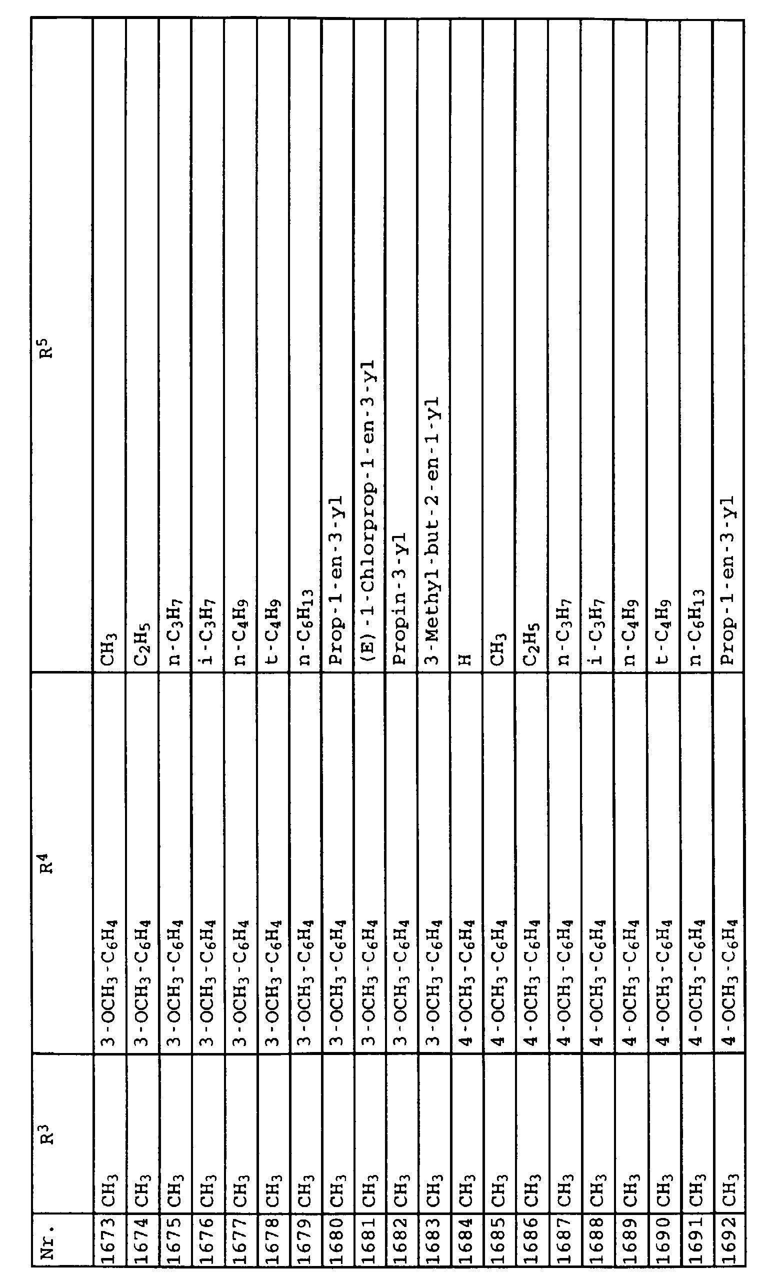

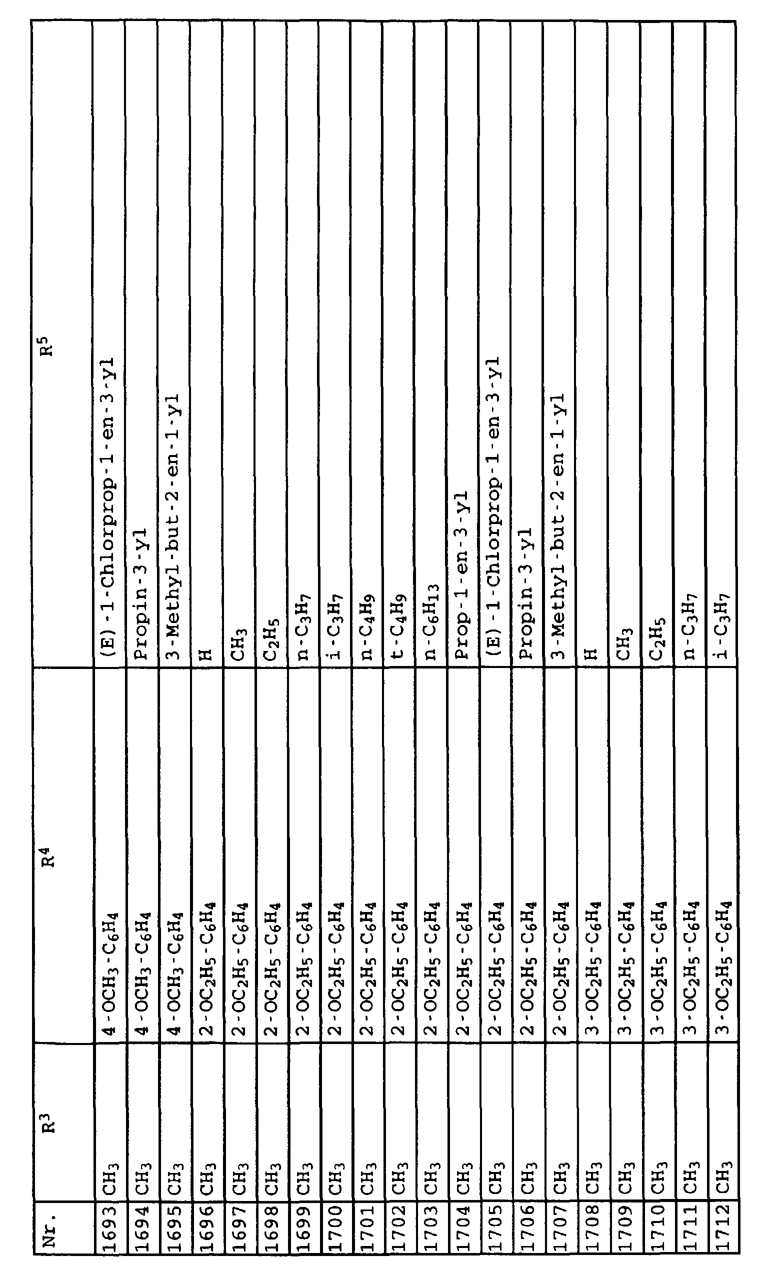

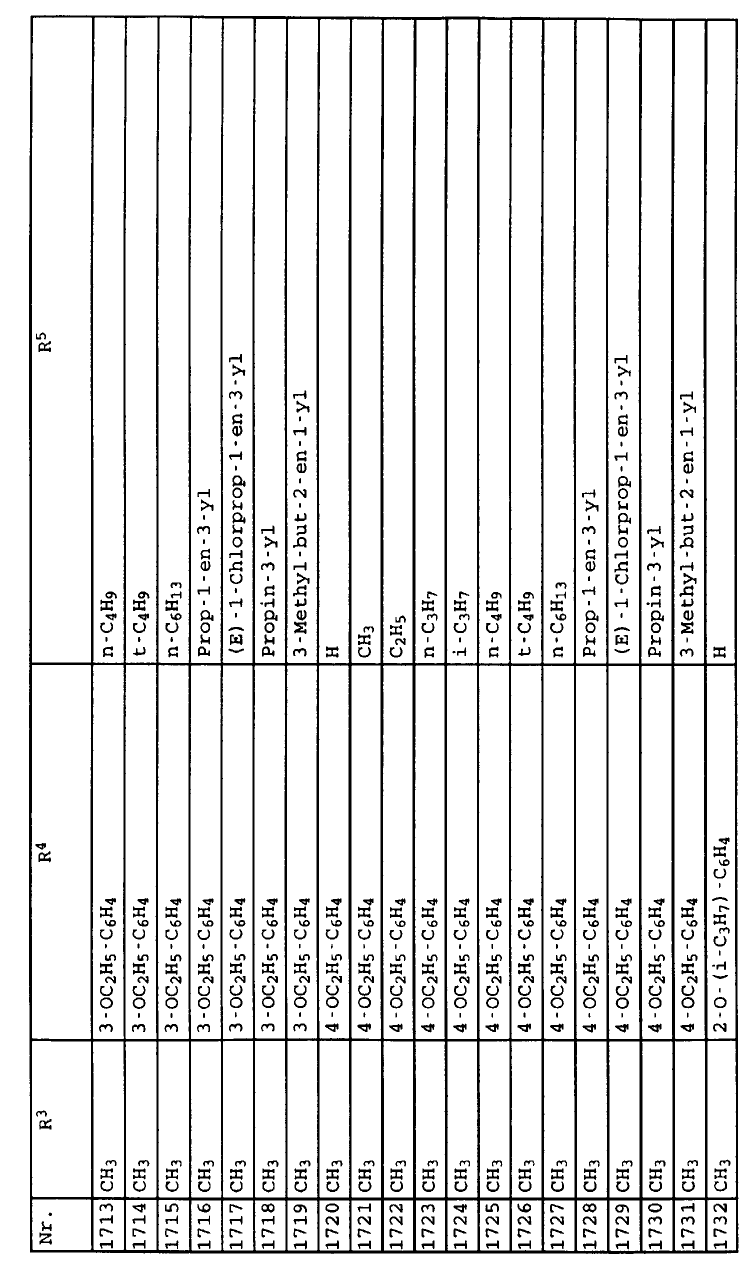

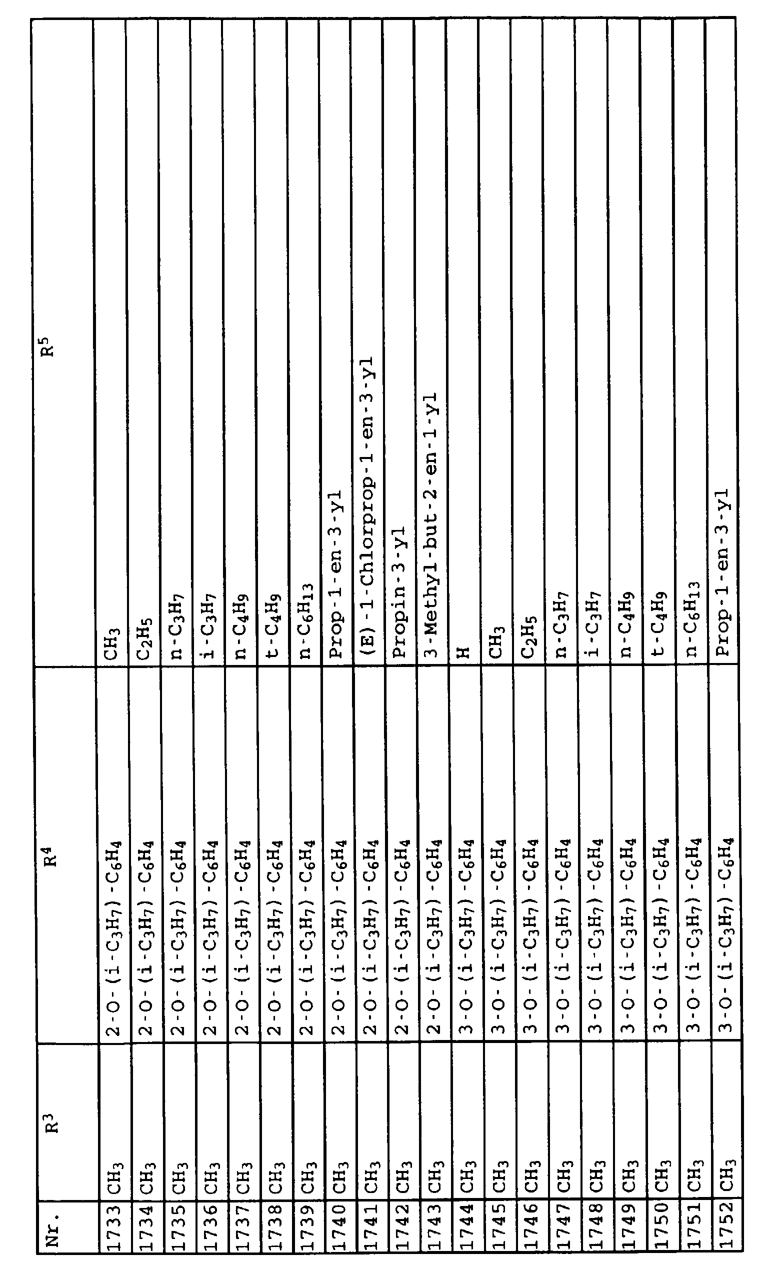

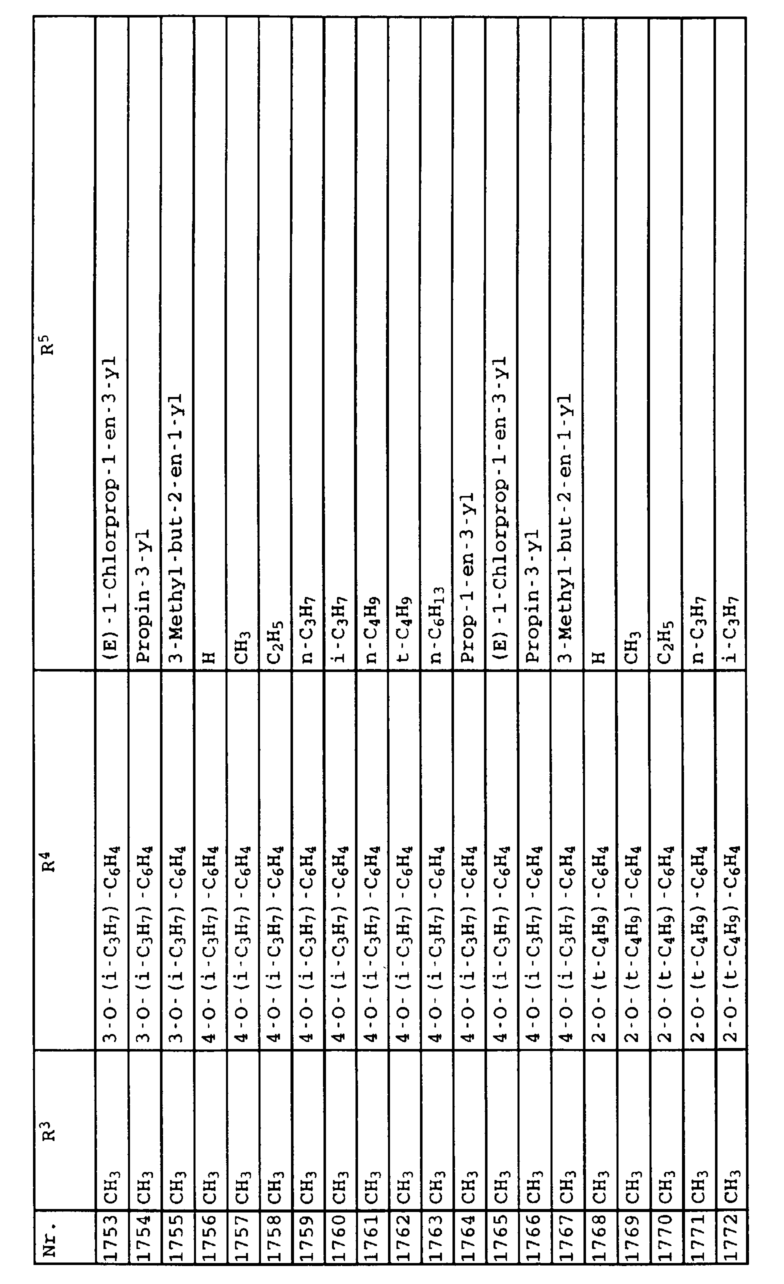

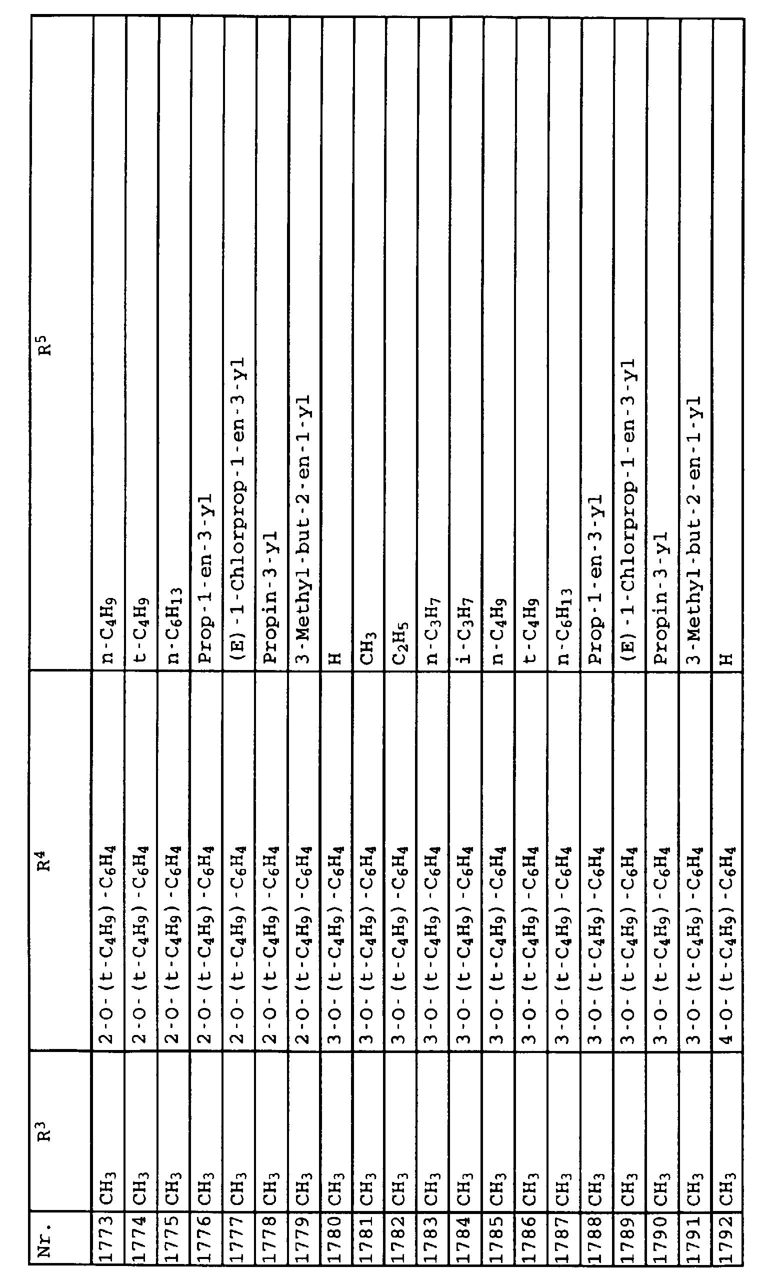

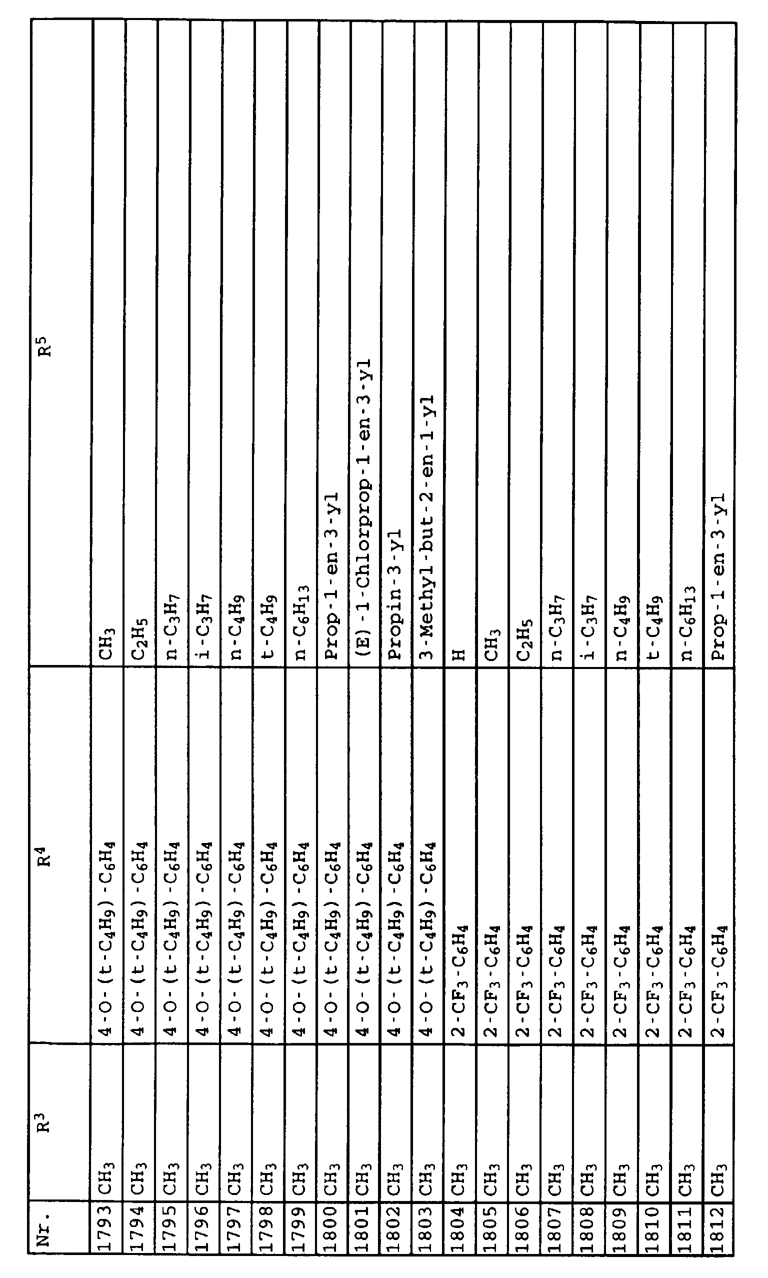

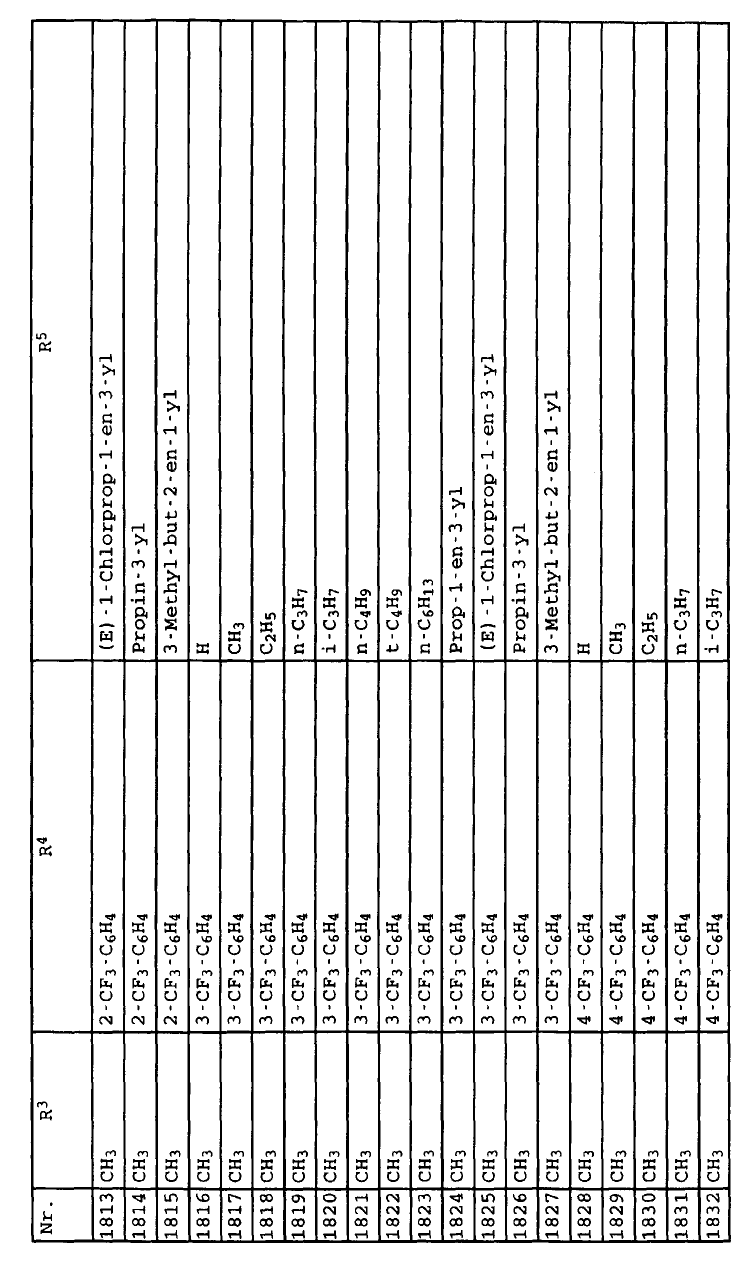

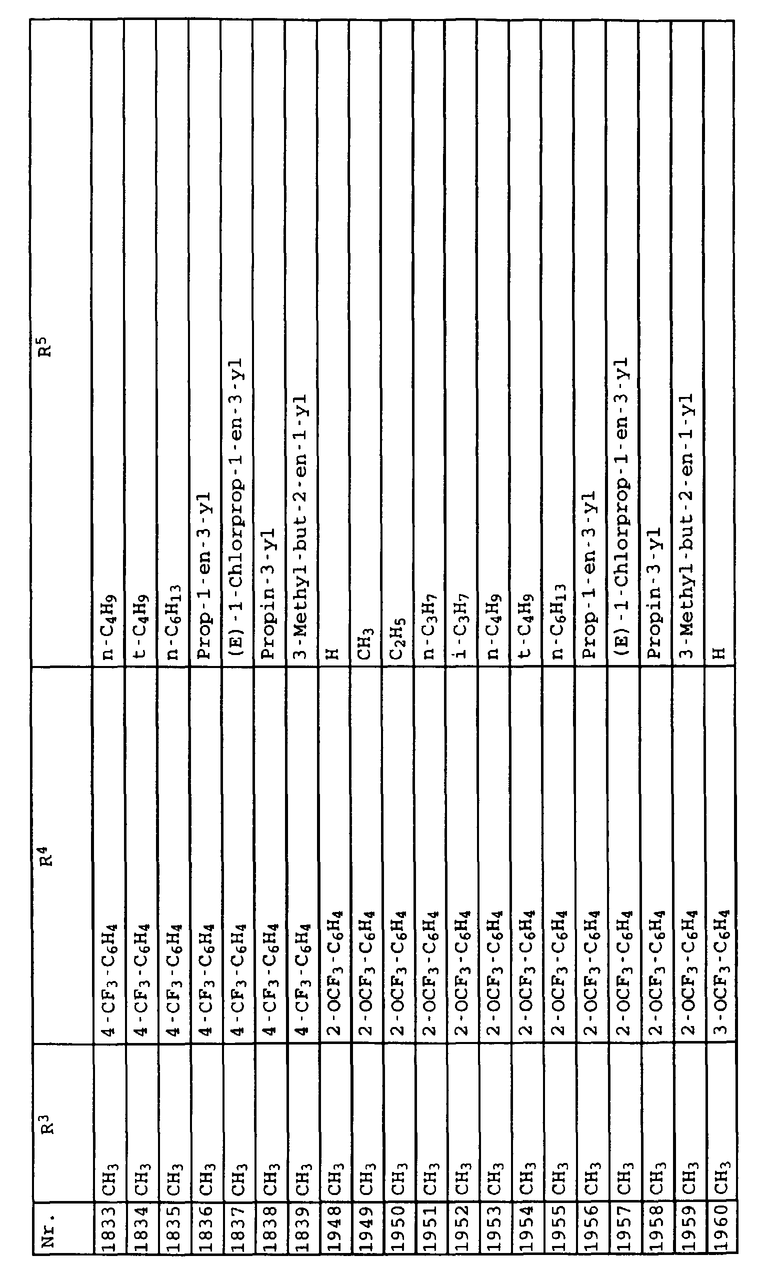

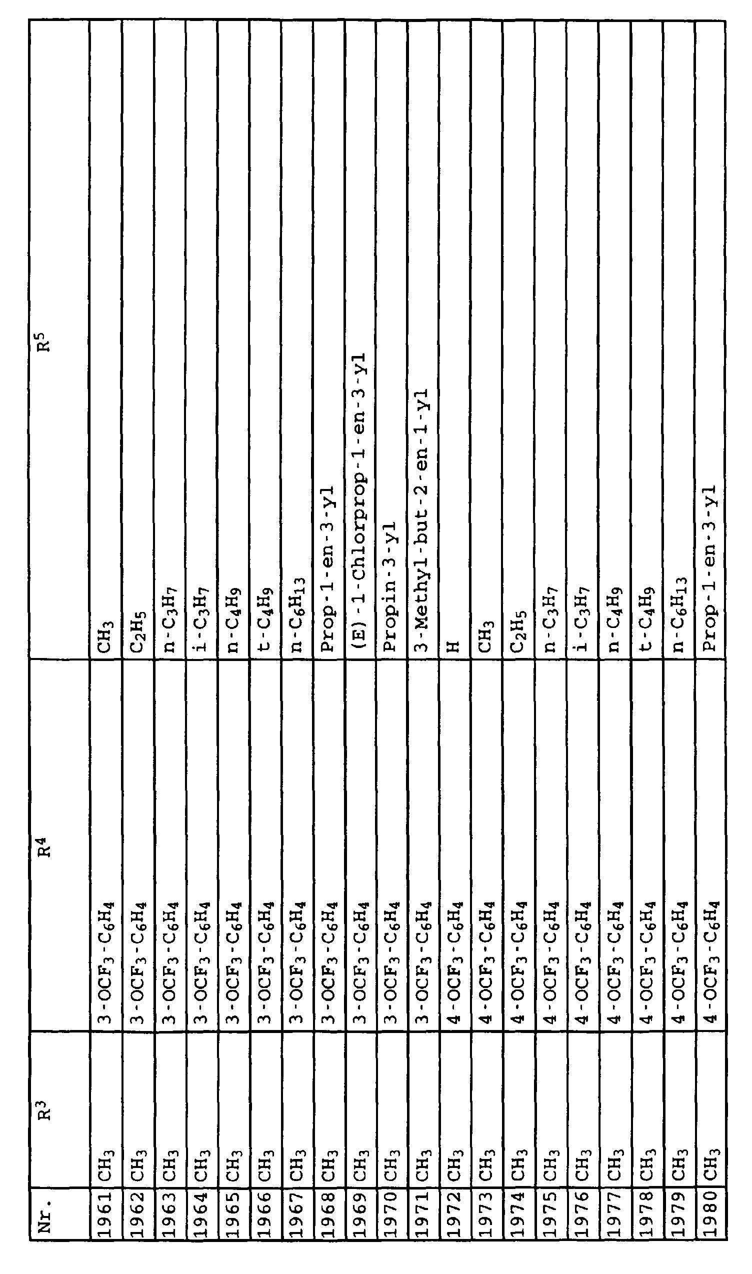

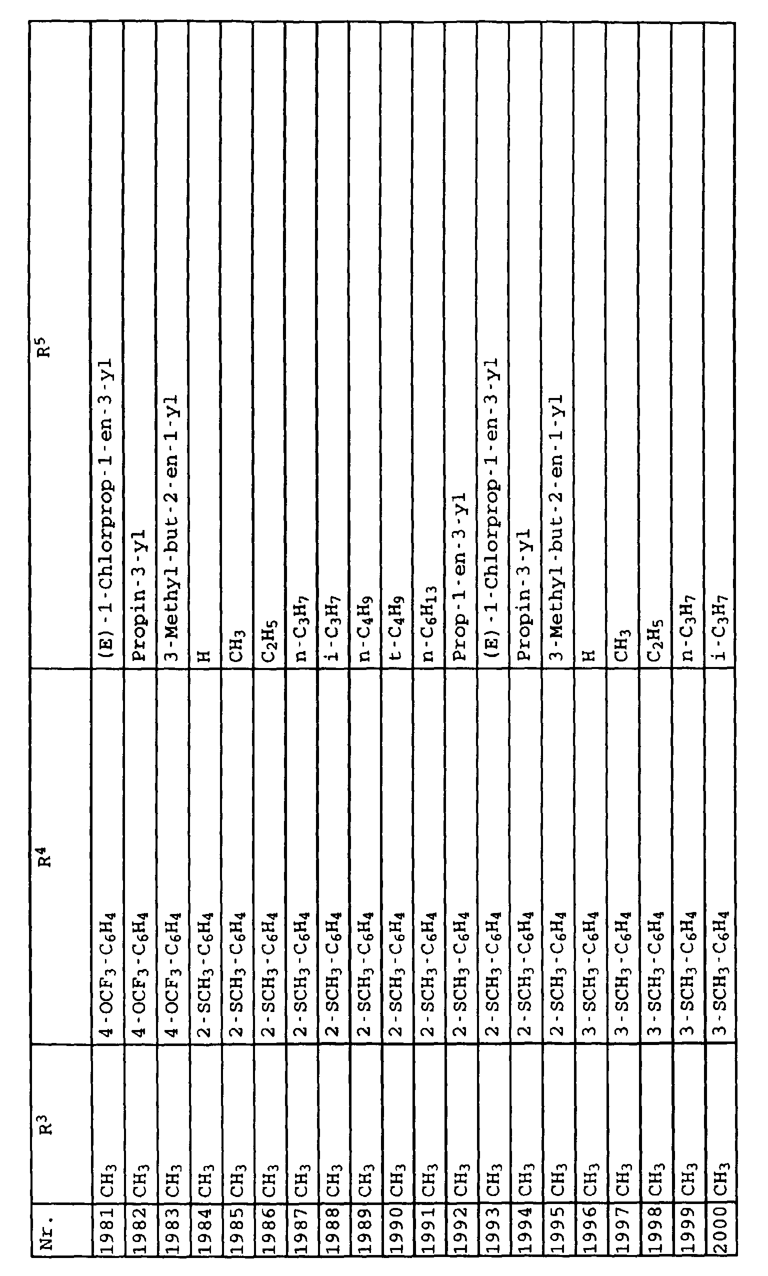

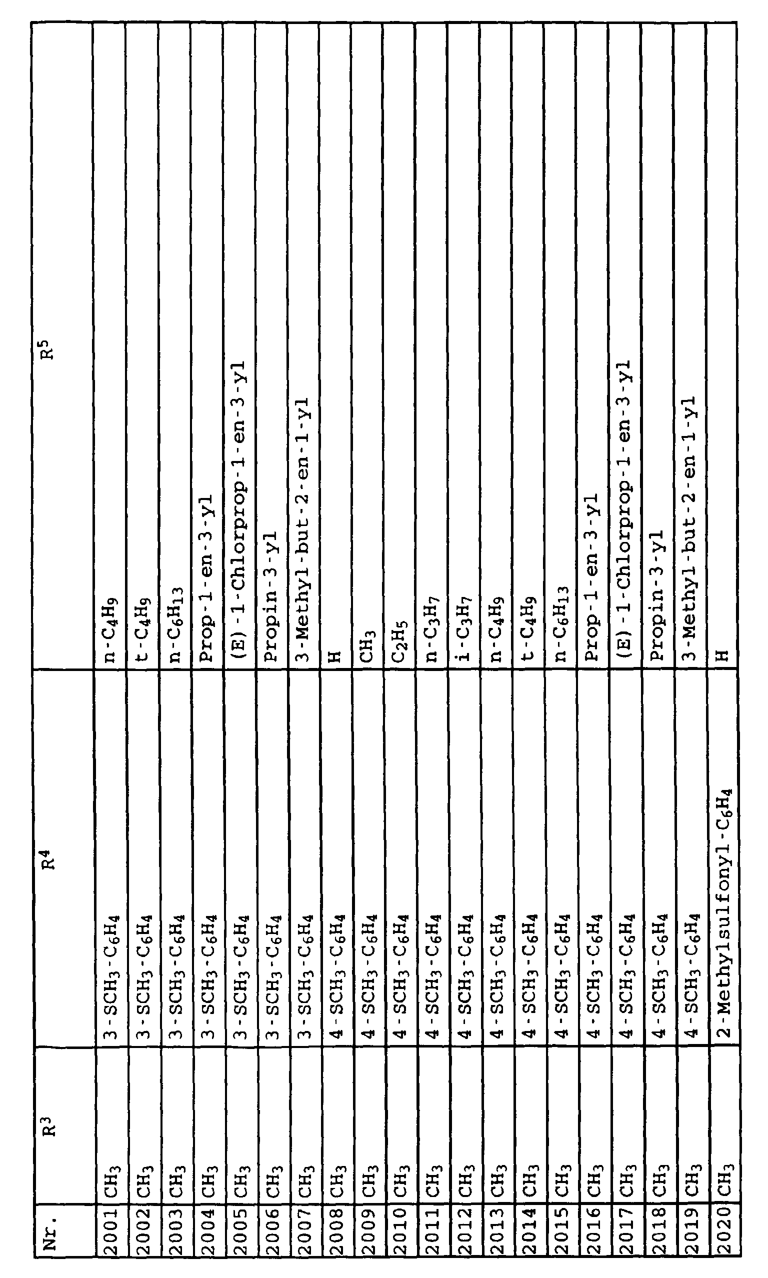

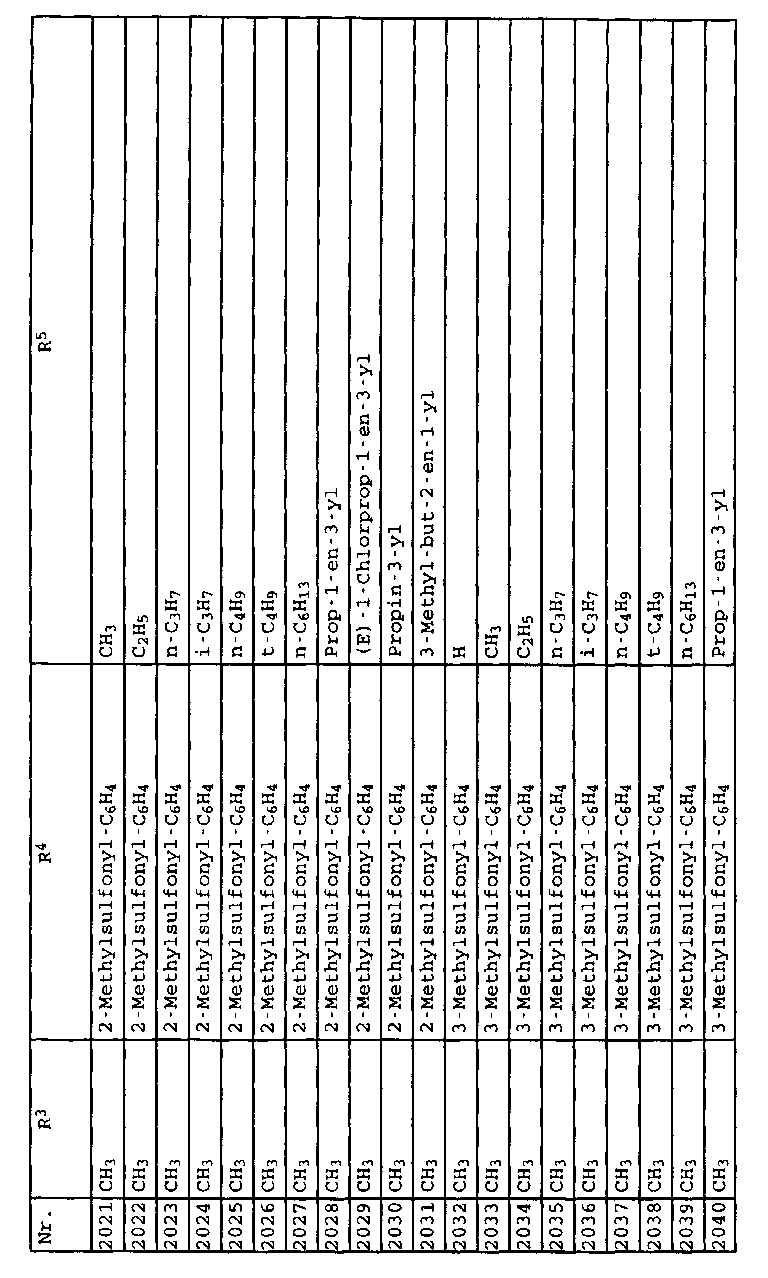

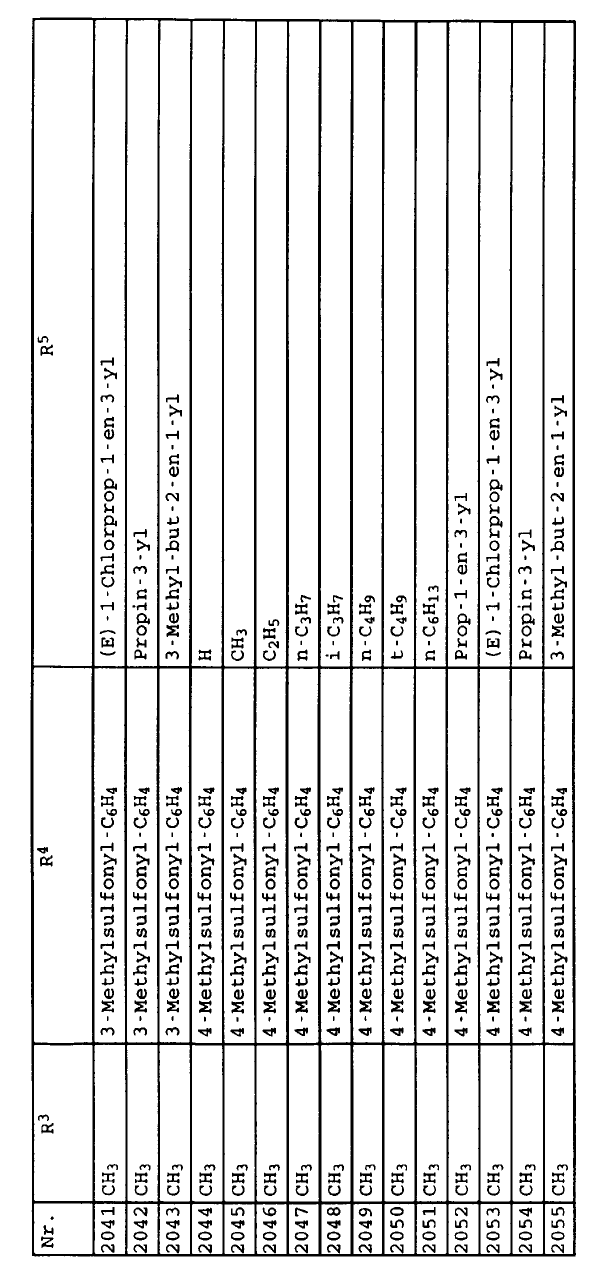

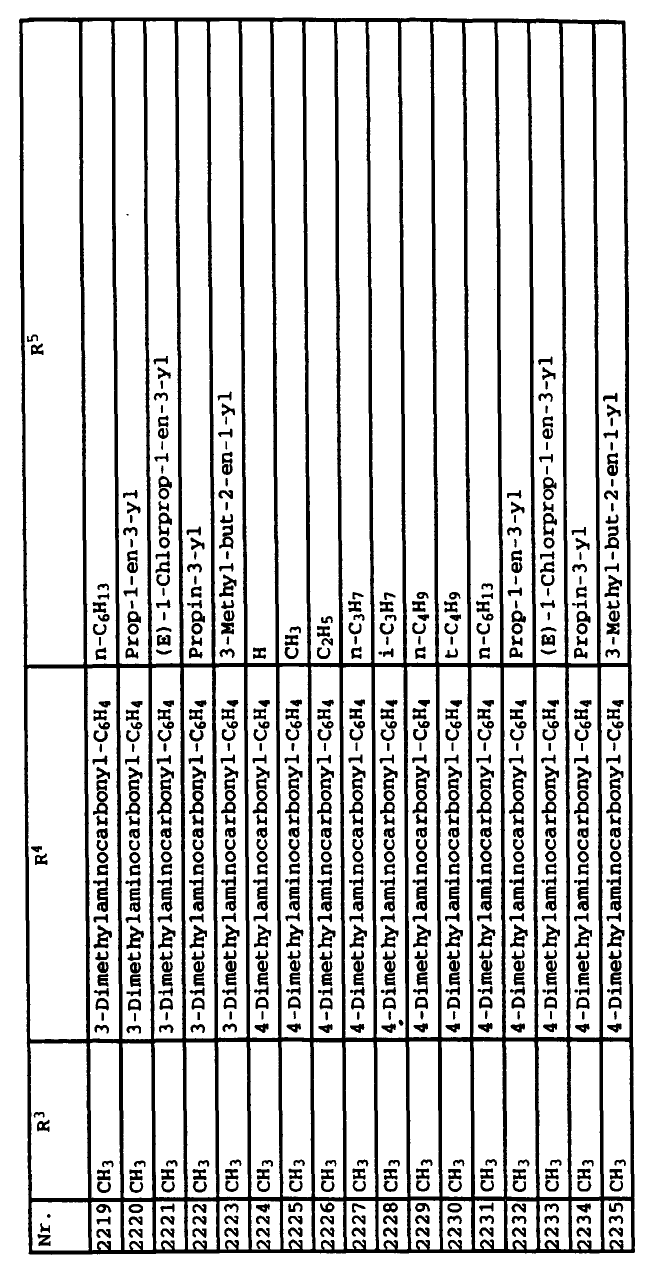

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Reben, Cercospora arachidicola an Erdnüssen, Pseudocercosporella herpotrichaides an Weizen, Gerste, Pyricularia oryzae an Reis, Phytophthora infestans an Kartoffeln und Tomaten, Fusa7rium- und Verticillium-Arten an verschiedenen Pflanzen, Plasmopara viticola an Reben, Alternaria-Arten an Gemüse und Obst.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung des ortho-substituierten Benzylesters einer Cyclopropancarbonsäure gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfestoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidin-methanol, 5-Butyl-2-dimethylamino-4-hydroxy-6-methyl-pyrimidin, Bis-(p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol, sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethyl-phenyl)-N-furoyl(2)-alaninat, DL-N-(2,6-Dimethyl-phenyl)-N-(2'-methoxyacetyl)-alaninmethyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin- 2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-1H-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregnina, Stauronotus maroccanus, Tachycines asynamorus.

Aus der Klasse der Arachnoidea beispielsweise Spinnentiere (Acarina) wie Amblyomma americanum, Amblyonmma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungs formen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulieringen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B. Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylazylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt.

Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6 Gew.-Teilen des Anlagerungsproduktes von 8 bis 10 Mol Ethylenoxid an 1 Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelöst, die aus 60 Gew.-Teilen Cyclohexanon, 30 Gew.-Teilen Isobutanol, 5 Gew.-Teilen des Anlagerungsproduktes von 7 Mol Ethylenoxid an 1 Mol Isooctylphenol und 5 Gew.-Teilen des Anlagerungsproduktes von 40 Mol Ethylenoxid an 1 Mol Ricinusöl besteht (Wirkstoffgehalt 16 Gew.%).
V. 80 Gew.-Teile einer erfindungsgemäßen Verbindung werden mit 3 Gew.-Teilen des Natriumsalzes der Diisobutylnaphthalinalpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-α-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelöst, die aus 40 Gew.-Teilen Cyclohexanon, 30 Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält man eine wäßrige Dispersion, die 0,02 Gew.% des Wirkstoffs enthält.
VIII.20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20 000 Gew.-Teilen Wasser erhält man eine Spritzbrühe, die 0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1 (nicht erfindungsgemäß)

### Darstellung von (E,E)-2-Methoxyimino-2-[2'-(1''-methyl, 1''-acetyl)-iminooxymethyl]phenylessigsäuremethylester

Zu 6,4 g (0,21 mol) Natriumhydrid (80 %) in 150 ml trockenem Dimethylformamid gibt man unter Schutzgas und leichtem Kühlen bei Raumtemperatur 21 g (0,21 mol) Diacetylmonoxim und rührt den Ansatz 30 min bei Raumtemperatur. Anschließend wird eine Lösung von 60 g (0,21 mol) 2-Methoxyimino-2-(2'brommethyl)phenylessigsäuremethylester in 360 ml Dimethylformamid zugetropft und 16 h bei Raumtemperatur gerührt. Nach Zugabe von 10 %iger Salzsäure wird mit Methyl-tert.-butyl-ether extrahiert. Die vereinigten organischen Phasen werden mit Wasser gewaschen, über Na₂SO₄ getrocknet und eingeengt. Der Rückstand wird in wenig kaltem Methanol suspendiert. Nach Absaugen erhält man 38 g (59 %) der Titelverbindung als hellbraune Kristalle mit einem Schmelzpunkt von 69 - 71°C.
¹H-NMR (CDCl₃):δ= 1,87(s,3H); 2,30(s,3H); 3,85(s,3H); 4,05(s,3H); 5,15(s,2H); 7,17-7,48(m,4H) ppm.

### Beispiel 2 (nicht erfindungsgemäß)

### Darstellung von (E,E,E)-2-Methoxyimino-2-[2'-(1''-methyl, 1''-(1'''-ethoxyiminoethyl))iminooxymethyl]phenylessigsäuremethylester

Zur Lösung von 2,5 g (8,2 mmol) (E,E)-2-Methoxyimino-2-[2'-(1''-methyl, 1''-acetyl)iminooxymethyl]phenylessigsäuremethylester in 60 ml warmen Methanol gibt man nach Abkühlen auf Raumtemperatur 0,96 g (9,8 mmol) O-Ethylhydroxylamin-Hydrochlorid sowie 0,6 g trockene Molekularsiebperlen (3 A) und läßt 5 Tage bei Raumtemperatur stehen. Nach Abfiltrieren des Molekularsiebes wird die Lösung eingeengt, der Rückstand zwischen Methyl-tert.-butyl-ether und Wasser verteilt, die organische Phase mit Wasser gewaschen, über Na₂SO₄ getrocknet und eingeengt. Nach Verreiben des Rückstandes mit n-Hexan und Absaugen erhält man 1,8 g (63 %) der Titelverbindung als hellgelbe Kristalle mit einem Schmelzpunkt von 69 - 72°C.
¹H-NMR (CDCl₃):δ= 1,27(t,3H); 1,96(s,3H); 1,99(s,3H); 3,84(s,3H); 4,04(s,3H); 4,17(q,2H); 5,06(s,2H); 7,17-7,49(m,4H) ppm

### Beispiel 3 (nicht erfindungsgemäß)

### Darstellung von (E,E,E)-2-Methoxyimino-2-[2'-(1''-methyl, 1''-(1'''-hydroxyiminoethyl))iminooxymethyl]phenylessigsäuremethylester

Zu 0,60 g (20 mmol) Natriumhydrid (80 %) in 14 ml trockenem Dimethylformamid gibt man portionsweise 2,0 g (17 mmol) Diacetyldioxim und läßt 30 min bei Raumtemperatur rühren. Anschließend gibt man eine Lösung von 5,0 g (17 mmol) 2-Methoxyimino-2-(2'-brommethyl)phenylessigsäuremethylester in 30 ml Dimethylformamid zu und läßt 2 h bei Raumtemperatur rühren. Nach Zugabe von 10 %iger Salzsäure wird mit Methyl-tert.-butyl-ether extrahiert. Die vereinigten organischen Phasen werden mit Wasser gewaschen, über Na₂SO₄ getrocknet und eingeengt. Nach Verreiben des Rückstandes mit Methanol wird abgesaugt und das Filtrat nach Einrotieren säulenchromatographisch an Kieselgel (Methyl-tert.-butyl-ether/n-Hexan) gereinigt. Man erhält so 1,0 g (18 %) der Titelverbindung als weißes Pulver vom Schmelzpunkt 107-111°C.
¹H-NMR (CDCl₃):δ = 1,97(s,3H); 2,00(s,3H); 3,84(s,3H); 4,05(s,3H); 5,08(s,2H); 7,17-7,45(m,4H), 8,56(s,1H) ppm.

### Beispiel 4

### Darstellung von 4-Hydroxyimino-2,2-dimethylpentan-3-on

Zu 96 g (0,84 mol) 2,2-Dimethyl-3-pentanon in 960 g Toluol wird eine Lösung aus 40 g Chlorwasserstoff in 156 g Diethylether bei Raumtemperatur zugetropft. Nach Abkühlen auf -10°C wird eine Lösung von 95 g n-Butylnitrit in 470 g Diethylether zugetropft. Man läßt 4 Stunden bei -10°C bis 0°C rühren und läßt anschließend auf Raumtemperatur kommen. Nach insgesamt 16 h wird der Reaktionsansatz dreimal mit je 1 l Eiswasser gewaschen und anschließend zweimal mit je 1 l 1M-Natronlauge extrahiert. Die alkalische Phase wird abgetrennt und mit 20 %iger Schwefelsäure neutralisiert. Das Rohprodukt wird abgesaugt und nach dem Trocknen aus n-Hexan umkristallisiert. Man erhält 66 g (55 %) der Titelverbindung als hellgelbes Pulver vom Schmelzpunkt 107 - 110°C.
¹H-NMR (CDCl₃):δ= 1,29(s,9H); 1,99(s,3H); 8,30(s,1H) ppm.

### Beispiel 5 (nicht erfindungsgemäß)

### Darstellung von (E)-2-Methoxyimino-2-[2'-(1''-methyl, 1''-(1''',1'''-dimethylethylcarbonyl))iminooxymethyl]phenylessigsäuremethylester

Zu 6,4 g (0,21 mol) Natriumhydrid (80 %) in 150 ml trockenem Dimethylformamid gibt man unter Schutzgas portionsweise 25 g (0,17 mol) 4-Hydroxyimino-2,2-dimethylpentan-3-on, wobei sich das Reaktionsgemisch bis auf 50°C erwärmt. Man läßt 30 min nachrühren, tropft anschließend eine Lösung von 50 g (0,17 mol) 2-Methoxyimino-2-(2'-brommethyl)phenylessigsäuremethylester in 300 ml Dimethylformamid zu und läßt 16 h bei Raumtemperatur rühren. Nach Zugabe von 10 %iger Salzsäure wird mit Methyl-tert.-butyl-ether extrahiert. Die vereinigten organischen Phasen werden mit Wasser gewaschen, über Na₂SO₂ getrocknet und eingeengt. Der schwarze ölige Rückstand wird säulenchromatographisch an Kieselgel (Methyl-tert-butyl-ether/n-Hexan) gereinigt und das so erhaltene Rohrprodukt in eiskaltem Methanol suspendiert. Nach Absaugen erhält man 24 g (41 %) der Titelverbindung als fast farbloses Pulver vom Schmelzpunkt 58 - 62°C.
¹H-NMR (CDCl₃):δ= 1,19(s,9H); 1,90(s,3H); 3,83(s,3H); 4,04(s,3H); 5,11(s,2H); 7,18-7,45(m,4H) ppm.

### Beispiel 6

### Darstellung von (E)-2-Methoxyimino-2-[2'-(1''-methyl, 1''-(1'''-(6''''-(4'''''-chlorphenyl)hexyloxyimino), 2''', 2'''-dimethylpropyl))iminooxymethyl]phenylessigsäuremethylester

Zur Lösung von 3,0 g (8,6 mmol) (E)-2-Methoxyimino-2-[2'-(1''-methyl, 1''-(1''', 1'''-dimethylethylcarbonyl))-iminooxymethyl]-phenylessigsäuremethylester in 60 ml warmen Methanol gibt man nach Abkühlen auf Raumtemperatur 5,9 g (26 mmol) O-6-(4'-Chlorphenyl)-hexylhydroxylamin, 3,6 g trockene Molekularsiebperlen (3 A) sowie 1,6 g (8,6 mmol) p-Toluolsulfonsäurehydrat und erhitzt 3 h am Rückfluß. Nach Abfiltrieren des Molekularsiebes wird die Lösung eingeengt, der Rückstand zwischen Methyl-tert.-butyl-ether und Wasser verteilt, die organische Phase mit Wasser gewaschen, über Na₂SO₄ getrocknet und eingeengt. Nach Säulenchromatographie an Kieselgel (Hexan/Methyl-tert.-butylether) erhält man 3,8 g (79 %) der Titelverbindung als hellgelbes Öl.
¹H-NMR (CDCl₃):δ= 1,09(s,9H); 1,26-1,42(m,4H); 1,52-1,67(m,4H); 1,90(s,3H); 2,57(t,2H); 3,84(s,3H); 3,99(t,2H); 4,03(s,3H); 5,02(s,2H); 7,07-7,47(m,8H) ppm.

### Beispiel 7

### Darstellung von (E)-2-Methoxyimino-2-[2'-(1''-methyl, 1''-methoxycarbonyl)iminooxymethyl]phenylessigsäuremethylester

Zu 2,0 g (67 mmol) Natriumhydrid (80 %) in 100 ml trockenem Dimethylformamid gibt man portionsweise 6,1 g (52 mmol) 2-Hydroxyiminopropionsäuremethylester, wobei sich das Reaktionsgemisch bis auf 50°C erwärmt. Man läßt 30 min nachrühren, tropft anschließend eine Lösung von 15 g (52 mmol) 2-Methoxyimino-2-(2'-brommethyl)phenylessigsäuremethylester in 90 ml Dimethylformamid zu und läßt 16 h bei Raumtemperatur rühren. Nach Zugabe von 10 %iger Salzsäure wird mit Methyl-tert.-butyl-ether extrahiert. Die vereinigten organischen Phasen werden mit Wasser gewaschen, über Na₂SO₄ getrocknet und eingeengt. Der Rückstand wird in eiskaltem Methanol suspendiert. Nach Absaugen erhält man 7,2 g (43 %) der Titelverbindung als beigefarbenes Pulver vom Schmelzpunkt 78-82°C.
¹H-NMR (CDCl₃):δ= 2,04(s,3H); 3,85(s,3H); 3,86(s,3H); 5,19(s,2H); 7,16-7,49(m,4H) ppm.

### Beispiel 8 (nicht erfindungsgemäß)

### Darstellung von 2-[2'-(1''-Methyl, 1''-(1'''-methoxyimino, 1'''-phenyl)-methyl)iminooxymethyl]-phenyl-3-methoxy-prop-E-2-ensäuremethylester

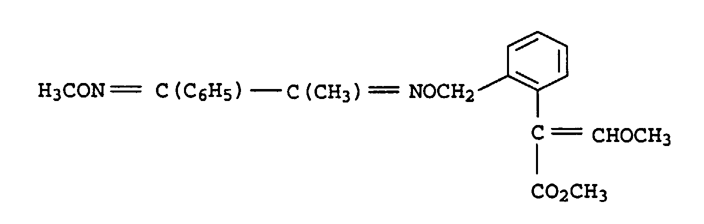

Zu einer Lösung von 2,5 g (7 mmol) 2-[2'-(1''-Methyl, 1''-benzoyl)iminooxymethyl]-phenyl-3-methoxy-prop-E-2-ensäuremethylester in 100 ml Methanol gibt man 2,4 g (28 mmol) O-Methylhydroxylamin-Hydrochlorid sowie 3 g trockenes Molekularsieb (3 Å). Man läßt die Reaktionslösung 3 Tage bei Raumtemperatur stehen und rührt dann 8 Stunden bei 60°C nach.

Nach Abfiltrieren des Molekularsiebes gibt man die Lösung auf 200 ml Wasser und extrahiert mit Methyl-tert-Butylether.

Die vereinigten organischen Extrakte werden mit Wasser gewaschen, über Na₂SO₄ getrocknet und eingeengt. Nach Säulenchromatographie an Kieselgel (n-Hexan / Methyl-tert-Butylether 9:1) erhält man 1,1 g (40 %) der Titelverbindung in Form eines Isomerengemisches (Isomerie in Seitenkette, Isomere 1:10). Farbloses Öl.
IR [cm⁻¹] (Film)
   768, 1036, 1057, 1111, 1130, 1190, 1256, 1284, 1634, 1709, 2930

### Beispiel 9

### Darstellung von α-[2'-(1''-Methyl, 1''-(1'''-methoxyimino, 1'''-phenyl)-methyl)iminooxymethyl]-phenyl-E-β-methylacrylsäuremethylester

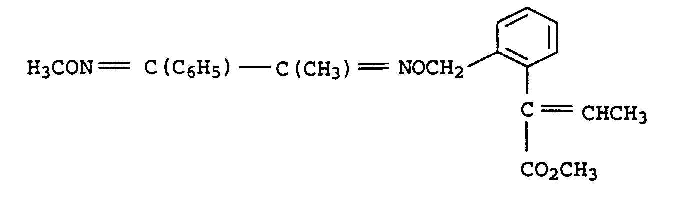

Zu einer Lösung von 1 g (2,9 mmol) α-[2'-(1''-Methyl, 1''-benzoyl)iminooxymethyl]-phenyl-E-β-methylacrylsäuremethylester in 50 ml Methanol gibt man 1 g (11,4 mmol) O-Methylhydroxylamin-Hydrochlorid sowie 2 g trockenes Molekularsieb (3 Å). Man läßt die Lösung zunächst 2 Stunden bei Raumtemperatur stehen, erhitzt dann 6 Stunden auf 60°C. Nach Abfiltrieren des Molekularsiebes versetzt man mit Wasser und extrahiert mit Methyl-tert-Butylether. Die vereinigten organ. Extrakte werden mit Wasser gewaschen, über Na₂SO₄ getrocknet und eingeengt.

Nach Säulenchromatographie an Kieselgel (n-Hexan / Methyl-tert-Butylether 9:1) erhält man 0,9 g (85 %) der Titelverbindung in Form eines Isomerengemisches (1:1, Isomerie in Seitenkette). Farbloses Öl.
- IR [cm⁻¹] (Film) :: 693, 764, 872, 892, 1005, 1035, 1207 1253, 1435, 1716, 2920

### Beispiel 10 (nicht erfindungsgemäß)

Isomerisierung von (E)-2-Methoxyimino-2-[2'-(1''-Methyl, 1''-(1'''(2)-methoxyimino, 1'''-phenyl)-methyl)(E)-iminooxymethyl]-phenylessigsäuremethylester (Tab I, Verb. I 48) zu (E)-2-Methoxyimino-2-[2'-(1''-Methyl, 1''-(1'''-(E)-methoxyimino, 1'''-phenyl)-methyl](E)-iminooxymethyl]-phenylessigsäuremethylester:
46 g der Ausgangsverbindung werden in 600 ml Diethylether suspendiert, mit 200 ml gesättigter ether. HCl versetzt und 19 Stunden bei Raumtemperatur stehengelassen. Man gibt die Reaktionslösung auf Eiswasser, extrahiert mit Dichlormethan und trocknet über Na₂SO₄. Nach Einengen am Rotationsverdampfer erhält man einen öligen Rückstand. Das gewünschte (E,E,E)-Isomer kristallisiert bei Zugabe von Methanol in Form eines weißen Feststoffes aus (35 g ≙ 75 % d.Th.)
Smp: 118-120°C (95 % E,E,E)

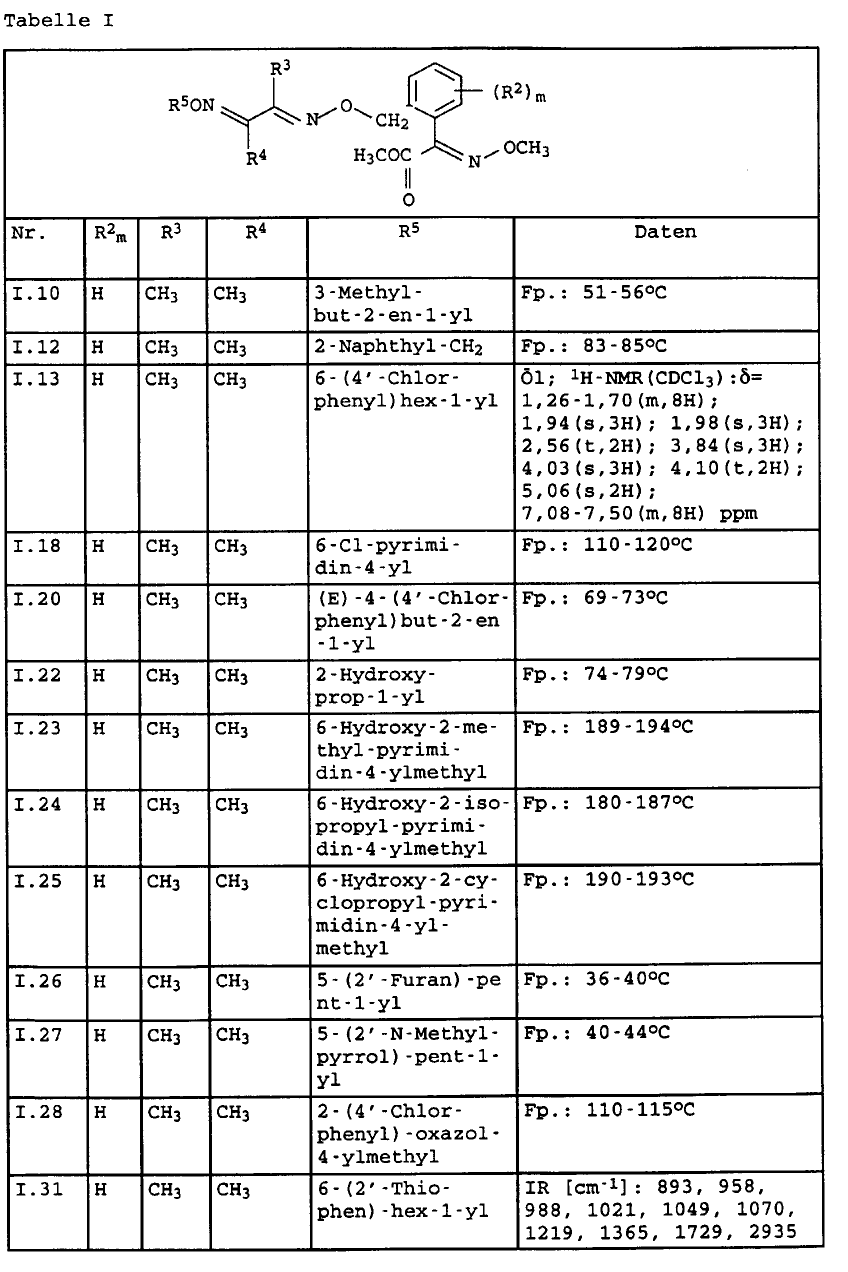

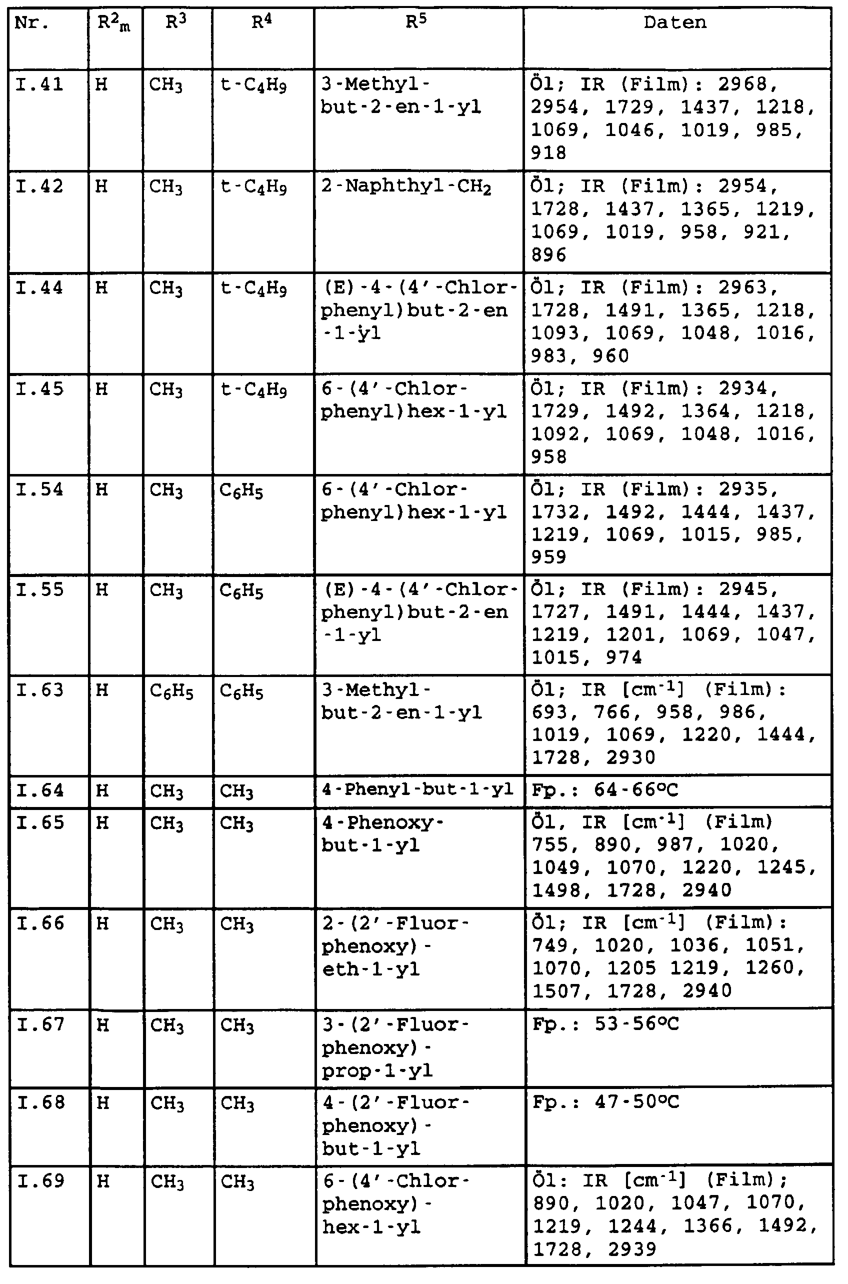

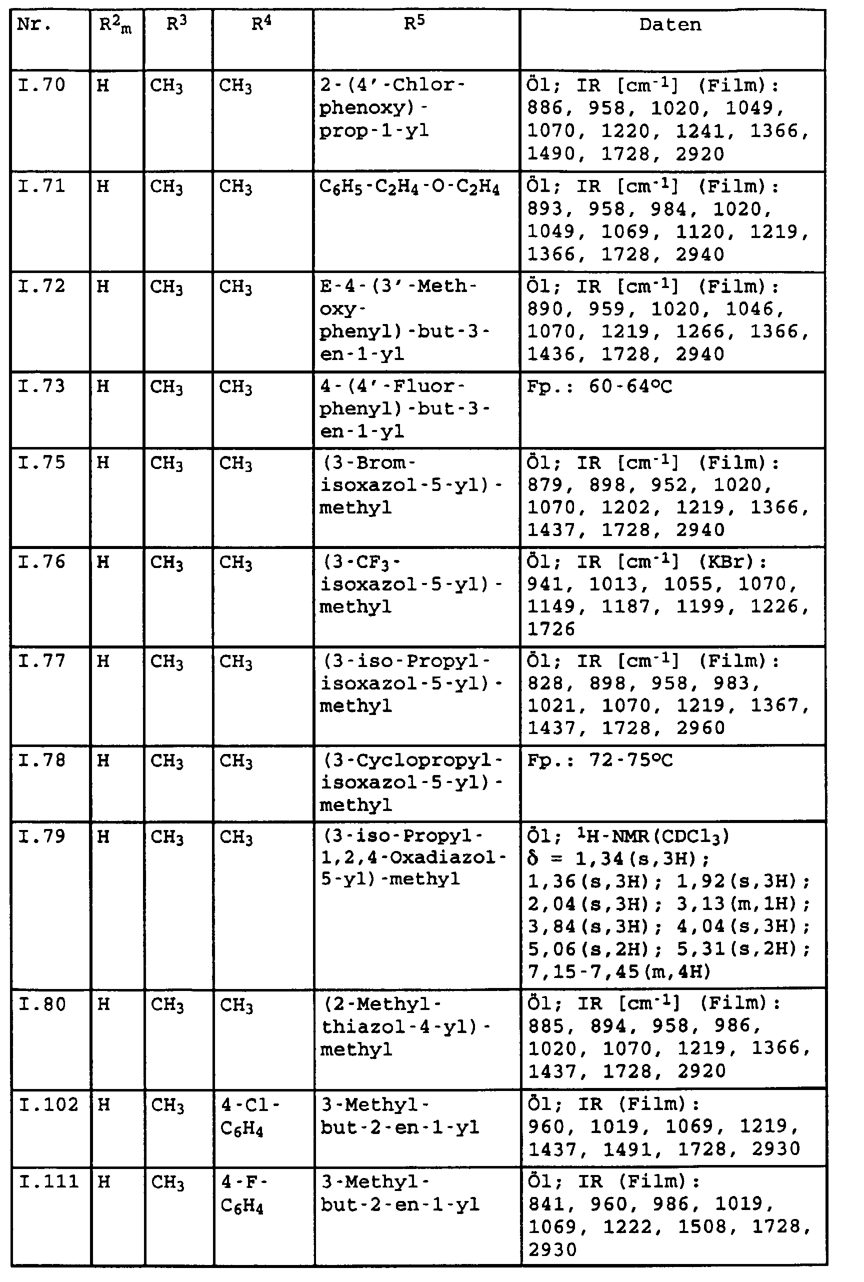

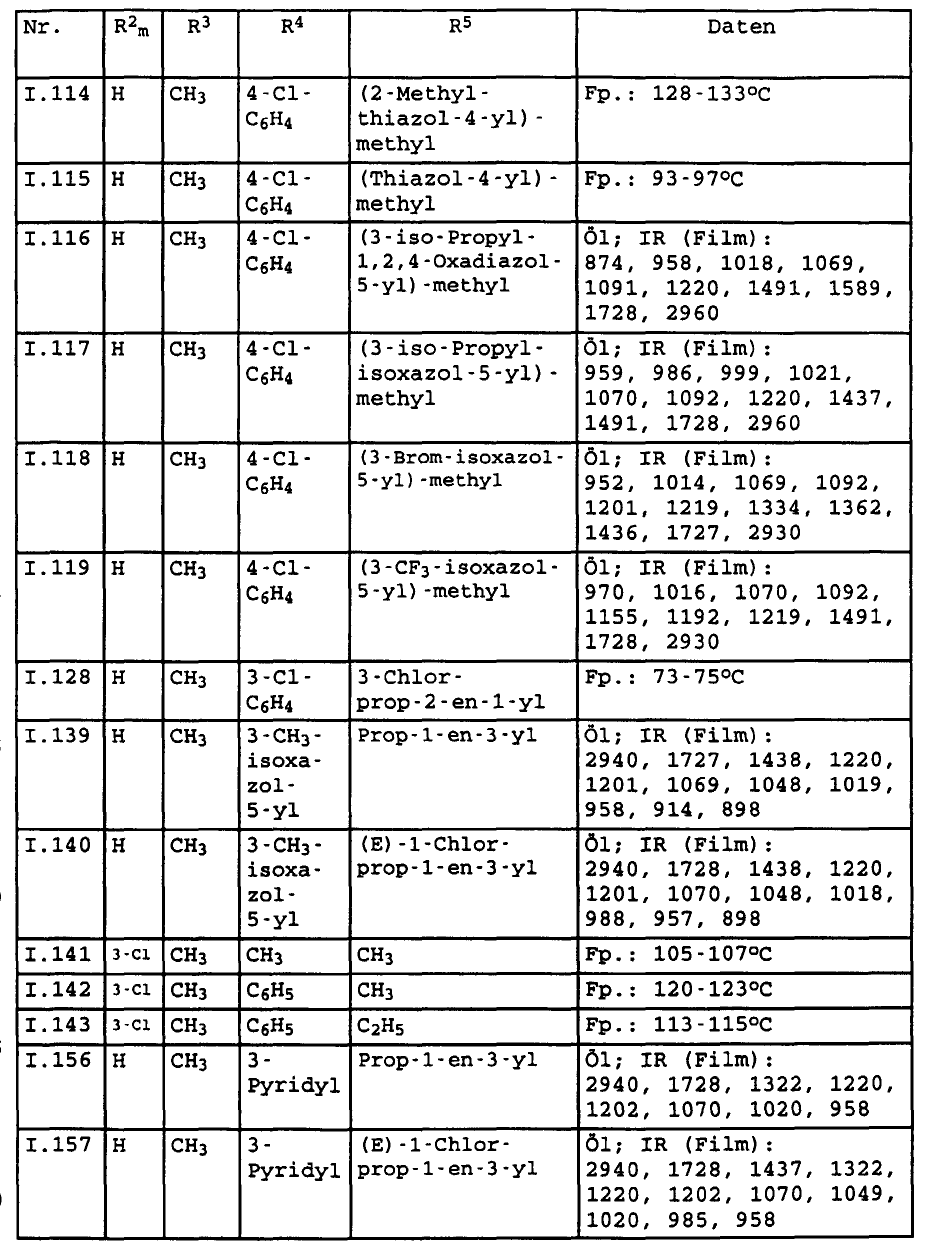

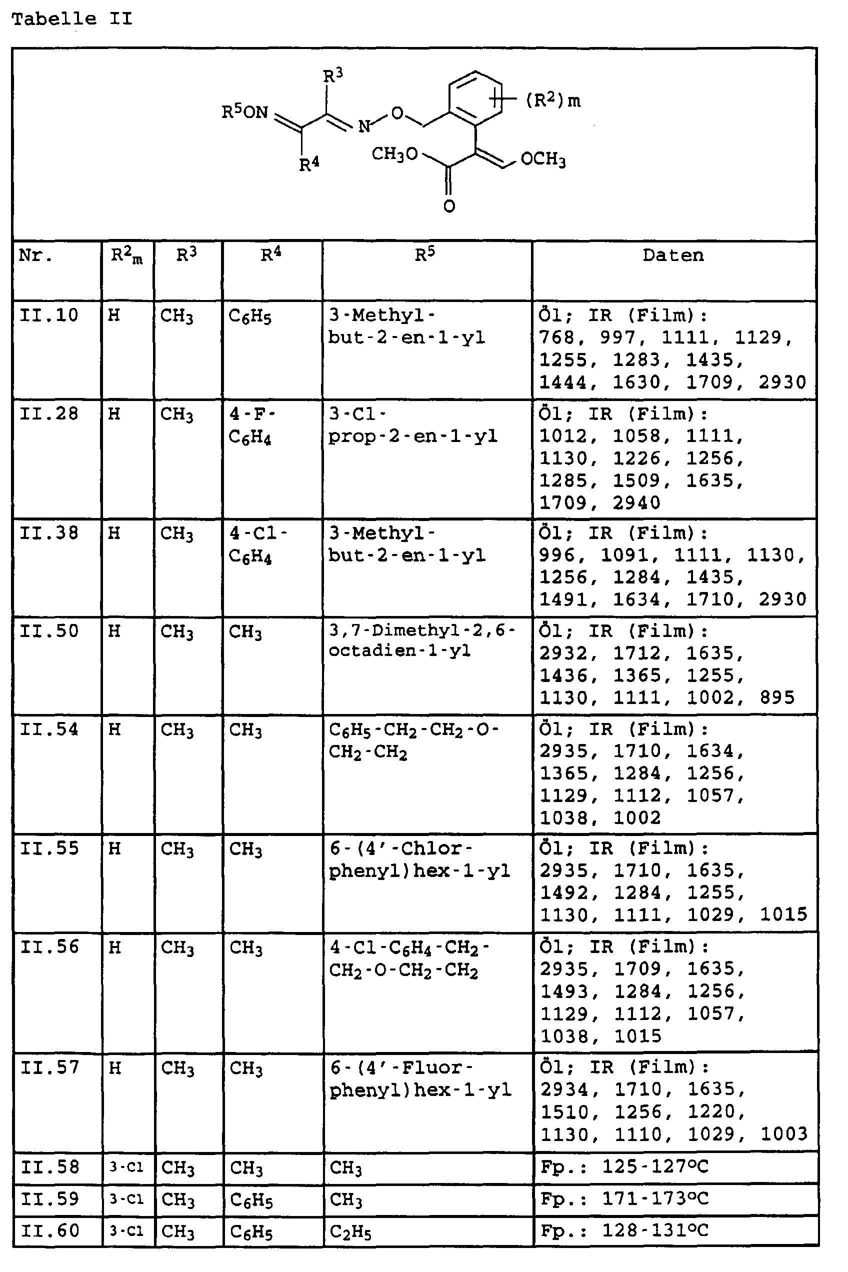

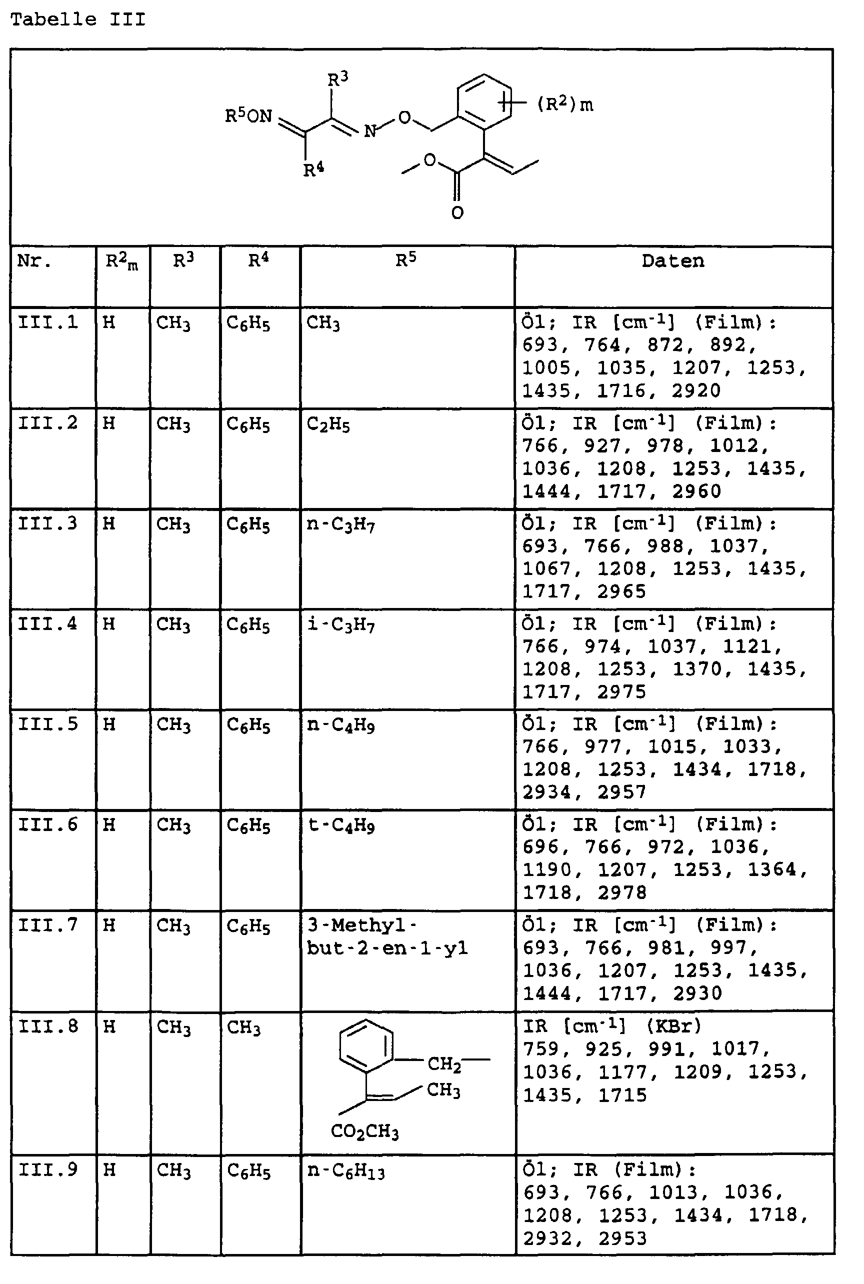

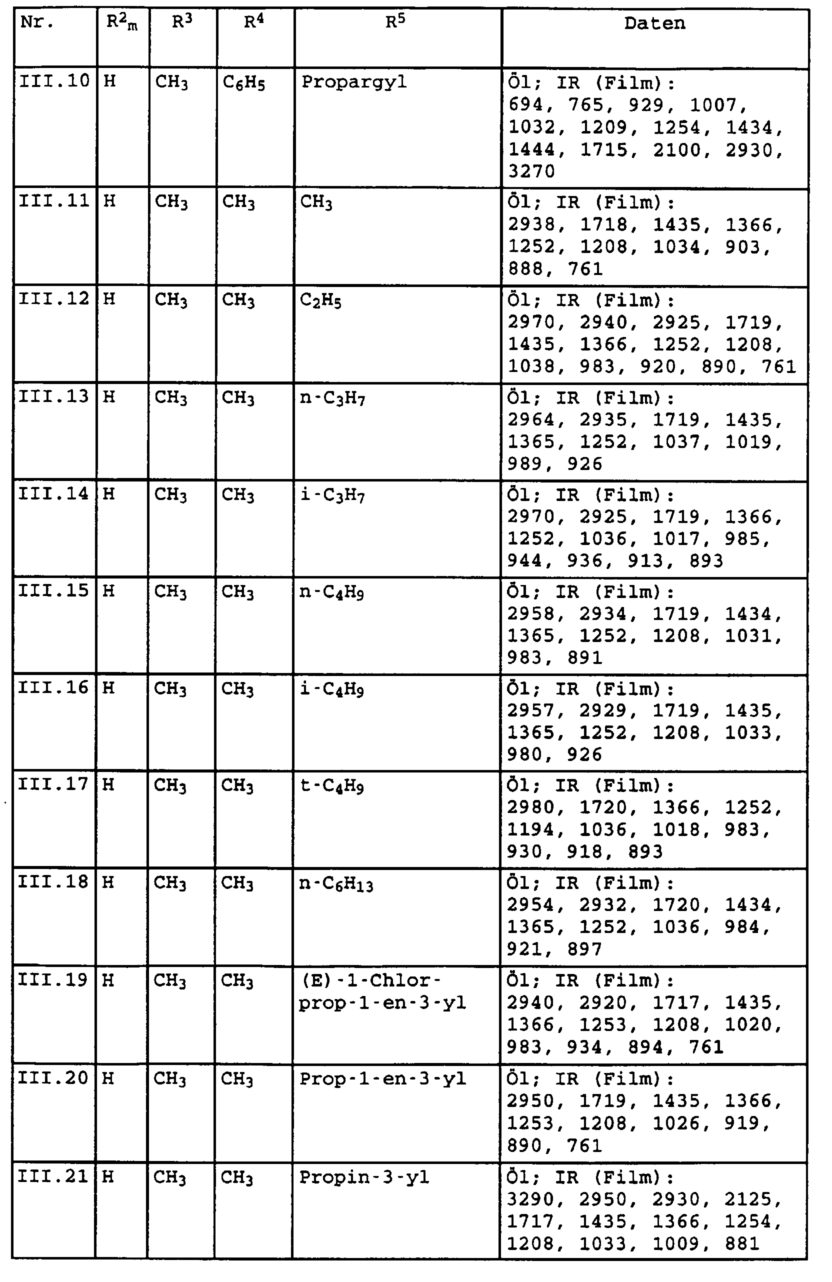

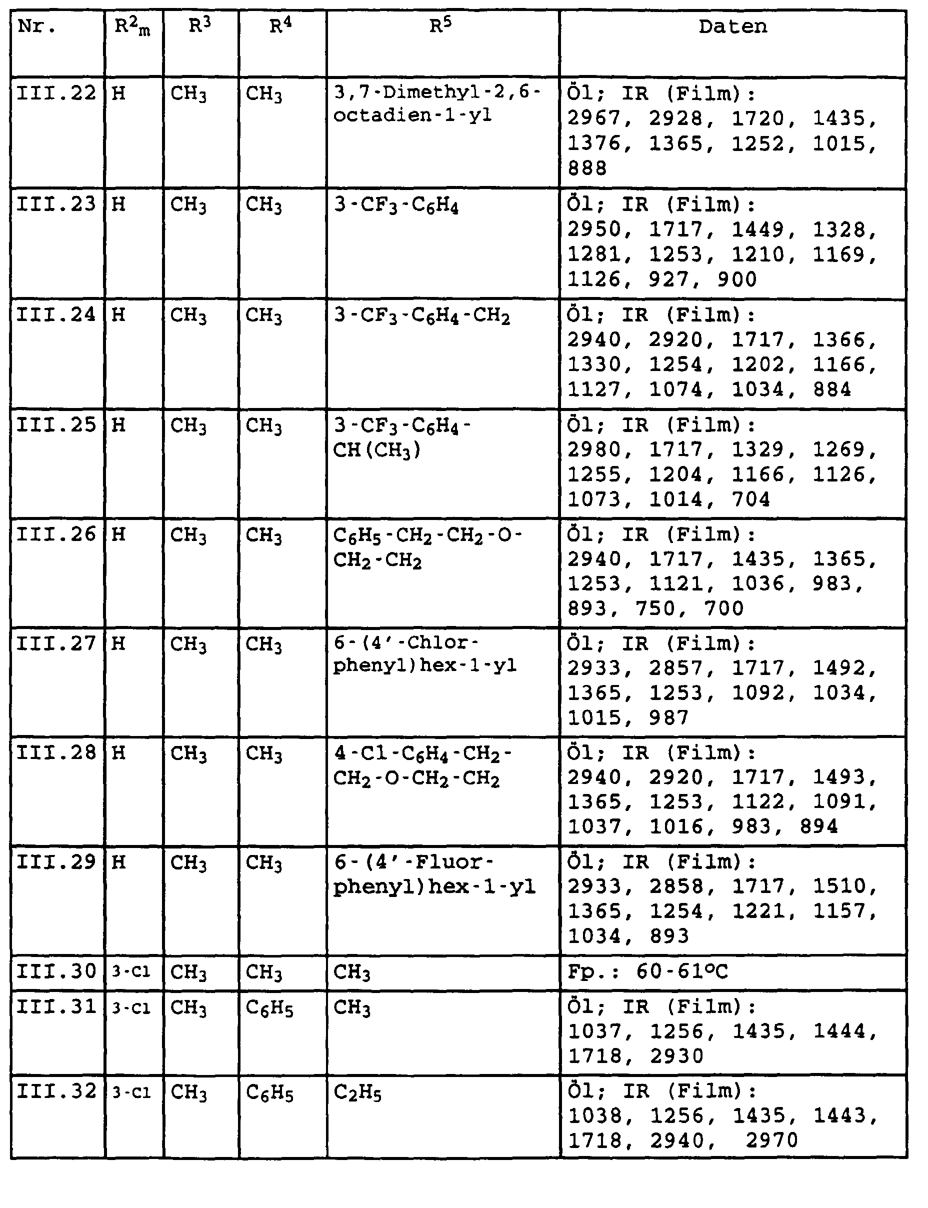

### Beispiele zur Wirkung gegen Schadpilze

Die fungizide Wirkung der Verbindungen der allgemeinen Formel I ließ sich durch folgende Versuche zeigen:

Die Wirkstoffe wurden als 20 %-ige Emulsion in einem Gemisch aus 70 Gew.-% Cyclohexanon, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL (Emulan® EL, Emulgator auf der Basis ethoxylierter Fettalkohole) aufbereitet und entsprechend der gewünschten Konzentration mit Wasser verdünnt.

### 1. Erysiphe graminis var. tritici

Blätter von Weizenkeimlingen (Sorte "Kanzler") wurden zunächst mit der wäßrigen Aufbereitung der Wirkstoffe (250 ppm-haltig) behandelt. Nach ca. 24 h wurden die Pflanzen mit Sporen des Weizenmehltaus (Erysiphe graminis var. tritici) bestäubt. Die so behandelten Pflanzen wurden anschließend für 7 Tage bei 20-22°C und einer relativen Luftfeuchtigkeit von 75-80 % inkubiert. Anschließend wurde das Ausmaß der Pilzentwicklung ermittlelt.

In diesem Test zeigten die mit den erfindungsgemäßen Verbindungen behandelten Pflanzen einen Befall von 15 % und weniger, die mit einem bekannten Wirkstoff (Verbindung Nr. 195, Tabelle 3, EP-A 463 488) behandelten Pflanzen 40 % Befall und die unbehandelten Pflanzen 70 % Befall.

In einem entsprechenden Test (Weizenkeimlinge der Sorte "Kanzler", Aufwandmenge 63 ppm), bei dem die Pflanzen zunächst infiziert und incubiert wureden und anschließend mit den Wirkstoffen behandelt wurden zeigten die mit den erfindunsgemäßen Verbindungen behandelten Pflanzen einen Befall von 5 % und weniger, die mit einem bekannten Wirkstoff (Verbindung Nr. 195, Tabelle 3, EP-A 463 488) behandelten Pflanten 25 % Befall und die unbehandelten Pflanzen 60% Befall.

### Beispiele zur Wirkung gegen tierische Schädlinge

Die insektizide Wirkung der Verbindungen der allgemeinen Formel I ließ sich durch folgende Versuche zeigen:

Die Wirkstoffe wurden
a) als 0,1 %-ige Lösung in Aceton oder
b) als 10 %-ige Emulsion in einem Gemisch aus 70 Gew.-% Cyclohexanol, 20 Gew.-% Nekanil® LN (Lutensol® AP6, Netzmittel mit Emulgier- und Dispergierwirkung auf der Basis ethoxylierter Alkylphenole) und 10 Gew.-% Emulphor® EL, Emulgator auf der Basis ethoxylierter Fettalkohole)
aufbereitet und entsprechend der gewünschten Konzentration mit Aceton im Fall von a) bzw. mit Wasser im Fall von b) verdünnt.

Nach Abschluß der Versuche wurde die jeweils niedrigste Konzentration ermittelt, bei der die Verbindungen im Vergleich zu unbehandelten Kontrollversuchen noch eine 80 - 100 %-ige Hemmung bzw. Mortalität hervorriefen (Wirkschwelle bzw. Minimalkonzentration).

### Aphis fabae (Schwarze Laus), Kontaktwirkung

Stark befallene Buschbohnen (Vicia faba) wurden mit der wässrigen Wirkstoffaufbereitung behandelt. Nach 24 h wurde die Mortalitätsrate bestimmt.

In diesem Test zeigten die erfindungsgemäßen Verbindungen I.67, I.68, I.71, I.72, I.73, III.01, III.02 und III.10 Wirkschwellen von 400 ppm und weniger.

### Nephotettix cincticeps (Grüne Reiszikade), Kontaktwirkung

Rundfilter wurden mit der wäßrigen Wirkstoffaufbereitung behandelt und anschließend mit 5 adulten Zikaden belegt. Nach 24 h wurde die Mortalität beurteilt.

In diesem Test zeigten die erfindungsgemäßen Verbindungen I.68 und III.02 Wirkschwellen von 0,4 mg und weniger.

### Prodenia litura (Ägyptischer Baumwollwurm), Kontaktwirkung

Mit der wäßrigen Wirkstoffaufbereitung behandelte Filter wurden mit 5 Raupen belegt. Die erste Beurteilung erfolgt nach 4 h. Sofern noch mindestens eine Raupe lebt, wird eine Futtermischung zugegeben. Nach 24 h wurde die Mortalität bestimmt.

In diesem Test zeigten die erfindungsgemäßen Verbindungen II.28 und III.10 Wirkschwellen von 0,4 mg und weniger.

### Tetranychus telarius (Rote Spinne), Kontaktwirkung

Getopfte Buschbohnen, die das zweite Folgeblattpaar zeigten, wurden mit wässrigen Wirkstoffaufbereitung behandelt. Nach 24 h wurden die Pflanzen mit Hilfe von stark befallenen Blattstücken infiziert. Nach 12 Tagen im Gewächshaus wurde der Befall bestimmt.

In diesem Test zeigten die Verbindungen II.10 und II.28 Wirkschwellen von 400 ppm und weniger.

## Patentansprüche

1. Phenylessigsäurederivate der Formel I 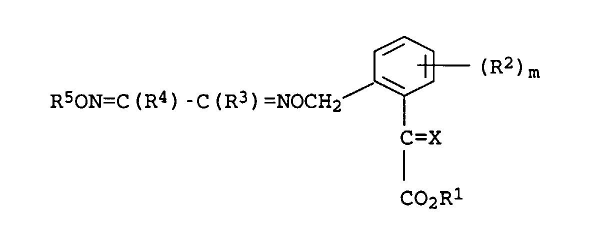 in der die Substituenten und der Index die folgende Bedeutung haben:
X NOCH₃, CHOCH₃, CHCH₃ und CHCH₂CH₃;
R¹ Wasserstoff und C₁-C₄-Alkyl;
R² Cyano, Nitro, Trifluormethyl, Halogen, C₁-C₄-Alkyl und C₁-C₄-Alkoxy;
m 0, 1 oder 2, wobei die Reste R² verschieden sein können, wenn m für 2 steht;
R³ Wasserstoff, Cyano, Nitro, Hydroxy, Amino, Halogen,
C₁-C₄-Alkyl, C₁-C₄-Halogenalkyl, C₁-C₄-Alkoxy, C₁-C₄-Halogenalkoxy, C₁-C₄-Alkylthio, C₁-C₄-Alkylamino oder Di-C₁-C₄-alkylamino;
R⁴ Wasserstoff, Cyano, Nitro, Hydroxy, Amino, Halogen,
C₁-C₆-Alkyl, C₁-C₆-Alkoxy, C₁-C₆-Alkylthio, C₁-C₆-Alkylamino, Di-C₁-C₆-alkylamino, C₂-C₆-Alkenyl, C₂-C₆-Alkenyloxy, C₂-C₆-Alkenylthio, C₂-C₆-Alkenylamino, N-C₂-C₆-Alkenyl-N-C₁-C₆-alkylamino, C₂-C₆-Alkinyl, C₂-C₆-Alkinyloxy, C₂-C₆-Alkinylthio, C₂-C₆-Alkinylamino, N-C₂-C₆-Alkinyl-N-C₁-C₆-alkylamino, wobei die Kohlenwasserstoffreste dieser Gruppen partiell oder vollständig halogeniert sein können oder einen bis drei der folgenden Reste tragen können: Cyano, Nitro, Hydroxy, Merkapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR⁶)-Aₙ-R⁷;
C₃-C₆-Cycloalkyl, C₃-C₆-Cycloalkyloxy, C₃-C₆-Cycloalkylthio, C₃-C₆-Cycloalkylamino, N-C₃-C₆-Cycloalkyl-N-C₁-C₆-alkylamino, C₃-C₆-Cycloalkenyl, C₃-C₆-Cycloalkenyloxy, C₃-C₆-Cycloalkenylthio, C₃-C₆-Cycloalkenylamino, N-C₃-C₆-Cycloalkenyl-N-C₁-C₆-alkylamino, Heterocyclyl, Heterocyclyloxy, Heterocyclylthio, Heterocyclylamino, N-Heterocyclyl-N-C₁-C₆-alkylamino, Aryl, Aryloxy, Arylthio, Arylamino, N-Aryl-N-C₁-C₆-alkylamino, Hetaryl, Hetaryloxy, Hetarylthio, Hetarylamino, N-Hetaryl-N-C₁-C₆-alkylamino, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
R⁵ Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶) -Aₙ-R⁷;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl. Hetaryloxy oder C(=NOR⁶)-Aₙ-R⁷;
wobei
A für Sauerstoff, Schwefel oder Stickstoff steht und wobei der Stickstoff Wasserstoff oder C₁-C₆-Alkyl trägt;
n 0 oder 1 bedeutet;
R⁶ Wasserstoff oder C₁-C₆-Alkyl bedeutet und
R⁷ Wasserstoff oder C₁-C₆-Alkyl bedeutet,
wobei X nicht CHOCH₃ oder NOCH₃ bedeutet, wenn
der Index m Null bedeutet, und
R³ Wasserstoff, Cyano, C₁-C₄-Alkyl, C₁-C₄-Halogenalkyl oder Methylthio,
R⁴ Wasserstoff, unsubstituiertes C₁-C₆-Alkyl, unsubstituiertes C₃-C₆-Cycloalkyl, Thienyl oder
Phenyl, das ggf. substituiert ist durch:
Halogen, Cyano, Nitro, C₁-C₄-Alkyl, C₁-C₄-Alkoxy, C₁-C₂-Halogenalkyl, C₁-C₂-Halogenalkoxy, C₃-C₆-Alkenyloxy, C₃-C₆-Alkinyloxy, C₃-C₄-Alkylendioxy; oder
folgende über Sauerstoff, C₁-C₄-Alkoxy, C₁-C₄-Oxyalkyl, S(O)ₙ, C₁-C₄-Alkyl-S(O)ₙ, S(O)ₙ-C₁-C₄-Alkyl, wobei n für 0, 1 oder 2 steht, gebundene Reste:
C₁-C₆-Alkyl, C₁-C₆-Halogenalkyl, C₃-C₆-Cycloalkyl oder C₂-C₆-Alkenyl oder C₂-C₄-Alkinyl-C₁-C₂-alkyl, wobei diese Reste 1 bis 3-fach halogeniert sein können; oder
ggf. durch Halogen, Trimethylsilyl, C₁-C₆-Alkyl, C₁-C₆-Halogenalkyl, C₁-C₆-Alkoxy, C₁-C₆-Halogenalkoxy, ggf. substituiertes Cyclopropyl oder Cyclopropyl-C₁-C₃-alkyl substituierte Aryl- oder Heterocyclylreste;
und
R⁵ Wasserstoff, C₁-C₆-Alkyl, C₁-C₆-Halogenalkyl; C₁-C₄-Alkoxy-C₁-C₂-alkyl, ggf. 1- bis 3-fach halogeniertes C₂-C₄-Alkenyl-C₁-C₂-alkyl, C₂-C₄-Alkinyl-C₁-C₂-alkyl, ggf. 1- bis 4-fach halogeniertes C₃-C₆-Cycloalkyl, ggf. 1- bis 4-fach halogeniertes C₃-C₆-Cycloalkyl-C₁-C₂-alkyl, Cyano-C₁-C₄-alkyl, C₁-C₄-Alkoxycarbonyl-C₁-C₂-alkyl; Phenyl-C₁-C₃-alkyl, das substituiert sein kann durch: Halogen, Cyano, Nitro, C₁-C₃-Alkyl, C₁-C₄-Alkoxy, C₁-C₄-Halogenalkyl, wobei die Phenylgruppe ggf. 1-bis 3-fach durch gleiche oder verschiedene Reste substituiert sein kann;
Phenyl, das ggf. 1- oder 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt;
oder
Pyridyl, das 1- bis 2-fach substituiert sein kann durch: Halogen, Cyano, Nitro, C₁-C₄-Alkyl, C₁-C₄-Alkoxy, C₁-C₂-Halogenalkyl, wobei dieser Rest 1 bis 3 Halogenatome trägt;
oder
R³ Wasserstoff, C₁-C₄-Alkyl oder Cyano,
R⁴ Cyano oder Heterocyclyl und
R⁵ C₁-C₆-Alkyl, C₁-C₆-Halogenalkyl, C₁-C₄-Alkoxy-C₁-C₂-alkyl, unsubstituiertes oder mit 1 bis 3 Halogenatomen substituiertes C₂-C₆-Alkenyl, C₃-C₆-Alkinyl, unsubstituiertes oder mit 1 bis 4 Halogenatomen substituiertes C₃-C₆-Cycloalkyl-C₁-C₄-alkyl bedeuten;
sowie deren Salze.

2. Verbindungen der Formel I gemäß Anspruch 1, in denen R³, R⁴ und R⁵ die folgende Bedeutung haben:
R³ Wasserstoff, Hydroxy, Halogen, C₁-C₄-Alkyl, C₁-C₄-Alkoxy oder C₁-C₄-Alkylthio;
R⁴ Wasserstoff, Hydroxy, Halogen, Cyclopropyl, Cyclohexyl, C₁-C₄-Alkyl, C₁-C₄-Alkoxy, C₁-C₄-Alkylthio,
Aryl oder Hetaryl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁- C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
R⁵ Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di- C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyboxy, Aryl, Aryloxy, Arylthio, Hetaryl, Hetaryloxy und Hetarylthio, wobei die aromatischen und heteroaromatischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁶)-Aₙ-R⁷;
Aryl, Hetaryl, Arylcarbonyl, Hetarylcarbonyl, Arylsulfonyl oder Hetarylsulfonyl, wobei diese Gruppen partiell oder vollständig halogeniert sein können oder ein bis 3 der folgenden Gruppen tragen können: Cyano, Nitro, Hydroxy, Merkapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁶)-Aₙ-R⁷;
sowie deren Salze.

3. Verbindungen der Formel I gemäß Anspruch 1, in denen m für 0 steht.

4. Verbindungen der Formel I gemäß Anspruch 1, in denen R¹ für Methyl steht.

5. Verbindungen der Formel I gemäß Anspruch 1, in denen X CHCH₃ oder CHCH₂CH₃ bedeutet.

6. Verfahren zur Herstellung der Verbindungen der Formel I gemäß Anspruch 1, in denen R³ nicht Halogen bedeutet, dadurch gekennzeichnet, daß man ein Benzylderivat der Formel II 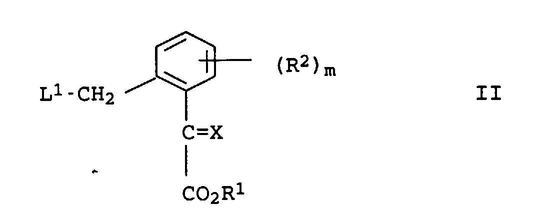 in der L¹ für eine nucleophil austauschbare Abgangsgruppe steht, in an sich bekannter Weise mit einem Hydroxyimim der Formel III
R⁵ON=C(R⁴)-C(R³)=NOH III
umsetzt.

7. Verfahren zur Herstellung der Verbindungen der Formel I gemäß Anspruch 1, in denen R³ und R⁴ nicht Halogen bedeutet, dadurch gekennzeichnet, daß man ein Benzylderivat der Formel II gemäß Anspruch 1 in an sich bekannter Weise mit einem Dihydroxyimim der Formel IV
HON=C(R⁴)-C(R3)=NOH IV
zu einer Verbindung der Formel V 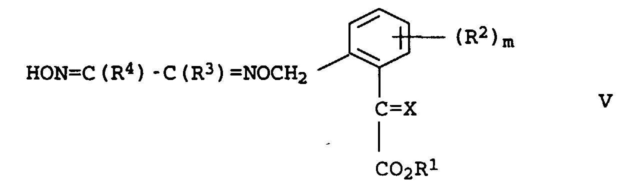 umsetzt und V anschließend mit einer Verbindung der Formel VI
R⁵-L² VI
in der L² für eine nucleophil austauschbare Abgangsgruppe steht, zu I umsetzt.

8. Verfahren zur Herstellung der Verbindungen der Formel I gemäß Anspruch 1, in denen R³ nicht Halogen bedeutet, dadurch gekennzeichnet, daß man ein Benzylderivat der Formel II gemäß Anspruch 1 in an sich bekannter Weise mit einem Carbonylhydroxyimin der Formel VII
O=C(R⁴)-C(R³)=NOH VII
zu einer Verbindung der Formel VIII 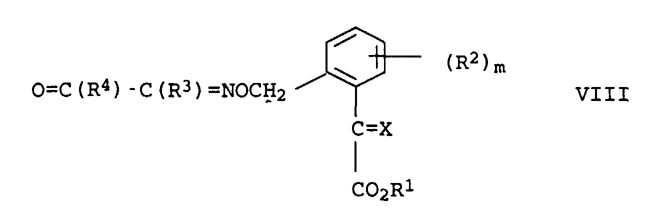 umsetzt, VIII anschließend entweder
a) zunächst mit Hydroxylamin oder dessen Salz und danach mit einer Verbindung der Formel VI (R⁵-L²) gemäß Anspruch 7 oder
b) mit einem Hydroxylamin oder einem Hydroxylammoniumsalz der Formel IXa bzw. IXb 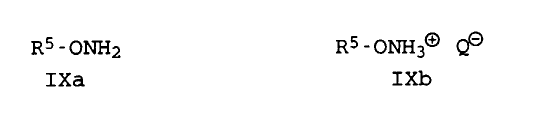 in der Q^{⊖} für das Anion einer Säure steht,
zu I umsetzt.

9. Mittel gegen tierische Schädlinge oder Schadpilze, enthaltend übliche Zusatzstoffe und eine wirksame Menge einer Verbindung der Formel I gemäß Anspruch 1.

10. Mittel nach Anspruch 9 zur Bekämpfung tierischer Schädlinge aus der Klasse der Insekten, Spinntiere oder Nematoden.

11. Verfahren zur Bekämpfung von Schadpilzen, dadurch gekennzeichnet, daß man Schadpilze, deren Lebensraum oder die von ihnen freizuhaltenden Pflanzen, Flächen, Materialien oder Räume mit einer wirksamen Menge einer Verbindung der Formel I gemäß Anspruch 1 behandelt.

12. Verfahren zur Bekämpfung von Schädlingen, dadurch gekennzeichnet, daß man die Schädlinge, deren Lebensraum oder die von ihnen freizuhaltenden Pflanzen, Flächen, Materialien oder Räume mit einer wirksamen Menge einer Verbindung der Formel I behandelt.

13. Verwendung der Verbindungen I gemäß Anspruch 1 zur Bekämpfung von tierischen Schädlingen oder Schadpilzen.

14. Verbindungen der Formel VIII gemäß Anspruch 8.

15. Verwendung der Verbindungen der Formel VIII gemäß Anspruch 8 als Zwischenprodukte.

16. Hydroxyimine der Formel III gemäß Anspruch 6, in der R³ die in Anspruch 1 gegebene Bedeutung hat und
R⁴ Hydroxy, Ethyl, iso-Propyl, ggf. subst. Aryl oder Hetaryl, wobei Thienyl und
Phenyl, das ggf. substituiert ist durch:
Halogen, Cyano, Nitro, C₁-C₄-Alkyl, C₁-C₄-Alkoxy, C₁-C₂-Halogenalkyl, C₁-C₂-Halogenalkoxy, C₃-C₆-Alkenyloxy, C₃-C₆-Alkinyloxy, C₃-C₄-Alkylendioxy; oder
folgende über Sauerstoff, C₁-C₄-Alkoxy, C₁-C₄-Oxyalkyl, S(O)ₙ, C₁-C₄-Alkyl-S(O)ₙ, S(O)ₙ-C₁-C₄-Alkyl, wobei n für 0, 1 oder 2 steht, gebundene Reste:
C₁-C₆-Alkyl, C₁-C₆-Halogenalkyl, C₃-C₆-Cycloalkyl oder C₂-C₆-Alkenyl oder C₂-C₄-Alkinyl-C₁-C₂-alkyl, wobei diese Reste 1 bis 3-fach halogeniert sein können; oder
ggf. durch Halogen, Trimethylsilyl, C₁-C₆-Alkyl, C₁-C₆-Halogenalkyl, C₁-C₆-Alkoxy, C₁-C₆-Halogenalkoxy, ggf. substituiertes Cyclopropyl oder Cyclopropyl-C₁-C₃-alkyl substituierte Aryl- oder Heterocyclylreste;
ausgenommen sind und
R⁵ C₁-C₆-Alkyl, Arylalkyl, Hetarylalkyl, Aryloxyalkyl, Hetaryloxyalkyl, Aryl oder Hetaryl bedeuten.

17. Verwendung der Verbindungen der Formel III gemäß Anspruch 6 als Zwischenprodukte zur Herstellung von Verbindungen gemäß Anspruch 1.

18. Verwendung der Verbindungen der Formel IV gemäß Anspruch 7 als Zwischenprodukte zur Herstellung von Verbindungen gemäß Anspruch 1.

## Claims

1. A phenylacetic acid derivative of the formula I 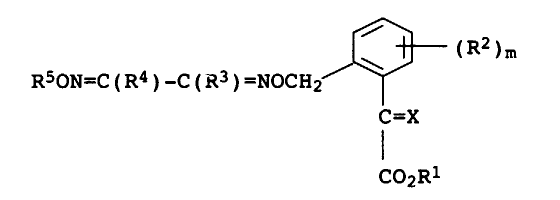 where the substituents and the index have the following meanings:
X is NOCH₃, CHOCH₃, CHCH₃ or CHCH₂CH₃;
R¹ is hydrogen or C₁-C₄-alkyl;
R² is cyano, nitro, trifluoromethyl, halogen, C₁-C₄-alkyl or C₁-C₄-alkoxy;
m is 0, 1 or 2, it being possible for the R² radicals to be different if m is 2;
R³ is hydrogen, cyano, nitro, hydroxyl, amino, halogen,
C₁-C₄-alkyl, C₁-C₄-haloalkyl, C₁-C₄-alkoxy, C₁-C₄-haloalkoxy, C₁-C₄-alkylthio, C₁-C₄-alkylamino or di-C₁-C₄-alkylamino;
R⁴ is hydrogen, cyano, nitro, hydroxyl, amino, halogen,
C₁-C₆-alkyl, C₁-C₆-alkoxy, C₁-C₆-alkylthio, C₁-C₆-alkylamino, di-C₁-C₆-alkylamino, C₂-C₆-alkenyl, C₂-C₆-alkenyloxy, C₂-C₆-alkenylthio, C₂-C₆-alkenylamino, N-C₂-C₆-alkenyl-N-C₁-C₆-alkylamino, C₂-C₆-alkynyl, C₂-C₆-alkynyloxy, C₂-C₆-alkynylthio, C₂-C₆-alkynylamino, N-C₂-C₆-alkynyl-N-C₁-C₆-alkylamino, it being possible for the hydrocarbon radicals of these groups to be partly or completely halogenated or to carry one to three of the following radicals: cyano, nitro, hydroxyl, mercapto, amino, carboxyl, aminocarbonyl, aminothiocarbonyl, halogen, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkoxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio, hetaryl-C₁-C₄-alkylthio, it being possible for the cyclic radicals in turn to be partly or completely halogenated and/or to carry one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C(=NOR⁶)-Aₙ-R⁷;
C₃-C₆-cycloalkyl, C₃-C₆-cycloalkoxy, C₃-C₆-cycloalkylthio, C₃-C₆-cycloalkylamino, N-C₃-C₆-cycloalkyl-N-C₁-C₆-alkylamino, C₃-C₆-cycloalkenyl, C₃-C₆-cycloalkenyloxy, C₃-C₆-cycloalkenylthio, C₃-C₆-cycloalkenylamino, N-C₃-C₆-cycloalkenyl-N-C₁-C₆-alkylamino, heterocyclyl, heterocyclyloxy, heterocyclylthio, heterocyclylamino, N-heterocyclyl-N-C₁-C₆-alkylamino, aryl, aryloxy, arylthio, arylamino, N-aryl-N-C₁-C₆-alkylamino, hetaryl, hetaryloxy, hetarylthio, hetarylamino, N-hetaryl-N-C₁-C₆-alkylamino, it being possible for the cyclic radicals to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl and hetaryloxy;
R⁵ is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these radicals to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkoxy, heterocyclyl, heterocyclyloxy, benzyl, benzyloxy, aryl, aryloxy, arylthio, hetaryl, hetaryloxy and hetarylthio, it being possible for the cyclic groups in turn to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR⁶)-Aₙ-R⁷;
aryl, arylcarbonyl, arylsulfonyl, hetaryl, hetarylcarbonyl or hetarylsulfonyl, it being possible for these radicals to be partly or completely halogenated or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR⁶)-Aₙ-R⁷;
where
A is oxygen, sulfur or nitrogen and where the nitrogen carries hydrogen or C₁-C₆-alkyl;
n is 0 or 1;
R⁶ is hydrogen or C₁-C₆-alkyl and
R⁷ is hydrogen or C₁-C₆-alkyl,
where X is not CHOCH₃ or NOCH₃ when
the index m is zero, and
R³ is hydrogen, cyano, C₁-C₄-alkyl, C₁-C₄-haloalkyl or methylthio,
R⁴ is hydrogen, unsubstituted C₁-C₆-alkyl, unsubstituted C₃-C₆-cycloalkyl, thienyl or
phenyl, which is optionally substituted by:
halogen, cyano, nitro, C₁-C₄-alkyl, C₁-C₄-alkoxy, C₁-C₂-haloalkyl, C₁-C₂-haloalkoxy, C₃-C₆-alkenyloxy, C₃-C₆-alkynyloxy, C₃-C₄-alkylenedioxy; or
the following radicals bonded via oxygen, C₁-C₄-alkoxy, C₁-C₄-oxyalkyl, S(O)ₙ, C₁-C₄-alkyl-S(O)ₙ, S(O)ₙ-C₁-C₄-alkyl, n being 0, 1 or 2:
C₁-C₆-alkyl, C₁-C₆-haloalkyl, C₃-C₆-cycloalkyl or C₂-C₆-alkenyl or C₂-C₄-alkynyl-C₁-C₂-alkyl, it being possible for these radicals to be halogenated 1 to 3 times; or
aryl or heterocyclyl radicals which are optionally substituted by halogen, trimethylsilyl, C₁-C₆-alkyl, C₁-C₆-haloalkyl, C₁-C₆-alkoxy, C₁-C₆-haloalkoxy, optionally substituted cyclopropyl or cyclopropyl-C₁-C₃-alkyl;
and
R⁵ is hydrogen, C₁-C₆-alkyl, C₁-C₆-haloalkyl; C₁-C₄-alkoxy-C₁-C₂-alkyl, C₂-C₄-alkenyl-C₁-C₂-alkyl which is optionally halogenated 1 to 3 times, C₂-C₄-alkynyl-C₁-C₂-alkyl, C₃-C₆-cycloalkyl which is optionally halogenated 1 to 4 times, C₃-C₆-cycloalkyl-C₁-C₂-alkyl which is optionally halogenated 1 to 4 times, cyano-C₁-C₄-alkyl, C₁-C₄-alkoxycarbonyl-C₁-C₂-alkyl;
phenyl-C₁-C₃-alkyl which can be substituted by: halogen, cyano, nitro, C₁-C₃-alkyl, C₁-C₄-alkoxy, C₁-C₄-haloalkyl, it being possible for the phenyl group to be optionally substituted 1 to 3 times by identical or different radicals;
phenyl which can optionally be substituted 1 or 2 times by: halogen, cyano, nitro, C₁-C₄-alkyl, C₁-C₄-alkoxy, C₁-C₂-haloalkyl, this radical carrying 1 to 3 halogen atoms;
or
pyridyl which can be substituted 1 to 2 times by: halogen, cyano, nitro, C₁-C₄-alkyl, C₁-C₄-alkoxy, C₁-C₂-haloalkyl, this radical carrying 1 to 3 halogen atoms;
or
R³ is hydrogen, C₁-C₄-alkyl or cyano,
R⁴ is cyano or heterocyclyl and
R⁵ is C₁-C₆-alkyl, C₁-C₆-haloalkyl,
C₁-C₄-alkoxy-C₁-C₂-alkyl, C₂-C₆-alkenyl which is unsubstituted or substituted by 1 to 3 halogen atoms, C₃-C₆-alkynyl, C₃-C₆-cycloalkyl-C₁-C₄-alkyl which is unsubstituted or substituted by 1 to 4 halogen atoms;
or its salts.

2. A compound of the formula I as claimed in claim 1, in which R³, R⁴ and R⁵ have the following meanings:
R³ is hydrogen, hydroxyl, halogen, C₁-C₄-alkyl, C₁-C₄-alkoxy or C₁-C₄-alkylthio,
R⁴ is hydrogen, hydroxyl, halogen, cyclopropyl, cyclohexyl, C₁-C₄-alkyl, C₁-C₄-alkoxy, C₁-C₄-alkylthio,
aryl or hetaryl, it being possible for these groups to be partly or completely halogenated or to carry one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, c₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl and hetaryloxy;
R⁵ is hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these groups to be partly or completely halogenated or to carry one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy and hetarylthio, it being possible for the aromatic and heteroaromatic radicals, in turn, to be partly or completely halogenated and/or to carry one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR⁶)-Aₙ-R⁷;
aryl, hetaryl, arylcarbonyl, hetarylcarbonyl, arylsulfonyl or hetarylsulfonyl, it being possible for these groups to be partly or completely halogenated or to carry one to 3 of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR⁶)-Aₙ-R⁷;
or its salts.

3. A compound of the formula I as claimed in claim 1, in which m is 0.

4. A compound of the formula I as claimed in claim 1, in which R¹ is methyl.

5. A compound of the formula I as claimed in claim 1, in which X is CHCH₃ OR CHCH₂CH₃.

6. A process for preparing the compounds of the formula I as claimed in claim 1, in which R³ is not halogen, which comprises reacting a benzyl derivative of the formula II 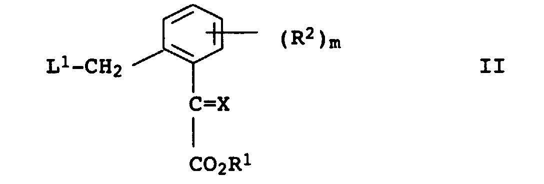 in which L¹ is a nucleophilically replaceable leaving group, in a manner known per se with a hydroxyimine of the formula III
R⁵ON=C(R⁴)-C(R³)=NOH III

7. A process for preparing the compounds of the formula I as claimed in claim 1, in which R³ and R⁴ are not halogen, which comprises reacting a benzyl derivative of the formula II as in claim 6 in a manner known per se with a dihydroxyimine of the formula IV
HON=C(R⁴)-C(R³)=NOH IV
to give a compound of the formula V 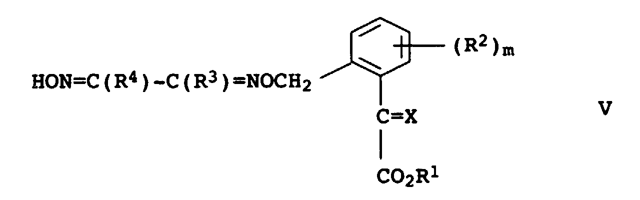 and then reacting V with a compound of the formula VI
R⁵-L³ VI
where L² is a nucleophilically replaceable leaving group, to give I.

8. A process for preparing the compounds of the formula I as claimed in claim 1, in which R³ is not halogen, which comprises reacting a benzyl derivative of the formula II as in claim 6 in a manner known per se with a carbonylhydroxyimine of the formula VII
O=C(R⁴)-C(R³)=NOH VII
to give a compound of the formula VIII 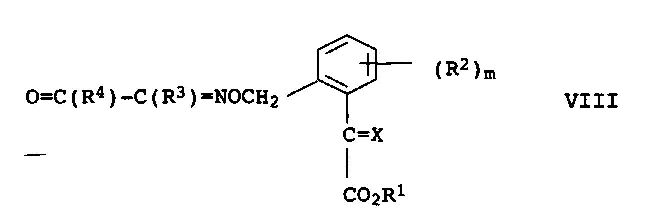 then either reacting VIII
a) first with hydroxylamine or its salt and then with a compound of the formula VI (R⁵-L²) as in claim 7 or
b) with a hydroxylamine or a hydroxylammonium salt of the formula IXa or IXb 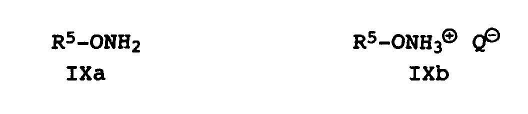 where Q^{⊖} is the anion of an acid,
to give I.

9. A composition against animal pests or harmful fungi, containing customary additives and an effective amount of a compound of the formula I as claimed in claim 1.

10. A composition as claimed in claim 9 for controlling animal pests of the insects, arachnids or nematodes class.

11. A method for controlling harmful fungi, which comprises treating harmful fungi, their environment or the plants, surfaces, materials or spaces to be kept free from them with an effective amount of a compound of the formula I as claimed in claim 1.

12. A method for controlling pests, which comprises treating the pests, their environment or the plants, surfaces, materials or spaces to be kept free from them with an effective amount of a compound of the formula I.

13. The use of the compounds I as claimed in claim 1 for the control of animal pests or harmful fungi.

14. A compound of the formula VIII as in claim 8.

15. The use of the compounds of the formula VIII as claimed in claim 8 as intermediates.

16. A hydroxyimine of the formula III as claimed in claim 6, in which R³ has the meaning given in claim 1 and
R⁴ is hydroxyl, ethyl, isopropyl, optionally subst. aryl or heteroaryl, with thienyl and
phenyl, which is optionally substituted by:
halogen, cyano, nitro, C₁-C₄-alkyl, C₁-C₄-alkoxy, C₁-C₂-haloalkyl, C₁-C₂-haloalkoxy, C₃-C₆-alkenyloxy, C₃-C₆-alkynyloxy, C₃-C₄-alkylenedioxy; or
the following radicals bonded via oxygen, C₁-C₄-alkoxy, C₁-C₄-oxyalkyl, S(O)ₙ, C₁-C₄-alkyl-S(O)ₙ, S(O)ₙ-C₁-C₄-alkyl, n being 0, 1 or 2:
C₁-C₆-alkyl, C₁-C₆-haloalkyl, C₃-C₆-cycloalkyl or C₂-C₆-alkenyl or C₂-C₄-alkynyl-C₁-C₂-alkyl, it being possible for these radicals to be halogenated 1 to 3 times; or
aryl or heterocyclyl radicals which are optionally substituted by halogen, trimethylsilyl, C₁-C₆-alkyl, C₁-C₆-haloalkyl, C₁-C₆-alkoxy, C₁-C₆-haloalkoxy, optionally substituted cyclopropyl or cyclopropyl-C₁-C₃-alkyl;
being excluded and
R⁵ is C₁-C₆-alkyl, arylalkyl, hetarylalkyl, aryloxyalkyl, hetaryloxyalkyl, aryl or hetaryl.

17. The use of the compounds of the formula III as in claim 6 as intermediates for the preparation of compounds as claimed in claim 1.

18. The use of the compounds of the formula IV as in claim 7 as intermediates for the preparation of compounds as claimed in claim 1.

## Revendications

1. Dérivés de l'acide phénylacétique répondant à la formule I 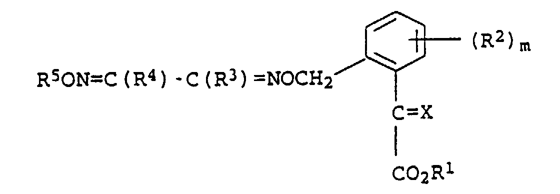 dans laquelle les symboles et l'indice ont les significations suivantes :
Y : NOCH₃, CHOCH₃, CHCH₃ et CHCH₂CH₃ ;
R¹ : hydrogène ou alkyle en C1-C4 ;
R² : cyano, nitro, trifluorométhyle, halogène, alkyle en C1-C4 ou alcoxy en C1-C4 ;
m : 0, 1 ou 2, les symboles R² pouvant avoir des significations différentes lorsque m est égal à 2 ;
R³ : hydrogène, cyano, nitro, hydroxy, amino, halogène,
alkyle en C1-C4, halogénoalkyle en C1-C4, alcoxy en C1-C4, halogénoalcoxy en C1-C4, alkylthio en C1-C4, alkylamino en C1-C4 ou di-(alkyle en C1-C4)amino ;
R⁴ : hydrogène, cyano, nitro, hydroxy, amino, halogène, alkyle en C1-C6, alcoxy en C1-C6, alkylthio en C1-C6, alkylamino en C1-C6, di-(alkyle en C1-C6)amino, alcényle en C2-C6, alcényloxy en C2-C6, alcénylthio en C2-C6, alcénylamino en C2-C6, N-(alcényle en C2-C6)-N-alkylamino en C1-C6, alcynyle en C2-C6, alcynyloxy en C2-C6, alcynylthio en C2-C6, alcynylamino en C2-C6, N-(alcynyle en C2-C6)-N-alkylamino en C1-C6, les radicaux hydrocarbonés de ces groupes pouvant être halogénés en totalité ou en partie ou pouvant porter eux-mêmes un à trois des substituants suivants : cyano, nitro, hydroxy, mercapto, amino, carboxyle, aminocarbonyle, aminothiocarbonyle, halogène, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, aikylamino en C1-C6, di-(alkyle en C1-C6)amino, alcényloxy en C2-C6, cycloalkyle en C3-C6, cycloalcoxy en C3-C6, hétérocyclyle, hétérocyclyloxy, aryle, aryloxy, aryl-alcoxy en C1-C4, arylthio, aryl-alkylthio en C1-C4, hétéroaryle, hétéroaryloxy, hétéroaryl-alcoxy en C1-C4, hétéroarylthio, hétéroarylalkylthio en C1-C4, les radicaux cycliques pouvant eux-mêmes être partiellement ou totalement halogénés et/ou pouvant porter un à trois des substituants suivants : cyano, nitro, hydroxy, mercapto, amino, carboxyle, aminocarbonyle, aminothiocarbonyle, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio et C(=NOR⁶)-Aₙ-R⁷ ;
cycloalkyle en C3-C6, cycloalcoxy en C3-C6, cycloalkylthio en C3-C6, cycloalkylamino en C3-C6, N-(cycloalkyle en C3-C6)-N-alkylamino en C1-C6, cycloalcényle en C3-C6, cycloalcényloxy en C3-C6, cycloalcénylthio en C3-C6, cycloalcénylamino en C3-C6, N-(cycloalcényle en C3-C6)-N-alkylamino en C1-C6, hétérocyclyle, hétérocyclyloxy, hétérocyclylthio, hétérocyclylamino, N-hétérocyclyl-N-alkylamino en C1-C6, aryle, aryloxy, arylthio, arylamino, N-aryl-N-alkylamino en C1-C6, hétéroaryle, hétéroaryloxy, hétéroarylthio, hétéroarylamino, N-hétéroaryl-N-alkylamino en C1-C6, les radicaux cycliques pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyde en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle et hétéroaryloxy ;
R⁵ : hydrogène,
alkyle en C1-C10, cycloalkyle en C3-C6, alcényle en C2-C10, alcynyle en C2-C10, (alkyle en C1-C10)carbonyle, (alcényle en C2-C6)carbonyle, (alcynyle en C3-C10)carbonyle ou alkylsulfonyle en C1-C10, ces radicaux pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, cycloalkyle en C3-C6, cycloalcoxy en C3-C6, hétérocyclyle, hétérocyclyloxy, benzyle, benzyloxy, aryle, aryloxy, arylthio, hétéroaryle, hétéroaryloxy et hétéroarylthio, les groupes cycliques pouvant eux-mêmes être partiellement ou totalement halogénés ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alkyle en C1-C6)oxycarbonyle, alkylthio en C1-C6, alkylamino en C1-C6, di-(alkyle en C1-C6)amino. (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio ou C(=NOR⁶)-Aₙ-R⁷ ;
aryle, arylcarbonyle, arylsulfonyle, hétéroaryle, hétéroarylcarbonyle ou hétéroarylsulfonyle, ces radicaux pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alkyle en C1-C6)-oxycarbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle, hétéroaryloxy ou C(=NOR⁶)-Aₙ-R⁷ ;
dans lequel
A représente l'oxygène, le soufre ou l'azote, ce dernier portant de l'hydrogène ou un groupe alkyle en C1-C6 ;
n est égal à 0 ou 1 ;
R⁶ représente l'hydrogène ou un groupe alkyle en C1-C6 et
R⁷ représente l'hydrogène ou un groupe alkyle en C1-C6, sous réserve que X ne peut représenter CHOCH₃ ou NOCH₃ lorsque l'indice m est égal à 0, et
R³ représente l'hydrogène, un groupe cyano, alkyle en C1-C4, halogénoalkyle en C1-C4 ou méthylthio,
R⁴ représente l'hydrogène, un groupe alkyle en C1-C6 non substitué, un groupe cycloalkyle en C3-C6 non substitué, un groupe thiényle ou bien un groupe phényle éventuellement substitué par :
des halogènes, des groupes cyano, nitro, alkyle en C1-C4, alcoxy en C1-C4, halogénoalkyle en C1-C2, halogénoalcoxy en C1-C2, alcényloxy en C3-C6, alcynyloxy en C3-C6, (alkylène en C3-C4)-dioxy ; ou bien
les groupes suivants reliés par l'intermédiaire de l'oxygène, d'un groupe alcoxy en C1-C4, oxyalkyle en C1-C4, S(O)ₙ, (alkyle en C1-C4)-S(O)ₙ, S(O)ₙ-alkyle en C1-C4, n étant égal à 0, 1 ou 2 :
alkyle en C1-C6, halogénoalkyle en C1-C6, cycloalkyle en C3-C6 ou alcényle en C2-C6 ou (alcynyle en C2-C4)-alkyle en C1-C2, ces groupes pouvant être halogénés une à trois fois ; ou bien
des groupes aryle ou hétérocyclyle éventuellement substitués par des halogènes, des groupes triméthylsilyle, alkyle en C1-C6, halogénoalkyle en C1-C6, alcoxy en C1-C6, halogénoalcoxy en C1-C6, cyclopropyle lui-même éventuellement substitué ou cyclopropyl-alkyle en C1-C3 ; et
R⁵ : hydrogène, alkyle en C1-C6, halogénoalkyle en C1-C6 ; (alcoxy en C1-C4)-alkyle en C1-C2, (alcényle en C2-C4)alkyle en C1-C2 éventuellement halogéné une à trois fois, (alcynyle en C2-C4)alkyle en C1-C2, cycloalkyle en C3-C6 éventuellement halogéné une à quatre fois, (cycloalkyle en C3-C6)alkyle en C1-C2 éventuellement halogéné une à quatre fois, cyano-alkyle en C1-C4, (alcoxy en C1-C4)carbonyl-alkyle en C1-C2 ; phényl-alkyle en C1-C3 éventuellement substitué par : halogènes, cyano, nitro, alkyle en C1-C3, alcoxy en C1-C4, halgoénoalkyle en C1-C4, le groupe phényle pouvant, éventuellement porter un à trois substituants identiques ou différents ;
phényle, portant éventuellement un ou deux substituants choisis parmi : halogènes, cyano, nitro, alkyle en C1-C4, alcoxy en C1-C4, halogénoalkyle en C1-C2, ce dernier portant un à trois atomes d'halogènes ;
ou bien
pyridyle portant éventuellement un à deux substituants choisis parmi : halogènes, cyano, nitro, alkyle en C1-C4, alcoxy en C1-C4, halogénoalkyle en C1-C2, ce dernier portant un à trois atomes d'halogènes ;
ou bien
R³ : hydrogène, alkyle en C1-C4 ou cyano,
R⁴ : cyano ou hétérocyclyle et
R⁵ : alkyle en C1-C6, halogénoalkyle en C1-C6, (alcoxy en C1-C4)-alkyle en C1-C2, alcényle en C2-C6 non substitué ou substitué par un à trois atomes d'halogènes,
alcynyle en C3-C6, (cycloalkyle en C3-C6)-alkyle en C1-C4 non substitué ou substitué par un à quatre atomes d'halogènes ;
et leurs sels.

2. Composés de formule I de la revendication 1 dans laquelle R³, R⁴ et R⁵ ont les significations suivantes :
R³ : hydrogène, hydroxy, halogène, alkyle en C1-C4, alcoxy en C1-C4 ou alkylthio en C1-C4 ;
R⁴ : hydrogène, hydroxy, halogène, cyclopropyle, cyclohexyle, alkyle en C1-C4, alcoxy en C1-C4, alkylthio en C1-C4,
aryle ou hétéroaryle, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle et hétéroaryloxy ;
R⁵ : hydrogène,
alkyle en C1-C10, cycloalkyle en C3-C6, alcényle en C2-C10, alcynyle en C2-C10, (alkyle en C1-C10)carbonyle, (alcényle en C2-C10)carbonyle, (alcynyle en C3-C10)carbonyle ou alkylsulfonyle en C1-C10, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy et hétéroarylthio, les radicaux aromatiques et hétéroaromatiques pouvant eux-mêmes être partiellement ou totalement halogénés et/ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alkyle en C1-C6)oxycarbonyle, alkylthio en C1-C6, alkylamino en C1-C6, di-(alkyle en C1-C6)amino, (alkyle en C1-06)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio ou C(=NOR⁶)-Aₙ-R⁷ ; aryle, hétéroaryle, arylcarbonyle, hétéroaryl-carbonyle, arylsulfonyle ou hétéroarylsulfonyle, ces groupes pouvant être halogénés en totalité ou en partie ou pouvant porter un à rois des substituants suivants : cyano, nitro, hydroxy, mercapto, amino, rboxyle, aminocarbonyle, aminothiocarbonyle, halogéno, alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle, hétéroaryloxy ou C(=NOR⁶)-Aₙ-R⁷ ;
et leurs sels.

3. Composés de formule I de la revendication 1 dans laquelle m est égal à 0.

4. Composés de formule I de la revendication 1, dans laquelle R¹ représente un groupe méthyle.

5. Composés de formule I de la revendication 1, dans laquelle X représente CHCH₃ ou CHCH₂CH₃.

6. Procédé de préparation des composés de formule I de la revendication 1 dans laquelle R³ a une signification autre qu'un halogène, caractérisé par le fait que l'on fait réagir de manière connue en soi un dérivé benzylique de formule II 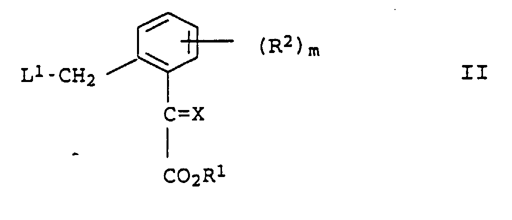 dans laquelle L¹ représente un groupe éliminable par échange nucléophile, avec une hydroxyimine de formule III
R⁵ON=C(R⁴)-C(R³)=NOH III

7. Procédé de préparation des composés de formule I de la revendication 1, dans laquelle R³ et R⁴ ont des significations autres que des halogènes, caractérisé par le fait que l'on fait réagir de manière connue en soi un dérivé benzylique de formule II de la revendication 1 avec une dihydroxyimine de formule IV
HON=C(R⁴)-C(R³)=NOH IV
la réaction donnant un composé de formule V 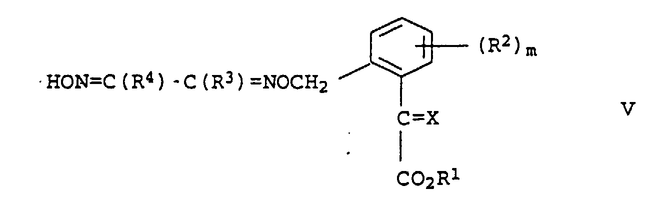 qu'on fait ensuite réagir avec un composé de formule VI
R⁵-L² VI
dans laquelle L² représente un groupe éliminable par échange nucléophile, ce qui donne le composé I.

8. Procédé de préparation des composés de formule I de la revendication 1 dans laquelle R³ a une signification autre qu'un halogène, caractérisé par le fait que l'on fait réagir, de manière connue en soi, un dérivé benzylique de formule II de la revendication 1 avec une carbonylhydroxyimine de formule VII
O=C(R⁴)-C(R³)=NOH VII
la réaction donnant un composé de formule VIII 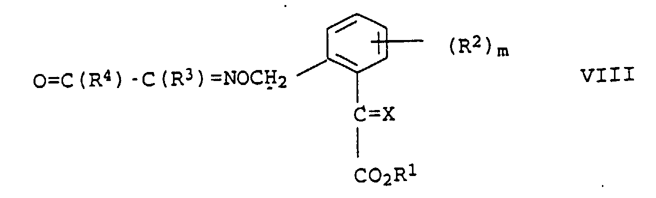 qu'on fait ensuite réagir,
a) d'abord avec l'hydroxylamine ou l'un de ses sels puis avec un composé de formule VI (R⁵-L²) de la revendication 7,
b) ou bien avec une hydroxylamine ou un sel d'hydroxylammonium de formules respectives IXa et IXb 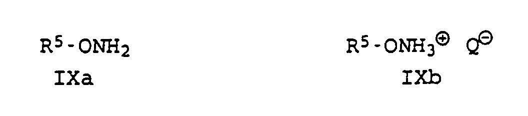 Q^{⊖} représentant l'anion d'un acide,
ce qui donne le composé I.

9. Produit prévu pour la lutte contre les parasites animaux ou les mycètes nuisibles, contenant des additifs usuels et une quantité efficace d'un composé de formule I selon la revendication 1.

10. Produit selon la revendication 9, pour la lutte contre les parasites animaux des classes des insectes, des arachnides ou des nématodes.

11. Procédé pour combattre les mycètes nuisibles, caractérisé par le fait que l'on traite les mycètes nuisibles, leur habitat ou les végétaux, aires, matériaux ou locaux qu'on veut protéger contre les mycètes par une quantité efficace d'un composé de formule I de la revendication 1.

12. Procédé pour combattre les parasites, caractérisé par le fait que l'on traite les parasites, leur habitat ou les végétaux, aires, matériaux ou locaux qu'on veut protéger contre ces parasites par une quantité efficace d'un composé de formule I.

13. Utilisation des composés I selon la revendication 1, pour la lutte contre les parasites animaux ou les mycètes nuisibles.

14. Composés de formule VIII de la revendication 8.

15. Utilisation des composés de formule VIII de la revendication 8 en tant que produits intermédiaires.

16. Hydroxyimines de formule III de la revendication 6 dans laquelle R³ a les significations indiquées dans la revendication 1, et
R⁴ représente un groupe hydroxy, éthyle, isopropyle, aryle ou hétéroaryle éventuellement substitué, les groupes thiényle et phényle, lui-même éventuellement substitué par :
des halogènes, des groupes cyano, nitro, alkyle en C1-C4, alcoxy en C1-C4, halogénoalkyle en C1-C2, halogénoalcoxy en C1-C2, alcényloxy en C3-C6, alcynyloxy en C3-C6, (alkylène en C3-C4)-dioxy ; ou bien
les groupes suivants reliés par l'intermédiaire de l'oxygène, d'un groupe alcoxy en C1-C4, oxyalkyle en C1-C4, S(O)ₙ), alkyle en C1-C4-S(O)ₙ, S(O)ₙ-alkyle en C1-C4, n étant égal à 0, 1 ou 2 :
alkyle en C1-C6, halogénoalkyle en C1-C6, cycloalkyle en C3-C6 ou alcényle en C2-C6 ou (alcényle en C2-C4)alkyle en C1-C2, ces groupes pouvant être halogénés une à trois fois ; ou bien
des groupes aryle ou hétérocyclyle éventuellement substitués par des halogènes, des groupes triméthylsilyle, alkyle en C1-C6, halogénoalkyle en C1-C6, alcoxy en C1-C6, halogénoalcoxy en C1-C6, cyclopropyle ou cyclopropyl-alkyle en C1-C3 lui-même éventuellement substitué ;
étant exclus et
R⁵ représente un groupe alkyle en C1-C6, arylalkyle, hétéroarylalkyle, aryloxyalkyle, hétéroaryloxyalkyle, aryle ou hétéroaryle.

17. Utilisation des composés de formule III de la revendication 6 en tant que produits intermédiaires de la préparation de composés selon la revendication 1.

18. Utilisation des composés de formule IV de la revendication 7 en tant que produits intermédiaires de la préparation de composés selon la revendication 1.
